# EUROPÄISCHE PATENTSCHRIFT

(11) **EP 1 356 067 B1**
(45) Veröffentlichungstag und Bekanntmachung des Hinweises auf die Patenterteilung: **28.04.2010**
(21) Anmeldenummer: 02704657.2
(22) Anmeldetag: 18.01.2002
(51) Int. Cl.: C12N 15/82, C12N 15/53, C12N 15/11, C12N 9/02, C12P 7/62, C12P 7/64, C12Q 1/68, C07K 16/40, G01N 33/573, C11B 1/00

(54) **VERFAHREN ZUR HERSTELLUNG MEHRFACH UNGESAETTIGTER FETTSAEUREN, NEUE BIOSYNTHESEGENE SOWIE NEUE PFLANZLICHE EXPRESSIONSKONSTRUKTE**
METHOD FOR PRODUCING POLYUNSATURATED FATTY ACIDS, NOVEL BIOSYNTHESIS GENES AND NOVEL PLANT EXPRESSION CONSTRUCTS
PROCEDE DE PRODUCTION D'ACIDES GRAS POLYINSATURES, NOUVEAUX GENES BIOSYNTHETIQUES ET NOUVELLES CONSTRUCTIONS D'EXPRESSION VEGETALES

(30) Priorität: 19.01.2001 DE 10102337
(43) Veröffentlichungstag der Anmeldung: 29.10.2003
(73) Patentinhaber: BASF Plant Science GmbH, 67056 Ludwigshafen (DE)
(72) Erfinder: LERCHL, Jens, S-26831 Svalöv (SE); RENZ, Andreas, 67117 Limburgerhof (DE); HEINZ, Ernst, 22609 Hamburg (DE); DOMERGUE, Frederic, 22761 Hamburg (DE); ZÄHRINGER, Ulrich, 22926 Ahrensburg (DE)
(74) Vertreter: Dörper, Thomas Michael
(86) Internationale Anmeldenummer: PCT/EP2002/000462
(87) Internationale Veröffentlichungsnummer: WO 2002/057465

(56) Entgegenhaltungen:
- WO-A-98/46765
- OBUKOWICZ MARK G ET AL: "Identification and characterization of a novel DELTA6/DELTA5 fatty acid desaturase inhibitor as a potential anti-inflammatory agent" BIOCHEMICAL PHARMACOLOGY, PERGAMON, OXFORD, GB, Bd. 55, Nr. 7, 1. April 1998 (1998-04-01), Seiten 1045-1058, XP002215907 ISSN: 0006-2952
- MORENO V J ET AL: "BIOSYNTHESIS OF UNSATURATED FATTY-ACIDS IN THE DIATOM PHAEODACTYLUM-TRICORNUTUM" LIPIDS, Bd. 14, Nr. 1, 1979, Seiten 15-19, XP009004733 EN ISSN: 0024-4201
- ABBADI AMINE ET AL: "Transgenic oilseeds as sustainable source of nutritionally relevant C20 and C22 polyunsaturated fatty acids?" EUROPEAN JOURNAL OF LIPID SCIENCE AND TECHNOLOGY, Bd. 103, Nr. 2, Februar 2001 (2001-02), Seiten 106-113, XP002228744 ISSN: 1438-7697
- DOMERGUE FREDERIC ET AL: "Cloning and functional characterization of Phaeodactylum tricornutum front-end desaturases involved in eicosapentaenoic acid biosynthesis." EUROPEAN JOURNAL OF BIOCHEMISTRY, Bd. 269, Nr. 16, August 2002 (2002-08), Seiten 4105-4113, XP002228745 ISSN: 0014-2956

## Beschreibung

Die vorliegende Erfindung betrifft ein Verfahren zur Herstellung von ungesättigten Fettsäuren mit mindestens zwei Doppelbindungen und/oder ein Verfahren zur Herstellung von Triglyceriden mit erhöhtem Gehalt an mehrfach ungesättigten Fettsäuren mit mindestens zwei Doppelbindungen. Die Erfindung betrifft weiterhin die vorteilhafte Verwendung der Nukleinsäuresequenzen SEQ ID NO: 1, SEQ ID NO: 3, SEQ ID NO: 5 oder 11 im Verfahren sowie zur Herstellung eines transgenen Organismuses bevorzugt einer transgenen Pflanze oder eines transgenen Mikroorganismus mit erhöhtem Gehalt an Fettsäuren, Ölen oder Lipiden mit ungesättigten C₁₈-, C₂₀-, oder C₂₂-Fettsäuren.

Die Erfindung betrifft weiterhin neue Desaturasen mit den in den Sequenzen SEQ ID NO: 1, SEQ ID NO: 3, SEQ ID NO: 5 und SEQ ID NO: 11 oder seine Homologen, Derivate oder Analoga sowie Genkonstrukte, die diese Gene oder ihre Homologen, Derivate oder Analoga umfasst sowie Ihre Verwendung allein oder in Kombination mit Biosynthesegenen polyungesättigter Fettsäuren wie vorteilhaft in SEQ ID NO: 7 und SEQ ID NO: 9 dargestellt.

Außerdem betrifft die Erfindung isolierte Nukleinsäuresequenzen; Expressionskassetten enthaltend die Nukleinesäuresequenzen, Vektoren und transgene Organismen enthaltend mindestens eine Nukleinsäuresequenz bzw. eine Expressionskassette. Außerdem betrifft die Erfindung ungesättigte Fettsäuren mit mindestens zwei Doppelbindungen sowie Triglyceride mit einem erhöhten Gehalt an ungesättigten Fettsäuren mit mindestens zwei Doppelbindungen und deren Verwendung.

Die Erfindung betrifft zudem Multiexpressionskassetten zur samenspezifischen Expression und Vektoren oder Organismen, die ein Desaturasegen allein oder in Kombination mit weiteren Desaturasen mit der Sequenz SEQ ID NO:7 und/oder Elongasegenen mit der Sequenz ID NO: 9 oder seine Homologen, Derivate oder Analoga unter Verwendung besagter Expressionskassetten umfassen.

Eine Reihe von Produkten und Nebenprodukten natürlich vorkommender Stoffwechselprozesse in Mikroorganismen, tierischen und pflanzlichen Zellen sind für viele Industriezweige, einschließlich der Futtermittel-, Nahrungsmittel-, Kosmetik- und pharmazeutischen Industrie, nützlich. Zu diesen gemeinsam als "Feinchemikalien" bezeichneten Molekülen gehören beispielsweise Lipide und Fettsäuren, unter denen eine beispielhafte Klasse die mehrfach ungesättigten Fettsäuren sind. Fettsäuren und Triglyceride haben eine Vielzahl von Anwendungen in der Lebensmittelindustrie, der Tierernährung, der Kosmetik und im Pharmabereich. Je nachdem ob es sich um freie gesättigte oder ungesättigte Fettsäuren oder um Triglyceride mit einem erhöhten Gehalt an gesättigten oder ungesättigten Fettsäuren handelt, sind sie für die unterschiedlichsten Anwendungen geeignet, so werden beispielsweise mehrfach ungesättigte Fettsäuren (polyunsaturated fatty acids, PUFAs) Babynahrung zur Erhöhung des Nährwertes zugesetzt. PUFAs haben weiterhin einen positiven Einfluss auf den Cholesterinspiegel im Blut von Menschen und eignen sich daher zum Schutz gegen Herzkrankheiten. So finden sie in verschiedenen diätischen Lebensmitteln oder Medikamenten Anwendung.

Besonders geeignete Mikroorganismen zur Herstellung von PUFAs sind Mikroorganismen wie Thraustochytrien oder Schizochytrien-Stämme, Algen wie Phaeodactylum tricornutum oder Crypthecodinium-Arten, Ciliaten, wie Stylonychia oder Colpidium, Pilze, wie Mortierella, Entomophthora oder Mucor. Durch Stammselektion ist eine Anzahl von Mutantenstämmen der entsprechenden Mikroorganismen entwickelt worden, die eine Reihe wünschenswerter Verbindungen, einschließlich PUFAs, produzieren. Die Selektion von Stämmen mit verbesserter Produktion eines bestimmten Moleküls ist jedoch ein zeitraubendes und schwieriges Verfahren.

Alternativ kann die Produktion von Feinchemikalien geeigneterweise über die Produktion von Pflanzen, die so entwickelt sind, dass sie die vorstehend genannten PUFAs herstellen, im großen Maßstab durchgeführt werden. Besonders gut für diesen Zweck geeignete Pflanzen sind Ölfruchtpflanzen, die große Mengen an Lipidverbindungen enthalten wie Raps, Canola, Lein, Soja, Sonnenblumen, Borretsch und Nachtkerze. Aber auch andere Nutzpflanzen, die Öle oder Lipide und Fettsäuren enthalten, sind gut geeignet, wie in der eingehenden Beschreibung dieser Erfindung erwähnt. Mittels herkömmlicher Züchtung ist eine Reihe von Mutantenpflanzen entwickelt worden, die ein Spektrum an wünschenswerten Lipiden und Fettsäuren, Cofaktoren und Enzymen produzieren.
Die Selektion neuer Pflanzensorten mit verbesserter Produktion eines bestimmten Moleküls ist jedoch ein zeitaufwändiges und schwieriges Verfahren oder sogar unmöglich, wenn die Verbindung in der entsprechenden Pflanze nicht natürlich vorkommt, wie im Fall von mehrfach ungesättigten C₁₈-, C₂₀-Fettsäuren und C₂₂-Fettsäuren und solchen mit längeren Kohlenstoffketten.

Aufgrund der positiven Eigenschaften ungesättigter Fettsäuren hat es in der Vergangenheit nicht an Ansätzen gefehlt, Gene, die an der Synthese von Fettsäuren bzw. Triglyceriden beteiligt sind, für die Herstellung von Ölen in verschiedenen Organismen mit geändertem Gehalt an ungesättigten Fettsäuren verfügbar zu machen. So wird in WO 91/13972 und seinem US-Äquivalent eine Δ-9-Desaturase beschrieben. In WO 93/11245 wird eine Δ-15-Desaturase in WO 94/11516.wird eine Δ-12-Desaturase beansprucht. Δ-6-Desaturasen werden in WO 93/06712, US 5,614,393, WO 96/21022 und WO 99/27111 beschrieben. Weitere Desaturasen werden beispielsweise in EP-A-0 550 162, WO 94/18337, WO 97/30582, WO 97/21340, WO 95/18222, EP-A-0 794 250, Stukey et al., J. Biol. Chem., 265, 1990: 20144-20149, Wada et al., Nature 347, 1990: 200-203 oder Huang et al., Lipids 34, 1999: 649-659 beschrieben. In WO 96/13591 wird eine Δ-6-Palmitoyl-ACP-Desaturase beschrieben und beansprucht. Die biochemische Charakterisierung der verschiedenen Desaturasen ist jedoch bisher nur unzureichend erfolgt, da die Enzyme als membrangebundene Proteine nur sehr schwer zu isolieren und charakterisieren sind (McKeon et al., Methods in Enzymol. 71, 1981: 12141-12147, Wang et al., Plant Physiol. Biochem., 26, 1988: 777-792).

In Hefen konnte sowohl eine Verschiebung des Fettsäurespektrums zu ungesättigten Fettsäuren hin als auch eine Steigerung der Produktivität nachgewiesen werden (siehe Huang et al., Lipids 34, 1999: 649-659, Napier et al., Biochem. J., Vol. 330, 1998: 611-614). Die Expression der verschiedenen Desaturasen in transgenen Pflanzen zeigte allerdings nicht den gewünschten Erfolg. Eine Verschiebung des Fettsäurespektrum zu ungesättigten Fettsäuren hin konnte gezeigt werden, gleichzeitig zeigte sich aber, dass die Syntheseleistung der transgenen Pflanzen stark nachließ, das heißt gegenüber den Ausgangspflanzen konnten nur geringere Mengen an Ölen isoliert werden.

Weder in Hefen noch in Pflanzen werden natürlicherweise mehrfach ungesättigte C₂₀- und/oder C₂₂-Fettsäuren mit mindestens zwei Doppelbindungen im Fettsäuremolekül wie Arachidonsäure (ARA) und/oder Eicosapentaensäure (EPA) und/oder Docosahexaensäure (DHA) hergestellt.

Nach wie vor besteht daher ein großer Bedarf an neuen Genen, die für Enzyme kodieren, die an der Biosynthese ungesättigter Fettsäuren beteiligt sind und es ermöglichen, diese in einem technischen Maßstab herzustellen. Keines der bisher bekannten biotechnologischen Verfahren zur Herstellung von mehrfach ungesättigten Fettsäuren liefert die vorgenannten Fettsäuren in wirtschaftlich nutzbaren Mengen.

Es bestand daher die Aufgabe weitere Enzyme für die Synthese mehrfach ungesättigter Fettsäuren zur Verfügung zu stellen. Und diese Enzyme gegebenenfalls mit anderen Enzymen in einem Verfahren zur Herstellung mehrfach ungesättigter Fettsäuren zu verwenden. Diese Aufgabe wurde durch das erfindungsgemäße Verfahren zur Herstellung von Fettsäureestern mit einem erhöhtem Gehalt an mehrfach ungesättigten Fettsäuren mit mindestens zwei Doppelbindungen gelöst, dadurch gekennzeichnet, man mindestens eine Nukleinsäuresequenz ausgewählt aus der Gruppe
a) einer Nukleinsäure mit der in SEQ ID NO: 1, SEQ ID NO: 3, SEQ ID NO: 5 oder SEQ ID NO: 11 dargestellten Sequenz,
b) Nukleinsäuren, die aufgrund des degenerierten genetischen Codes durch Rückübersetzung der in SEQ ID NO: 2, SEQ ID NO: 4, SEQ ID NO: 6 oder SEQ ID NO: 12 dargestellten Aminosäuresequenzen erhalten werden,
c) Derivate der in SEQ ID NO: 1, SEQ ID NO: 3, SEQ ID NO: 5 oder SEQ ID NO: 11 dargestelltenNukleinsäuren, die für biologisch aktive Teile einer Desaturase kodieren und mindestens 50 % Identität zu der Aminosäuresequenz der SEQ ID NO: 2, 4, 6 oder 12 aufweisen
in einen Fettsäureester produzierenden nicht-humanen Organismus einbringt, anzieht und die in dem Organismus enthaltenen Fettsäureester isoliert.

Bei den im erfindungsgemäßen Verfahren verwendeten Nukleinsäuresequenzen handelt es sich um isolierte Nukleinsäuresequenzen, die für Polypeptide mit Δ-5-, Δ-6- oder Δ-12-Desaturaseaktivität codieren.

Im erfindungsgemäßen Verfahren werden vorteilhaft Fettsäureester mit mehrfach ungestättigten C₁₈-, C₂₀- und/oder C₂₂-Fettsäuremolekülen mit mindestens zwei Doppelbindungen im Fettsäureester hergestellt. Bevorzugt enthalten diese Fettsäuremoleküle drei, vier oder fünf Doppelbindungen und führen vorteilhaft zur Synthese von Arachidonsäure (ARA), Eicosapentaensäure (EPA) oder Docosahexaensäure (DHA).

Die Fettsäureester mit mehrfach ungesättigten C₁₈-, C₂₀- und/oder C₂₂-Fettsäuremolekülen können aus den Organismen, die für die Herstellung der Fettsäureester verwendet wurden, in Form eines Öls oder Lipids beispielsweise in Form von Verbindungen wie Sphingolipide, Phosphoglyceride, Lipide, Glycolipide, Phospholipide, Monoacylglyceride, Diacylglyceride, Triacylglyceride oder sonstige Fettsäureester, die die mehrfach ungesättigten Fettsäuren mit mindestens zwei Doppelbindungen enthalten, isoliert werden.

Als Organismus für die Herstellung im erfindungsgemäßen Verfahren kommen prinzipell alle prokaryontischen oder eurkaryontischen Organismen wie prokaryontische oder eurkaryontische Mikroorganismen wie gram-positive oder gram-negative Bakterien, Pilze, Hefen, Algen, Ciliaten, tierische oder pflanzliche Zellen, Tiere oder Pflanzen wie Moose, zweikeimblättrige oder einkeimblättrige Pflanzen in Frage. Vorteilhaft werden Organismen im erfindungsgemäßen Verfahren verwendet, die zu den Öl-produzierenden Organismen gehören, das heißt die für die Herstellung von Ölen verwendet werden, wie Mikroorganismen wie Crypthecodinium, Thraustochytrium, Phaeodactylum und Mortierella, Entomophthora, Mucor, Crypthecodinium sowie andere Algen oder Pilze sowie Tiere oder Pflanzen, insbesondere Pflanzen bevorzugt Ölfruchtpflanzen, die große Mengen an Lipidverbindungen enthalten, wie Sojabohne, Erdnuss, Raps, Canola, Sonnenblume, Safflor, Nachtkerze, Lein, Soja, Borretsch, Bäume (Ölpalme, Kokosnuss) oder Feldfrüchte, wie Mais, Weizen, Roggen, Hafer, Triticale, Reis, Gerste, Baumwolle, Maniok, Pfeffer, Tagetes, Solanaceen-Pflanzen, wie Kartoffel, Tabak, Aubergine und Tomate, Vicia-Arten, Erbse, Alfalfa oder Buschpflanzen (Kaffee, Kakao, Tee), Salix-Arten, Bäume (Ölplame, Kokosnuß) sowie ausdauernde Gräser und Futterfeldfrüchte. Besonders bevorzugte erfindungsgemäße Pflanzen sind Ölfruchtpflanzen, wie Soja, Erdnuß, Raps, Canola, Lein, Nachtkerze, Sonnenblume, Safflor oder Bäume (Ölpalme, Kokosnuß).

Das erfindungsgemäße Verfahren beinhaltet entweder die Züchtung eines geeigneten transgenen Organismus bzw. transgenen Mikroorganismus oder die Züchtung von transgenen Pflanzenzellen, -geweben, -organen oder ganzen Pflanzen, umfassend die erfindungsgemäßen Nukleotidsequenzen der SEQ ID NO: 1, 3, 5 oder 11 gegebenenfalls in Verbindungen mit den in SEQ ID NO: 7 und/oder SEQ ID NO: 9 dargestellten Sequenzen allein oder in Kombination mit Sequenzen von Expressionskonstrukten aus SEQ ID NO: 13-17 oder ihre Homologen, Derivate oder Analoga oder ein Genkonstrukt, das die SEQ ID NO: 1, 3, 5 oder 11 ggf. in Verbindung mit SEQ ID NO: 7 und/oder 9 oder ihre Homologen, Derivate oder Analoga umfasst, oder einen Vektor, der diese Sequenz oder das Genkonstrukt umfasst, welches die Expression erfindungsgemäßer Nukleinsäuremoleküle herbeiführt, so dass eine Feinchemikalie produziert wird. Bei einer bevorzugten Ausführungsform umfasst das Verfahren ferner den Schritt des Gewinnens einer Zelle, die eine solche erfindungsgemäße Nukleinsäuresequenzen enthält, wobei eine Zelle mit einer Desaturasenukleinsäuresequenz, einem Genkonstrukt oder einem Vektor, welche die Expression einer erfindungsgemäßen Desaturasenukleinsäure allein oder in Kombination herbeiführen, transformiert wird. Bei einer weiteren bevorzugten Ausführungsform umfasst dieses Verfahren ferner den Schritt des Gewinnens der Feinchemikalie aus der Kultur. Bei einer besonders bevorzugten Ausführungsform gehört die Zelle zur Ordnung der Ciliaten, zu Mikroorganismen, wie Pilzen, oder zum Pflanzenreich, insbesondere zu Ölfruchtpflanzen, besonders bevorzugt sind Mikroorganismen oder Ölfruchtpflanzen beispielsweise Erdnuss, Raps, Canola, Lein, Soja, Safflower (Distel), Sonnenblumen oder Borretsch.

Unter transgen im Sinne der Erfindung ist zu verstehen, daß die erfindungsgemäßen Nukleinsäuren nicht an ihrer natürlichen Stelle im Genom eines Organismus sind, dabei können die Nukleinsäuren homolog oder heterolog exprimiert werden. Tansgen bedeutet aber auch, dass die erfindungsgemäßen Nukleinsäuren an ihrem natürlichen Platz im Genom eines Organismus sind, dass jedoch die Sequenz gegenüber der natürlichen Sequenz verändert wurde und/oder das die Regulationssequenzen, der natürlichen Sequenzen verändert wurden. Bevorzugt ist unter transgen die Expression der erfindungsgemäßen Nukleinsäuren an nicht natürlicher Stelle im Genom zu verstehen, das heißt eine homologe oder bevorzugt heterologe Expression der Nukleinsäuren liegt vor. Bevorzugte transgene Organismen sind die oben genannten transgenen Pflanzen bevorzugt Ölfruchtpflanzen.

Aus den im erfindungsgemäßen Verfahren hergestellten Fettsäureestern lassen sich die enthaltenden mehrfach ungesättigten Fettsäuren beispielsweise über eine Alkalibehandlung wie wässrige KOH oder NaOH vorteilhaft in Gegenwart eines Alkohols wie Methanol oder Ethanol freisetzen und isolieren über beispielsweise Phasentrennung und anschließender Ansäuerung über z.B. H₂SO₄.

Ein weiterer Gegenstand der Erfindung sind Öle, Lipide und/oder Fettsäuren, die mindestens zwei Doppelbindungen in den Fettsäuremolekülen bevorzugt drei, vier, fünf oder sechs Doppelbindungen enthalten, die nach dem oben beschriebenen erfindungsgemäßen Verfahren hergestellt wurden. Auch sind Zusammensetzungen, die die genannten Öl-, Lipid- und/oder Fettsäuren enthalten, sowie die Verwendung der Öle, Lipide und/oder Fettsäuren oder der Zusammensetzungen in Futter, Nahrungsmitteln, Kosmetika oder Pharmazeutika ein weiterer Erfindungsgegenstand.

Ein weiterer Aspekt der Erfindung betrifft Verfahren zur Modulation der Produktion eines Moleküls durch einen Mikroorganismus. Diese Verfahren umfassen das Zusammenbringen der Zelle mit einer Substanz, welche die erfindungsgemäßen Desaturaseaktivität allein oder in Kombination oder die Desaturasenukleinsäureexpression moduliert, so dass eine zellassoziierte Aktivität relativ zu der gleichen Aktivität in Abwesenheit der Substanz verändert wird. Bei einer bevorzugten Ausführungsform wird/werden ein oder zwei Stoffwechselweg(e) der Zelle für Lipide und Fettsäuren, Cofaktoren und Enzyme moduliert oder der Transport von Verbindungen über diese Membranen moduliert, so dass die Ausbeute oder die Rate der Produktion einer gewünschten Feinchemikalie durch diesen Mikroorganismus verbessert ist. Die Substanz, welche die Desaturaseaktivität moduliert, kann eine Substanz sein, welche die Desaturaseaktivität oder Desaturasenukleinsäureexpression stimuliert oder die als Zwischenprodukt bei der Fettsäurebiosynthese verwendet werden kann. Beispiele für Substanzen, welche die Desaturaseaktivität oder Desaturasenukleinsäureexpression stimulieren, sind u.a. kleine Moleküle, aktive Desaturasen sowie desaturasenkodierende Nukleinsäuren, die in die Zelle eingebracht worden sind. Beispiele für Substanzen, welche die Desaturaseaktivität oder -Expression hemmen, sind u.a. kleine Moleküle und Antisense- Desaturasenukleinsäuremoleküle.

Ein weiterer Aspekt der Erfindung betrifft Verfahren zur Modulation der Ausbeuten einer gewünschten Verbindung aus einer Zelle, umfassend das Einbringen eines Wildtyp- oder Mutanten-Desaturasegens, das entweder auf einem separaten Plasmid gehalten oder in das Genom der Wirtszelle integriert wird, in eine Zelle. Bei Integration in das Genom kann die Integration zufallsgemäß sein oder durch derartige Rekombination erfolgen, dass das native Gen durch die eingebrachte Kopie ersetzt wird, wodurch die Produktion der gewünschten Verbindung durch die Zelle moduliert wird, oder durch Verwendung eines Gens in trans, so dass das Gen mit einer funktionellen Expressionseinheit, welche mindestens eine die Expression eines Gens gewährleistende Sequenz und mindestens eine die Polyadenylierung eines funktionell transkribierten Gens gewährleistende Sequenz enthält, funktionell verbunden ist.

Bei einer bevorzugten Ausführungsform sind die Ausbeuten modifiziert. Bei einer weiteren bevorzugten Ausführungsform wird die gewünschte Chemikalie vermehrt, wobei unerwünschte störende Verbindungen vermindert werden können. Bei einer besonders bevorzugten Ausführungsform ist die gewünschte Feinchemikalie ein Lipid oder eine Fettsäure, ein Cofaktor oder ein Enzym. Bei besonders bevorzugten Ausführungsform ist diese Chemikalie eine mehrfach ungesättigte Fettsäure. Stärker bevorzugt ist sie ausgewählt aus Arachidonsäure (ARA), Eicosapentaensäure (EPA) oder Docosahexaensäure (DHA).

Die vorliegende Erfindung stellt neue Nukleinsäuremoleküle bereit, die zur Identifizierung und Isolierung von Desaturasen der PUFA-Biosynthese geeignet sind und zur Modifikation von Ölen, Fettsäuren, Lipiden, von Lipiden stammenden Verbindungen und am stärksten bevorzugt zur Herstellung mehrfach ungesättigter Fettsäuren verwendet werden können.

Ferner stellt die Erfindung Multiexpressionskassetten und Konstrukte zur multiparallelen samenspezifischen Expression von Genkombinationen in Pflanzen zur Verfügung.

Mikroorganismen, wie Crypthecodinium, Thraustochytrium, Phaeodactylum und Mortierella, Entomophthora, Mucor, Crypthecodinium sowie andere Algen und Pilze und Pflanzen, insbesondere Ölfruchtpflanzen sind bevorzugte Organismen für das erfindungsgemäße Verfahren.

In WO 98/01572, oder in Falciatore et al., 1999, Marine Biotechnology 1(3):239-251, sowie Dunahay et al., 1995, Genetic transformation of diatoms, J. Phycol. 31:10004-1012 und den Zitaten darin werden Klonierungsvektoren und Techniken zur genetischen Manipulation der obengenannten Mikroorganismen und Ciliaten, Algen oder verwandten Organismen, wie Phaeodactylum tricornutum, beschrieben. Dadurch können die oben genannten Nukleinsäuremoleküle im erfindungsgemäßen Verfahren, indem die Organismen zur gentechnologisch verändert werden, verwendet werden, so dass sie bessere oder effizientere Produzenten einer oder mehrerer Feinchemikalien werden. Diese verbesserte Produktion oder Effizienz der Produktion einer Feinchemikalie kann durch eine direkte Wirkung der Manipulation eines erfindungsgemäßen Gens oder durch eine indirekte Wirkung dieser Manipulation hervorgerufen werden. Unter Feinchemikalien sind im Sinne der Erfindung Fettsäureester, die mehrfach ungesättigte Fettsäuren mit mindestens zwei Doppelbindungen enthalten wie Sphingolipide, Phosphoglyceride, Lipide, Glycolipide, Phospholipide, Monoacylglyceride, Diacylglyceride, Triacylglyceride oder sonstige Fettsäureester, die die mehrfach ungesättigten Fettsäuren mit mindestens zwei Doppelbindungen enthalten, zu verstehen. Weiter sind darunter zu verstehen Verbindungen wie Vitamine beispielsweise Vitamin E, Vitamin C, Vitamin B2, Vitamin B6, Pantolacton, Carotinoide wie Astaxanthin, β-carotin, Zeaxanthin und andere.

Moose und Algen sind die einzigen bekannten Pflanzensysteme, die erhebliche Mengen an mehrfach ungesättigten Fettsäuren, wie Arachidonsäure (ARA) und/oder Eicosapentaensäure (EPA) und/oder Docosahexaensäure (DHA) herstellen. Moose enthalten PUFAs in Membranlipiden während Algen, algenverwandte Organismen und einige Pilze auch nennenswerte Mengen an PUFAs in der Triacylglycerolfraktion akkumulieren. Daher eignen sich Nukleinsäuremoleküle, die aus solchen Stämmen isoliert werden, die PUFAs auch in der Triacylglycerolfraktion akkumulieren, besonders vorteilhaft zur Modifikation des Lipid- und PUFA-Produktionssystems in einem Wirt, insbesondere in Mikroorganismen, wie den vorstehend erwähnten Mikroorganismen, und Pflanzen, wie Ölfruchtpflanzen, beispielsweise Raps, Canola, Lein, Soja, Sonnenblumen, Borretsch. Sie sind deshalb vorteilhaft im erfindungsgemäßen Verfahren verwendbar.

Weiterhin eignen sich deshalb die erfindungsgemäßen Nukleinsäuren besonders vorteilhaft zur Isolierung von Nukleinsäuren aus Triacylglycerol-akkumulierenden Mikroorganismen und zur Identifikation solcher DNA-Sequenzen und den durch sie codierten Enzyme in anderen Arten, die sich zur Modifikation der Biosynthese von Vorläufermolekülen von PUFAs in den entsprechenden Organismen eignen.

Mikroorganismen wie Crypthecodinium cohnii, Thraustochytrium und Phaeodactylum-Species sind Mikroganismen, die PUFAs wie ARA, EPA oder DHA in Triacylglycerolen akkumulieren können. Thraustochytrien sind phylogenetisch auch eng verwandt mit Schizochytrien Stämmen. Die Fähigkeit, anhand der erfindungsgemäßen Nukleinsäuren Desaturasen zu identifizieren, z.B. die Vorhersage der Substratspezifität von Enzymen, kann daher von signifikanter Bedeutung sein. Ferner können diese Nukleinsäuremoleküle als Bezugssequenzen zur Kartierung verwandter Genome oder zur Ableitung von PCR-Primern dienen.

Die erfindungsgemäßen Nukleinsäuremoleküle kodieren für Proteine, die als Desaturasen bezeichnet werden. Diese Desaturasen können beispielsweise eine Funktion ausüben, die am Stoffwechsel (z.B. an der Biosynthese oder am Abbau) von Verbindungen, die zur Lipid- oder Fettsäuresynthese notwendig sind, wie PUFAs, beteiligt sind oder am Transmembrantransport einer oder mehrerer Lipid-/Fettsäureverbindungen entweder in die oder aus der Zelle teilnehmen.

Die erfindungsgemäßen Nukleinsäuresequenzen codieren für Desaturasen, die zur Produktion langkettiger mehrfach ungesättigter Fettsäuren, vorzugsweise mit mehr als sechzehn, achtzehn oder zwanzig Kohlenstoffatomen im Kohlenstoffgrundgerüst der Fettsäure und/oder mindestens zwei Doppelbindungen in der Kohlenstoffkette, geeignet sind, wobei eine erfindungsgemäße Nukleinsäure für ein Enzym codiert, das Doppelbindungen in die Δ-5-Position, in einem anderen Fall in die Δ-6-Position und in einem weiteren Fall in die Δ-12-Position einführen kann. Mithilfe dieser Nukleinsäuren können hohe Mengen an PUFAs in der Triacylgycerolfraktion erhalten werden. Weiterhin wurden weitere Desaturasen isoliert, die allein oder zusammen mit einer Δ-4-Desaturase für ein Verfahren zur Produktion polyungesättigter Fettsäuren genutzt werden können. Dabei ist in der Anmeldung unter dem Singular d.h. unter einem Desaturasegen oder -Protein auch der Plural d.h. die Desaturasegenen oder -Proteinen zu verstehen.

Die Herstellung einer Triensäure mit C₁₈-Kohlenstoffkette mithilfe von Desaturasen konnte bisher gezeigt werden. In diesen literaturbekannten Verfahren wurde die Herstellung von γ-Linolensäure beansprucht. Bisher konnte jedoch niemand die Herstellung sehr langkettiger mehrfach ungesättigter Fettsäuren (mit C₂₀- und längerer Kohlenstoffkette sowie von Triensäuren und höher ungesättigten Typen) allein durch modifizierte Organismen zeigen.

Zur Herstellung der erfindungsgemäßen langkettiger PUFAs müssen die mehrfach ungesättigten C₁₈-Fettsäuren zunächst durch die enzymatische Aktivität einer Elongase um mindestens zwei Kohlenstoffatome verlängert werden. Nach einer Elongationsrunde führt diese Enzymaktivität zu C₂₀-Fettsäuren, und nach zwei, drei und vier Elongationsrunden zu C₂₂-, C₂₄- oder C₂₆-Fettsäuren. Die in dieser Erfindung offenbarten Nukleinsäuresequenzen, die für verschiedene Desaturasen codieren, können im Konzert mit Elongasen zu sehr langkettigen, polyungesättigten führen. Die Aktivität der erfindungsgemäßen Desaturasen führt vorzugsweise zu C₁₈-, C₂₀- und/oder C₂₂-Fettsäuren mit mindestens zwei Doppelbindungen im Fettsäuremolekül, vorzugsweise mit drei, vier, fünf oder sechs Doppelbindungen, besonders bevorzugt zu C₁₈- und/oder C₂₀-Fettsäuren mit mindestens zwei Doppelbindungen im Fettsäuremolekül, vorzugsweise mit drei, vier oder fünf Doppelbindungen im Molekül. Die Fettsäureelongation kann durch Kombination der erfindungsgemäßen Desaturasen mit einer Elongaseaktivität erfolgen, wobei die durch die in SEQ ID NO: 9 codierte Elongase vorteilhaft verwendet werden kann. Nachdem die Verlängerung mit dem erfindungsgemäßen Enzym(en) stattgefunden hat, können weitere Desaturierungsschritte wie z.B. eine solche in Δ-5-Position erfolgen. Auch die Kombination mit anderen Elongasen wie solche, die zu einer Verlängerung von C₁₈- auf C₂₀- oder von C₂₀- auf C₂₂₋₂₄ Ketten wie in WO0012720 offenbart führt, kann Verwendung finden und/oder einer Desaturase mit Aktivität für Δ-4-Position kann vorteilhaft eingesetzt werden, um die hoch desaturierten Fettsäuren zu erhalten. Daher führen die Produkte der Desaturaseaktivitäten und der möglichen weiteren Desaturierung zu bevorzugten PUFAs mit einem höheren Desaturierungsgrad, wie Dihomo-gamma-Lonolensäure, Docosadiensäure, Arachidonsäure, ω6-Eicosatriendihomo-γ-linolensäure, Eicosapentaensäure, ω3-Eicosatriensäure, ω3-Eicosatetraensäure, Docosapentaensäure oder Docosahexaensäure. Substrate der erfindungsgemäßen Enzymaktivität sind zum Beispiel Taxolsäure; 6,9-Octadecadiensäure, Linolsäure, Pinolensäure, α-oder γ-Linolensäure oder Stearidonsäure sowie Arachidonsäure, Eicosatetraensäure, Docosapentaensäure, Eicosapentaensäure. Bevorzugte Substrate sind Linolsäure, γ-Linolensäure und/oder α-Linolensäure sowie Arachidonsäure, Eicosatetraensäure, Docosapentaensäure, Eicosapentaensäure. Besonders bevorzugt als Produkte des erfindungsgemäßen Verfahrens sind Arachidonsäure, Docosapentaensäure, Eicosapentaensäure. Die C₁₈-Fettsäuren mit mindestens zwei Doppelbindungen in der Fettsäure können durch die erfindungsgemäße enzymatische Aktivität in Form der freien Fettsäure oder in Form der Ester, wie Phospholipide, Glycolipide, Sphingolipide, Phosphoglyceride, Monoacylglyceride, Diacylglyceride oder Triacylglyceride, verlängert werden.

Für die menschliche Ernährung ist konjugierte Linolsäure "CLA" von besonderer Bedeutung. Unter CLA versteht man insbesondere Fettsäuren wie C18:2 ^{9 cis}, 11trans oder das Isomer C18:2 10trans, 12 cis, die aufgrund menschlicher Enzymsysteme nach Aufnahme im Körper desaturiert bzw. elongiert werden können und zu gesundheitsfördernden Effekten beitragen können. Mit den erfindungsgemäßen Desaturasen (Δ-12-Desaturase) können auch solche konjugierten Fettsäuren mit wenigstens zwei Doppelbindungen im Molekül desaturiert werden und damit solche gesundheitsfördernden Fettsäuren der menschlichen Ernährung zugeführt werden. Weitere Beispiel für konjugierte Fettsäuren sind alpha-Parinarsäure, Punicasäure, Eleostearinsäure und Calendulasäure.

Unter der Verwendung von Klonierungsvektoren in Pflanzen und bei der Pflanzentransformation, wie denjenigen, die veröffentlicht sind in und dort zitiert sind: Plant Molecular Biology and Biotechnology (CRC Press, Boca Raton, Florida), Kapitel 6/7, S. 71-119 (1993); F.F. White, Vectors for Gene Transfer in Higher Plants; in: Transgenic Plants, Bd. 1, Engineering and Utilization, Hrsgb.: Kung und R. Wu, Academic Press, 1993, 15-38; B. Jenes et al., Techniques for Gene Transfer, in: Transgenic Plants, Bd. 1, Engineering and Utilization, Hrsgb.: Kung und R. Wu, Academic Press (1993), 128-143; Potrykus, Annu. Rev. Plant Physiol. Plant Molec. Biol. 42 (1991), 205-225)), lassen sich die erfindungsgemäßen Nukleinsäuren zur gentechnologischen Veränderung eines breiten Spektrums an Pflanzen verwenden, so dass diese ein besserer oder effizienterer Produzent eines oder mehrerer von Lipiden hergeleiteter Produkte, wie PUFAs, wird. Diese verbesserte Produktion oder Effizienz der Produktion eines von Lipiden hergeleiteten Produktes, wie PUFAs, kann durch direkte Wirkung der Manipulation oder eine indirekte Wirkung dieser Manipulation hervorgerufen werden.

Es gibt eine Reihe von Mechanismen, durch die die Veränderung eines erfindungsgemäßen Desaturaseproteins die Ausbeute, Produktion und/oder Effizienz der Produktion einer Feinchemikalie aus einer Ölfruchtpflanze oder einem Mikroorganismus aufgrund eines veränderten Proteins direkt beeinflussen kann. Die Anzahl oder Aktivität des Desaturaseproteins oder -Gens sowie von Genkombinationen von Desaturasen und Elongasen kann erhöht sein, so dass größere Mengen dieser Verbindungen de novo hergestellt werden, weil den Organismen diese Aktivität und Fähigkeit zur Biosynthese vor dem Einbringen des entsprechenden Gens fehlte. Entsprechendes gilt für die Kombination mit weiteren Desaturasen oder Elongasen oder weiteren Enzymen aus dem Lipidstoffwechsel. Auch die Verwendung verschiedener divergenter, d.h. auf DNA-Sequenzebene unterschiedlicher Sequenzen kann dabei vorteilhaft sein bzw. die Verwendung von Promotoren zur Genexpression, die eine andere zeitliche Genexpression z.B. abhängig vom Reifegrad eines Samens oder Öl-speichernden Gewebes ermöglicht.

Durch das Einbringen eines erfindungsgemäßen Desaturasegens oder mehrerer Desaturasegene in einen Organismus allein oder in Kombination mit anderen Genen in eine Zelle kann nicht nur den Biosynthesefluss zum Endprodukt erhöhen, sondern auch die entsprechende Triacylglycerin-Zusammensetzung erhöhen oder de novo schaffen. Ebenso kann die Anzahl oder Aktivität anderer Gene, die am Import von Nährstoffen, die zur Biosynthese einer oder mehrerer Feinchemikalien (z.B. Fettsäuren, polaren und neutralen Lipiden) nötig sind, erhöht sein, so dass die Konzentration dieser Vorläufer, Cofaktoren oder Zwischenverbindungen innerhalb der Zellen oder innerhalb des Speicherkompartiments erhöht ist, wodurch die Fähigkeit der Zellen zur Produktion von PUFAs, wie im folgenden beschrieben, weiter gesteigert wird. Fettsäuren und Lipide sind selbst als Feinchemikalien wünschenswert; durch Optimierung der Aktivität oder Erhöhung der Anzahl einer oder mehrerer Desaturasen, die an der Biosynthese dieser Verbindungen beteiligt sind, oder durch Zerstören der Aktivität einer oder mehrerer Desaturasen, die am Abbau dieser Verbindungen beteiligt sind, kann es möglich sein, die Ausbeute, Produktion und/oder Effizienz der Produktion von Fettsäure- und Lipidmolekülen aus Pflanzen oder Mikroorganismen zu steigern.

Die Mutagenese der/des erfindungsgemäßen Desaturasegene(s) kann weiterhin zu einem Desaturaseprotein mit geänderten Aktivitäten führen, welche die Produktion einer oder mehrerer gewünschter Feinchemikalien direkt oder indirekt beeinflussen. Beispielsweise kann die Anzahl oder Aktivität der/des erfindungsgemäßen Desaturasegens(e) gesteigert werden, so dass die normalen Stoffwechselabfälle oder -nebenprodukte der Zelle (deren Menge möglicherweise aufgrund der Überproduktion der gewünschten Feinchemikalie erhöht ist) effizient exportiert werden, bevor sie andere Moleküle oder Prozesse innerhalb der Zelle (welche die Lebensfähigkeit der Zelle senken würden) zerstören oder die Biosynthesewege der Feinchemikalie stören würden (wodurch die Ausbeute, Produktion oder Effizienz der Produktion der gewünschten Feinchemikalie verringert wird). Ferner können die relativ großen intrazellulären Mengen der gewünschten Feinchemikalie selbst toxisch für die Zelle sein oder Enzym-Rückkopplungsmechanismen, wie die allosterische Regulation, stören, beispielsweise könnte sie durch Steigerung der Aktivität oder Anzahl anderer stromabwärts folgender Enzyme oder Entgiftungsenzyme des PUFA-Wegs die Allokation der PUFA in die Triacylgylcerin-Fraktion steigern, man könnte die Lebensfähigkeit von Saatzellen erhöhen, was wiederum zu besserer Entwicklung von Zellen in Kultur oder zu Saaten führt, die die gewünschte Feinchemikalie produzieren. Das erfindungsgemäße Desaturasegen kann auch so manipuliert werden, dass die entsprechenden Mengen der verschiedenen Lipid- und Fettsäuremoleküle hergestellt werden. Dies kann eine einschneidende Wirkung auf die Lipidzusammensetzung der Membran der Zelle haben und erzeugt neue Öle zusätzlich zum Auftreten neusynthetisierter PUFAs. Da jeder Lipidtyp unterschiedliche physikalische Eigenschaften hat, kann eine Veränderung der Lipidzusammensetzung einer Membran die Membranfluidität erheblich verändern. Änderungen der Membranfluidität können sich auf den Transport von Molekülen über die Membran sowie auf die Unversehrtheit der Zelle auswirken, die beide eine entscheidende Wirkung auf die Produktion von Feinchemikalien besitzen. In Pflanzen können diese Änderungen überdies auch andere Merkmale, wie Toleranz gegenüber abiotischen und biotischen Stresssituationen, beeinflussen.

Biotische und abiotische Stresstoleranz ist ein allgemeines Merkmal, das man an ein breites Spektrum von Pflanzen, wie Mais, Weizen, Roggen, Hafer, Triticale, Reis, Gerste, Sojabohne, Erdnuss, Baumwolle, Öl- und Faserlein, Raps und Canola, Lein, Maniok, Pfeffer, Sonnenblume und Tagetes, Solanaceen-Pflanzen, wie Kartoffel, Tabak, Aubergine und Tomate, Vicia-Arten, Erbse, Alfalfa, Buschpflanzen (Kaffee, Kakao, Tee), Salix-Arten, Bäume (Ölpalme, Kokosnuss) und ausdauernde Gräser und Futterfeldfrüchte, vererben möchte. Diese Feldfrüchte sind als weitere erfindungsgemäße Ausführungsform auch bevorzugte Zielpflanzen für die Gentechnologie. Besonders bevorzugte erfindungsgemäße Pflanzen sind Ölfruchtpflanzen, wie Sojabohne, Erdnuss, Raps, Canola, Sonnenblume, Lein, Safflor, Bäume (Ölpalme, Kokosnuss) oder Feldfrüchte, wie Mais, Weizen, Roggen, Hafer, Triticale, Reis, Gerste, Alfalfa, oder Buschpflanzen (Kaffee, Kakao, Tee).

Folglich betrifft ein Aspekt der Erfindung isolierte Nukleinsäuremoleküle (z.B. cDNAs), umfassend Nukleotidsequenzen, die eine Desaturase oder mehrere Desaturasen oder biologisch aktive Teile davon codieren, oder Nukleinsäurefragmente, die sich als Primer oder Hybridisierungssonden zum Nachweis oder zur Amplifikation desaturasekodierender Nukleinsäuren (z.B. DNA oder mRNA) eignen. Bei besonders bevorzugten Ausführungsformen umfasst das Nukleinsäuremolekül eine der in Sequenz ID NO:1 bzw 3 und 5 dargestellten Nukleotidsequenzen oder die kodierende Region oder ein Komplement einer dieser Nukleotidsequenzen. Bei anderen besonders bevorzugten Ausführungsformen umfasst das erfindungsgemäße isolierte Nukleinsäuremolekül eine Nukleotidsequenz, die an eine Nukleotidsequenz, wie in der Sequenz SEQ ID NO: 1, 3, 5 oder 11 dargestellt, oder einen Teil davon hybridisiert oder zu mindestens etwa 50 %, vorzugsweise mindestens etwa 60 %, stärker bevorzugt mindestens etwa 70 %, 80 % oder 90 % und noch stärker bevorzugt mindestens etwa 95 %, 96 %, 97 %, 98 %, 99 % oder mehr homolog dazu ist. Bei anderen bevorzugten Ausführungsformen kodiert das isolierte Nukleinsäuremolekül eine der in der Sequenz SEQ ID NO: 2, 4, 6 oder 12 dargestellten Aminosäuresequenzen. Das bevorzugte erfindungsgemäße Desaturasegen besitzt vorzugsweise auch mindestens eine der hier beschriebenen Desaturaseaktivitäten.

Bei einer weiteren Ausführungsform kodiert das isolierte Nukleinsäuremolekül ein Protein oder einen Teil davon, wobei das Protein oder der Teil davon eine Aminosäuresequenz enthält, die ausreichend homolog zu einer Aminosäuresequenz der Sequenz SEQ ID NO: 2, 4, 6 oder 12 ist, dass das Protein oder der Teil davon eine Desaturaseaktivität beibehält. Vorzugsweise behält das Protein oder der Teil davon, das/der von dem Nukleinsäuremolekül kodiert wird, die Fähigkeit, am Stoffwechsel von zum Aufbau von Zellmembranen von Pflanzen notwendigen Verbindungen oder am Transport von Molekülen über diese Membranen teilzunehmen. Bei einer Ausführungsform ist das von dem Nukleinsäuremolekül kodierte Protein zu mindestens etwa 50 %, vorzugsweise mindestens etwa 60 % und stärker bevorzugt mindestens etwa 70 %, 80 % oder 90 % und am stärksten bevorzugt mindestens etwa 95 %, 96 %, 97 %, 98 %, 99 % oder mehr homolog zu einer Aminosäuresequenz der Sequenz SEQ ID NO: 2, 4, 6 oder 12. Bei einer weiteren bevorzugten Ausführungsform ist das Protein ein Volllängen-Protein, das im wesentlichen in Teilen homolog zu einer gesamten Aminosäuresequenz der SEQ ID NO: 2, 4, 6 oder 12 (die von dem in SEQ ID NO: 1, 3, 5 oder 11 gezeigten offenen Leserahmen herrührt) ist.

Bei anderen Ausführungsformen umfasst die isolierte Desaturase eine Aminosäuresequenz, die zu mindestens etwa 50 % homolog zu einer der Aminosäuresequenzen der SEQ ID NO: 2, 4, 6 oder 12 ist und am Stoffwechsel von zum Aufbau von Fettsäuren in einem Mikroorganismus oder einer Pflanzenzelle notwendigen Verbindungen oder am Transport von Molekülen über diese Membranen teilnehmen kann, wobei desaturierte C₁₈- oder C₂₀₋₂₂-Kohlenstoffketten mit Doppelbindungen an mindestens zwei Stellen gemeint ist.

Bei einer anderen bevorzugten Ausführungsform rührt das isolierte Nukleinsäuremolekül von Phaeodactylum tricornutum UTEX646 her und kodiert ein Protein (z.B. ein Desaturasefusionsprotein), das eine biologisch aktive Domäne enthält, die zu mindestens etwa 50 % oder mehr homolog zu einer Aminosäuresequenz der Sequenz SEQ ID NR 2, 4, 6 oder 12 ist und die Fähigkeit, am Stoffwechsel von zum Aufbau von Zellmembranen von Pflanzen notwendigen Verbindungen oder am Transport von Molekülen über diese Membranen teilzunehmen, beibehält oder zumindest eine der Desaturierungsaktivitäten resultierend in PUFAs wie GLA, ALA, Dihomo-gamma Linolensäure, ARA, EPA oder DHA oder deren Vorläufermoleküle besitzt, und umfasst auch heterologe Nukleinsäuresequenzen, die ein heterologes Polypeptid oder regulatorische Proteine kodieren.

Alternativ kann die isolierte Desaturase eine Aminosäuresequenz umfassen, die von einer Nukleotidsequenz kodiert wird, die an eine Nukleotidsequenz der SEQ ID NO: 1, 3, 5 oder 11 hybridisiert, z.B. unter stringenten Bedingungen hybridisiert, oder zu mindestens etwa 50 %, vorzugsweise mindestens etwa 60 %, stärker bevorzugt mindestens etwa 70 %, 80 % oder 90 % und noch stärker bevorzugt mindestens etwa 95 %, 96 %, 97 %, 98 %, 99 % oder mehr homolog dazu ist. Es ist ebenfalls bevorzugt, dass die bevorzugten Desaturaseformen ebenfalls eine der hier beschriebenen Desaturaseaktivitäten besitzen.

Bei einer anderen Ausführungsform ist das isolierte Nukleinsäuremolekül mindestens 15, 25, 50, 100, 250 oder mehr Nukleotide lang und hybridisiert unter stringenten Bedingungen an ein Nukleinsäuremolekül, das eine Nukleotidsequenz der SEQ ID NO: 1, 3, 5 oder 17 umfasst. Vorzugsweise entspricht das isolierte Nukleinsäuremolekül einem natürlich vorkommenden Nukleinsäuremolekül. Stärker bevorzugt kodiert das isolierte Nukleinsäuremolekül natürlich vorkommende Phaeodactylum-Desaturase oder einen biologisch aktiven Teil davon.

Eine weitere Ausführungsform der Erfindung sind Expressionskassetten, die die Expression der erfindungsgemäßen Nukleinsäuren mit den Sequenzen SEQ ID NO: 1, 3, 5 oder 11 in den verschiedenen Organismen wie Mikroorganismen beispielsweise Bakterien, Pilze, Hefen, Ciliaten, Algen oder tierische oder pflanzliche Zellen oder in Tieren oder Pflanzen ermöglichen.

Unter der erfindungsgemäßen Expressionskassette (= Nukleinsäurekonstrukt oder -fragment) sind die in SEQ ID NO: 1, SEQ ID NO: 3, SEQ ID NO: 5 oder SEQ ID NO: 11 genannten Sequenzen, die sich als Ergebnis des genetischen Codes und/oder deren funktionellen oder nicht funktionellen Derivate zu verstehen, die mit einem oder mehreren Regulationssignalen vorteilhafterweise zur Erhöhung der Genexpression funktionell verknüpft wurden und welche die Expression der codierenden Sequenz in der Wirtszelle steuern. Diese regulatorischen Sequenzen sollen die gezielte Expression der Gene und der Proteinexpression ermöglichen. Dies kann beispielsweise je nach Wirtsorganismus bedeuten, dass das Gen erst nach Induktion exprimiert und/oder überexprimiert wird, oder dass es sofort exprimiert und/oder überexprimiert wird. Beispielsweise handelt es sich bei diesen regulatorischen Sequenzen um Sequenzen an die Induktoren oder Repressoren binden und so die Expression der Nukleinsäure regulieren. Zusätzlich zu diesen neuen Regulationssequenzen oder anstelle dieser Sequenzen kann die natürliche Regulation dieser Sequenzen vor den eigentlichen Strukturgenen noch vorhanden sein und gegebenenfalls genetisch verändert worden sein, so dass die natürliche Regulation ausgeschaltet und die Expression der Gene erhöht wurde. Das Genkonstrukt kann aber auch einfacher aufgebaut sein, das heißt es wurden keine zusätzlichen Regulationssignale vor die Nukleinsäuresequenz oder dessen Derivate inseriert und der natürliche Promotor mit seiner Regulation wurde nicht entfernt. Stattdessen wurde die natürliche Regulationssequenz so mutiert, dass keine Regulation mehr erfolgt und/oder die Genexpression gesteigert wird. Diese veränderten Promotoren können in Form von Teilsequenzen (= Promotor mit Teilen der erfindungsgemäßen Nukleinsäuresequenzen) auch allein vor das natürliche Gen zur Steigerung der Aktivität gebracht werden. Das Genkonstrukt kann außerdem vorteilhafterweise auch eine oder mehrere sogenannte "enhancer Sequenzen" funktionell verknüpft mit dem Promotor enthalten, die eine erhöhte Expression der Nukleinsäuresequenz ermöglichen. Auch am 3'-Ende der DNA-Sequenzen können zusätzliche vorteilhafte Sequenzen inseriert werden wie weitere regulatorische Elemente oder Terminatoren. Die Δ-5-Desaturase-/Δ-6-Desaturase und/oder Δ-12-Desaturasegene können in einer oder mehreren Kopien in der Expressionskassette (= Genkonstrukt) enthalten sein.

Die regulatorischen Sequenzen bzw. Faktoren können dabei wie oben beschrieben vorzugsweise die Genexpression der eingeführten Gene positiv beeinflussen und dadurch erhöhen. So kann eine Verstärkung der regulatorischen Elemente vorteilhafterweise auf der Transkriptionsebene erfolgen, indem starke Transkriptionssignale wie Promotoren und/oder "Enhancer" verwendet werden. Daneben ist aber auch eine Verstärkung der Translation möglich, indem beispielsweise die Stabilität der mRNA verbessert wird.

Ein weiterer Aspekt der Erfindung betrifft Vektoren, z.B. rekombinante Expressionsvektoren, die mindestens ein erfindungsgemäßes Nukleinsäuremolekül enthalten, und Wirtszellen, in die diese Vektoren eingebracht worden sind, insbesondere Mikroorganismen, Pflanzenzellen, Pflanzengewebe, -organe oder ganze Pflanzen. Bei einer Ausführungsform kann eine solche Wirtszelle Feinchemikalien-Verbindungen, insbesondere PUFAs, speichern; zur Isolation der gewünschten Verbindung werden die Zellen geerntet. Die Verbindung (Öle, Lipide, Triacylglyceride, Fettsäuren) oder die Desaturase können dann aus dem Medium oder der Wirtszelle, welche bei Pflanzen Zellen sind, die Feinchemikalien enthalten oder speichern, am stärksten bevorzugt Zellen von Speichergeweben, wie Samenhüllen, Knollen, Epidermis- und Samenzellen, Endosperm oder Embyrogewebe isoliert werden.

Noch ein weiterer Aspekt der Erfindung betrifft eine genetisch veränderte transgene Pflanze, bevorzugt ein Ölfruchtpflanze, wie vorstehend erwähnt, besonders bevorzugt eine Raps- oder Leinpflanze, in die ein Desaturasegen eingebracht worden ist. Bei einer Ausführungsform ist das Genom von Raps oder Lein durch Einbringen eines erfindungsgemäßen Nukleinsäuremoleküls, das eine Wildtyp- oder mutierte Desaturasesequenz kodiert, als Transgen verändert worden. Bei einer anderen Ausführungsform ist ein endogenes Desaturasegen im Genom des Spenderorganismus Phaeodactylum Mutagenese und Detektion mittels DNA-Sequenzen funktionell zerstört worden oder mittels Antisensetechnologie reprimiert worden. Bei einer bevorzugten Ausführungsform wird Raps oder Lein auch zur Produktion einer gewünschten Verbindung, wie Lipiden und Fettsäuren, wobei PUFAs besonders bevorzugt sind, verwendet.

Bei noch einer weiteren bevorzugten Ausführungsform kann das Moos Physcomitrella patens zur Demonstration der Funktion eines Desaturasesegens unter Verwendung homologer Rekombination auf der Basis der in dieser Erfindung beschriebenen Nukleinsäuren verwendet werden.

Noch ein weiterer Aspekt der Erfindung betrifft ein isoliertes Desaturasegen oder einen Teil, z.B. einen biologisch aktiven Teil, davon. Bei einer bevorzugten Ausführungsform kann die isolierte Desaturase oder ein Teil davon am Stoffwechsel von zum Aufbau von Zellmembranen in einem Mikroorganismus oder einer Pflanzenzelle notwendigen Verbindungen oder am Transport von Molekülen über dessen/deren Membranen teilnehmen. Bei einer weiteren bevorzugten Ausführungsform ist die isolierte Desaturase oder der Teil davon ausreichend homolog zu einer Aminosäuresequenz der SEQ ID NO: 2, 4, 6 oder 12 das dieses Protein oder der Teil davon die Fähigkeit, am Stoffwechsel von zum Aufbau von Zellmembranen in Mikroorganismen oder Pflanzenzellen notwendigen Verbindungen oder am Transport von Molekülen über diese Membranen teilzunehmen, beibehält.

Die Erfindung stellt auch eine isolierte Präparation einer Desaturase in Form eines Rohextraktes oder als reines Protein bereit.

Das Desaturasepolypeptid oder ein biologisch aktiver Teil davon kann vorteilhaft funktionsfähig mit einem weiteren Polypeptid, das eine andere enzymatische Aktivität als die Desaturasen hat beispielsweise eine Elongase-, Acyltransferase- oder sonstige Aktivität verbunden werden, so dass ein Fusionsprotein gebildet wird. Vorteilhaft hat dieses Fusionsprotein eine Aktivität, die sich von derjenigen der Desaturase allein unterscheidet. Bei anderen bevorzugten Ausführungsform nimmt dieses Fusionsprotein am Stoffwechsel von Verbindungen, die zur Synthese von Lipiden und Fettsäuren, Cofaktoren und Enzymen in Mikroorganismen oder Pflanzen notwendig sind, oder am Transport von Molekülen über diese Membranen teil. Besonders vorteilhaft moduliert das Einbringen dieses Fusionsproteins in einer Wirtszelle die Produktion einer gewünschten Verbindung innerhalb einer und durch die Zelle. Bei einer bevorzugten Ausführungsform enthalten diese Fusionsproteine auch Δ-4-, Δ-5- oder Δ-6, Δ-8-, Δ-15, Δ-17 oder Δ-19-Desaturaseaktivitäten allein oder in Kombination. Insbesondere solche Genkombinationen sind bevorzugte Ausführungsformen, die aus SEQ ID NO: 7 oder 9 gewählt sind, bzw Teilen davon, Derivate oder ihren Homologen. Insbesondere solche Kombinationen sind bevorzugt, die die vollständige Proteinaktivität wie in SEQ ID NO: 1, 3, 5 oder 11 enthalten und in Multiexpressionskassetten definiert durch SEQ ID NO: 13, 14, 15, 16 und 17 eingefügt zur Transformation von Pflanzen und Expression in Pflanzen geeignet sind.

### Eingehende Beschreibung der Erfindung

Ein erfindungsgemäßer Gegenstand ist/sind isolierte Nukleinsäuren, die für ein Polypeptid mit Desaturaseaktivität codiert, ausgewählt aus der Gruppe:
a) einer Nukleinsäuren mit der in SEQ ID NO: 1, SEQ ID NO: 3, SEQ ID NO: 5 oder SEQ ID NO: 11 dargestellten Sequenz,
b) Nukleinsäuren, die aufgrund des degenerierten genetischen Codes durch Rückübersetzung der in SEQ ID NO: 2, SEQ ID NO: 4, SEQ ID NO: 6 oder SEQ ID NO: 12 dargestelltenAminosäuresequenzen erhalten werden,
c) Derivate der in SEQ ID NO: 1, SEQ ID NO:3 , SEQ ID NO : 5 oder SEQ ID NO: 11 dargestellten Nukleinsäuren, die für biologisch aktive Teile einer Desaturase kodieren und mindestens 50% Identität zu der Aminosäuresequenz der SEQ ID NO: 2, SEQ ID NO: 4, SEQ ID NO: 6 oder SEQ ID NO: 12 aufweisen.

Weiterhin betrifft die Erfindung eine Aminosäuresequenz, die durch die oben genannte (n) Nukleinsäuresequenz (en) codiert werden (für die Erfindung soll der Singular den Plural und umgekehrt umfassen). Speziell betrifft die Erfindung Aminosäuresequenzen, die durch die in SEQ ID NO: 1, SEQ ID NO: 3, SEQ ID NO: 5 oder SEQ ID NO: 11 dargestellte Sequenz codiert werden.

Die vorliegende Erfindung stellt Nukleinsäuren und Proteinmoleküle mit Desaturaseaktivität bereit, die am Stoffwechsel von Lipiden und Fettsäuren, PUFA-Cofaktoren und Enzymen in dem Moos Physcomitrella patens oder am Transport lipophiler Verbindungen über Membranen beteiligt sind. Die erfindungsgemäßen Verbindungen lassen sich zur Modulation der Produktion von Feinchemikalien aus Organismen, beispielsweise Mikroorganismen, wie Ciliaten, Pilzen, Hefen, Bakterien, Algen, und/oder Pflanzen, wie Mais, Weizen, Roggen, Hafer, Triticale, Reis, Gerste, Sojabohne, Erdnuss, Baumwolle, Linum Arten wie Öl- oder Faserlein, Brassica-Arten, wie Raps, Canola und Rübsen, Pfeffer, Sonnenblume, Borretsch, Nachtkerze und Tagetes, Solanacaen-Pflanzen, wie Kartoffel, Tabak, Aubergine und Tomate, Vicia-Arten, Erbse, Maniok, Alfalfa, Buschpflanzen (Kaffee, Kakao, Tee), Salix-Arten, Bäume (Ölpalme, Kokosnuss) und ausdauernden Gräsern und Futterfeldfrüchten, entweder direkt (z.B. wenn die Überexpression oder Optimierung eines Fettsäurebiosynthese-Proteins einen direkten Einfluss auf die Ausbeute, Produktion und/oder Effizienz der Produktion der Fettsäure aus modifizierten Organismen hat) verwenden oder können eine indirekt Auswirkung haben, die dennoch zu einer Steigerung der Ausbeute, Produktion und/oder Effizienz der Produktion einer gewünschten Verbindung oder einer Abnahme unerwünschter Verbindungen führt (z.B. wenn die Modulation des Stoffwechsels von Lipiden und Fettsäuren, Cofaktoren und Enzymen zu Veränderungen der Ausbeute, Produktion und/oder Effizienz der Produktion oder der Zusammensetzung der gewünschten Verbindungen innerhalb der Zellen führt, was wiederum die Produktion einer oder mehrerer Feinchemikalien beeinflussen kann). Aspekte der Erfindung sind nachstehend weiter erläutert.

### I. Feinchemikalien und PUFAs

Der Begriff "Feinchemikalie" ist im Fachgebiet bekannt und umfasst Moleküle, die durch einen Organismus produziert worden sind und Anwendungen in verschiedenen Industrien finden, wie, aber nicht beschränkt auf, die pharmazeutische, Landwirtschafts-, Nahrungsmittel- und Kosmetik-Industrie. Diese Verbindungen umfassen Lipide, Fettsäuren, Cofaktoren und Enzyme usw. (wie z.B. beschrieben in Kuninaka, A. (1996) Nucleotides and related compounds, S. 561-612, in Biotechnology Bd. 6, Rehm et al., Hrsgb., VCH: Weinheim und darin enthaltenen Literaturstellen), Lipide, gesättigte und ungesättigte Fettsäuren (z.B. Arachidonsäure), Vitamine und Cofaktoren (wie beschrieben in Ullmann's Encyclopedia of Industrial Chemistry, Bd. A27, Vitamins, S. 443-613 (1996) VCH: Weinheim und darin enthaltenen Literaturstellen; und Ong, A.S., Niki, E., & Packer, L. (1995) Nutrition, Lipids, Health and Disease Proceedings of the UNESCO/Confederation of Scientific and Technological Associations in Malaysia and the Society for Free Radical Research - Asien, abgehalten am 1.-3. Sept. 1994 in Penang, Malysia, AOCS Press (1995)), Enzyme und sämtliche anderen von Gutcho (1983) in Chemicals by Fermentation, Noyes Data Corporation, ISBN: 0818805086, und darin angegebenen Literaturstellen beschriebenen Chemikalien. Der Stoffwechsel und die Verwendungen bestimmter Feinchemikalien sind nachstehend weiter erläutert.

Die Kombination verschiedener Vorläufermoleküle und Biosyntheseenzyme führt zur Herstellung verschiedener Fettsäuremoleküle, was eine entscheidende Auswirkung auf die Zusammensetzung der Membran hat. Es kann angenommen werden, dass PUFAs nicht nur einfach in Triacylglycerin, sondern auch in Membranlipide eingebaut werden.

Die Synthese von Membranen ist ein gut charakterisierter Prozess, an dem eine Anzahl von Komponenten, einschließlich Lipiden als Teil der Bilayer-Membran, beteiligt sind. Die Produktion neuer Fettsäuren, wie PUFAs, kann daher neue Eigenschaften von Membranfunktionen innerhalb einer Zelle oder eines Organismus erzeugen.

Zellmembranen dienen einer Vielzahl von Funktionen in einer Zelle. Zuerst und in erster Linie grenzt eine Membran den Inhalt einer Zelle von der Umgebung ab, wodurch sie der Zelle Integrität verleiht. Membranen können auch als Schranken gegenüber dem Einstrom gefährlicher oder unerwünschter Verbindungen und auch gegenüber dem Ausstrom gewünschter Verbindungen dienen.

Detailliertere Beschreibungen und Beteiligungen von Membranen und die beteiligten Mechanismen siehe in: Bamberg, E., et al. (1993) Charge transport of ion pumps on lipid bilayer membranes, Q. Rev. Biophys. 26:1-25; Gennis, R.B. (1989) Pores, Channels and Transporters, in: Biomembranes, Molecular Structure and Function, Springer: Heidelberg, S. 270-322; und Nikaido, H., und Saier, H. (1992) Transport proteins in bacteria: common themes in their design, Science 258:936-942, und den in jeder dieser Literaturstellen enthaltenen Zitaten.

Die Lipidsynthese lässt sich in zwei Abschnitte unterteilen: die Synthese von Fettsäuren und ihre Bindung an sn-Glycerin-3-Phosphat sowie die Addition oder Modifikation einer polaren Kopfgruppe. Übliche Lipide, die in Membranen verwendet werden, umfassen Phospholipide, Glycolipide, Sphingolipide und Phosphoglyceride. Die Fettsäuresynthese beginnt mit der Umwandlung von Acetyl-CoA in Malonyl-CoA durch die Acetyl-CoA-Carboxylase oder in Acetyl-ACP durch die Acetyltransacylase. Nach einer Kondensationsreaktion bilden diese beiden Produktmoleküle zusammen Acetoacetyl-ACP, das über eine Reihe von Kondensations-, Reduktions- und Dehydratisierungsreaktionen umgewandelt wird, so dass ein gesättigtes Fettsäuremolekül mit der gewünschten Kettenlänge erhalten wird. Die Produktion der ungesättigten Fettsäuren aus diesen Molekülen wird durch spezifische Desaturasen katalysiert, und zwar entweder aerob mittels molekularem Sauerstoff oder anaerob (bezüglich der Fettsäuresynthese in Mikroorganismen siehe F.C. Neidhardt et al. (1996) E. coli und Salmonella. ASM Press: Washington, D.C., S. 612-636 und darin enthaltene Literaturstellen; Lengeler et al. (Hrsgb.) (1999) Biology of Procaryotes. Thieme: Stuttgart, New York, und die enthaltene Literaturstellen, sowie Magnuson, K., et al. (1993) Microbiological Reviews 57:522-542 und die enthaltenen Literaturstellen).

Vorläufer für die PUFA-Biosynthese sind beispielsweise Ölsäure, Linol- und Linolensäure. Diese C₁₈-Kohlenstoff-Fettsäuren müssen auf C₂₀ und C₂₂ verlängert werden, damit Fettsäuren vom Eicosa- und Docosa-Kettentyp erhalten werden. Mithilfe verschiedener Desaturasen, wie Enzymen, welche Δ-12-Desaturase, Δ-15-Desaturase, Δ-6-Desaturase-, Δ-5- und Δ-4-Desaturaseaktivität aufweisen, können Arachidonsäure, Eicosapentaensäure und Docosahexaensäure sowie verschiedene andere langkettige PUFAs erhalten, extrahiert und für verschiedene Zwecke bei Nahrungsmittel-, Futter-, Kosmetik- oder pharmazeutischen Anwendungen verwendet werden.

Zur Herstellung langkettiger PUFAs müssen, wie oben erwähnt, die mehrfach ungesättigten C₁₈- bzw C₂₀-Fettsäuren mehrfach desaturiert werden. Die erfindungsgemäßen Nukleinsäuresequenzen kodieren erste funktionell aktive Desaturasen aus Phyeodactylum tricornutum, einem Mikroorganismus, der PUFAs in der Triacylglycerolfraktion enthält. Mit den erfindungsgemäßen Desaturasen können Doppelbindungen in die Δ-5-, Δ-6- oder Δ-12-Position eingeführt werden. Die Aktivitäten der erfindungsgemäßen Desaturasen führt vorzugsweise zu C₁₈- + C₂₀-Fettsäuren mit mindestens zwei, drei, vier oder fünf Doppelbindungen im Fettsäuremolekül, vorzugsweise zu C₂₀-Fettsäuren mit vorteilhaft drei, vier oder fünf Doppelbindungen im Fettsäuremolekül. Die Desaturierung kann vor oder nach Elongation der entsprechenden Fettsäure erfolgen. Daher führen die Produkte der Desaturaseaktivitäten und der möglichen weiteren Desaturierung und Elongation zu bevorzugten PUFAs mit höherem Desaturierungsgrad, einschließlich einer weiteren Elongation von C₂₀ zu C₂₂-Fettsäuren, zu Fettsäuren wie Linolsäure, Docosadiensäure, dihomo-γ-linolensäure, Arachidonsäure, ω6-Eicosatriendihomo-γ-linolensäure, Eicosapentaensäure, ω3-Eicosatriensäure, ω3-Eicosatetraensäure, Docosapentaensäure oder Docosahexaensäure. Substrate dieser erfindungsgemäßen Enzymaktivität sind zum Beispiel Taxolsäure, 6,9-Octadecadiensäure, Ölsäure, Linolsäure, γ-Linolensäure, Pinolensäure, α-Linolensäure, Arachidonsäure, Eicosapentaensäure oder Stearidonsäure. Bevorzugte Substrate sind Linolsäure, γ-Linolensäure und/oder α-Linolensäure, dihomo-γ-linolensäure bzw. Arachidonsäure, Eicosatetraensäure oder Eicosapentaensäure. Die C₁₈-oder C₂₀-Fettsäuren mit mindestens zwei Doppelbindungen in der Fettsäure können durch die erfindungsgemäße Enzymaktivität in Form der freien Fettsäure oder in Form der Ester, wie Phospholipide, Glykolipide, Sphingolipide, Phosphoglyceride, Monoacylglyceride, Diacylglyceride, Triacylglyceride oder sonstige Ester, verlängert werden.

Ferner müssen Fettsäuren anschließend an verschiedene Modifikationsorte transportiert und in das Triacylglycerin-Speicherlipid eingebaut werden. Ein weiterer wichtiger Schritt bei der Lipidsynthese ist der Transfer von Fettsäuren auf die polaren Kopfgruppen, beispielsweise durch Glycerin-Fettsäure-Acyltransferase (siehe Frentzen, 1998, Lipid, 100(4-5):161-166).

Veröffentlichungen über die Pflanzen-Fettsäurebiosynthese, Desaturierung, den Lipidstoffwechsel und Membrantransport von fetthaltigen Verbindungen, die Betaoxidation, Fettsäuremodifikation und Cofaktoren, Triacylglycerin-Speicherung und -Assemblierung einschließlich der Literaturstellen darin siehe in den folgenden Artikeln: Kinney, 1997, Genetic Engeneering, Hrsgb.: JK Setlow, 19:149-166; Ohlrogge und Browse, 1995, Plant Cell 7:957-970; Shanklin und Cahoon, 1998, Annu. Rev. Plant Physiol. Plant Mol. Biol. 49:611-641; Voelker, 1996, Genetic Engeneering, Hrsgb.: JK Setlow, 18:111-13; Gerhardt, 1992, Prog. Lipid R. 31:397-417; Gühnemann-Schäfer & Kindl, 1995, Biochim. Biophys Acta 1256:181-186; Kunau et al., 1995, Prog. Lipid Res. 34:267-342; Stymne et al., 1993, in: Biochemistry and Molecular Biology of Membrane and Storage Lipids of Plants, Hrsgb.: Murata und Somerville, Rockville, American Society of Plant Physiologists, 150-158, Murphy & Ross 1998, Plant Journal. 13 (1) :1-16.

Vitamine, Cofaktoren und Nutraceutical wie PUFAs, umfassen eine Gruppe von Molekülen, die höhere Tiere nicht mehr synthetisieren können und somit aufnehmen müssen oder die höhere Tiere nicht mehr ausreichend selbst herstellen können und somit zusätzlich aufnehmen müssen, obwohl sie leicht von anderen Organismen, wie Bakterien, synthetisiert werden. Die Biosynthese dieser Moleküle in Organismen, die sie produzieren können, wie in Bakterien, ist im großen und ganzen charakterisiert worden (Ullmann's Encyclopedia of Industrial Chemistry, "Vitamins", Bd. A27, S. 443-613, VCH: Weinheim, 1996; Michal, G. (1999) Biochemical Pathways: An Atlas of Biochemistry and Molecular Biology, John Wiley & Sons; Ong, A.S., Niki, E., & Packer, L. (1995) "Nutrition, Lipids, Health and Disease" Proceedings of the UNESCO/Confederation of Scientific and Technological Associations in Malaysia and the Society for Free Radical Research Asia, abgehalten am 1.-3. Sept. 1994 in Penang, Malaysia, AOCS Press, Champaign, IL X, 374 S).

Die oben erwähnten Moleküle sind entweder selbst biologisch aktive Moleküle oder Vorstufen biologisch aktiver Substanzen, die entweder als Elektronenüberträger oder Zwischenprodukte bei einer Vielzahl von Stoffwechselwegen dienen. Diese Verbindungen haben neben ihrem Nährwert auch einen signifikanten industriellen Wert als Farbstoffe, Antioxidantien und Katalysatoren oder andere Verarbeitungshilfsstoffe. (Einen Überblick über Struktur, Aktivität und industrielle Anwendungen dieser Verbindungen siehe z.B. in Ullmann's Encyclopedia of Industrial Chemistry, "Vitamins", Bd. A27, S. 443-613, VCH: Weinheim, 1996). Mehrfach ungesättigte Fettsäuren haben verschiedene Funktionen und gesundheitsfördernde Wirkungen, beispielsweise bei koronarer Herzerkrankung, Entzündungsmechanismen, Kinderernährung usw. Veröffentlichungen und Literaturstellen, einschließlich darin zitierter Literaturstellen, siehe in: Simopoulos, 1999, Am. J. Clin. Nutr. 70 (3. Suppl.):560-569, Takahata et al., Biosc. Biotechnol. Biochem. 1998, 62(11):2079-2085, Willich und Winther, 1995, Deutsche Medizinische Wochenschrift 120(7):229ff.

### II. Elemente und Verfahren der Erfindung

Die vorliegende Erfindung beruht unter anderem auf der Entdeckung neuer Moleküle, die hier als Desaturasenukleinsäure- und -proteinmoleküle bezeichnet werden, welche eine Wirkung auf die Produktion von Zellmembranen und Lipiden Phaeodactylum tricornutum ausüben und beispielsweise die Bewegung von Molekülen über diese Membranen beeinflussen. Bei einer Ausführungsform nehmen die Desaturasemoleküle am Stoffwechsel von zum Aufbau von Zellmembranen in Organismen, wie Mikroorganismen und Pflanzen, notwendigen Verbindungen teil oder beeinflussen indirekt den Transport von Molekülen über diese Membranen. Bei einer bevorzugten Ausführungsform hat die Aktivität der erfindungsgemäßen Desaturasemoleküle zur Regulation der Produktion von Membrankomponenten und des Membrantransports eine Auswirkung auf die Produktion der gewünschten Feinchemikalie durch diesen Organismus. Bei einer besonders bevorzugten Ausführungsform ist die Aktivität der erfindungsgemäßen Desaturasemoleküle moduliert, so dass die Ausbeute, Produktion und/oder Effizienz der Produktion der Stoffwechselwege von Mikroorganismen oder Pflanzen, welche die erfindungsgemäßen Desaturasen regulieren, moduliert sind und die Effizienz des Transport von Verbindungen durch die Membranen verändert ist, was entweder direkt oder indirekt die Ausbeute, Produktion und/oder Effizienz der Produktion einer gewünschten Feinchemikalie durch Mikroorganismen und Pflanzen moduliert.

Der Begriff "Desaturase" oder "Desaturasepolypeptid" umfasst Proteine, die an der Desaturierung von Fettsäuren teilnehmen. Beispiele für Desaturasen sind in der SEQ ID NO: 1, 3, 5, 11 oder ihren Homologen, Derivaten oder Analoga offenbart. Die Begriffe Desaturase oder Desaturasenukleinsäuresequenz(en) umfassen Nukleinsäuresequenzen, die eine Desaturase kodieren und bei denen ein Teil eine kodierende Region und ebenfalls entsprechende 5'- und 3'-untranslatierte Sequenzbereiche sein können. Beispiele für Desaturase-Gene sind die in SEQ ID NO: 1, 3, 5 oder 11 dargestellten. Die Begriffe Produktion oder Produktivität sind im Fachgebiet bekannt und beinhalten die Konzentration des Fermentationsproduktes (zum Beispiel der gewünschten Feinchemikalie), das in einer bestimmten Zeitspanne und einem bestimmten Fermentationsvolumen gebildet wird (z.B. kg Produkt pro Stunde pro Liter). Der Begriff Effizienz der Produktion umfasst die Zeit, die zur Erzielung einer bestimmten Produktionsmenge nötig ist (z.B. wie lange die Zelle zur Aufrichtung einer bestimmten Durchsatzrate einer Feinchemikalie benötigt). Der Begriff Ausbeute oder Produkt/Kohlenstoff-Ausbeute ist im Fachgebiet bekannt und umfasst die Effizienz der Umwandlung der Kohlenstoffquelle in das Produkt (d.h. die Feinchemikalie). Dies wird gewöhnlich beispielsweise ausgedrückt als kg Produkt pro kg Kohlenstoffquelle. Durch Erhöhen der Ausbeute oder Produktion der Verbindung wird die Menge der gewonnenen Moleküle oder der geeigneten gewonnenen Moleküle dieser Verbindung in einer bestimmten Kulturmenge über einen festgelegten Zeitraum erhöht. Die Begriffe Biosynthese oder Biosyntheseweg sind im Fachgebiet bekannt und umfassen die Synthese einer Verbindung, vorzugsweise einer organischen Verbindung, durch eine Zelle aus Zwischenverbindungen, beispielsweise in einem Mehrschritt- und stark regulierten Prozess. Die Begriffe Abbau oder Abbauweg sind im Fachgebiet bekannt und umfassen die Spaltung einer Verbindung, vorzugsweise einer organischen Verbindung, durch eine Zelle in Abbauprodukte (allgemeiner gesagt, kleinere oder weniger komplexe Moleküle) beispielsweise in einem Mehrschritt- und stark regulierten Prozess. Der Begriff Stoffwechsel ist im Fachgebiet bekannt und umfasst die Gesamtheit der biochemischen Reaktionen, die in einem Organismus stattfinden. Der Stoffwechsel einer bestimmten Verbindung (z.B. der Stoffwechsel einer Fettsäure) umfasst dann die Gesamtheit der Biosynthese-, Modifikations- und Abbauwege dieser Verbindung in der Zelle, die diese Verbindung betreffen.

Bei einer anderen Ausführungsform können die erfindungsgemäßen Nukleinsäuresequenzen, die für Desaturase-Moleküle codieren, die Produktion eines gewünschten Moleküls, wie einer Feinchemikalie, in einem Mikroorganismus oder in Pflanzen modulieren. Es gibt eine Reihe von Mechanismen, durch die die Veränderung einer erfindungsgemäßen Sequenz die Ausbeute, Produktion und/oder Effizienz der Produktion einer Feinchemikalie aus einem Mikroorganismus- oder Pflanzenstamm, die dieses veränderte Protein enthalten, direkt beeinflussen kann. Die Anzahl oder Aktivität von Desaturasen, die am Transport von Feinchemikalienmolekülen innerhalb oder aus der Zelle beteiligt sind, kann erhöht werden, so dass größere Mengen dieser Verbindungen über Membranen transportiert werden, aus denen sie leichter gewonnen und ineinander umgewandelt werden. Ferner sind Fettsäuren, Triacylglycerine und/oder Lipide selbst wünschenswerte Feinchemikalien; durch Optimierung der Aktivität oder Steigern der Anzahl einer oder mehrerer erfindungsgemäßer Desaturasen, die an der Biosynthese dieser Verbindungen beteiligt sind, oder durch Stören der Aktivität einer oder mehrerer Desaturasen, die am Abbau dieser Verbindungen beteiligt sind, kann es möglich sein, die Ausbeute, Produktion und/oder Effizienz der Produktion von Fettsäure- und Lipidmolekülen aus Organismen, wie Mikroorganismen oder Pflanzen, zu erhöhen.

Die Mutagenese des erfindungsgemäßen Nukleinsäuresequenzen kann Desaturasen mit veränderten Aktivitäten hervorbringen, welche die Produktion einer oder mehrerer gewünschter Feinchemikalien aus Mikroorganismen oder Pflanzen indirekt beeinflussen. Beispielsweise können erfindungsgemäße Desaturasen, die am Export von Abfallprodukten beteiligt sind, eine größere Anzahl oder höhere Aktivität aufweisen, so dass die normalen Stoffwechselabfälle der Zelle (deren Menge möglicherweise aufgrund der Überproduktion der gewünschten Feinchemikalie erhöht ist) effizient exportiert werden, bevor sie die Moleküle in der Zelle schädigen können (was die Lebensfähigkeit der Zelle herabsetzen würde) oder die Feinchemikalien-Biosynthesewege stören können (was die Ausbeute, Produktion oder Effizienz der Produktion einer gewünschten Feinchemikalie senken würde). Die relativ großen intrazellulären Mengen der gewünschten Feinchemikalie selbst können ferner für die Zelle toxisch sein, so dass man durch Steigern der Aktivität oder Anzahl von Transportern, die diese Verbindungen aus der Zelle exportieren können, die Lebensfähigkeit der Zelle in Kultur steigern kann, was wiederum zu einer größeren Anzahl an Zellen in der Kultur führt, welche die gewünschte Feinchemikalie produzieren. Die erfindungsgemäßen Desaturasen können auch so manipuliert werden, dass die entsprechenden Mengen unterschiedlicher Lipid- und Fettsäuremoleküle produziert werden. Dies kann eine erhebliche Auswirkung auf die Lipidzusammensetzung der Zellmembran haben. Da jeder Lipidtyp unterschiedliche physikalische Eigenschaften aufweist, kann eine Veränderung der Lipidzusammensetzung einer Membran die Membranfluidität signifikant verändern. Änderungen der Membranfluidität können den Transport von Molekülen über die Membran sowie die Integrität der Zelle beeinflussen, was jeweils eine erhebliche Auswirkung auf die Produktion von Feinchemikalien aus Mikroorganismen und Pflanzen in Fermentationskultur im großen Maßstab hat. Pflanzenmembranen verleihen spezifische Eigenschaften, wie Toleranz gegenüber Wärme, Kälte, Salz, Trockenheit sowie Toleranz gegen Pathogene, wie Bakterien und Pilze. Daher kann die Modulation der Membrankomponenten eine grundlegende Wirkung auf die Überlebensfähigkeit der Pflanzen unter den oben genannten Stressparametern haben. Dies kann über Änderungen in Signalkaskaden oder direkt über die veränderte Membranzusammensetzung erfolgen (siehe zum Beispiel: Chapman, 1998, Trends in Plant Science, 3(11):419-426) und Signalkaskaden (siehe Wang 1999, Plant Physiology, 120:645-651) oder die Kältetoleranz, wie offenbart in WO 95/18222, beeinflussen.

Die erfindungsgemäßen isolierten Nukleinsäuresequenzen sind im Genom eines Phaeodactylum tricornutum UTEX646-Stammes enthalten, der über die Algensammlung der University of Texas, Austin verfügbar ist.

Die Nukleotidsequenz der Phaeodactylum tricornutum-cDNA und die abgeleiteten Aminosäuresequenzen der Desaturasen sind in den SEQ ID NO: 1 bis 6 sowie 11 und 12 gezeigt. Es wurden Computeranalysen durchgeführt, die diese Nukleotidsequenzen als Sequenzen klassifizieren und/oder identifizieren, die am Stoffwechsel von Zellmembrankomponenten beteiligte Proteine oder am Transport von Verbindungen über Zellmembranen beteiligte Proteine bzw. der PUFA Biosynthese codieren. EST's mit der Datenbankeingabe-NO: PT001070010R und PT001078032R durch die Erfinder stellen die erfindungsgemäßen Sequenzen in SEQ ID NO: 1 und 3 dar. Die Sequenz des Fragments aus EST PT001070010R wurde ermittelt und ist wie dargestellt in SEQ ID NO: 5. Analog ist die Sequenz des Klones PT001078032R dargestellt in SEQ ID NO: 1. Den Klonen wurden Gennamen zugewiesen. Abkürzungen bedeuten: Pp = Physcomitrella patens, Pt = Phaeodactylum tricornutum. PT001070010R aus SEQ ID NO: 5 codiert für ein neues Gen homolog zu Δ-12-Desaturase und PT001078032R codiert für eine neuartige Δ-5-Desaturase. Pt_des6 kann gemäß Beispiel 5a mittels Polymerase Kettenreaktion unter Zuhilfenahme degenerierter Oligonukleotide isoliert werden. Ein so erhaltenes Fragment kann zum Sichten einer cDNA Bank aus Phaeodactylum tricornutum isoliert werden und die codierende Region einer Phaeodactylum tricornutum Δ-6-Desaturase erhalten werden. Ein so isoliertes Gen wird in Tabelle 1 als Pt_des6 bezeichnet und ist in SEQ ID NO: 3 dargestellt. Die korrespondierenden Aminosäuresequenzen werden durch Übersetzung des genetischen Codes der Sequenz ID NO: 1, 3 und 5 erhalten und sind als SEQ ID NO: 2, 4 und 6 definiert (siehe auch Tabelle 1). Auch eine weitere Nukleinsäuresequenz, die für eine Δ-12-Desaturase codiert, ist Tabelle 1 zu entnehmen. Sie trägt die Klon-Nummer PT001072031R.

**Tabelle 1**

| | Genname | Klonname | Nukleinsäure SEQ ID NO: | Polypeptid SEQ ID NO: |
|---|---|---|---|---|
| D5 Desaturase | Pt_des5 | PT001078032R | 1 | 2 |
| D6 Desaturase | Pt_des6 | Pt_des6 | 3 | 4 |
| D12 Desaturase | Pt_des12 | PT001070010R | 5 | 6 |
| D6 Desaturase | Pp_des6 | Pp_des6 | 7 | 8 |
| D6 Elongase | Pp_PSE1 | PP001019019F | 9 | 10 |
| Δ12 Desaturase | Pt des12.2 | PT001072013R | 11 | 12 |

Die vorliegende Erfindung betrifft auch Proteine mit einer Aminosäuresequenz, die im wesentlichen homolog zu einer Aminosäuresequenz der SEQ ID NO:2, 4, 6 oder 12 ist. Wie hier verwendet, ist ein Protein mit einer Aminosäuresequenz, die im wesentlichen homolog zu einer ausgewählten Aminosäuresequenz ist, zu mindestens etwa 50 % homolog zu der ausgewählten Aminosäuresequenz, z.B. der gesamten ausgewählten Aminosäuresequenz. Ein Protein mit einer Aminosäuresequenz, die im wesentlichen homolog zu einer ausgewählten Aminosäuresequenz ist, kann auch zu mindestens etwa 50 bis 60 %, vorzugsweise mindestens etwa 60 bis 70 % und stärker bevorzugt mindestens etwa 70 bis 80 %, 80 bis 90 % oder 90 bis 95 % und am stärksten bevorzugt mindestens etwa 96 %, 97 %, 98 %, 99 % oder mehr homolog zu einer ausgewählten Aminosäuresequenz sein.

Die erfindungsgemäße Desaturase oder der biologisch aktive Teil oder das Fragment davon kann am Stoffwechsel von Lipiden zum Aufbau von Zellmembranen oder Speicherlipiden in Mikroorganismen teilnehmen und in Kombination mit weiteren Genen, insbesondere solchen mit Elongaseaktivität zur Elongation von C₁₈-bzw C₂₀₋₂₂-PUFAs benötigten Aktivitäten beitragen, so dass C₁₈, C₂₀-, C₂₂- oder C₂₄-PUFAs sowie verwandte PUFAs erhalten werden. Dabei können erfindungsgemäße Desaturasen in Kombination mit Elongasen und anderen Desaturasen in erfindungsgemäßen Expressionskassetten kloniert werden und zur Transformation von Pflanzen mithilfe von Agrobakterium eingesetzt werden.

Verschiedene Aspekte der Erfindung sind eingehender in den folgenden Unterabschnitten beschrieben.

### A. Isolierte Nukleinsäuremoleküle

Eine Ausführungsform der Erfindung sind isolierte Nukleinsäuren, die von PUFA produzierenden Mikroorganismen stammen und für Polypeptide kodieren, die C₁₈-oder C₂₀₋₂₂-Fettsäuren mit mindestens einer, zwei, drei oder vier Doppelbindungen in der Fettsäure desaturieren.

Eine weitere erfindungsgemäße Ausführungsform sind isolierte Nukleinsäuren, umfassend Nukleotidsequenzen, die für Polypeptide kodieren, die C₁₈-bzw C₂₀-Fettsäuren mit mindestens ein, zwei, drei oder vier Doppelbindungen in der Fettsäure desaturieren und sind aus der Gruppe, bestehend aus
a) einer Nukleinsäuresequenz mit der in SEQ ID NO: 1, SEQ ID NO: 3, SEQ ID NO: 5 oder SEQ ID NO: 11 dargestellten Sequenz,
b) Nukleinsäuresequenzen, die aufgrund des degenerierten genetischen Codes durch Rückübersetzung der in SEQ ID NO: 2, SEQ ID NO: 4, SEQ ID NO: 6 oder SEQ ID NO: 12 dargestellten Aminosäuresequenzen erhalten werden,
c) Derivate der in SEQ ID NO: 1, SEQ ID NO: 3, SEQ ID NO: 5 oder SEQ ID NO: 11 dargestellten Nukleinsäuresequenz, die für Polypeptide mit der in SEQ ID NO: 2, SEQ ID NO: 4, SEQ ID NO: 6 oder SEQ ID NO: 12 dargestellten Aminosäuresequenzen codieren und mindestens 50 % Homologie auf Aminosäureebene aufweisen, ohne dass die enzymatische Wirkung der Polypeptide wesentlich reduziert ist.

Die oben genannte erfindungsgemäße Nukleinsäure stammt von Organismen, wie Ciliaten, Pilzen, Algen oder Dinoflagellaten, die PUFAs synthetisieren können, vorzugsweise von Phaeodactylum tricornutum oder nah verwandten Organismen.

Ein Aspekt der Erfindung betrifft isolierte Nukleinsäuremoleküle, die Desaturase-Polypeptide oder biologisch aktive Teile davon kodieren, sowie Nukleinsäurefragmente, die zur Verwendung als Hybridisierungssonden oder Primer zur Identifizierung oder Amplifizierung einer Desaturase-kodierenden Nukleinsäure (z.B. Desaturase-DNA) ausreichen. Der Begriff "Nukleinsäuremolekül", wie hier verwendet, soll DNA-Moleküle (z.B. cDNA oder genomische DNA) und RNA-Moleküle (z.B. mRNA) sowie DNA- oder RNA-Analoga, die mittels Nukleotidanaloga erzeugt werden, umfassen. Dieser Begriff umfasst zudem die am 3'- und am 5'-Ende des kodierenden Genbereichs gelegene untranslatierte Sequenz: mindestens 500, bevorzugt 200, besonders bevorzugt 100 Nukleotide der Sequenz stromaufwärts des 5'-Endes des kodierenden Bereichs und mindestens 100, bevorzugt 50, besonders bevorzugt 20 Nukleotide der Sequenz stromabwärts des 3'-Endes des kodierenden Genbereichs. Das Nukleinsäuremolekül kann einzelsträngig oder doppelsträngig sein, ist aber vorzugsweise doppelsträngige DNA. Ein "isoliertes" Nukleinsäuremolekül wird von anderen Nukleinsäuremolekülen abgetrennt, die in der natürlichen Quelle der Nukleinsäure vorliegen. Eine "isolierte" Nukleinsäure hat vorzugsweise keine Sequenzen, welche die Nukleinsäure in der genomischen DNA des Organismus, aus dem die Nukleinsäure stammt, natürlicherweise flankieren (z.B. Sequenzen, die sich an den 5'- und 3'-Enden der Nukleinsäure befinden). Bei verschiedenen Ausführungsformen kann das isolierte Desaturase-Nukleinsäuremolekül zum Beispiel weniger als etwa 5 kb, 4 kb, 3 kb, 2 kb, 1 kb, 0,5 kb oder 0,1 kb an Nukleotidsequenzen enthalten, die natürlicherweise das Nukleinsäuremolekül in der genomischen DNA der Zelle, aus der die Nukleinsäure stammt (z.B. eine Physcomitrella patens-Zelle) flankieren. Ein "isoliertes" Nukleinsäuremolekül, wie ein cDNA-Molekül, kann überdies im wesentlichen frei von anderem zellulären Material oder Kulturmedium sein, wenn es durch rekombinante Techniken hergestellt wird, oder frei von chemischen Vorstufen oder anderen Chemikalien sein, wenn es chemisch synthetisiert wird.

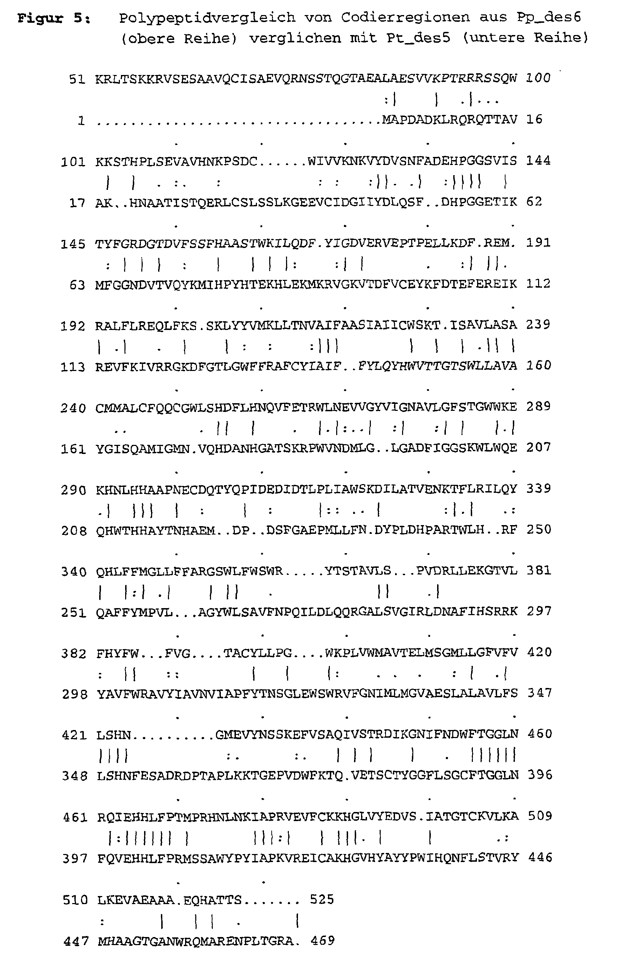

Ein erfindungsgemäßes Nukleinsäuremolekül, z.B. ein Nukleinsäuremolekül mit einer Nukleotidsequenz der SEQ ID NO:1 oder eines Teils davon, kann unter Verwendung molekularbiologischer Standardtechniken und der hier bereitgestellten Sequenzinformation isoliert werden. Auch kann mithilfe von Vergleichsalgorithmen beispielsweise eine homologe Sequenz oder homologe, konservierte Sequenzbereiche auf DNA oder Aminosäureebene identifiziert werden. Beispielsweise kann aus einer Phaeodactylum tricornutum cDNA aus einer Phaeodactylum tricornutum-Bank isoliert werden, indem die vollständige SEQ ID NO:1, 3, 5 oder 11 oder ein Teil davon als Hybridisierungssonde sowie Standard-Hybridisierungstechniken (wie z.B. beschrieben in Sambrook et al., Molecular Cloning: A Laboratory Manual. 2. Aufl., Cold Spring Harbor Laboratory, Cold Spring Harbor Laboratory Press, Cold Spring Harbor, NY, 1989) verwendet werden. Überdies lässt sich ein Nukleinsäuremolekül, umfassend eine vollständige Sequenz der SEQ ID NO: 1, 3, 5 oder 11 oder einen Teil davon, durch Polymerasekettenreaktion isolieren, wobei Oligonukleotidprimer, die auf der Basis dieser Sequenz oder von Teilen davon, insbesondere Regionen um Motive aus Beispiel 5a erstellt werden oder Modifikationen ebensolcher in einzelnen definierten Aminosäuren, verwendet werden (z.B. kann ein Nukleinsäuremolekül, umfassend die vollständigen Sequenz der SEQ ID NO:1, 3, 5 oder 11 oder einen Teil davon, durch Polymerasekettenreaktion unter Verwendung von Oligonukleotidprimern isoliert werden, die auf der Basis dieser gleichen Sequenz der SEQ ID NO: 1, 3, 5 oder 11 erstellt worden sind). Zum Beispiel lässt sich mRNA aus Zellen isolieren (z.B. durch das Guanidiniumthiocyanat-Extraktionsverfahren von Chirgwin et al. (1979) Biochemistry 18:5294-5299) und cDNA mittels Reverser Transkriptase (z.B. Moloney-MLV-Reverse-Transkriptase, erhältlich von Gibco/BRL, Bethesda, MD, oder AMV-Reverse-Transkriptase, erhältlich von Seikagaku America, Inc., St.Petersburg, FL) herstellen. Synthetische Oligonukleotidprimer zur Amplifizierung mittels Polymerasekettenreaktion lassen sich auf der Basis einer der in SEQ ID NO: 1, 3, 5 oder 11 sowie der in Figur 5a gezeigten Sequenzen oder mithilfe der in SEQ ID NO: 2, 4, 6 oder 12 dargestellten Aminosäuresequenzen erstellen. Eine erfindungsgemäße Nukleinsäure kann unter Verwendung von cDNA oder alternativ von genomischer DNA als Matrize und geeigneten Oligonukleotidprimern gemäß Standard-PCR-Amplifikationstechniken amplifiziert werden. Die so amplifizierte Nukleinsäure kann in einen geeigneten Vektor kloniert werden und mittels DNA-Sequenzanalyse charakterisiert werden. Oligonukleotide, die einer Desaturase-Nukleotidsequenz entsprechen, können durch Standard-Syntheseverfahren, beispielsweise mit einem automatischen DNA-Synthesegerät, hergestellt werden.

Die in SEQ ID NO: 1,3, 5 oder 11 gezeigte cDNA umfasst Sequenzen, die Desaturasen kodieren, (d.h. den "kodierenden Bereich") sowie 5'-untranslatierte Sequenzen und 3'-untranslatierte Sequenzen. Alternativ kann das Nukleinsäuremolekül nur den kodierenden Bereich einer der Sequenzen in SEQ ID NO: 1, 3, 5 oder 11 umfassen oder kann ganze genomische Fragmente, die aus genomischer DNA isoliert sind, enthalten.

Bei einer weiteren bevorzugten Ausführungsform umfasst ein erfindungsgemäßes isoliertes Nukleinsäuremolekül ein Nukleinsäuremolekül, das ein Komplement einer der in SEQ ID NO: 1, 3, 5 oder 11 gezeigten Nukleotidsequenzen oder eines Teils davon ist. Ein Nukleinsäuremolekül, das zu einer der in SEQ ID NO: 1, 3, 5 oder 11 gezeigten Nukleotidsequenzen komplementär ist, ist dann ausreichend komplementär, wenn es mit einer der in SEQ ID NO: 1, 3, 5 oder 11 angegebenen Sequenzen hybridisieren kann, wodurch ein stabiler Duplex entsteht.'

Homologe der neuen Desaturase-Nukleinsäuresequenzen mit der Sequenz SEQ ID NO: 1, 3, 5 oder 11 bedeutet beispielsweise allelische Varianten mit mindestens etwa 50 bis 60 %, vorzugsweise mindestens etwa 60 bis 70 %, stärker bevorzugt mindestens etwa 70 bis 80 %, 80 bis 90 % oder 90 bis 95 % und noch stärker bevorzugt mindestens etwa 95 %, 96 %, 97 %, 98 %, 99 % oder mehr Homologie zu einer in SEQ ID NO: 1, 3, 5 oder 11 gezeigten Nukleotidsequenzen oder ihren Homologen, Derivaten oder Analoga oder Teilen davon. Bei einer weiteren bevorzugten Ausführungsform umfasst ein isoliertes erfindungsgemäßes Nukleinsäuremolekül eine Nukleotidsequenz, die an eine der in SEQ ID NO: 1, 3, 5 oder 11 gezeigten Nukleotidsequenzen oder einen Teil davon hybridisiert, z.B. unter stringenten Bedingungen hybridisiert. Allelische Varianten umfassen insbesondere funktionelle Varianten, die sich durch Deletion, Insertion oder Substitution von Nukleotiden aus/in der in SEQ ID NO: 1, 3, 5 oder 11 dargestellten Sequenz erhalten lassen, wobei aber die Absicht ist, dass die Enzymaktivität der davon herrührenden synthetisierten Proteine für die Insertion eines oder mehrerer Gene vorteilhafterweise beibehalten wird. Proteine, die noch die enzymatische Aktivität der Desaturase besitzen, das heißt deren Aktivität im wesentlichen nicht reduziert ist, bedeutet Proteine mit mindestens 10 %, vorzugsweise 20 %, besonders bevorzugt 30 %, ganz besonders bevorzugt 40 % der ursprünglichen Enzymaktivität, verglichen mit dem durch SEQ ID NO: 2, 4, 6 oder 12 kodierten Protein.

Homologen der SEQ ID NO: 1, 3, 5 oder 11 bedeuten beispielsweise auch bakterielle, Pilz- und Pflanzenhomologen, verkürzte Sequenzen, einzelsträngige DNA oder RNA der kodierenden und nicht-kodierenden DNA-Sequenz.

Homologen der SEQ ID NO: 1, 3, 5 oder 11 bedeutet auch Derivate, wie beispielsweise Promotorvarianten. Die Promotoren stromaufwärts der angegebenen Nukleotidsequenzen können durch einen oder mehrere Nukleotidaustausche, durch Insertion(en) und/oder Deletion(en) modifiziert werden, ohne dass jedoch die Funktionalität oder Aktivität der Promotoren gestört wird. Es ist weiterhin möglich, dass die Aktivität der Promotoren durch Modifikation ihrer Sequenz erhöht ist oder dass sie vollständig durch aktivere Promotoren, sogar aus heterologen Organismen, ersetzt werden.

Überdies kann das erfindungsgemäße Nukleinsäuremolekül nur einen Teil des kodierenden Bereichs einer der Sequenzen in SEQ ID NO: 1, 3, 5 oder 11 umfassen, zum Beispiel ein Fragment, das als Sonde oder Primer verwendet werden kann, oder ein Fragment, welches einen biologisch aktiven Abschnitt einer Desaturase kodiert. Die aus der Klonierung des Desaturase-Gens von Phaeodactylum tricornutum ermittelten Nukleotidsequenzen ermöglichen die Erzeugung von Sonden und Primern, die zur Identifizierung und/oder Klonierung von Desaturase-Homologen in anderen Zelltypen und Organismen sowie Desaturase-Homologen aus anderen Mikroalgen oder verwandten Arten gestaltet sind. Die Sonde/der Primer umfasst gewöhnlich im wesentlichen gereinigtes Oligonukleotid. Das Oligonukleotid umfasst gewöhnlich einen Nukleotidsequenzbereich, der unter stringenten Bedingungen an mindestens etwa 12, vorzugsweise etwa 16, stärker bevorzugt etwa 25, 40, 50 oder 75 aufeinanderfolgende Nukleotide eines Sense-Stranges einer der in SEQ ID NO: 1, 3, 5 oder 11 angegebenen Sequenzen, eines Antisense-Stranges einer der in SEQ ID NO: 1, 3, 5 oder 11 angegebenen Sequenzen oder seiner Homologen, Derivate oder Analoga oder natürlich vorkommender Mutanten davon hybridisiert. Primer auf der Basis einer Nukleotidsequenz der SEQ ID NO: 1, 3, 5 oder 11 können in PCR-Reaktionen zur Klonierung von Desaturase-Homologen verwendet werden. Sonden auf der Basis der Desaturase-Nukleotidsequenzen können zum Nachweis von Transkripten oder genomischen Sequenzen, die das gleiche oder homologe Proteine kodieren, verwendet werden. Bei bevorzugten Ausführungsformen umfasst die Sonde zudem eine daran gebundene Markierungsgruppe, z.B. ein Radioisotop, eine fluoreszierende Verbindung, ein Enzym oder einen Enzym-Cofaktor. Diese Sonden können als Teil eines Test-Kits für genomische Marker zur Identifizierung von Zellen, die eine Desaturase misexprimieren, beispielsweise durch Messen einer Menge einer Desaturase-kodierenden Nukleinsäure in einer Zellenprobe, z.B. Messen der Desaturase-mRNA-Spiegel, oder zur Bestimmung, ob ein genomisches Desaturase-Gen mutiert oder deletiert ist, verwendet werden.

Bei einer Ausführungsform kodiert das erfindungsgemäße Nukleinsäuremolekül ein Protein oder einen Teil davon, das/der eine Aminosäuresequenz umfasst, die ausreichend homolog zu einer Aminosäuresequenz der SEQ ID NO: 2, 4, 6 oder 12 ist, dass das Protein oder der Teil davon die Fähigkeit, am Stoffwechsel von zum Aufbau von Zellmembranen in Mikroorganismen oder Pflanzen notwendigen Verbindungen oder am Transport von Molekülen über diese Membranen teilzunehmen, beibehält. Wie hier verwendet, betrifft der Begriff "ausreichend homolog" Proteine oder Teile davon, deren Aminosäuresequenzen eine minimale Anzahl identischer oder äquivalenter Aminosäurereste (z.B. einen Aminosäurerest mit einer ähnlichen Seitenkette, wie ein Aminosäurerest in einer der Sequenzen der SEQ ID NO:2) zu einer Aminosäuresequenz der SEQ ID NO: 2 aufweisen, so dass das Protein oder der Teil davon am Stoffwechsel von zum Aufbau von Zellmembranen in Mikroorganismen oder Pflanzen notwendigen Verbindungen oder am Transport von Molekülen über diese Membranen teilnehmen kann. Proteinbestandteile dieser Stoffwechselwege für Membrankomponenten oder Membrantransportsysteme können, wie hier beschrieben, eine Rolle bei der Produktion und Sekretion einer oder mehrerer Feinchemikalien spielen. Beispiele für diese Aktivitäten sind hier ebenfalls beschrieben. Somit trägt die "Funktion einer Desaturase" entweder direkt oder indirekt zur Ausbeute, Produktion und/oder Effizienz der Produktion einer oder mehrerer Feinchemikalien bei. Beispiele für Desaturase-Substratspezifitäten der katalytischen Aktivität sind in Tabelle 5 und 6 angegeben.

Bei einer weiteren Ausführungsform kodieren Derivate des erfindungsgemäßen Nukleinsäuremoleküls Proteine mit mindestens etwa 50 bis 60 %, vorzugsweise mindestens etwa 60 bis 70 % und stärker bevorzugt mindestens etwa 70 bis 80 %, 80 bis 90 %, 90 bis 95 % und am stärksten bevorzugt mindestens etwa 96 %, 97 %, 98 %, 99 % oder mehr Homologie zu einer vollständigen Aminosäuresequenz der SEQ ID NO:2. Die Homologie der Aminosäuresequenz kann über den gesamten Sequenzbereich mit dem Programm PileUp (J. Mol. Evolution., 25, 351-360, 1987, Higgins et al., CABIOS, 5, 1989:151-153) oder BESTFIT oder GAP bestimmt (Henikoff, S. and Henikoff, J. G. (1992). Amino acid substitution matrices from protein blocks. Proc. Natl. Acad. Sci. USA 89: 10915-10919.)

Teile von Proteinen, die von den erfindungsgemäßen Desaturase-Nukleinsäuremolekülen kodiert werden, sind vorzugsweise biologisch aktive Teile einer der Desaturasen. Wie hier verwendet, soll der Begriff "biologisch aktiver Teil einer Desaturase", einen Abschnitt, z.B. eine Domäne/ein Motiv, einer Desaturase umfassen, der am Stoffwechsel von zum Aufbau von Zellmembranen in Mikroorganismen oder Pflanzen notwendigen Verbindungen oder am Transport von Molekülen über diese Membranen teilnehmen kann oder eine in Tabelle 5 und 6 angegebene Aktivität aufweist. Zur Bestimmung, ob eine Desaturase oder ein biologisch aktiver Teil davon am Stoffwechsel von zum Aufbau von Zellmembranen in Mikroorganismen oder Pflanzen notwendigen Verbindungen oder am Transport von Molekülen über diese Membranen teilnehmen kann, kann ein Test der enzymatischen Aktivität durchgeführt werden. Diese Testverfahren, wie eingehend in Beispiel 8 des Beispielteils beschrieben, sind dem Fachmann geläufig.

Zusätzliche Nukleinsäurefragmente, die biologisch aktive Abschnitte einer Desaturase kodieren, lassen sich durch Isolierung eines Teils einer der Sequenzen in SEQ ID NO: 1, 3, 5 oder 11, Exprimieren des kodierten Abschnitt der Desaturase oder des Peptids (z.B. durch rekombinante Expression in vitro) und Bestimmen der Aktivität des kodierten Teils der Desaturase oder des Peptids herstellen.

Die Erfindung umfasst zudem Nukleinsäuremoleküle, die sich von einer der in SEQ ID NO: 1, 3, 5 oder 11 gezeigten Nukleotidsequenzen (und Teilen davon) aufgrund des degenerierten genetischen Codes unterscheiden und somit die gleiche Desaturase kodieren wie diejenige, die von den in SEQ ID NO: 1, 3, 5 oder 11 gezeigten Nukleotidsequenzen kodiert wird. Bei einer anderen Ausführungsform hat ein erfindungsgemäßes isoliertes Nukleinsäuremolekül eine Nukleotidsequenz, die für ein Protein mit einer in SEQ ID NO: 2, 4, 6 oder 12 gezeigten Aminosäuresequenz kodiert. Bei einer weiteren Ausführungsform kodiert das erfindungsgemäße Nukleinsäuremolekül ein Vollängen-Desaturase-Protein, das zu einer Aminosäuresequenz der SEQ ID NO: 2,4, 6 oder 12 (die von einem in SEQ ID NO: 1, 3, 5 oder 11 gezeigten offenen Leseraster kodiert wird) im wesentlichen homolog ist und durch gängige Methoden identifizierbar und isolierbar ist.

Zusätzlich zu den in SEQ ID NO: 1, 3, 5 oder 11 gezeigten Desaturase-Nukleotidsequenzen erkennt der Fachmann, dass DNA-Sequenzpolymorphismen, die zu Änderungen in den Aminosäuresequenzen der Desaturasen führen, innerhalb einer Population (z.B. der Phaeodactylum tricornutum-Population) existieren können. Diese genetischen Polymorphismen im Desaturase-Gen können zwischen Individuen innerhalb einer Population aufgrund von natürlicher Variation existieren. Wie hier verwendet, bedeuten die Begriffe "Gen" und "rekombinantes Gen" Nukleinsäuremoleküle mit einem offenen Leserahmen, der eine Desaturase, vorzugsweise eine Phaeodactylum tricornutum -Desaturase, kodiert. Diese natürlichen Varianten bewirken üblicherweise eine Varianz von 1 bis 5 % in der Nukleotidsequenz des Desaturase-Gens. Sämtliche und alle dieser Nukleotidvariationen und daraus resultierende Aminosäurepolymorphismen in der Desaturase, die das Ergebnis natürlicher Variation sind und die funktionelle Aktivität von Desaturasen nicht verändern, sollen im Umfang der Erfindung enthalten sein.

Nukleinsäuremoleküle, die den natürlichen Varianten entsprechen, und nicht-Phaeodactylum tricornutum-Homologen, -Derivate und -Analoga der Phaeodactylum tricornutum-cDNA können auf der Grundlage ihrer Homologie zu der hier offenbarten Phaeodactylum tricornutum-Desaturase-Nukleinsäure unter Verwendung der Phaeodactylum tricornutum-cDNA oder eines Teils davon als Hybridisierungssonde gemäß Standard-Hybridisierungstechniken unter stringenten Hybridisierungsbedingungen isoliert werden. Bei einer anderen Ausführungsform ist ein erfindungsgemäßes isoliertes Nukleinsäuremolekül mindestens 15 Nukleotide lang und hybridisiert unter stringenten Bedingungen mit dem Nukleinsäuremolekül, das eine Nukleotidsequenz der SEQ ID NO:1, 3, 5 oder 11 umfasst. Bei anderen Ausführungsformen ist die Nukleinsäure mindestens 25, 50, 100, 250 oder mehr Nukleotide lang. Der Begriff "hybridisiert unter stringenten Bedingungen", wie hier verwendet, soll Hybridisierungs- und Waschbedingungen beschreiben, unter denen Nukleotidsequenzen, die mindestens 60 % homolog zueinander sind, gewöhnlich aneinander hybridisiert bleiben. Die Bedingungen sind vorzugsweise derart, dass Sequenzen, die mindestens etwa 65%, stärker bevorzugt mindestens etwa 70% und noch stärker bevorzugt mindestens etwa 75 % oder stärker zueinander homolog sind, gewöhnlich aneinander hybridisiert bleiben. Diese stringenten Bedingungen sind dem Fachmann bekannt und lassen sich in Current Protocols in Molecular Biology, John Wiley & Sons, N. Y. (1989), 6.3.1-6.3.6., finden. Ein bevorzugtes, nicht einschränkendes Beispiel für stringente Hybridisierungsbedingungen sind Hybridisierungen in 6 x Natriumchlorid/Natriumcitrat (sodium chloride/ sodiumcitrate = SSC) bei etwa 45°C, gefolgt von einem oder mehreren Waschschritten in 0,2 x SSC, 0,1 % SDS bei 50 bis 65°C. Dem Fachmann ist bekannt, dass diese Hybridisierungsbedingungen sich je nach dem Typ der Nukleinsäure und, wenn beispielsweise organische Lösungsmittel vorliegen, hinsichtlich der Temperatur und der Konzentration des Puffers unterscheiden. Die Temperatur unterscheidet sich beispielsweise unter "Standard-Hybridisierungsbedingungen" je nach dem Typ der Nukleinsäure zwischen 42°C und 58°C in wässrigem Puffer mit einer Konzentration von 0,1 bis 5 x SSC (pH 7,2). Falls organisches Lösungsmittel im obengenannten Puffer vorliegt, zum Beispiel 50 % Formamid, ist die Temperatur unter Standardbedingungen etwa 42°C. Vorzugsweise sind die Hybridisierungsbedingungen für DNA:DNA-Hybride zum Beispiel 0,1 x SSC und 20°C bis 45°C, vorzugsweise zwischen 30°C und 45°*C*. Vorzugsweise sind die Hybridisierungsbedingungen für DNA:RNA-Hybride zum Beispiel 0,1 x SSC und 30°C bis 55°C, vorzugsweise zwischen 45°C und 55°C. Die vorstehend genannten Hybridisierungstemperaturen sind beispielsweise für eine Nukleinsäure mit etwa 100 bp (= Basenpaare) Länge und einem G + C-Gehalt von 50 % in Abwesenheit von Formamid bestimmt. Der Fachmann weiß, wie die erforderlichen Hybridisierungsbedingungen anhand von Lehrbüchern, wie dem vorstehend erwähnten oder aus den folgenden Lehrbüchern Sambrook et al., "Molecular Cloning", Cold Spring Harbor Laboratory, 1989; Hames und Higgins (Hrsgb.) 1985, "Nucleic Acids Hybridization: A Practical Approach", IRL Press at Oxford University Press, Oxford; Brown (Hrsgb.) 1991, "Essential Molecular Biology: A Practical Approach", IRL Press at Oxford University Press, Oxford, bestimmt werden können.

Vorzugsweise entspricht ein erfindungsgemäßes isoliertes Nukleinsäuremolekül, das unter stringenten Bedingungen an eine Sequenz der SEQ ID NO:1, 3, 5 oder 11 hybridisiert, einem natürlich vorkommenden Nukleinsäuremolekül. Wie hier verwendet, betrifft ein "natürlich vorkommendes" Nukleinsäuremolekül ein RNA- oder DNA-Molekül mit einer Nukleotidsequenz, die in der Natur vorkommt (z.B. ein natürliches Protein kodiert). Bei einer Ausführungsform kodiert die Nukleinsäure eine natürliche vorkommendes Phyaeodactylum tricornutum-Desaturase.

Zusätzlich zu natürlich vorkommenden Varianten der Desaturasesequenz, die in der Population existieren können, erkennt der Fachmann ferner, dass auch Änderungen durch Mutation in eine Nukleotidsequenz der SEQ ID NO: 1, 3, 5 oder 11 eingebracht werden können, was zu Änderungen der Aminosäuresequenz der kodierten Desaturase führt, ohne dass die Funktionsfähigkeit des Desaturaseproteins beeinträchtigt wird. Beispielsweise lassen sich Nukleotidsusbtitutionen, die an "nicht-essentiellen" Aminosäureresten zu Aminosäuresubstitutionen führen, in einer Sequenz der SEQ ID NO: 2, 4, 6 oder 12 herstellen. Ein "nicht-essentieller" Aminosäurerest ist ein Rest, der sich in einer Wildtyp-Desaturasequenz einer der Desaturasen (SEQ ID NO: 2, 4, 6 oder 12) verändern lässt, ohne dass die Aktivität der Desaturase verändert das heißt wesentlich reduziert wird, wohingegen ein "essentieller" Aminosäurerest für die Desaturaseaktivität erforderlich ist. Andere Aminosäurereste (z.B. diejenigen, die in der Domäne mit Desaturaseaktivität nicht konserviert oder lediglich semikonserviert sind) können jedoch für die Aktivität nicht essentiell sein und lassen sich somit verändern, ohne dass die Desaturaseaktivität verändert wird.

Folglich betrifft ein weiterer Aspekt der Erfindung Nukleinsäuremoleküle, die Desaturasen kodieren, die veränderte Aminosäurereste enthalten, die für die Desaturaseaktivität nicht essentiell sind. Diese Desaturasen unterscheiden sich in der Aminosäuresequenz von einer Sequenz in SEQ ID NO: 2, 4, 6 oder 12 und behalten dennoch zumindest eine der hier beschriebenen Desaturaseaktivitäten. Das isolierte Nukleinsäuremolekül umfasst bei einer Ausführungsform eine Nukleotidsequenz, die ein Protein kodiert, wobei das Protein eine Aminosäuresequenz mit mindestens etwa 50 % Homologie zu einer Aminosäuresequenz der SEQ ID NO: 2, 4, 6 oder 12 umfasst und am Stoffwechsel von zum Aufbau von Zellmembranen in Phaeodactylum tricornutum notwendigen Verbindungen oder am Transport von Molekülen über diese Membranen teilnehmen kann. Das von dem Nukleinsäuremolekül kodierte Protein ist vorzugsweise mindestens etwa 50 bis 60 % homolog zu einer der Sequenzen in SEQ ID NO:2, 4, 6 oder 12 stärker bevorzugt mindestens etwa 60 bis 70 % homolog zu einer der Sequenzen in SEQ ID NO:2, 4, 6 oder 12 noch stärker bevorzugt mindestens etwa 70 bis 80 %, 80 bis 90 %, 90 bis 95 % homolog zu einer der Sequenzen in SEQ ID NO: 2, 4, 6 oder 12 und am stärksten bevorzugt mindestens etwa 96 %, 97 %, 98 % oder 99 % homolog zu einer der Sequenzen in SEQ ID NO: 2, 4, 6 oder 12.

Zur Bestimmung der prozentualen Homologie von zwei Aminosäuresequenzen (z.B. einer der Sequenzen der SEQ ID NO: 2, 4, 6 oder 12 und einer mutierten Form davon) oder von zwei Nukleinsäuren werden die Sequenzen zum Zweck des optimalen Vergleichs untereinander geschrieben (z.B. können Lücken in die Sequenz eines Proteins oder einer Nukleinsäure eingefügt werden, um ein optimales Alignment mit dem anderen Protein oder der anderen Nukleinsäure zu erzeugen). Die Aminosäurereste oder Nukleotide an den entsprechenden Aminosäurepositionen oder Nukleotidpositionen werden dann verglichen. Wenn eine Position in einer Sequenz (z.B. einer der Sequenzen der SEQ ID NO: 2, 4, 6 oder 12) durch den gleichen Aminosäurerest oder das gleiche Nukleotid wie die entsprechende Stelle in der anderen Sequenz (z.B. einer mutierten Form der aus SEQ ID NO: 2, 4, 6 oder 12 ausgewählten Sequenz) belegt wird, dann sind die Moleküle an dieser Position homolog (d.h. Aminosäure- oder Nukleinsäure-"Homologie", wie hier verwendet, entspricht Aminosäure- oder Nukleinsäure-"Identität"). Die prozentuale Homologie zwischen den beiden Sequenzen ist eine Funktion der Anzahl an identischen Positionen, die den Sequenzen gemeinsam sind (d.h. % Homologie = Anzahl der identischen Positionen/Gesamtanzahl der Positionen x 100). Die Begriffe Homologie und Identität sind damit als Synonym anzusehen.

Ein isoliertes Nukleinsäuremolekül, das eine Desaturase kodiert, die zu einer Proteinsequenz der SEQ ID NO: 2, 4, 6 oder 12 homolog ist, kann durch Einbringen einer oder mehrerer Nukleotidsubstitutionen, -additionen oder -deletionen in eine Nukleotidsequenz der SEQ ID NO: 1, 3, 5 oder 11 erzeugt werden, so dass eine oder mehrere Aminosäuresubstitutionen, -additionen oder -deletionen in das kodierte Protein eingebracht werden. Mutationen können in eine der Sequenzen der SEQ ID NO: 1, 3, 5 oder 11 durch Standardtechniken, wie stellenspezifische Mutagenese und PCR-vermittelte Mutagenese, eingebracht werden. Vorzugsweise werden konservative Aminosäuresubstitutionen an einer oder mehreren der vorhergesagten nicht-essentiellen Aminosäureresten hergestellt. Bei einer "konservativen Aminosäuresubstitution" wird der Aminosäurerest gegen einen Aminosäurerest mit einer ähnlichen Seitenkette ausgetauscht. Im Fachgebiet sind Familien von Aminosäureresten mit ähnlichen Seitenketten definiert worden. Diese Familien umfassen Aminosäuren mit basischen Seitenketten (z.B. Lysin, Arginin, Histidin), sauren Seitenketten (z.B. Asparaginsäure, Glutaminsäure), ungeladenen polaren Seitenketten (z.B. Glycin, Asparagin, Glutamin, Serin, Threonin, Tyrosin, Cystein), unpolaren Seitenketten, (z.B. Alanin, Valin, Leucin, Isoleucin, Prolin, Phenylalanin, Methionin, Tryptophan), beta-verzweigten Seitenketten (z.B. Threonin, Valin, Isoleucin) und aromatischen Seitenketten (z.B. Tyrosin, Phenylalanin, Tryptophan, Histidin). Ein vorhergesagter nicht-essentieller Aminosäurerest in einer Desaturase wird somit vorzugsweise durch einen anderen Aminosäurerest aus der gleichen Seitenkettenfamilie ausgetauscht. Alternativ können bei einer anderen Ausführungsform die Mutationen zufallsgemäß über die gesamte oder einen Teil der Desaturase-kodierenden Sequenz eingebracht werden, z.B. durch Sättigungsmutagenese, und die resultierenden Mutanten können nach der hier beschriebenen Desaturase-Aktivität durchmustert werden, um Mutanten zu identifizieren, die Desaturaseaktivität beibehalten. Nach der Mutagenese einer der Sequenzen der SEQ ID NO: 1, 3, 5 oder 11 kann das kodierte Protein rekombinant exprimiert werden, und die Aktivität des Proteins kann z.B. unter Verwendung der hier beschriebenen Tests (siehe Beispielteil) bestimmt werden.

Zusätzlich zu den Nukleinsäuremolekülen, welche die vorstehend beschriebenen Desaturasen kodieren, betrifft ein weiterer Aspekt der Erfindung isolierte Nukleinsäuremoleküle, die "Antisense" zu den erfindungsgemäßen Nukleinsäuresequenzen sind. Eine "Antisense"-Nukleinsäure umfasst eine Nukleotidsequenz, die zu einer "Sense"-Nukleinsäure, welche ein Protein kodiert, komplementär ist, z.B. komplementär zum kodierenden Strang eines doppelsträngigen cDNA-Moleküls oder komplementär zu einer mRNA-Sequenz. Eine Antisense-Nukleinsäure kann folglich über Wasserstoffbrückenbindungen an eine Sense-Nukleinsäure binden. Die Antisense-Nukleinsäure kann zu einem gesamten Desaturase-kodierenden Strang oder nur zu einem Teil davon komplementär sein. Bei einer Ausführungsform ist ein Antisense-Nukleinsäuremolekül "Antisense" zu einem "kodierenden Bereich" des kodierenden Strangs einer Nukleotidsequenz, die eine Desaturase kodiert. Der Begriff "kodierender Bereich" betrifft den Bereich der Nukleotidsequenz, der Codons umfasst, die in Aminosäurereste translatiert werden (z.B. den gesamten kodierenden Bereich, der mit dem Stopcodon beginnt und endet, d.h. dem letzten Codon vor dem Stopcodon). Bei einer weiteren Ausführungsform ist das Antisense-Nukleinsäuremolekül "Antisense" zu einem "nicht-kodierenden Bereich" des kodierenden Strangs einer Nukleotidsequenz, die Desaturase kodiert. Der Begriff "nicht-kodierender Bereich" betrifft 5'-und 3'-Sequenzen, die den kodierenden Bereich flankieren und nicht in Aminosäuren translatiert werden (d.h. die man auch als 5'- und 3'-untranslatierte Bereiche bezeichnet).

Unter Voraussetzung der hier offenbarten Desaturase-kodierenden Sequenzen des kodierenden Stranges (z.B. die in SEQ ID NO: 1, 3, 5 oder 11 dargestellten Sequenzen) können erfindungsgemäße Antisense-Nukleinsäuren gemäß den Regeln der Watson-Crick-Basenpaarung gestaltet werden. Das Antisense-Nukleinsäuremolekül kann komplementär zum gesamten kodierenden Bereich von Desaturase-mRNA sein, ist aber stärker bevorzugt ein Oligonukleotid, das nur zu einem Teil des kodierenden oder nicht-kodierenden Bereichs von Desaturase-mRNA "Antisense" ist. Das Antisense-Oligonukleotid kann z.B. zu dem Bereich, der die Translationsstartstelle von Desaturase-mRNA umgibt, komplementär sein. Ein Antisense-Oligonukleotid kann z.B. etwa 5, 10, 15, 20, 25, 30, 35, 40, 45 oder 50 und mehr Nukleotide lang sein. Eine erfindungsgemäße Antisense-Nukleinsäure kann unter Verwendung chemischer Synthese und enzymatischer Ligationsreaktionen mittels im Fachgebiet bekannter Verfahren konstruiert werden. Eine Antisense-Nukleinsäure (z.B. ein Antisense-Oligonukleotid) kann z.B. chemisch synthetisiert werden, wobei natürlich vorkommende Nukleotide oder verschiedentlich modifizierte Nukleotide verwendet werden, die so gestaltet sind, dass sie die biologische Stabilität der Moleküle erhöhen oder die physikalische Stabilität des zwischen der Antisense- und der Sense-Nukleinsäure gebildeten Duplexes erhöhen, beispielsweise können Phosphorthioat-Derivate und acridinsubstituierte Nukleotide verwendet werden. Beispiele für modifizierte Nukleotide, die zur Erzeugung der Antisense-Nukleinsäure verwendet werden können, sind u.a. 5-Fluoruracil, 5-Bromuracil, 5-Chloruracil, 5-Ioduracil, Hypoxanthin, Xanthin, 4-Acetylcytosin, 5-(Carboxyhydroxylmethyl)uracil, 5-Carboxymethylaminomethyl-2-thiouridin, 5-Carboxymethylaminomethyluracil, Dihydrouracil, Beta-D-Galactosylqueosin, Inosin, N6-Isopentenyladenin, 1-Methylguanin, 1-Methylinosin, 2,2-Dimethylguanin, 2-Methyladenin, 2-Methylguanin, 3-Methylcytosin, 5-Methylcytosin, N6-Adenin, 7-Methylguanin, 5-Methylaminomethyluracil, 5-Methoxyaminomethyl-2-thiouracil, Beta-D-Mannosylqueosin, 5'-Methoxycarboxymethyluracil, 5-Methoxyuracil, 2-Methylthio-N6-isopentyladenin, Uracil-5-oxyessigsäure (v), Wybutoxosin, Desaturaseudouracil, Queosin, 2-Thiocytosin, 5-Methyl-2-thiouracil, 2-Thiouracil, 4-Thiouracil, 5-Methyluracil, Uracil-5-oxyessigsäuremethylester, Uracil-5-oxyessigsäure (v), 5-Methyl-2-thiouracil, 3-(3-Amino-3-N-2-carboxypropyl)uracil, (acp3)w und 2,6-Diaminopurin. Die Antisense-Nukleinsäure kann alternativ biologisch unter Verwendung eines Expressionsvektors hergestellt werden, in den eine Nukleinsäure in Antisense-Richtung subkloniert worden ist (d.h. RNA, die von der eingebrachten Nukleinsäure transkribiert wird, ist zu einer Zielnukleinsäure von Interesse in Antisense-Richtung orientiert, was im nachstehenden Unterabschnitt weiter beschrieben ist).

Die erfindungsgemäßen Antisense-Nukleinsäuremoleküle werden üblicherweise an eine Zelle verabreicht oder in situ erzeugt, so dass sie mit der zellulären mRNA und/oder der genomischen DNA, die eine Desaturase kodiert, hybridisieren oder daran binden, um dadurch die Expression des Proteins, z.B. durch Hemmung der Transkription und/oder Translation, zu hemmen. Die Hybridisierung kann durch herkömmliche Nukleotidkomplementarität unter Bildung eines stabilen Duplexes oder z.B. im Fall eines Antisense-Nukleinsäuremoleküls, das DNA-Duplices bindet, durch spezifische Wechselwirkungen in der großen Furche der Doppelhelix erfolgen. Das Antisense-Molekül kann so modifiziert sein, dass es spezifisch an einen Rezeptor oder an ein auf einer ausgewählten Zelloberfläche exprimiertes Antigen bindet, z.B. durch Binden des Antisense-Nukleinsäuremoleküls an ein Peptid oder einen Antikörper, das/der an einen Zelloberflächenrezeptor oder ein Antigen bindet. Das Antisense-Nukleinsäuremolekül kann auch unter Verwendung der hier beschriebenen Vektoren den Zellen zugeführt werden. Zur Erzielung ausreichender intrazellulärer Konzentrationen der Antisense-Moleküle sind Vektorkonstrukte, in denen sich das Antisense-Nukleinsäuremolekül unter der Kontrolle eines starken prokaryotischen, viralen oder eukaryotischen, einschließlich pflanzlichen, Promotors befindet, bevorzugt.

Bei einer weiteren Ausführungsform ist das erfindungsgemäße Antisense-Nukleinsäuremolekül ein α-anomeres Nukleinsäuremolekül. Ein α-anomeres Nukleinsäuremolekül bildet spezifische doppelsträngige Hybride mit komplementärer RNA, wobei die Stränge im Gegensatz zu gewöhnlichen β-Einheiten parallel zueinander verlaufen. (Gaultier et al. (1987) Nucleic Acids Res. 15:6625-6641). Das Antisense-Nukleinsäuremolekül kann zudem ein 2¹-o-Methylribonukleotid (Inoue et al. (1987) Nucleic Acids Res. 15:6131-6148) oder ein chimäres RNA-DNA-Analogon (Inoue et al. (1987) FEBS Lett. 215:327-330) umfassen.

Bei einer weiteren Ausführungsform ist eine erfindungsgemäße Antisense-Nukleinsäure ein Ribozym. Ribozyme sind katalytische RNA-Moleküle mit Ribonukleaseaktivität, die eine einzelsträngige Nukleinsäure, wie eine mRNA, spalten können, zu der sie einen komplementären Bereich haben. Somit können Ribozyme (z.B. Hammerhead-Ribozyme (beschrieben in Haselhoff und Gerlach (1988) Nature 334:585-591)) zur katalytischen Spaltung von Desaturase-mRNA-Transkripten verwendet werden, um dadurch die Translation von Desaturase-mRNA zu hemmen. Ein Ribozym mit Spezifität für eine Desaturase-kodierende Nukleinsäure kann auf der Basis der Nukleotidsequenz einer der in SEQ ID NO: 1, 3, 5 oder 11 offenbarten Desaturase-cDNA (d.h. oder auf der Basis einer gemäß den in dieser Erfindung gelehrten Verfahren zu isolierenden heterologen Sequenz gestaltet werden. Beispielsweise kann ein Derivat einer Tetrahymena-L-19-IVS-RNA konstruiert werden, wobei die Nukleotidsequenz der aktiven Stelle komplementär zu der Nukleotidsequenz ist, die in einer Desaturase-kodierenden mRNA gespalten werden soll. Siehe z.B. Cech et al., US-Patent Nr. 4,987,071 und Cech et al., US-Patent Nr. 5,116,742. Alternativ kann Desaturase-mRNA zur Selektion einer katalytischen RNA mit einer spezifischen Ribonukleaseaktivität aus einem Pool von RNA-Molekülen verwendet werden. Siehe z.B. Bartel, D., und Szostak, J.W. (1993) Science 261:1411-1418.

Alternativ lässt sich die Desaturase-Gen-Expression hemmen, indem Nukleotidsequenzen, die komplementär zum regulatorischen Bereich einer Desaturase-Nukleotidsequenz (z.B. einem Desaturase-Promotor und/oder -Enhancer) sind, so dirigiert werden, dass Dreifachhelix-Strukturen gebildet werden, welche die Transkription eines Desaturase-Gens in Zielzellen hemmen. Siehe allgemein Helene, C. (1991) Anticancer Drug Res. 6(6) 569-84; Helene, C., et al. (1992) Ann. N. Y. Acad. Sci. 660:27-36; und Maher. L.J. (1992) Bioassays 14(12):807-815.

### B. Genkonstrukt (= Nukleinsäurekonstrukt, -fragment oder Expressionskassette)

Unter der erfindungsgemäßen Expressionskassette sind die in SEQ ID NO: 1, SEQ ID NO: 3, SEQ ID NO: 5 oder SEQ ID NO: 11 genannten Sequenzen, die sich als Ergebnis des genetischen Codes und/oder deren funktionellen oder nicht funktionellen Derivate zu verstehen, die mit einem oder mehreren Regulationssignalen vorteilhafterweise zur Erhöhung der Genexpression funktionell verknüpft wurden und welche vorteilhaft die Expression der codierenden Sequenz in der Wirtszelle steuern. Diese regulatorischen Sequenzen sollen die gezielte Expression der Gene und der Proteinexpression ermöglichen. Dies kann beispielsweise je nach Wirtsorganismus bedeuten, dass das Gen erst nach Induktion exprimiert und/oder überexprimiert wird, oder dass es sofort exprimiert und/oder überexprimiert wird. Beispielsweise handelt es sich bei diesen regulatorischen Sequenzen um Sequenzen an die Induktoren oder Repressoren binden und so die Expression der Nukleinsäure regulieren. Zusätzlich zu diesen neuen Regulationssequenzen oder anstelle dieser Sequenzen kann die natürliche Regulation dieser Sequenzen vor den eigentlichen Strukturgenen noch vorhanden sein und gegebenenfalls genetisch verändert worden sein, so dass die natürliche Regulation ausgeschaltet und die Expression der Gene erhöht wurde. Das Genkonstrukt kann aber auch einfacher aufgebaut sein, das heißt es wurden keine zusätzlichen Regulationssignale vor die Nukleinsäuresequenz oder dessen Derivate inseriert und der natürliche Promotor mit seiner Regulation wurde nicht entfernt. Stattdessen wurde die natürliche Regulationssequenz so mutiert, dass keine Regulation mehr erfolgt und/oder die Genexpression gesteigert wird. Diese veränderten Promotoren können in Form von Teilsequenzen (= Promotor mit Teilen der erfindungsgemäßen Nukleinsäuresequenzen) auch allein vor das natürliche Gen zur Steigerung der Aktivität gebracht werden. Das Genkonstrukt kann außerdem vorteilhafterweise auch eine oder mehrere sogenannte "enhancer Sequenzen" funktionell verknüpft mit dem Promotor enthalten, die eine erhöhte Expression der Nukleinsäuresequenz ermöglichen. Auch am 3'-Ende der DNA-Sequenzen können zusätzliche vorteilhafte Sequenzen inseriert werden wie weitere regulatorische Elemente oder Terminatoren. Die Δ-5-Desaturase-/Δ-6-Desaturase und/oder Δ-12-Desaturasegene können in einer oder mehreren Kopien in der Expressionskassette (= Genkonstrukt) enthalten sein.

Die regulatorischen Sequenzen bzw. Faktoren können dabei wie oben beschrieben vorzugsweise die Genexpression der eingeführten Gene positiv beeinflussen und dadurch erhöhen. So kann eine Verstärkung der regulatorischen Elemente vorteilhafterweise auf der Transkriptionsebene erfolgen, indem starke Transkriptionssignale wie Promotoren und/oder "Enhancer" verwendet werden. Daneben ist aber auch eine Verstärkung der Translation möglich, indem beispielsweise die Stabilität der mRNA verbessert wird.

Eine weitere Ausführungsform der Erfindung sind ein oder mehrere Genkonstrukte, die eine oder mehrere Sequenzen enthalten, die durch Seq ID NO: 1, 3, 5, 7, 9 oder 11 definiert sind und gem. SEQ ID NO: 2, 4, 6, 8, 10 oder 12 Polypeptide kodieren. Dabei stammen SEQ ID NO: 1, 3, 5, 7 und 11 von Desaturasen während SEQ ID NO: 9 für eine Elongase codiert. Desaturasen codierende Enzyme, die eine Doppelbindung in Δ-5-, Δ-6- oder Δ-12-Position einführen, wobei das Substrat ein, zwei, drei oder vier Doppelbindungen aufweisen. Die in SEQ ID NO: 9 dargestellte Sequenz codiert für eine Enzymaktivität, die eine Fettsäure um mindestens zwei Kohlenstoffatome verlängert sowie ihre Homologen, Derivate oder Analoga, die funktionsfähig mit einem oder mehreren Regulationssignalen, vorteilhafterweise zur Steigerung der Genexpression, verbunden sind. Beispiele für diese Regulationssequenzen sind Sequenzen, an die Induktoren oder Repressoren binden und so die Expression der Nukleinsäure regulieren. Zusätzlich zu diesen neuen Regulationssequenzen kann die natürliche Regulation dieser Sequenzen vor den eigentlichen Strukturgenen noch vorhanden sein und, wenn geeignet, genetisch modifiziert worden sein, so dass die natürliche Regulation ausgeschaltet worden ist und die Expression der Gene gesteigert worden ist.

Das Genkonstrukt kann jedoch auch eine einfachere Struktur haben, d.h. dass keine zusätzlichen Regulationssignale vor der Sequenz SEQ ID NO: 1, 3, 5 oder 11 oder ihren Homologen inseriert worden sind und der natürliche Promotor mit seiner Regulation nicht deletiert worden ist. Statt dessen ist die natürliche Regulationssequenz so mutiert worden, dass keine Regulation mehr stattfindet und die Genexpression verstärkt ist. Das Genkonstrukt kann außerdem vorteilhafterweise eine oder mehrere sogenannte Enhancer-Sequenzen, die funktionsfähig mit dem Promotor verbunden sind und die gesteigerte Expression der Nukleinsäuresequenz ermöglichen, umfassen. Es ist auch möglich, am 3'-Ende der DNA-Sequenzen zusätzlich vorteilhafte Sequenzen zu inserieren, beispielsweise weitere Regulationselemente oder Terminatoren. Die Desaturasegene und das Elongasegen können im Genkonstrukt in einer oder mehreren Kopien vorliegen. Sie können in einem Genkonstrukt oder mehreren Genkonstrukten vorliegen. Dieses Genkonstrukt oder die Genkonstrukte können zusammen im Wirtsorganismus exprimiert werden. Dabei kann das Genkonstrukt oder die Genkonstrukte in einem oder mehreren Vektoren inseriert sein und frei in der Zelle vorliegen oder aber im Genom inseriert sein. Es ist vorteilhaft für die Insertion weiterer Gene in Organismen, wenn weitere Gene im Genkonstrukt vorliegen.

Vorteilhafte Regulationssequenzen für das neue Verfahren liegen beispielsweise in Promotoren vor, wie dem cos-, tac-, trp-, tet-, trp-tet-, lpp-, lac-, lpp-lac-, lacI^{q-}, T7-, T5-, T3-, gal-, trc-, ara-, SP6-, λ-P_{R}- oder λ-P_{L}-Promotor und werden vorteilhafterweise in Gram-negativen Bakterien angewendet. Weitere vorteilhafte Regulationssequenzen liegen beispielsweise in den Gram-positiven Promotoren amy und SPO2, in den Hefe- oder Pilzpromotoren ADC1, MFα, AC, P-60, CYC1, GAPDH, TEF, rp28, ADH oder in den Pflanzenpromotoren CaMV/35S [Franck et al., Cell 21 (1980) 285-294], PRP1 [Ward et al., Plant. Mol. Biol. 22 (1993)], SSU, OCS, lib4, usp, STLS1, B33, nos oder im Ubiquitin- oder Phaseolin-Promotor vor. In diesem Zusammenhang vorteilhaft sind ebenfalls induzierbare Promotoren, wie die in EP-A-0 388 186 (Benzylsulfonamidinduzierbar), Plant J. 2, 1992:397-404 (Gatz et al., Tetracyclin-induzierbar), EP-A-0 335 528 (Abzisinsäure-induzierbar) oder WO 93/21334 (Ethanol- oder Cyclohexenol-induzierbar) beschriebenen Promotoren. Weitere geeignete Pflanzenpromotoren sind der Promotor von cytosolischer FBPase oder der ST-LSI-Promotor der Kartoffel (Stockhaus et al., EMBO J. 8, 1989, 2445), der Phosphoribosylpyrophosphatamidotransferase-Promotor aus Glycine max (Genbank-Zugangsnr. U87999) oder der in EP-A-0 249 676 beschriebene nodienspezifische Promotor. Besonders vorteilhafte Promotoren sind Promotoren, welche die Expression in Geweben ermöglichen, die an der Fettsäurebiosynthese beteiligt sind. Ganz besonders vorteilhaft sind samenspezifische Promotoren, wie der ausführungsgemäße USP Promotor aber auch andere Promotoren wie der LeB4- (Baeumlein et al., Plant J., 1992, 2 (2) (2):233-239), DC3 (Thomas, Plant Cell 1996, 263:359-368), Phaseolin- oder Napin-Promotor. Weitere besonders vorteilhafte Promotoren sind samenspezifische Promotoren, die für monokotyle oder dikotyle Pflanzen verwendet werden können und in US 5,608,152 (Napin-Promotor aus Raps), WO 98/45461 (Oleosin-Promotor aus Arobidopsis), US 5,504,200 (Phaseolin-Promotor aus Phaseolus vulgaris), WO 91/13980 (Bce4-Promotor aus Brassica), von Baeumlein et al., Plant J., 1992, 2 (2):233-239 (LeB4-Promotor aus einer Leguminose) beschrieben sind, wobei sich diese Promotoren für Dikotyledonen eignen. Die folgenden Promotoren eignen sich beispielsweise für Monokotyledonen lpt-2-oder 1pt-1-Promotor aus Gerste (WO 95/15389 und WO 95/23230), Hordein-Promotor aus Gerste und andere, in WO 99/16890 beschriebene geeignete Promotoren.

Es ist im Prinzip möglich, alle natürlichen Promotoren mit ihren Regulationssequenzen, wie die oben genannten, für das neue Verfahren zu verwenden. Es ist ebenfalls möglich und vorteilhaft, zusätzlich synthetische Promotoren zu verwenden.

Das Genkonstrukt kann, wie oben beschrieben, auch weitere Gene umfassen, die in die Organismen eingebracht werden sollen. Es ist möglich und vorteilhaft, in die Wirtsorganismen Regulationsgene, wie Gene für Induktoren, Repressoren oder Enzyme, welche durch ihre Enzymaktivität in die Regulation eines oder mehrerer Gene eines Biosynthesewegs eingreifen, einzubringen und darin zu exprimieren. Diese Gene können heterologen oder homologen Ursprungs sein. Weiterhin können vorteilhaft im Nukleinsäurekonstrukt bzw. Genkonstrukt weitere Biosynthesegene des Fettsäure- oder Lipidstoffwechsels enthalten sein oder aber diese Gene können auf einem weiteren oder mehreren weiteren Nukleinsäurekonstrukten liegen. Vorteilhaft werden als Biosynthesegene des Fettsäure- oder Lipidstoffwechsels ein Gen ausgewählt aus der Gruppe Acyl-CoA-Dehydrogenase(n), Acyl-ACP[= acyl carrier protein]-Desaturase(n), Acyl-ACP-Thioesterase(n), Fettsäure-AcylTransferase(n), Fettsäure-Synthase(n), Fettsäure-Hydroxylase(n), Acetyl-Coenzym A-Carboxylase(n), Acyl-Coenzym, A-Oxidase(n), Fettsäure-Desaturase(n), Fettsäure-Acetylenasen, Lipoxygenasen, Triacylglycerol-Lipasen, Allenoxid-Synthasen, Hydroperoxid-Lyasen oder Fettsäure-Elongase(n) oder deren Kombinationen verwendet. Genkonstrukte umfassen vorteilhafterweise zur Expression der anderen vorliegenden Gene weitere 3'- und/oder 5'-terminale Regulationssequenzen zur Steigerung der Expression, die in Abhängigkeit vom gewählten Wirtsorganismus und dem Gen oder den Genen für die optimale Expression ausgewählt werden. Diese Regulationssequenzen sollen, wie oben erwähnt, die spezifische Expression der Gene und die Proteinexpression ermöglichen. Dies kann je nach dem Wirtsorganismus beispielsweise bedeuten, dass das Gen nur nach Induktion exprimiert oder überexprimiert wird oder dass es sofort exprimiert und/oder überexprimiert wird.

Die Regulationssequenzen oder -faktoren können außerdem vorzugsweise eine vorteilhafte Wirkung auf die Expression der eingebrachten Gene haben und diese somit steigern. Auf diese Weise ist es möglich, dass die Regulationselemente unter Verwendung starker Transkriptionssignale, wie Promotoren und/oder Enhancer, vorteilhafterweise auf Transkriptionsebene verstärkt werden. Es ist jedoch weiterhin auch möglich, die Translation zum Beispiel durch Verbesserung der mRNA-Stabilität zu verstärken.

### C. Rekombinante Expressionsvektoren und Wirtszellen

Ein weiterer Aspekt der Erfindung betrifft Vektoren, vorzugsweise Expressionsvektoren, die eine Nukleinsäure enthalten, die eine Desaturase allein (oder einen Teil davon) oder ein unter Punkt b beschriebenes Nukleinsäurekonstrukt in dem die erfindungsgemäße Nukleinsäure allein oder in Kombination mit weiteren Biosynthesegenen des Fettsäure- oder Lipidstoffwechsels wie Desaturasen oder Elongasen enthalten ist. Wie hier verwendet, betrifft der Begriff "Vektor" ein Nukleinsäuremolekül, das eine andere Nukleinsäure transportieren kann, an welche es gebunden ist. Ein Vektortyp ist ein "Plasmid", was für eine zirkuläre doppelsträngige DNA-Schleife steht, in die zusätzlichen DNA-Segmente ligiert werden können. Ein weiterer Vektortyp ist ein viraler Vektor, wobei zusätzliche DNA-Segmente in das virale Genom ligiert werden können. Bestimmte Vektoren können in einer Wirtszelle, in die sie eingebracht worden sind, autonom replizieren (z.B. Bakterienvektoren mit bakteriellem Replikationsursprung und episomale Säugervektoren). Andere Vektoren (z.B. nicht-episomale Säugervektoren) werden beim Einbringen in die Wirtszelle in das Genom einer Wirtszelle integriert und dadurch zusammen mit dem Wirtsgenom repliziert. Zudem können bestimmte Vektoren die Expression von Genen, mit denen sie funktionsfähig verbunden sind, steuern. Diese Vektoren werden hier als "Expressionsvektoren" bezeichnet. Gewöhnlich haben Expressionsvektoren, die für DNA-Rekombinationstechniken geeignet sind, die Form von Plasmiden. In der vorliegenden Beschreibung können "Plasmid" und "Vektor" austauschbar verwendet werden, da das Plasmid die am häufigsten verwendete Vektorform ist. Die Erfindung soll jedoch diese anderen Expressionsvektorformen, wie virale Vektoren (z.B. replikationsdefiziente Retroviren, Adenoviren und adenoverwandte Viren), die ähnliche Funktionen ausüben, umfassen. Ferner soll der Begriff Vektor auch andere Vektoren, die dem Fachmann bekannt sind, wie Phagen, Viren, wie SV40, CMV, Baculovirus, Adenovirus, Transposons, IS-Elemente, Phasmide, Phagemide, Cosmide, lineare oder zirkuläre DNA, umfassen.

Die erfindungsgemäßen rekombinanten Expressionsvektoren umfassen eine erfindungsgemäße Nukleinsäure oder ein erfindungsgemäßes Genkonstrukt in einer Form, die sich zur Expression der Nukleinsäure in einer Wirtszelle eignet, was bedeutet, dass die rekombinanten Expressionsvektoren eine oder mehrere Regulationssequenzen, ausgewählt auf der Basis der zur Expression zu verwendenden Wirtszellen, die mit der zu exprimierenden Nukleinsäuresequenz funktionsfähig verbunden ist, umfasst. In einem rekombinanten Expressionsvektor bedeutet "funktionsfähig verbunden", dass die Nukleotidsequenz von Interesse derart an die Regulationssequenz(en) gebunden ist, dass die Expression der Nukleotidsequenz möglich ist und sie aneinander gebunden sind, so dass beide Sequenzen die vorhergesagte, der Sequenz zugeschriebene Funktion erfüllen (z.B. in einem In-vitro-Transkriptions-/Translationssystem oder in einer Wirtszelle, wenn der Vektor in die Wirtszelle eingebracht wird). Der Begriff "Regulationssequenz" soll Promotoren, Enhancer und andere Expressionskontrollelemente (z.B. Polyadenylierungssignale) umfassen. Diese Regulationssequenzen sind z.B. beschrieben in Goeddel: Gene Expression Technology: Methods in Enzymology 185, Academic Press, San Diego, CA (1990), oder siehe: Gruber und Crosby, in: Methods in Plant Molecular Biology and Biotechnolgy, CRC Press, Boca Raton, Florida, Hrsgb. : Glick und Thompson, Kapitel 7, 89-108, einschließlich der Literaturstellen darin. Regulationssequenzen umfassen solche, welche die konstitutive Expression einer Nukleotidsequenz in vielen Wirtszelltypen steuern, und solche, welche die direkte Expression der Nukleotidsequenz nur in bestimmten Wirtszellen unter bestimmten Bedingungen steuern. Der Fachmann weiß, dass die Gestaltung des Expressionsvektors von Faktoren, wie der Auswahl der zu transformierenden Wirtszelle, dem Ausmaß der Expression des gewünschten Proteins usw., abhängen kann. Die erfindungsgemäßen Expressionsvektoren können in Wirtszellen eingebracht werden, um dadurch Proteine oder Peptide, einschließlich Fusionsproteinen oder -peptiden, herzustellen, die von den Nukleinsäuren, wie hier beschrieben, kodiert werden (z.B. Desaturasen, mutante Formen von Desaturasen, Fusionsproteine usw.).

Die erfindungsgemäßen rekombinanten Expressionsvektoren können zur Expression von Desaturasen und Elongasen in prokaryotischen oder eukaryotischen Zellen gestaltet sein. Beispielsweise können Desaturasegene in bakteriellen Zellen, wie C. glutamicum, Insektenzellen (unter Verwendung von Baculovirus-Expressionsvektoren), Hefe- und anderen Pilzzellen (siehe Romanos, M.A., et al. (1992) "Foreign gene expression in yeast: a review", Yeast 8:423-488; van den Hondel, C.A.M.J.J., et al. (1991) "Heterologous gene expression in filamentous fungi", in: More Gene Manipulations in Fungi, J.W. Bennet & L.L. Lasure, Hrsgb., S. 396-428: Academic Press: San Diego; und van den Hondel, C.A.M.J.J., & Punt, P.J. (1991) "Gene transfer systems and vector development for filamentous fungi, in: Applied Molecular.Genetics of Fungi, Peberdy, J.F., et al., Hrsgb., S. 1-28, Cambridge University Press: Cambridge), Algen (Falciatore et al., 1999, Marine Biotechnology.1, 3:239-251), Ciliaten der Typen: Holotrichia, Peritrichia, Spirotrichia, Suctoria, Tetrahymena, Paramecium, Colpidium, Glaucoma, Platyophrya, Potomacus, Desaturaseudocohnilembus, Euplotes, Engelmaniella und Stylonychia, insbesondere der Gattung Stylonychia lemnae, mit Vektoren nach einem Transformationsverfahren, wie beschrieben in WO 98/01572, sowie Zellen vielzelliger Pflanzen (siehe Schmidt, R. und Willmitzer, L. (1988) "High efficiency Agrobacterium tumefaciens-mediated transformation of Arabidopsis thaliana leaf and cotyledon explants" Plant Cell Rep.:583-586; Plant Molecular Biology and Biotechnology, C Press, Boca Raton, Florida, Kapitel 6/7, S.71-119 (1993); F.F. White, B. Jenes et al., Techniques for Gene Transfer, in: Transgenic Plants, Bd. 1, Engineering and Utilization, Hrsgb.: Kung und R. Wu, Academic Press (1993), 128-43; Potrykus, Annu. Rev. Plant Physiol. Plant Molec. Biol. 42 (1991), 205-225 (und darin zitierte Literaturstellen)) oder Säugerzellen exprimiert werden. Geeignete Wirtszellen werden ferner erörtert in Goeddel, Gene Expression Technology: Methods in Enzymology 185, Academic Press, San Diego, CA (1990). Der rekombinante Expressionsvektor kann alternativ, zum Beispiel unter Verwendung von T7-Promotor-Regulationssequenzen und T7-Polymerase, in vitro transkribiert und translatiert werden.

Die Expression von Proteinen in Prokaryoten erfolgt meist mit Vektoren, die konstitutive oder induzierbare Promotoren enthalten, welche die Expression von Fusions- oder nicht-Fusionsproteinen steuern. Fusionsvektoren fügen eine Reihe von Aminosäuren an ein darin kodiertes Protein an, gewöhnlich am Aminoterminus des rekombinanten Proteins, aber auch am C-Terminus oder fusioniert innerhalb geeigneter Bereiche in den Proteinen. Diese Fusionsvektoren haben gewöhnlich drei Aufgaben: 1) die Verstärkung der Expression von rekombinantem Protein; 2) die Erhöhung der Löslichkeit des rekombinanten Proteins und 3) die Unterstützung der Reinigung des rekombinanten Proteins durch Wirkung als Ligand bei der Affinitätsreinigung. Bei Fusions-Expressionsvektoren wird oft eine proteolytische Spaltstelle an der Verbindungsstelle der Fusionseinheit und des rekombinanten Proteins eingebracht, so dass die Abtrennung des rekombinanten Proteins von der Fusionseinheit nach der Reinigung des Fusionsproteins möglich ist. Diese Enzyme und ihre entsprechenden Erkennungssequenzen umfassen Faktor Xa, Thrombin und Enterokinase.

Typische Fusions-Expressionsvektoren sind u.a. pGEX (Pharmacia Biotech Inc; Smith, D.B., und Johnson, K.S. (1988) Gene 67:31-40), pMAL (New England Biolabs, Beverly, MA) und pRIT5 (Pharmacia, Piscataway, NJ), bei denen Glutathion-S-Transferase (GST), Maltose E-bindendes Protein bzw. Protein A an das rekombinante Zielprotein fusioniert wird. Bei einer Ausführungsform ist die Desaturase-kodierende Sequenz in einen pGEX-Expressionsvektor kloniert, so dass ein Vektor erzeugt wird, der ein Fusionsprotein kodiert, das vom N-Terminus zum C-Terminus GST-Thrombin-Spaltstelle-X-Protein umfasst. Das Fusionsprotein kann durch Affinitätschromatographie unter Verwendung von Glutathion-Agarose-Harz gereinigt werden. Rekombinante Desaturase, die nicht an GST fusioniert ist, kann durch Spaltung des Fusionsproteins mit Thrombin gewonnen werden.

Beispiele für geeignete induzierbare nicht-Fusions-E. coli-Expressionsvektoren sind u.a. pTrc (Amann et al. (1988) Gene 69:301-315) und pET 11d (Studier et al., Gene Expression Technology: Methods in Enzymology 185, Academic Press, San Diego, Kalifornien (1990) 60-89). Die Zielgenexpression vom pTrc-Vektor beruht auf der Transkription durch Wirts-RNA-Polymerase von einem Hybrid-trp-lac-Fusionspromotor. Die Zielgenexpression aus dem pET 11d-Vektor beruht auf der Transkription von einem T7-gn10-lac-Fusions-Promotor, die von einer coexprimierten viralen RNA-Polymerase (T7 gn1) vermittelt wird. Diese virale Polymerase wird von den Wirtsstämmen BL21 (DE3) oder HMS174 (DE3) von einem residenten λ-Prophagen bereitgestellt, der ein T7 gnl-Gen unter der Transkriptionskontrolle des lacUV 5-Promotors birgt.

Andere in prokaryotischen Organismen geeignete Vektoren sind dem Fachmann bekannt, diese Vektoren sind beispielsweise in E. coli pLG338, pACYC184, die pBR-Reihe, wie pBR322, die pUC-Reihe, wie pUC18 oder pUC19, die M113mp-Reihe, pKC30, pRep4, pHS1, pHS2, pPLc236, pMBL24, pLG200, pUR290, pIN-III¹¹³-B1, λgt11 or pBdCI, in Streptomyces pIJ101, pIJ364, pIJ702 oder pIJ361, in Bacillus pUB110, pC194 oder pBD214, in Corynebacterium pSA77 oder pAJ667. Eine Strategie zur Maximierung der Expression von rekombinantem Protein ist die Expression des Proteins in einem Wirtsbakterium, dessen Fähigkeit zur proteolytischen Spaltung des rekombinanten Proteins gestört ist (Gottesman, S., Gene Expression Technology: Methods in Enzymology 185, Academic Press, San Diego, Kalifornien (1990) 119-128). Eine weitere Strategie ist die Veränderung der Nukleinsäuresequenz der in einen Expressionsvektor zu inserierenden Nukleinsäure, so dass die einzelnen Codons für jede Aminosäure diejenigen sind, die vorzugsweise in einem zur Expression ausgewählten Bakterium, wie *C. glutamicum,* verwendet werden (Wada et al. (1992) Nucleic Acids Res. 20:2111-2118). Diese Veränderung der erfindungsgemäßen Nukleinsäuresequenzen erfolgt durch Standard-DNA-Synthesetechniken.

Bei einer weiteren Ausführungsform ist der Desaturase-Expressionsvektor ein Hefe-Expressionsvektor. Beispiele für Vektoren zur Expression in der Hefe S. cerevisiae umfassen pYeDesaturasecl (Baldari et al. (1987) Embo J. 6:229-234), pMFa (Kurjan und Herskowitz (1982) Cell 30:933-943), pJRY88 (Schultz et al. (1987) Gene 54:113-123) sowie pYES2 (Invitrogen Corporation, San Diego, CA). Vektoren und Verfahren zur Konstruktion von Vektoren, die sich zur Verwendung in anderen Pilzen, wie den filamentösen Pilzen, eignen, umfassen diejenigen, die eingehend beschrieben sind in: van den Hondel, C.A.M.J.J., & Punt, P.J. (1991) "Gene transfer systems and vector development for filamentous fungi, in: Applied Molecular Genetics of fungi, J.F. Peberdy et al., Hrsgb., S. 1-28, Cambridge University Press: Cambridge, oder in: More Gene Manipulations in Fungi [J.W. Bennet & L.L. Lasure, Hrsgb., S. 396-428: Academic Press: San Diego]. Weitere geeignete Hefevektoren sind beispielsweise pAG-1, YEp6, YEp13 oder pEMBLYe23.

Alternativ können die erfindungsgemäßen Desaturasen in Insektenzellen unter Verwendung von Baculovirus-Expressionsvektoren exprimiert werden. Baculovirus-Vektoren, die zur Expression von Proteinen in gezüchteten Insektenzellen (z.B. Sf9-Zellen) verfügbar sind, umfassen die pAc-Reihe (Smith et al. (1983) Mol. Cell Biol.. 3:2156-2165) und die pVL-Reihe (Lucklow und Summers (1989) Virology 170:31-39).

Die oben genannten Vektoren bieten nur einen kleinen Überblick über mögliche geeignete Vektoren. Weitere Plasmide sind dem Fachmann bekannt und sind zum Beispiel beschrieben in: Cloning Vectors (Hrsgb. Pouwels, P.H., et al., Elsevier, Amsterdam-New York-Oxford, 1985, ISBN 0 444 904018).

Bei noch einer weiteren Ausführungsform wird eine erfindungsgemäße Nukleinsäure in Säugerzellen unter Verwendung eines Säuger-Expressionsvektors exprimiert. Unter Säugern werden im Sinne der Erfindung alle nicht-humanen Säuger verstanden. Beispiele für Säuger-Expressionsvektoren umfassen pCDM8 (Seed, B. (1987) Nature 329:840) und pMT2PC (Kaufman et al. (1987) EMBO J. 6:187-195). Bei der Verwendung in Säugerzellen werden die Kontrollfunktionen des Expressionsvektors oft von viralen Regulationselementen bereitgestellt. Üblicherweise verwendete Promotoren stammen z.B. aus Polyoma, Adenovirus2, Cytomegalievirus und Simian Virus 40. Weitere geeignete Expressionssysteme für prokaryotische und eukaryotische Zellen siehe in den Kapiteln 16 und 17 von Sambrook, J., Fritsch, E.F., und Maniatis, T., Molecular Cloning: A Laboratory Manual, 2. Auflage, Cold Spring Harbor Laboratory, Cold Spring Harbor Laboratory Press, Cold Spring Harbor, NY, 1989.

Bei einer anderen Ausführungsform kann der rekombinante Säuger-Expressionsvektor die Expression der Nukleinsäure vorzugsweise in einem bestimmten Zelltyp steuern (z.B. werden gewebespezifische Regulationselemente zur Expression der Nukleinsäure verwendet). Gewebespezifische Regulationselemente sind im Fachgebiet bekannt. Nicht beschränkende Beispiele für geeignete gewebespezifische Promotoren sind u.a. der Albuminpromotor (leberspezifisch; Pinkert et al. (1987) Genes Dev. 1:268-277), lymphoidspezifische Promotoren (Calame und Eaton (1988) Adv. Immunol. 43:235-275), insbesondere Promotoren von T-Zellrezeptoren (Winoto und Baltimore (1989) EMBO J. 8:729-733) und Immunglobulinen (Banerji et al. (1983) Cell 33:729-740; Queen und Baltimore (1983) Cell 33:741-748), neuronspezifische Promotoren (z.B. Neurofilament-Promotor; Byrne und Ruddle (1989) PNAS 86:5473-5477), pankreasspezifische Promotoren (Edlund et al., (1985) Science 230:912-916) und milchdrüsenspezifische Promotoren (z.B. Milchserum-Promotor; US-Patent Nr. 4,873,316 und Europäische Patentanmeldung-Veröffentlichung Nr. 264,166). Auch entwicklungsregulierte Promotoren sind umfasst, z.B. die hox-Promotoren der Maus (Kessel und Gruss (1990) Science 249:374-379) und der Fetoprotein-Promotor (Campes und Tilghman (1989) Genes Dev. 3:537-546).

Bei einer weiteren Ausführungsform können die erfindungsgemäßen Desaturasen in einzelligen Pflanzenzellen (wie Algen), siehe Falciatore et al., 1999, Marine Biotechnology 1 (3):239-251 und darin zitierte Literaturangaben, und Pflanzenzellen aus höheren Pflanzen (z.B. Spermatophyten, wie Feldfrüchten) exprimiert werden. Beispiele für Pflanzen-Expressionsvektoren umfassen solche, die eingehend beschrieben sind in: Becker, D., Kemper, E., Schell, J., und Masterson, R. (1992) "New plant binary vectors with selectable markers located proximal to the left border", Plant Mol. Biol. 20:1195-1197; und Bevan, M.W. (1984) "Binary Agrobacterium vectors for plant transformation", Nucl. Acids Res. 12:8711-8721; Vectors for Gene Transfer in Higher Plants; in: Transgenic Plants, Bd. 1, Engineering and Utilization, Hrsgb.: Kung und R. Wu, Academic Press, 1993, S. 15-38.

Eine Pflanzen-Expressionskassette enthält vorzugsweise Regulationssequenzen, welche die Genexpression in Pflanzenzellen steuern können und funktionsfähig verbunden sind, so dass jede Sequenz ihre Funktion, wie Termination der Transkription, erfüllen kann, beispielsweise Polyadenylierungssignale. Bevorzugte Polyadenylierungssignale sind diejenigen, die aus Agrobacterium tumefaciens-t-DNA stammen, wie das als Octopinsynthase bekannte Gen 3 des Ti-Plasmids pTiACH5 (Gielen et al., EMBO J. 3 (1984) 835ff.) oder funktionelle Äquivalente davon, aber auch alle anderen in Pflanzen funktionell aktiven Terminatoren sind geeignet.

Da die Pflanzengenexpression sehr oft nicht auf Transkriptionsebenen beschränkt ist, enthält eine Pflanzen-Expressionskassette vorzugsweise andere funktionsfähig verbunden Sequenzen, wie Translationsenhancer, beispielsweise die Overdrive-Sequenz, welche die 5'-untranslatierte Leader-Sequenz aus Tabakmosaikvirus, die das Protein/RNA-Verhältnis erhöht, enthält (Gallie et al., 1987, Nucl. Acids Research 15:8693-8711).

Die Pflanzengenexpression muss funktionsfähig mit einem geeigneten Promotor verbunden sein, der die Genexpression auf rechtzeitige, zell- oder gewebespezifische Weise durchführt. Bevorzugt sind Promotoren, welche die konstitutive Expression herbeiführen (Benfey et al., EMBO J. 8 (1989) 2195-2202), wie diejenigen, die von Pflanzenviren stammen, wie 35S CAMV (Franck et al., Cell 21 (1980) 285-294), 19S CaMV (siehe auch US 5352605 und WO 84/02913) oder Pflanzenpromotoren, wie der in US 4,962,028 beschriebene der kleinen Untereinheit der Rubisco.

Andere bevorzugte Sequenzen für die Verwendung zur funktionsfähigen Verbindung in Pflanzengenexpressions-Kassetten sind Targeting-Sequenzen, die zur Steuerung des Genproduktes in sein entsprechendes Zellkompartiment notwendig sind (siehe eine Übersicht in Kermode, Crit. Rev. Plant Sci. 15, 4 (1996) 285-423 und darin zitierte Literaturstellen), beispielsweise in die Vakuole, den Zellkern, alle Arten von Plastiden, wie Amyloplasten, Chloroplasten, Chromoplasten, den extrazellulären Raum, die Mitochondrien, das Endoplasmatische Retikulum, Ölkörper, Peroxisomen und andere Kompartimente von Pflanzenzellen.

Die Pflanzengenexpression lässt sich auch über einen chemisch induzierbaren Promotor erleichtern (siehe eine Übersicht in Gatz 1997, Annu. Rev. Plant Physiol. Plant Mol. Biol., 48:89-108). Chemisch induzierbare Promotoren eignen sich besonders, wenn gewünscht wird, dass die Genexpression auf zeitspezifische Weise erfolgt. Beispiele für solche Promotoren sind ein Salicylsäureinduzierbarer Promotor (WO 95/19443), ein Tetracyclin-induzierbarer Promotor (Gatz et al. (1992) Plant J. 2, 397-404) und ein Ethanol-induzierbarer Promotor.

Auch Promotoren, die auf biotische oder abiotische Stressbedingungen reagieren, sind geeignete Promotoren, beispielsweise der pathogeninduzierte PRP1-Gen-Promotor (Ward et al., Plant. Mol. Biol. 22 (1993) 361-366), der hitzeinduzierbare hsp80-Promotor aus Tomate (US 5,187,267), der kälteinduzierbare Alphaamylase-Promotor aus Kartoffel (WO 96/12814) oder der durch Wunden induzierbare pinII-Promotor (EP-A-0 375 091).

Es sind insbesondere solche Promotoren bevorzugt, welche die Genexpression in Geweben und Organen herbeiführen, in denen die Lipid- und Ölbiosynthese stattfindet, in Samenzellen, wie den Zellen des Endosperms und des sich entwickelnden Embryos. Geeignete Promotoren sind der Napingen-Promotor aus Raps (US 5,608,152), der USP-Promotor aus Vicia faba (Baeumlein et al., Mol Gen Genet, 1991, 225 (3):459-67), der Oleosin-Promotor aus Arabidopsis (WO 98/45461), der Phaseolin-Promotor aus Phaseolus vulgaris (US 5,504,200), der Bce4-Promotor aus Brassica (WO 91/13980) oder der Legumin-B4-Promotor (LeB4; Baeumlein et al., 1992, Plant Journal, 2 (2):233-9) sowie Promotoren, welche die samenspezifische Expression in Monokotyledonen-Pflanzen, wie Mais, Gerste, Weizen, Roggen, Reis usw. herbeiführen. Geeignete beachtenswerte Promotoren sind der lpt2-oder lpt1-Gen-Promotor aus Gerste (WO 95/15389 und WO 95/23230) oder die in WO 99/16890 beschriebenen (Promotoren aus dem Gersten-Hordein-Gen, dem Reis-Glutelin-Gen, dem Reis-Oryzin-Gen, dem Reis-Prolamin-Gen, dem Weizen-Gliadin-Gen, Weizen-Glutelin-Gen, dem Mais-Zein-Gen, dem Hafer-Glutelin-Gen, dem Sorghum-Kasirin-Gen, dem Roggen-Secalin-Gen).

Insbesondere kann die multiparallele Expression von erfindungsgemäßen Desaturasen allein oder in Kombination mit anderen desaturasen oder Elongasen gewünscht sein. Die Einführung solcher Expressionskassetten kann über eine simultane Transformation mehrerer einzelner Expressionskonstrukte erfolgen oder durch Kombination mehrerer Expressionskassetten auf einem Konstrukt. Auch können mehrere Vektoren mit mit jeweils mehreren Exopressionskassetten transformiert und auf die Wirtszelle übertragen werden.

Ebenfalls besonders geeignet sind Promotoren, welche die plastidenspezifische Expression herbeiführen, da Plastiden das Kompartiment sind, in dem die Vorläufer sowie einige Endprodukte der Lipidbiosynthese synthetisiert werden. Geeignete Promotoren, wie der virale RNA-Polymerase-Promotor, sind beschrieben in WO 95/16783 und WO 97/06250, und der clpP-Promotor aus Arabidopsis, beschrieben in WO 99/46394.

Die Erfindung stellt zudem einen rekombinanten Expressionsvektor bereit, umfassend ein erfindungsgemäßes DNA Molekül, das in Antisense-Richtung in den Expressionsvektor kloniert ist. d.h. das DNA-Molekül ist derart mit einer regulatorischen Sequenz funktionsfähig verbunden, dass die Expression (durch Transkription des DNA-Moleküls) eines RNA-Moleküls, das zur Desaturase-mRNA "Antisense" ist, ermöglicht wird. Es können Regulationssequenzen ausgewählt werden, die funktionsfähig mit einer in Antisense-Richtung klonierten Nukleinsäure verbunden sind und die kontinuierliche Expression des Antisense-RNA-Moleküls in einer Vielzahl von Zelltypen steuern, zum Beispiel können virale Promotoren und/oder Enhancer oder Regulationssequenzen ausgewählt werden, welche die konstitutive, gewebespezifische oder zelltypspezifische Expression von Antisense-RNA steuern. Der Antisense-Expressionsvektor kann in Form eines rekombinanten Plasmids, Phagemids oder attenuierten Virus vorliegen, in dem Antisense-Nukleinsäuren unter der Kontrolle eines hochwirksamen regulatorischen Bereichs produziert werden, dessen Aktivität durch den Zelltyp bestimmt werden kann, in den der Vektor eingebracht worden ist. Eine Erläuterung der Regulation der Genexpression mittels Antisense-Genen siehe in Weintraub, H., et al., Antisense-RNA as a molecular tool for genetic analysis, Reviews - Trends in Genetics, Bd. 1(1) 1986.

Ein weiterer Aspekt der Erfindung betrifft Wirtszellen, in die ein erfindungsgemäßer rekombinanter Expressionsvektor eingebracht worden ist. Die Begriffe "Wirtszelle" und "rekombinante Wirtszelle" werden hier untereinander austauschbar verwendet. Selbstverständlich betreffen diese Begriffe nicht nur die bestimmte Zielzelle, sondern auch die Nachkommen oder potentiellen Nachkommen dieser Zelle. Da in aufeinanderfolgenden Generationen aufgrund von Mutation oder Umwelteinflüssen bestimmte Modifikationen auftreten können, sind diese Nachkommen nicht unbedingt mit der Parentalzelle identisch, sind jedoch immer noch vom Umfang des Begriffs, wie hier verwendet, umfasst.

Unter Rekombinant oder Transgen beispielsweise rekombinanten Expressionsvektor oder rekombinanten Wirt oder Wirtszellen im Sinne der Erfindung ist zu verstehen, dass die erfindungsgemäßen Nukleinsäuren und/oder deren natürliche Regulationssequenzen an 5' und 3'-Position der Nukleinsäuren nicht in ihrer natürlichen Umgebung sind, das heißt entweder wurde die Lage der Sequenzen im Herkunfstorganismus verändert oder in diesem wurden die Nukleinsäuresequenzen und/oder die Regulationssequenzen mutiert oder die erfindungsgemäßen Nukleinsäuresequenzen wurden in einen anderen Organismus als den Herkunfstorganismus verbracht oder deren Regulationssequenzen. Auch Kombinationen dieser Veränderungen sind möglich. Unter natürlicher Umgebung ist die Lage einer Nukleinsäuresequenz in einem Organismus zu verstehen, wie er in der Natur vorkommt.

Eine Wirtszelle kann eine prokaryotische oder eukaryotische Zelle sein. Zum Beispiel kann eine Desaturase in Bakterienzellen, wie C. glutamicum, Insektenzellen, Pilzzellen oder Säugerzellen (wie Chinesischer Hamster-Ovarzellen (CHO) oder COS-Zellen), Algen, Ciliaten, Pflanzenzellen, Pilzen oder anderen Mikroorganismen, wie C. glutamicum, exprimiert werden. Andere geeignete Wirtszellen sind dem Fachmann geläufig.

Vektor-DNA lässt sich in prokaryotische oder eukaryotische Zellen über herkömmliche Transformations- oder Transfektionstechniken einbringen. Die Begriffe "Transformation" und "Transfektion", Konjugation und Transduktion, wie hier verwendet, sollen eine Vielzahl von im Stand der Technik bekannten Verfahren zum Einbringen fremder Nukleinsäure (z.B. DNA) in eine Wirtszelle, einschließlich Calciumphosphat- oder Calciumchlorid-Copräzipitation, DEAE-Dextran-vermittelte Transfektion, Lipofektion, natürliche Kompetenz, chemisch vermittelter Transfer, Elektroporation oder Teilchenbeschuss, umfassen. Geeignete Verfahren zur Transformation oder Transfektion von Wirtszellen, einschließlich Pflanzenzellen, lassen sich finden in Sambrook et al. (Molecular Cloning: A Laboratory Manual., 2. Aufl., Cold Spring Harbor Laboratory, Cold Spring Harbor Laboratory Press, Cold Spring Harbor, NY, 1989) und anderen Labor-Handbüchern, wie Methods in Molecular Biology, 1995, Bd. 44, Agrobacterium protocols, Hrsgb: Gartland und Davey, Humana Press, Totowa, New Jersey.

Über die stabile Transfektion von Säugerzellen ist bekannt, dass je nach verwendetem Expressionsvektor und verwendeter Transfektionstechnik nur ein kleiner Teil der Zellen die fremde DNA in ihr Genom integriert. Zur Identifikation und Selektion dieser Integranten wird gewöhnlich ein Gen, das einen selektierbaren Marker (z.B. Resistenz gegen Antibiotika) kodiert, zusammen mit dem Gen von Interesse in die Wirtszellen eingebracht. Bevorzugte selektierbare Marker umfassen solche, welche Resistenz gegen Medikamente, wie G418, Hygromycin und Methotrexat, verleihen, oder in Pflanzen solche, welche Resistenz gegen ein Herbizid, wie Glyphosphat oder Glufosinat, verleihen. Weitere geeignete Marker sind beispielsweise Marker, welche Gene kodieren, die an Biosynthesewegen von zum Beispiel Zuckern oder Aminosäuren beteiligt sind, wie β-Galactodsidase, ura3 oder ilv2. Marker, welche Gene, wie Luziferase, gfp oder andere Fluoreszenzgene kodieren, sind ebenfalls geeignet. Diese Marker lassen sich in Mutanten verwenden, in denen diese Gene nicht funktionell sind, da sie beispielsweise mittels herkömmlicher Verfahren deletiert worden sind. Ferner können Marker, welche eine Nukleinsäure kodieren, die einen selektierbaren Marker kodiert, in eine Wirtszelle auf dem gleichen Vektor eingebracht werden, wie derjenige, der eine Desaturase kodiert, oder können auf einem gesonderten Vektor eingebracht werden. Zellen, die mit der eingebrachten Nukleinsäure stabil transfiziert worden sind, können zum Beispiel durch Medikamentenselektion identifiziert werden (z.B. überleben Zellen, die den selektierbaren Marker integriert haben, wohingegen die anderen Zellen absterben).

Zur Erzeugung eines homolog rekombinierten Mikroorganismus wird ein Vektor hergestellt, der zumindest einen Abschnitt eines Desaturasegens enthält, in den eine Deletion, Addition oder Substitution eingebracht worden ist, um dadurch das Desaturasegen zu verändern, z.B. funktionell zu disrumpieren. Dieses Desaturasegen ist vorzugsweise ein Phaeodactylum tricornutum Desaturasegen, es kann jedoch ein Homologon oder Analogon aus anderen Organismen, sogar aus einer Säuger-, Pilz- oder Insektenquelle verwendet werden. Bei einer bevorzugten Ausführungsform ist der Vektor so gestaltet, dass das endogene Desaturasegen bei homologer Rekombination funktionell disrumptiert wird (d.h. nicht länger ein funktionelles Protein kodiert, auch als Knock-out-Vektor bezeichnet). Alternativ kann der Vektor so gestaltet sein, dass das endogene Desaturasegen bei homologer Rekombination mutiert oder anderweitig verändert wird, aber immer noch ein funktionelles Protein kodiert (z.B. kann der stromaufwärts gelegene regulatorische Bereich so verändert sein, dass dadurch die Expression der endogenen Desaturase verändert wird). Zur Erzeugung einer Punktmutation über homologe Rekombination können auch als Chimeraplasty bekannte DNA-RNA-Hybride verwendet werden, die aus Cole-Strauss et al., 1999, Nucleic Acids Research 27(5);1323-1330 und Kmiec, Gene therapy, 19999, American Scientist, 87(3):240-247 bekannt sind.

Im Vektor für die homologe Rekombination ist der veränderte Abschnitt des Desaturasegens an seinem 5'- und 3'-Ende von zusätzlicher Nukleinsäure des Desaturasegens flankiert, so dass homologe Rekombination zwischen dem exogenen Desaturasegen, das auf dem Vektor vorliegt, und einem endogenen Desaturasegen in einem Mikroorganismus oder einer Pflanze möglich ist. Die zusätzliche flankierende Desaturase-Nukleinsäure ist für eine erfolgreiche homologe Rekombination mit dem endogenen Gen hinreichend lang. Gewöhnlich sind im Vektor mehrere hundert Basenpaare bis zu Kilobasen flankierende DNA (sowohl am 5'- als auch am 3'-Ende) enthalten (eine Beschreibung von Vektoren zur homologen Rekombination siehe z.B. in Thomas, K.R., und Capecchi, M.R. (1987) Cell 51:503 oder der Rekombination in Physcomitrella patens auf cDNA-Basis in Strepp et al., 1998, Proc. Natl. Acad. Sci. USA 95 (8):4368-4373). Der Vektor wird in einen Mikroorganismus oder eine Pflanzenzelle (z.B. mittels Polyethylenglycol-vermittelter DNA) eingebracht, und Zellen, in denen das eingebrachte Desaturasegen mit dem endogenen Desaturasegen homolog rekombiniert ist, werden unter Verwendung im Fachgebiet bekannter Techniken selektiert.

Bei einer anderen Ausführungsform können rekombinante Organismen, wie Mikroorganismen, hergestellt werden, die ausgewählte Systeme enthalten, welche eine regulierte Expression des eingebrachten Gens ermöglichen. Der Einschluß eines Desaturasegens in einem Vektor, wobei es unter die Kontrolle des lac-Operons gebracht wird, ermöglicht z.B. die Expression des Desaturasegens nur in Gegenwart von IPTG. Diese Regulationssysteme sind im Fachgebiet bekannt.

Eine erfindungsgemäße Wirtszelle, wie eine prokaryotische oder eukaryotische Wirtszelle, in Kultur oder auf einem Feld wachsend, kann zur Produktion (d.h. Expression) einer Desaturase verwendet werden. In Pflanzen kann zusätzlich ein alternatives Verfahren durch direkten Transfer von DNA in sich entwickelnde Blüten über Elektroporation oder Gentransfer mittels Agrobacterium angewendet werden. Die Erfindung stellt folglich ferner Verfahren zur Produktion von Desaturasen unter Verwendung der erfindungsgemäßen Wirtszellen bereit. Bei einer Ausführungsform umfasst das Verfahren die Anzucht der erfindungsgemäßen Wirtszelle (in die ein rekombinanter Expressionsvektor, der eine Desaturase kodiert, eingebracht worden ist, oder in deren Genom ein Gen eingebracht worden ist, das eine Wildtyp- oder veränderte Desaturase kodiert) in einem geeigneten Medium, bis die Desaturase produziert worden ist. Das Verfahren umfasst bei einer weiteren Ausführungsform das Isolieren der Desaturasen aus dem Medium oder der Wirtszelle.

Wirtszellen, die im Prinzip zum Aufnehmen der erfindungsgemäßen Nukleinsäure, des erfindungsgemäßen Genproduktes oder des erfindungsgemäßen Vektors geeignet sind, sind alle prokaryotischen oder eukaryotischen Organismen. Die vorteilhafterweise verwendeten Wirtsorganismen sind Organismen, wie Bakterien, Pilze, Hefen, Tier- oder Pflanzenzellen. Weitere vorteilhafte Organismen sind Tiere oder vorzugsweise Pflanzen oder Teile davon. Pilze, Hefen oder Pflanzen werden vorzugsweise verwendet, besonders bevorzugt Pilze oder Pflanzen, ganz besonders bevorzugt Pflanzen, wie Ölfruchtpflanzen, die große Mengen an Lipidverbindungen enthalten, wie Raps, Nachtkerze, Canola, Erdnuss, Lein, Soja, Safflor, Sonnenblume, Borretsch, oder Pflanzen, wie Mais, Weizen, Roggen, Hafer, Triticale, Reis, Gerste, Baumwolle, Maniok, Pfeffer, Tagetes, Solanaceen-Pflanzen, wie Kartoffel, Tabak, Aubergine und Tomate, Vicia-Arten, Erbse, Alfalfa, Buschpflanzen (Kaffee, Kakao, Tee), Salix-Arten, Bäume (Ölplame, Kokosnuß) sowie ausdauernde Gräser und Futterfeldfrüchte. Besonders bevorzugte erfindungsgemäße Pflanzen sind Ölfruchtpflanzen, wie Soja, Erdnuss, Raps, Canola, Lein, Nachtkerze, Sonnenblume, Safflor, Bäume (Ölpalme, Kokosnuß).

### D. Isolierte Desaturase

Ein weiterer Aspekt der Erfindung betrifft isolierte Desaturasen und biologisch aktive Teile davon. Ein "isoliertes" oder "gereinigtes" Protein oder ein biologisch aktiver Teil davon ist im wesentlichen frei von zellulärem Material, wenn es durch DNA-Rekombinationstechniken produziert wird, oder von chemischen Vorstufen oder andern Chemikalien, wenn es chemisch synthetisiert wird. Der Begriff "im wesentlichen frei von zellulärem Material" umfasst Desaturase-Präparationen, in denen das Protein von zellulären Komponenten der Zellen, in denen es natürlich oder rekombinant produziert wird, getrennt ist. Bei einer Ausführungsform umfasst der Ausdruck "im wesentlichen frei von zellulärem Material" Desaturase-Präparationen mit weniger als etwa 30 % (bezogen auf das Trockengewicht) nicht-Desaturase (hier auch als "verunreinigendes Protein" bezeichnet), stärker bevorzugt weniger als etwa 20 % nicht-Desaturase, noch stärker bevorzugt weniger als etwa 10 % nicht-Desaturase und am stärksten bevorzugt weniger als etwa 5 % nicht-Desaturase. Wenn die Desaturase oder ein biologisch aktiver Teil davon rekombinant hergestellt worden ist, ist sie/er auch im wesentlichen frei von Kulturmedium, d.h. das Kulturmedium macht weniger als etwa 20 %, stärker bevorzugt weniger als etwa 10 % und am stärksten bevorzugt weniger als etwa 5 % des Volumens der Proteinpräparation aus. Der Begriff "im wesentlichen frei von chemischen Vorstufen oder anderen Chemikalien" umfasst Desaturase-Präparationen, in denen das Protein von chemischen Vorstufen oder anderen Chemikalien getrennt ist, die an der Synthese des Proteins beteiligt sind. Bei einer Ausführungsform umfasst der Begriff "im wesentlichen frei von chemischen Vorstufen oder anderen Chemikalien" Desaturase-Präparationen mit weniger als etwa 30 % (bezogen auf das Trockengewicht) chemischen Vorstufen oder nicht-Desaturase-Chemikalien, stärker bevorzugt weniger als etwa 20 % chemischen Vorstufen oder nicht-Desaturase-Chemikalien, noch stärker bevorzugt weniger als etwa 10 % chemischen Vorstufen oder nicht-Desaturase-Chemikalien und am stärksten bevorzugt weniger als etwa 5 % chemischen Vorstufen oder nicht-Desaturase-Chemikalien. Bei bevorzugten Ausführungsformen weisen isolierte Proteine oder biologisch aktive Teile davon keine verunreinigenden Proteine aus dem gleichen Organismus auf, aus dem die Desaturase stammt. Diese Proteine werden gewöhnlich durch rekombinante Expression zum Beispiel Phaeodactylum tricornutum-Desaturase in Pflanzen wie Physcomitrella patens bzw. o.g. oder Mikroorganismen, beispielsweise Bakterien, wie E. coli, Bacillus subtilis, C. glutamicum, Pilzen, wie Mortierella, Hefe, wie Saccharomyces, oder Ciliaten wie Colpidium oder Algen wie Phaeodactylum hergestellt.

Eine erfindungsgemäße isolierte Desaturase oder ein Teil davon kann auch am Stoffwechsel von zum Aufbau von Zellmembranen in Phaeodactylum tricornutum notwendigen Verbindungen oder am Transport von Molekülen über diese Membranen teilnehmen. Bei bevorzugten Ausführungsformen umfasst das Protein oder der Teil davon eine Aminosäuresequenz, die ausreichend homolog zu einer Aminosäuresequenz der SEQ ID NO: 2, 4, 6 oder 12 ist, dass das Protein oder der Teil davon die Fähigkeit, am Stoffwechsel von zum Aufbau von Zellmembranen in Phaeodactylum tricornutum notwendigen Verbindungen oder am Transport von Molekülen über diese Membranen teilzunehmen, beibehält. Der Teil des Proteins ist vorzugsweise ein biologisch aktiver Teil, wie hier beschrieben. Bei einer weiteren bevorzugten Ausführungsform hat eine erfindungsgemäße Desaturase eine der in SEQ ID NO: 2, 4, 6 oder 12 gezeigten Aminosäuresequenzen. Bei einer weiteren bevorzugten Ausführungsform hat die Desaturase eine Aminosäuresequenz, die von einer Nukleotidsequenz kodiert wird, die, zum Beispiel unter stringenten Bedingungen, an eine Nukleotidsequenz der SEQ ID NO: 1, 3, 5 oder 11 hybridisiert. Bei noch einer weiteren bevorzugten Ausführungsform hat die Desaturase eine Aminosäuresequenz, die von einer Nukleotidsequenz kodiert wird, die mindestens etwa 50 bis 60 %, vorzugsweise mindestens etwa 60 bis 70 %, stärker bevorzugt mindestens etwa 70 bis 80 %, 80 bis 90 %, 90 bis 95 % und noch stärker bevorzugt mindestens etwa 96 %, 97 %, 98 %, 99 % oder noch homologer zu einer der Aminosäuresequenzen der SEQ ID NO: 2, 4, 6 oder 18 ist. Die erfindungsgemäße bevorzugte Desaturase besitzt vorzugsweise auch mindestens eine der hier beschriebenen Desaturase-Aktivitäten. Zum Beispiel umfasst eine erfindungsgemäße bevorzugte Desaturase eine Aminosäuresequenz, die von einer Nukleotidsequenz kodiert wird, die, zum Beispiel unter stringenten Bedingungen, an eine Nukleotidsequenz der SEQ ID NO: 1, 3, 5 oder 11 hybridisiert und am Stoffwechsel von zum Aufbau von Zellmembranen in Phaeodactylum tricornutum notwendigen Verbindungen oder am Transport von Molekülen über diese Membranen teilnehmen kann oder eine Doppelbindung in eine Fettsäure mit ein, zwei, drei oder vier Doppelbindungen und einer Kettenlänge von C₁₈, C₂₀ oder C₂₂ einführt.

Bei anderen Ausführungsformen ist die Desaturase im wesentlichen homolog zu einer Aminosäuresequenz der SEQ ID NO: 2, 4 oder 6 und behält die funktionelle Aktivität des Proteins einer der Sequenzen der SEQ ID NO: 2, 4 oder 6 bei, ihre Aminosäuresequenz unterscheidet sich jedoch aufgrund von natürlicher Variation oder Mutagenese, wie eingehend im obigen Unterabschnitt I beschrieben. Bei einer weiteren Ausführungsform ist die Desaturase folglich ein Protein, das eine Aminosäuresequenz umfasst, die mindestens etwa 50 bis 60 %, vorzugsweise mindestens etwa 60 bis 70 % und stärke bevorzugt mindestens etwa 70 bis 80 %, 80 bis 90 %, 90 bis 95 % und am stärksten bevorzugt mindestens etwa 96 %, 97 %, 98 %, 99 % oder noch homologer zu einer vollständigen Aminosäuresequenz der SEQ ID NO: 2, 4 oder 6 ist und zumindest eine der hier beschriebenen Desaturase-Aktivitäten aufweist. Bei einer anderen Ausführungsform betrifft die Erfindung ein vollständiges Phaeodactylum tricornutum-Protein, das im wesentlichen homolog zu einer vollständigen Aminosäuresequenz der SEQ ID NO: 2, 4 oder 6 ist.

Biologisch aktive Teile einer Desaturase umfassen Peptide, umfassend Aminosäuresequenzen, die von der Aminosäuresequenz einer Desaturase hergeleitet sind, z.B. eine in SEQ ID NO: 2, 4 oder 6 gezeigte Aminosäuresequenz oder die Aminosäuresequenz eines Proteins, das zu einer Desaturase homolog ist, welche weniger Aminosäuren als die Vollängen-Desaturase oder das Vollängenprotein aufweisen, das zu einer Desaturase homolog ist, und zumindest eine Aktivität einer Desaturase aufweisen.

Gewöhnlich umfassen biologisch aktive Teile (Peptide, z.B. Peptide, die zum Beispiel 5, 10, 15, 20, 30, 35, 36, 37, 38, 39, 40, 50, 100 oder mehr Aminosäuren lang sind) eine Domäne oder ein Motiv mit mindestens einer Aktivität einer Desaturase. Überdies können andere biologisch aktive Teile, in denen andere Bereiche des Proteins deletiert sind, durch rekombinante Techniken hergestellt und bezüglich einer oder mehrerer der hier beschriebenen Aktivitäten untersucht werden. Die biologisch aktiven Teile einer Desaturase umfassen vorzugsweise ein/eine oder mehrere ausgewählte Domänen/Motive oder Teile davon mit biologischer Aktivität.

Desaturasen werden vorzugsweise durch DNA-Rekombinationstechniken hergestellt. Zum Beispiel wird ein das Protein kodierendes Nukleinsäuremolekül in einen Expressionsvektor (wie vorstehend beschrieben) kloniert, der Expressionsvektor wird in eine Wirtszelle (wie vorstehend beschrieben) eingebracht, und die Desaturase wird in der Wirtszelle exprimiert. Die Desaturase kann dann durch ein geeignetes Reinigungsschema mittels Standard-Proteinreinigungstechniken aus den Zellen isoliert werden. Alternativ zur rekombinanten Expression kann eine Desaturase, ein -Polypeptid, oder -Peptid mittels Standard-Peptidsynthesetechniken chemisch synthetisiert werden. Überdies kann native Desaturase aus Zellen (z.B. Endothelzellen) z.B. unter Verwendung eines Anti-Desaturase-Antikörpers isoliert werden, der durch Standardtechniken produziert werden kann, wobei eine erfindungsgemäße Desaturase oder ein Fragment davon verwendet wird.

Die Erfindung stellt auch chimäre Desaturase-Proteine oder Desaturase-Fusionsproteine bereit. Wie hier verwendet, umfasst ein "chimäres Desaturase-Protein" oder "Desaturase-Fusionsprotein" ein Desaturase-Polypeptid, das funktionsfähig an ein nicht-Desaturase-Polypeptid gebunden ist. Ein "Desaturase-Polypeptid" betrifft ein Polypeptid mit einer Aminosäuresequenz, die einer Desaturase entspricht, wohingegen ein "nicht-Desaturase-Polypeptid" ein Polypeptid mit einer Aminosäuresequenz betrifft, die einem Protein entspricht, das im wesentlichen nicht homolog zu der Desaturase ist, z.B. ein Protein, das sich vom der Desaturase unterscheidet und aus dem gleichen oder einem anderen Organismus stammt. Innerhalb des Fusionsproteins soll der Begriff "funktionsfähig verbunden" bedeuten, dass das Desaturase-Polypeptid und das nicht-Desaturase-Polypeptid so miteinander fusioniert sind, dass beide Sequenzen die vorhergesagte, der verwendeten Sequenz zugeschriebene Funktion erfüllen. Das nicht-Desaturase-Polypeptid kann an den N-Terminus oder den C-Terminus des Desaturase-Polypeptids fusioniert sein. Bei einer Ausführungsform ist das Fusionsprotein zum Beispiel ein GST-Desaturase-Fusionsprotein, bei dem die Desaturase-Sequenzen an den C-Terminus der GST-Sequenzen fusioniert sind. Diese Fusionsproteine können die Reinigung der rekombinanten Desaturasen erleichtern. Bei einer weiteren Ausführungsform ist das Fusionsprotein eine Desaturase, die eine heterologe Signalsequenz an ihrem N-Terminus aufweist. In bestimmten Wirtszellen (z.B. Säuger-Wirtszellen) kann die Expression und/oder Sekretion einer Desaturase durch Verwendung einer heterologen Signalsequenz gesteigert werden.

Ein erfindungsgemäßes chimäres Desaturase-Protein oder Desaturase-Fusionsprotein wird durch Standard-DNA-Rekombinationstechniken hergestellt. Zum Beispiel werden DNA-Fragmente, die unterschiedliche Polypeptidsequenzen kodieren, gemäß herkömmlicher Techniken im Leseraster aneinander ligiert, indem beispielsweise glatte oder überhängende Enden zur Ligation, Restriktionsenzymspaltung zur Bereitstellung geeigneter Enden, Auffüllen kohäsiver Enden, wie erforderlich, Behandlung mit alkalischer Phosphatase, um ungewollte Verknüpfungen zu vermeiden, und enzymatische Ligation eingesetzt werden. Bei einer weiteren Ausführungsform kann das Fusionsgen durch herkömmliche Techniken, einschließlich DNA-Syntheseautomaten, synthetisiert werden. Alternativ kann eine PCR-Amplifizierung von Genfragmenten unter Verwendung von Ankerprimern durchgeführt werden, die komplementäre Überhänge zwischen aufeinanderfolgenden Genfragmenten erzeugen, die anschließend miteinander hybridisiert und reamplifiziert werden können, so dass eine chimäre Gensequenz erzeugt wird (siehe zum Beispiel Current Protocols in Molecular Biology, Hrsgb. Ausubel et al., John Wiley & Sons: 1992). Überdies sind viele Expressionsvektoren kommerziell erhältlich, die bereits eine Fusionseinheit (z.B. ein GST-Polypeptid) kodieren. Eine Desaturase-kodierende Nukleinsäure kann in einen solchen Expressionsvektor kloniert werden, so dass die Fusionseinheit im Leseraster mit dem Desaturase-Protein verbunden ist.

Homologe der Desaturase können durch Mutagenese, z.B. durch spezifische Punktmutation oder Verkürzung der Desaturase, erzeugt werden. Der Begriff "Homologe", wie hier verwendet, betrifft eine variante Form der Desaturase, die als Agonist oder Antagonist der Desaturase-Aktivität wirkt. Ein Agonist der Desaturase kann im wesentlichen die gleiche Aktivität wie die oder einen Teil der biologischen Aktivitäten der Desaturase beibehalten. Ein Antagonist der Desaturase kann eine oder mehrere Aktivitäten der natürlich vorkommenden Form der Desaturase durch zum Beispiel kompetitive Bindung an ein stromabwärts oder -aufwärts gelegenes Element der Stoffwechselkaskade für Zellmembrankomponenten, welche die Desaturase umfasst, oder durch Bindung an eine Desaturase, welche den Transport von Verbindungen über Zellmembranen vermittelt, hemmen, wodurch die Translokation gehemmt wird. Bei einer alternativen Ausführungsform können Homologe der Desaturase durch Sichten kombinatorischer Banken von Mutanten, z.B. Verkürzungsmutanten, der Desaturase hinsichtlich Desaturase-Agonisten- oder -Antagonisten-Aktivität identifiziert werden. Bei einer Ausführungsform wird eine variegierte Bank von Desaturase-Varianten durch kombinatorische Mutagenese auf Nukleinsäureebene erzeugt und durch eine variegierte Genbank kodiert. Eine variegierte Bank von Desaturase-Varianten kann z.B. durch enzymatische Ligation eines Gemisches von synthetischen Oligonukleotiden in Gensequenzen hergestellt werden, so dass sich ein degenerierter Satz potentieller Desaturase-Sequenzen als individuelle Polypeptide oder alternativ als Satz größerer Fusionsproteine (z.B. für das Phage-Display), die diesen Satz von Desaturase-Sequenzen enthalten, exprimieren lässt. Es gibt eine Vielzahl von Verfahren, die zur Herstellung von Banken potentieller Desaturase-Homologen aus einer degenerierten Oligonukleotidsequenz verwendet werden können. Die chemische Synthese einer degenerierten Gensequenz kann in einem DNA-Syntheseautomaten durchgeführt und das synthetische Gen dann in einen geeigneten Expressionsvektor ligiert werden. Die Verwendung eines degenerierten Satzes von Genen ermöglicht die Bereitstellung sämtlicher Sequenzen, die den gewünschten Satz an potentiellen Desaturase-Sequenzen kodieren, in einem Gemisch. Verfahren zur Synthese degenerierter Oligonukleotide sind im Fachgebiet bekannt (siehe z.B. Narang, S.A. (1983) Tetrahedron 39:3; Itakura et al. (1984) Annu. Rev. Biochem. 53:323; Itakura et al., (1984) Science 198:1056; Ike et al. (1983) Nucleic Acids Res. 11:477).

Zusätzlich können Banken von Desaturase-Fragmenten zur Herstellung einer variegierten Population von Desaturase-Fragmenten für das Sichten und für die anschließende Selektion von Homologen einer Desaturase verwendet werden. Bei einer Ausführungsform kann eine Bank von Fragmenten der kodierenden Sequenz durch Behandeln eines doppelsträngigen PCR-Fragmentes einer kodierenden Desaturase-Sequenz mit einer Nuklease unter Bedingungen, unter denen Doppelstrangbrüche nur etwa einmal pro Molekül erfolgen, Denaturieren der doppelsträngigen DNA, Renaturieren der DNA unter Bildung doppelsträngiger DNA, welche Sense/Antisense-Paare von verschiedenen Produkten mit Doppelstrangbrüchen umfassen kann, Entfernen einzelsträngiger Abschnitte aus neu gebildeten Duplices durch Behandlung mit S1-Nuklease und Ligieren der resultierenden Fragmentbank in einen Expressionsvektor erzeugt werden. Mit diesem Verfahren kann eine Expressionsbank hergeleitet werden, die N-terminale, C-terminale und interne Fragmente der Desaturase verschiedener Größen kodiert.

Im Fachgebiet sind mehrere Techniken für das Sichten von Genprodukten in kombinatorischen Banken, die durch Punktmutationen oder Verkürzung hergestellt worden sind, und für das Sichten von cDNA-Banken nach Genprodukten mit einer ausgewählten Eigenschaft bekannt. Diese Techniken lassen sich an das schnelle Sichtung der Genbanken anpassen, die durch kombinatorische Mutagenese von Desaturase-Homologen erzeugt worden sind. Die am häufigsten verwendeten Techniken zum Sichtung großer Genbanken, die einer Analyse mit hohem Durchsatz unterworfen werden können, umfassen gewöhnlich das Klonieren der Genbank in replizierbare Expressionsvektoren, Transformieren von geeigneten Zellen mit der resultierenden Vektorenbank und Exprimieren der kombinatorischen Gene unter Bedingungen, unter denen der Nachweis der gewünschten Aktivität die Isolation des Vektors, der das Gen kodiert, dessen Produkt nachgewiesen wurde, erleichtert. Recursive-Ensemble-Mutagenese (REM), eine neue Technik, die die Häufigkeit funktioneller Mutanten in den Banken erhöht, kann in Kombination mit den Sichtungstests zur Identifikation von Desaturase-Homologen verwendet werden (Arkin und Yourvan (1992) Proc. Natl. Acad. Sci. USA 89:7811-7815; Delgrave et al. (1993) Protein Engineering 6(3):327-331).

Eine weitere bekannte Technik zur Veränderung von katalytischen Eigenschaften von Enzymen bzw. deren codierenden Genen ist das "Gen-Shuffling" (siehe z.B. in Stemmer, PNAS 1994, 91: 10747-10751, WO9720078 oder WO9813487), das eine Kombination von Genfragmenten darstellt, wobei diese Neukombination zusätzlich noch durch fehlerhafte Polymerasekettenreaktionen variiert werden kann und somit eine hohe zu testende Sequenzdiversität schafft. Voraussetzung für den Einsatz eines solchen Ansatzes ist jedoch ein geeignetes Screeningsystem, um die erstellte Gendiversität auf Funktionalität zu überprüfen.

Insbesondere für die Sichtung von Desaturaseaktivitäten ist ein Sichtungsverfahren Voraussetzung, das PUFA-abhängig Enzymaktivität(en) erfaßt. Bzgl. Desaturaseaktivitäten mit Spezifität für PUFAs kann man in Mucor-Species, die durch bekannte Transformationsverfahren mit gewünschten Genkonstrukten transformierbar sind, die Toxizität von Arachidonsäure in Anwesenheit eines toxischen Metaboliten (hier: Salicylsäure oder Salicylsäurederivate) nutzen (Eroshin et al., Mikrobiologiya, Vol. 65, No.1 1996, Seiten 31-36), um eine wachstumsbasierte Erstsichtung durchzuführen. Resultierende Klone können dann einer Analyse ihrer Lipidinhaltstoffe mittels Gaschromatographie und Massenspektroskopie unterzogen werden, um Edukte und Produkte in Art und Menge zu erfassen.

Bei einer weiteren Ausführungsform können Tests auf Zellbasis zur Analyse einer variegierten Desaturase-Bank unter Verwendung von weiteren im Fachgebiet bekannten Verfahren ausgenutzt werden.

### E. Erfindungsgemäße Verwendungen und Verfahren

Die hier beschriebenen Nukleinsäuremoleküle, Proteine, Proteinhomologen, Fusionsproteine, Primer, Vektoren und Wirtszellen können bei einem oder mehreren der nachstehenden Verfahren verwendet werden: Identifikation von Phaeodactylum und verwandten Organismen, Kartierung der Genome von Organismen, die mit Phaeodactylum tricornutum verwandt sind, Identifikation und Lokalisierung von Phaeodactylum tricornutum-Sequenzen von Interesse, Evolutionsstudien, Bestimmung von Desaturase-Proteinbereichen, die für die Funktion notwendig sind, Modulation einer Desaturase-Aktivität; Modulation des Stoffwechsels einer oder mehrerer Zellmembrankomponenten; Modulation des Transmembrantransports einer oder mehrerer Verbindungen sowie Modulation der zellulären Produktion einer gewünschten Verbindung, wie einer Feinchemikalie. Die erfindungsgemäßen Desaturase-Nukleinsäuremoleküle haben eine Vielzahl von Verwendungen. Sie können zunächst zur Identifikation eines Organismus als Phaeodactylum tricornutum oder als naher Verwandter davon verwendet werden. Sie können auch zur Identifikation des Vorliegens von Phaeodactylum tricornutum oder eines Verwandten davon in einer Mischpopulation von Mikroorganismen verwendet werden. Die Erfindung stellt die Nukleinsäuresequenzen einer Reihe von Phaeodactylum tricornutum-Genen bereit; durch Sondieren der extrahierten genomischen DNA einer Kultur einer einheitlichen oder gemischten Population von Mikroorganismen unter stringenten Bedingungen mit einer Sonde, die einen Bereich eines Phaeodactylum tricornutum -Gens oder von Teilen davon überspannt, das für diesen Organismus einzigartig ist, kann man bestimmen, ob dieser Organismus vorliegt. Phaeodactylum tricornutum selbst werden zur kommerziellen Produktion mehrfach ungesättigter Säuren verwendet und eignen darüber hinaus zur PUFA-Produktion auch in anderen Organismen insbesondere wenn erreicht werden soll, dass resultierende PUFAs auch in die Triacylglycerolfraktion eingebaut werden sollen.

Ferner können die erfindungsgemäßen Nukleinsäure- und Proteinmoleküle als Marker für spezifische Bereiche des Genoms dienen. Dies ist nicht nur zur Kartierung des Genoms, sondern auch für funktionelle Phaeodactylum tricornutum-Proteinen geeignet. Zur Identifikation des Genombereichs, an den ein bestimmtes DNAbindendes Protein von Phaeodactylum tricornutum bindet, könnte das Phaeodactylum tricornutum-Genom zum Beispiel gespalten werden und die Fragmente mit dem DNA-bindenden Protein inkubiert werden. Diejenigen, die das Protein binden, können zusätzlich mit den erfindungsgemäßen Nukleinsäuremolekülen, vorzugsweise mit leicht nachweisbaren Markierungen, sondiert werden; die Bindung eines solchen Nukleinsäuremoleküls an das Genomfragment ermöglicht die Lokalisierung des Fragments auf der Genomkarte von Phaeodactylum tricornutum und erleichtert, wenn dies mehrmals mit unterschiedlichen Enzymen durchgeführt wird, eine rasche Bestimmung der Nukleinsäuresequenz, an die das Protein bindet. Die erfindungsgemäßen Nukleinsäuremoleküle können zudem ausreichend homolog zu den Sequenzen verwandter Arten sein, dass diese Nukleinsäuremoleküle als Marker für die Konstruktion einer genomischen Karte bei verwandten Pilzen oder Algen dienen können.

Die erfindungsgemäßen Desaturase-Nukleinsäuremoleküle eignen sich auch für Evolutions- und Proteinstruktur-Untersuchungen. Die Stoffwechsel- und Transportprozesse, an denen die erfindungsgemäßen Moleküle beteiligt sind, werden von vielen prokaryotischen und eukaryotischen Zellen genutzt; durch Vergleich der Sequenzen der erfindungsgemäßen Nukleinsäuremoleküle mit solchen, die ähnliche Enzyme aus anderen Organismen kodieren, kann der Evolutions-Verwandschaftsgrad der Organismen bestimmt werden. Entsprechend ermöglicht ein solcher Vergleich die Bestimmung, welche Sequenzbereiche konserviert sind und welche nicht, was bei der Bestimmung von Bereichen des Proteins hilfreich sein kann, die für die Enzymfunktion essentiell sind. Dieser Typ der Bestimmung ist für Proteinengineering-Untersuchungen wertvoll und kann einen Hinweis darauf geben, wieviel Mutagenese das Protein tolerieren kann, ohne die Funktion zu verlieren.

Die Manipulation der erfindungsgemäßen Desaturase-Nukleinsäuremoleküle kann zur Produktion von Desaturasen mit funktionellen Unterschieden zu den Wildtyp-Desaturasen führen. Die Effizienz oder Aktivität dieser Proteine kann verbessert sein, sie können in größeren Anzahlen als gewöhnlich in der Zelle zugegen sein, oder ihre Effizienz oder Aktivität kann verringert sein. Verbesserte Effizienz oder Aktivität bedeutet beispielsweise, dass das Enzym eine höhere Selektivität und/oder Aktivität, vorzugsweise eine mindestens 10 % höhere, besonders bevorzugt eine mindestens 20 % höhere Aktivität, ganz besonders bevorzugt eine mindestens 30 % höhere Aktivität als das ursprüngliche Enzym aufweist.

Es gibt eine Reihe von Mechanismen, durch die die Veränderung einer erfindungsgemäßen Desaturase die Ausbeute, Produktion und/oder Effizienz der Produktion einer Feinchemikalie, welche ein solches verändertes Protein enthält, direkt beeinflussen kann. Die Gewinnung von Feinchemikalien-Verbindungen aus Kulturen von Ciliaten, Algen oder Pilzen im großen Maßstab ist signifikant verbessert, wenn die Zelle die gewünschten Verbindungen sezerniert, da diese Verbindungen aus dem Kulturmedium (im Gegensatz zur Extraktion aus der Masse der gezüchteten Zellen) leicht gereinigt werden können. Ansonsten lässt sich die Reinigung verbessern, wenn die Zelle in vivo Verbindungen in einem spezialisierten Kompartiment mit einer Art Konzentrationsmechanismus speichert. Bei Pflanzen, die Desaturasen exprimieren, kann ein gesteigerter Transport zu besserer Verteilung innerhalb des Pflanzengewebes und der -organe führen. Durch Vergrößern der Anzahl oder der Aktivität von Transportermolekülen, welche Feinchemikalien aus der Zelle exportieren, kann es möglich sein, die Menge der produzierten Feinchemikalie, die im extrazellulären Medium zugegen ist, zu steigern, wodurch Ernte und Reinigung oder bei Pflanzen eine effizientere Verteilung erleichtert werden. Zur effizienten Überproduktion einer oder mehrerer Feinchemikalien sind dagegen erhöhte Mengen an Cofaktoren, Vorläufermolekülen und Zwischenverbindungen für die geeigneten Biosynthesewege erforderlich. Durch Vergrößern der Anzahl und/oder der Aktivität von Transporterproteinen, die am Import von Nährstoffen, wie Kohlenstoffquellen (d.h. Zuckern), Stickstoffquellen (d.h. Aminosäuren, Ammoniumsalzen), Phosphat und Schwefel, beteiligt sind, kann man die Produktion einer Feinchemikalie aufgrund der Beseitigung aller Einschränkungen des Nährstoffangebots beim Biosyntheseprozess verbessern. Fettsäuren, wie PUFAs, und Lipide, die PUFAs enthalten, sind selbst wünschenswerte Feinchemikalien; durch Optimieren der Aktivität oder Erhöhen der Anzahl einer oder mehrerer erfindungsgemäßer Desaturasen, die an der Biosynthese dieser Verbindungen beteiligt sind, oder durch Stören der Aktivität einer oder mehrerer Desaturasen, die am Abbau dieser Verbindungen beteiligt sind, kann man somit die Ausbeute, Produktion und/oder Effizienz der Produktion von Fettsäure- und Lipidmoleküle in Ciliaten, Algen, Pflanzen, Pilzen, Hefen oder anderen Mikroorganismen steigern.

Die Manipulation eines oder mehrerer erfindungsgemäßer Desaturase-Gene kann ebenfalls zu Desaturasen mit veränderten Aktivitäten führen, welche die Produktion einer oder mehrerer gewünschter Feinchemikalien aus Algen, Pflanzen, Ciliaten oder Pilzen indirekt beeinflussen. Die normalen biochemischen Stoffwechselprozesse führen z.B. zur Produktion einer Vielzahl an Abfallprodukten (z.B. Wasserstoffperoxid und andere reaktive Sauerstoffspezies), die diese Stoffwechselprozesse aktiv stören können (z.B. nitriert Peroxynitrit bekanntlich Tyrosin-Seitenketten, wodurch einige Enzyme mit Tyrosin im aktiven Zentrum inaktiviert werden (Groves, J.T. (1999) Curr. Opin. Chem. Biol. 3(2);226-235)). Diese Abfallprodukte werden zwar üblicherweise ausgeschieden, aber die zur fermentativen Produktion im großen Maßstab verwendeten Zellen werden für die Überproduktion einer oder mehrerer Feinchemikalien optimiert und können somit mehr Abfallprodukte produzieren als für eine Wildtypzelle üblich. Durch Optimieren der Aktivität einer oder mehrerer erfindungsgemäßer Desaturasen, die am Export von Abfallmolekülen beteiligt sind, kann man die Lebensfähigkeit der Zelle verbessern und eine effiziente Stoffwechselaktivität aufrechterhalten. Auch das Vorliegen hoher intrazellulärer Mengen der gewünschten Feinchemikalie kann tatsächlich für die Zelle toxisch sein, so dass man durch Steigern der Fähigkeit der Zelle zur Sekretion dieser Verbindungen die Lebensfähigkeit der Zelle verbessern kann.

Die erfindungsgemäßen Desaturasen können ferner so manipuliert sein, dass die relativen Mengen verschiedener Lipid- und Fettsäuremoleküle verändert werden. Dies kann eine entscheidende Auswirkung auf die Lipidzusammensetzung der Zellmembran haben. Da jeder Lipidtyp unterschiedliche physikalische Eigenschaften hat, kann eine Veränderung der Lipidzusammensetzung einer Membran die Membranfluidität signifikant verändern. Änderungen der Membranfluidität können den Transport von Molekülen über die Membran beeinflussen, was, wie vorstehend erläutert, den Export von Abfallprodukten oder der produzierten Feinchemikalie oder den Import notwendiger Nährstoffe modifizieren kann. Diese Änderungen der Membranfluidität können auch die Integrität der Zelle entscheidend beeinflussen; Zellen mit vergleichsweise schwächeren Membranen sind anfälliger gegenüber abiotischen und biotischen Stressbedingungen, welche die Zelle beschädigen oder abtöten können. Durch Manipulieren von Desaturasen, die an der Produktion von Fettsäuren und Lipiden für den Membranaufbau beteiligt sind, so dass die resultierende Membran eine Membranzusammensetzung hat, die für die in den Kulturen, die zur Produktion von Feinchemikalien verwendet werden, herrschenden Umweltbedingungen empfänglicher sind, sollte ein größerer Anteil der Zellen überleben und sich vermehren. Größere Mengen an produzierenden Zellen sollten sich in größeren Ausbeuten, höherer Produktion oder Effizienz der Produktion der Feinchemikalie aus der Kultur manifestieren.

Die vorstehend genannten Mutagenesestrategien für Desaturasen, die zu erhöhten Ausbeuten einer Feinchemikalie führen sollen, sollen nicht beschränkend sein; Variationen dieser Strategien sind dem Fachmann leicht ersichtlich. Unter Verwendung dieser Mechanismen und mithilfe der hier offenbarten Mechanismen können die erfindungsgemäßen Nukleinsäure- und Proteinmoleküle zur Erzeugung von Algen, Ciliaten, Pflanzen, Tieren, Pilzen oder anderen Mikroorganismen, wie C. glutamicum, verwendet werden, die mutierte Desaturase-Nukleinsäure- und Proteinmoleküle exprimieren, so dass die Ausbeute, Produktion und/oder Effizienz der Produktion einer gewünschten Verbindung verbessert wird. Diese gewünschte Verbindung kann ein beliebiges natürliches Produkt von Algen, Ciliaten, Pflanzen, Tieren, Pilzen oder Bakterien sein, welches die Endprodukte von Biosynthesewegen und Zwischenprodukte natürlich vorkommender Stoffwechselwege umfasst, sowie Moleküle, die im Stoffwechsel dieser Zellen nicht natürlich vorkommen, die jedoch von den erfindungsgemäßen Zellen produziert werden.

Eine weitere erfindungsgemäße Ausführungsform ist ein Verfahren zur Produktion von PUFAs, wobei das Verfahren das Züchten eines Organismus, der eine erfindungsgemäße Nukleinsäure, ein erfindungsgemäßes Genkonstrukt oder einen erfindungsgemäßen Vektor umfasst, welche ein Polypeptid kodieren, das C₁₈-, C₂₀- oder C₂₂-Fettsäuren mit mindestens zwei Doppelbindungen im Fettsäuremolekül um mindestens zwei Kohlenstoffatome unter Bedingungen, unter denen PUFAs in dem Organismus produziert werden, verlängert, umfasst. Durch dieses Verfahren hergestellte PUFAs lassen sich durch Ernten der Organismen entweder aus der Kultur, in der sie wachsen, oder von dem Feld, Aufbrechen und/oder Extrahieren des geernteten Materials mit einem organischen Lösungsmittel isolieren. Aus diesem Lösungsmittel kann das Öl, das Lipide, Phospholipide, Sphingolipide, Glycolipide, Triacylglycerine und/oder freie Fettsäuren mit höherem Gehalt an PUFAs enthält, isoliert werden. Durch basische oder saure Hydrolyse der Lipide, Phospholipide, Sphingolipide, Glycolipide, Triacylglycerine können die freien Fettsäuren mit höherem Gehalt an PUFAs isoliert werden. Ein höherer Gehalt an PUFAs bedeutet mindestens 5 %, vorzugsweise 10 %, besonders bevorzugt 20 %, ganz besonders bevorzugt 40 % mehr PUFAs als der ursprüngliche Organismus, der keine zusätzliche Nukleinsäure, die die erfindungsgemäße Desaturase kodiert, besitzt.

Vorzugsweise sind die durch dieses Verfahren produzierten PUFAs C₁₈- oder C₂₀₋₂₂-Fettsäuremoleküle mit mindestens zwei Doppelbindungen im Fettsäuremolekül, vorzugsweise drei, vier, bei Kombination mit einer weiteren Elongasen und einer Δ-4 Desaturase fünf oder sechs Doppelbindungen. Diese C₁₈- oder C₂₀₋₂₂-Fettsäuremoleküle lassen sich aus dem Organismus in Form eines Öls, Lipids oder einer freien Fettsäure isolieren. Geeignete Organismen sind beispielsweise die vorstehend erwähnten. Bevorzugte Organismen sind transgene Pflanzen.

Eine erfindungsgemäße Ausführungsform sind Öle, Lipide oder Fettsäuren oder Fraktionen davon, die durch das oben beschriebene Verfahren hergestellt worden sind, besonders bevorzugt Öl, Lipid oder eine Fettsäurezusammensetzung, die PUFAs umfassen und von transgenen Pflanzen herrühren.

Eine weitere erfindungsgemäße Ausführungsform ist die Verwendung des Öls, Lipids oder der Fettsäurezusammensetzung in Futtermitteln, Nahrungsmitteln, Kosmetika oder Pharmazeutika.

Ein weiterer Erfindungsgegenstand ist ein Verfahren zur Identifikation eines Antagonisten oder Agonisten von Desaturasen, umfassend
a) in Kontaktbringen der Zellen, die das Polypeptid der vorliegenden Erfindung exprimieren, mit einem Kandidatenstoff;
b) Testen der Desaturaseaktivität;
c) Vergleichen der Desaturaseaktivität mit einer Standardaktivität in Abwesenheit des Kandidatenstoffs, wobei ein Anstieg der Desaturaseaktivität über den Standard anzeigt, daß der Kandidatenstoff ein Agonist und ein Verringerung der Desaturaseaktivität anzeigt, daß der Kandidatenstoff ein Antagonist ist.

Der genannte Kandidatenstoff kann ein chemisch synthetisierter oder mikrobiologisch produzierter Stoff sein und z.B. in Zellextrakten von z.B. Pflanzen, Tieren oder Mikroorganismen auftreten. Weiterhin kann der genannte Stoff zwar im Stand der Technik bekannt sein, aber bisher nicht bekannt sein als die Aktivität der Desaturasen steigernd oder repremierend. Das Reaktionsgemisch kann ein zellfreier Extrakt sein oder eine Zelle oder Zellkultur umfassen. Geeignete Methoden sind dem Fachmann bekannt und werden z.B. allgemein beschrieben in Alberts, Molecular Biology the cell, 3rd Edition (1994), z.B. Kapitel 17. Die genannten Stoffe können z.B. zu dem Reaktionsgemisch oder dem Kulturmedium zugegeben werden oder den Zellen injiziert werden oder auf eine Pflanze gesprüht werden.

Wenn eine Probe, die ein nach der erfindungsgemäßen Methode aktiven Stoff beinhaltet, identifiziert wurde, dann ist es entweder möglich, den Stoff direkt von der ursprünglichen Probe zu isolieren oder man kann die Probe in verschiedene Gruppen teilen, z.B. wenn sie aus einer Vielzahl von verschiedenen Komponenten besteht, um so die Zahl der verschiedenen Substanzen pro Probe zu reduzieren und dann das erfindungsgemäße Verfahren mit einer solchen "Unterprobe" der ursprünglichen Probe zu wiederholen. Abhängig von der Komplexität der Probe können die oben beschriebenen Schritte mehrmals wiederholt werden, vorzugsweise bis die gemäß der erfindungsgemäßen Methode identifizierte Probe nur noch eine geringe Anzahl von Substanzen oder nur noch eine Substanz umfaßt. Vorzugsweise wird der gemäß der erfindungsgemäßen Methode identifizierte Stoff oder Derivate davon weiter formuliert, so, daß er für die Anwendung in der Pflanzenzüchtung oder Pflanzenzell- oder Gewebekultur geeignet ist.

Die Stoffe, die gemäß dem erfindungsgemäßen Verfahren getestet und identifiziert wurden, können sein: Expressionsbibliotheken, z.B. cDNA-Expressionsbibliotheken, Peptide, Proteine, Nukleinsäuren, Antikörper, kleine organische Stoffe, Hormone, PNAs oder ähnliches (Milner, Nature Medicin 1 (1995), 879-880; Hupp, Cell. 83 (1995), 237-245; Gibbs, Cell. 79 (1994), 193-198 und darin zitierte Referenzen). Diese Stoffe könne auch funktionelle Derivate oder Analogon der bekannten Inhibitoren oder Aktivatoren sein. Verfahren zur Herstellung von chemischen Derivaten oder Analogon sind dem Fachmann bekannt. Die genannten Derivate und Analogon können gemäß Verfahren nach dem Stand der Technik getestet werden. Weiterhin kann computergestütztes Design oder Peptidomimetics zur Herstellung geeigneter Derivate und Analogon verwendet werden. Die Zelle oder das Gewebe, die/das für das erfindungsgemäße Verfahren verwendet werden kann, ist vorzugsweise eine erfindungsgemäße Wirtszelle, Pflanzenzelle oder ein Pflanzengewebe, wie in den oben genannten Ausführungsformen beschrieben.

Entsprechend betrifft die vorliegende Erfindung auch einen Stoff, der gemäß den vorstehenden erfindungsgemäßen Verfahren identifiziert wurde. Der Stoff ist z.B. ein Homolog der erfindungsgemäßen Desaturasen. Homologe der Desaturasen können durch Mutagenese, z.B. durch Punktmutation oder Deletion der Desaturasen, erzeugt werden. Hierin verwendet wird der Begriff "Homolog" als eine variante Form der Desaturasen, die als Agonist oder Antagonist für die Aktivität der Desaturasen wirkt. Ein Agonist kann die im wesentlichen gleiche oder einen Teil der biologischen Aktivität der Desaturasen haben. Ein Antagonist der Desaturasen kann eine oder mehr Aktivitäten der natürlich vorkommenden Formen der Desaturasen inhibieren, z.B. kompetitiv an ein *Downstream* oder *Upstream* gelegenes Mitglied der Fettsäuresynthese-Stoffwechselwege, die die Desaturasen einschließen, binden oder an Desaturasen binden und dabei die Aktivität reduzieren oder inhibieren.

Außerdem betrifft die vorliegende Erfindung auch ein Antikörper oder ein Fragment davon, wie sie hierin beschrieben werden, der die Aktivität der erfindungsgemäßen Desaturasen inhibiert.

Bei einem Aspekt betrifft die vorliegende Erfindung ein Antikörper, der spezifisch den erfindungsgemäßen oben beschriebenen Agonisten oder Antagonisten erkennt bzw. bindet.

Ein weiterer Aspekt betrifft eine Zusammensetzung, die den Antikörper, den nach dem erfindungsgemäßen Verfahren identifizierten Stopp oder das Antisense-Molekül umfaßt.

In einer weiteren Ausführungsform betrifft die vorliegende Erfindung ein Kit, umfassend die erfindungsgemäße Nukleinsäure, das erfindungsgemäße Genkonstrukt, die erfindungsgemäße Aminosäuresequenz, das erfindungsgemäße Antisense-Nukleinsäuremolekül, den erfindungsgemäßen Antikörper und/oder Zusammensetzung, einen Antagonisten oder Agonisten, der nach dem erfindungsgemäßen Verfahren hergestellt wurde, und/oder erfindungsgemäße Öle, Lipide und/oder Fettsäuren oder eine Fraktion davon. Ebenso kann das Kit die erfindungsgemäßen Wirtszellen, Organismen, Pflanzen oder Teile davon, erntbare Teile der erfindungsgemäßen Pflanzen oder Vermehrungsmaterial oder aber auch den erfindungsgemäßen Antagonisten oder Agonisten umfassen. Die Komponenten des Kits der vorliegenden Erfindung können in geeigneten Containern, beispielsweise mit oder in Puffern oder anderen Lösungen verpackt sein. Ein oder mehr der genannten Komponenten können in ein und demselben Container verpackt sein. Zusätzlich oder alternativ können ein oder mehr der genannten Komponenten z.B. auf einer festen Oberfläche adsorbiert sein, z.B. Nitrozellulosefilter, Glasplatten, Chips, Nylonmembranen oder Mikrotiterplatten. Das Kit kann für jede der hierin beschriebenen Methoden und Ausführungsformen verwendet werden, z.B. für die Produktion von Wirtszellen, transgenen Pflanzen, zur Detektion von homologen Sequenzen, zur Identifikation von Antagonisten oder Agonisten usw. Weiterhin kann das Kit Anleitungen für die Verwendung des Kits für eine der genannten Anwendungen enthalten.

Diese Erfindung wird durch die nachstehenden Beispiele weiter veranschaulicht, die nicht als beschränkend aufgefaßt werden sollten. Der Inhalt sämtlicher in dieser Patentanmeldung zitierten Literaturstellen, Patentanmeldungen, Patente und veröffentlichten Patentanmeldungen ist hier durch Bezugnahme aufgenommen.

### Beispielteil

### Beispiel 1: Allgemeine Verfahren

### a) Allgemeine Klonierungsverfahren:

Klonierungsverfahren, wie beispielsweise Restriktionsspaltungen, Agarosegelelektrophorese, Reinigung von DNA-Fragmenten, Transfer von Nukleinsäuren auf Nitrocellulose- und Nylonmembranen, Verbindung von DNA-Fragmenten, Transformation von Escherichia coli- und Hefe-Zellen, Anzucht von Bakterien und Sequenzanalyse rekombinanter DNA, wurden durchgeführt wie beschrieben in Sambrook et al. (1989) (Cold Spring Harbor Laboratory Press: ISBN 0-87969-309-6) oder Kaiser, Michaelis und Mitchell (1994) "Methods in Yeast Genetics" (Cold Spring Harbor Laboratory Press: ISBN 0-87969-451-3). Die Transformation und Anzucht von Algen, wie Chlorella oder Phaeodactylum werden durchgeführt wie beschrieben von El-Sheekh (1999), Biologia Plantarum 42:209-216; Apt et al. (1996) Molecular and General Genetics 252 (5):872-9.

### b) Chemikalien

Die verwendeten Chemikalien wurden, wenn im Text nicht anders angegeben, in p. A.-Qualität von den Firmen Fluka (Neu-Ulm), Merck (Darmstadt), Roth (Karlsruhe), Serva (Heidelberg) und Sigma (Deisenhofen) bezogen. Lösungen wurden unter Verwendung von reinem pyrogenfreiem Wasser, im nachstehenden Text als H₂O bezeichnet, aus einer Milli-Q-Wassersystem-Wasserreinigungsanlage (Millipore, Eschborn) hergestellt. Restriktionsendonukleasen, DNA-modifizierende Enzyme und molekularbiologische Kits wurden bezogen von den Firmen AGS (Heidelberg), Amersham (Braunschweig), Biometra (Göttingen), Boehringer (Mannheim), Genomed (Bad Oeynhausen), New England Biolabs (Schwalbach/ Taunus), Novagen (Madison, Wisconsin, USA), Perkin-Elmer (Weiterstadt), Pharmacia (Freiburg), Qiagen (Hilden) und Stratagene (Amsterdam, Niederlande). Wenn nicht anders angegeben, wurden sie nach den Anweisungen des Herstellers verwendet.

### c) Zellmaterial

Die erfindungsgemäßen isolierten Nukleinsäuresequenzen sind im Genom eines Phaeodactylum tricornutum UTEX646-Stammes enthalten, der über die Algensammlung der University of Texas, Austin verfügbar ist.

Phaeodactylum tricornutum wurde bei 25°C mit einem Licht/Dunkel Rhythmus von 14:10 Stunden bei 22°C und 35 microEinstein (entspricht micromol Photonen pro Quadratmeter und Sekunde) in Glasröhren kultiviert, die von unten mit Luft begast wurden.

Als Kulturmedium für Phaeodactylum tricornutum wurde das f/2 Kulturmedium mit 10 % organischen Medium nach Guillard, R.R.L. verwendet (1975; Culture of phytoplankton for feeding marine invertebrates. In: Smith, W.L. and Chanley, M.H. (Eds.) Culture of marine Invertebrate animals, NY Plenum Press, pp. 29-60.): Es enthält
995,5 ml Seewasser (artifiziell)
1 ml NaNO₃ (75 g/l), 1 ml NaH₂PO₄ (5 g/l), 1 ml Spurenelementelösung, 1 ml Tris/Cl pH 8.0, 0.5 ml f/2 Vitaminlösung
Spurenelementelösung: Na₂EDTA (4,36 g/l), FeCl₃ (3,15 g/l), Primäre Spurenelemente: CuSO4 (10 g/l), ZnSO₄ (22 g/l), CoCl₂ (10 g/l), MnCl₂ (18 g/l), NaMoO4 (6,3 g/l)
f/2 Vitaminlösung: Biotin: 10 mg/l, Thiamin 200 mg/l, Vit B12 0,1 mg/l
org-Medium: Na-Acetat (1 g/l), Glucose (6 g/l), Na-Succinat (3 g/l), Bacto-Trypton (4 g/l), Hefe-Extrakt (2 g/l)

### Beispiel: 2 Isolierung von Gesamt-DNA aus Phaeodactylum tricornutum UTEX646 für Hybridisierungsexperimente

Die Einzelheiten der Isolierung von Gesamt-DNA betreffen die Aufarbeitung von Pflanzenmaterial mit einem Frischgewicht von einem Gramm.

CTAB-Puffer: 2 % (Gew./Vol.) N-Acetyl-N,N,N-trimethylammoniumbromid (CTAB); 100 mM Tris-HCl, pH 8,0; 1,4 M NaCl; 20 mM EDTA.

N-Laurylsarkosin-Puffer: 10 % (Gew./Vol.) N-Laurylsarkosin; 100 mM Tris-HCl, pH 8,0; 20 mM EDTA.

Phaeodactylum tricornutum-Zellmaterial wurde unter flüssigem Stickstoff in einem Mörser verrieben, so dass ein feines Pulver erhalten wurde, und in 2 ml-Eppendorfgefäße überführt. Das gefrorene Pflanzenmaterial wurde dann mit einer Schicht von 1 ml Zersetzungspuffer (1 ml CTAB-Puffer, 100 ml N-Laurylsarkosin-Puffer, 20 ml β-Mercaptoethanol und 10 ml Proteinase K-Lösung, 10 mg/ml) überschichtet und eine Stunde unter kontinuierlichem Schütteln bei 60°C inkubiert. Das erhaltene Homogenat wurde in zwei Eppendorfgefäße (2 ml) aufgeteilt und zweimal durch Schütteln mit dem gleichen Volumen Chloroform/Isoamylalkohol (24:1) extrahiert. Zur Phasentrennung wurde eine Zentrifugation bei 8000 x g und RT (= Raumtemperatur = - 23°C) jeweils 15 min lang durchgeführt. Die DNA wurde dann 30 min unter Verwendung von eiskaltem Isopropanol bei -70°C gefällt. Die gefällte DNA wurde bei 10000 g 30 min bei 4°C sedimentiert und in 180 ml TE-Puffer (Sambrook et al., 1989, Cold Spring Harbor Laboratory Press: ISBN 0-87969-309-6) resuspendiert. Zur weiteren Reinigung wurde die DNA mit NaCl (1,2 M Endkonzentration) behandelt und erneut 30 min unter Verwendung des zweifachen Volumens an absolutem Ethanol bei -70°C gefällt. Nach einem Waschschritt mit 70 % Ethanol wurde die DNA getrocknet und anschließend in 50 ml H₂O + RNAse (50 mg/ml Endkonzentration) aufgenommen. Die DNA wurde über Nacht bei 4°C gelöst und die RNAse-Spaltung wurde anschließend 1 Std. bei 37°C durchgeführt. Die Aufbewahrung der DNA erfolgte bei 4°C.

### Beispiel 3: Isolierung von Gesamt-RNA und poly(A)⁺-RNA aus Pflanzen und Phaeodactylum tricornutum

Die Isolierung von Gesamt-RNA aus Pflanzen wie Lein und Raps etc. erfolgt nach einer bei Logemann et al beschriebenen Methode (1987, Anal. Biochem. 163, 21) isoliert. Aus Moos kann die Gesamt-RNA Protonema-Gewebe nach dem GTC-Verfahren (Reski et al., 1994, Mol. Gen. Genet., 244:352-359) gewonnen werden.

### RNA Isolierung aus Phaeodactylum tricornutum:

Tiefgefrorene Algenproben (- 70°C) wurden in einem eiskaltem Mörser unter Flüssigstickstoff zu feinem Pulver zerreiben. 2 Volumen Homogenisationsmedium (12,024 g Sorbitol, 40,0 ml 1M Tris-HCl, pH 9 (0,2 M); 12,0 ml 5 M NaCl (0,3 M), 8,0 ml 250 mM EDTA, 761,0 mg EGTA, 40,0 ml 10 % SDS wurden auf 200 ml mit H₂O aufgefüllt und der pH auf 8,5 eingestellt) und 4 Volumen Phenol mit 0,2 % Mercaptoethanol wurden bei 40 bis 50°C unter gutem Mischen zu gefrorenem Zellpulver gegeben. Danach wurden 2 Volumen Chloroform hinzufügen und für 15 min kräftig gerührt. Es wurde 10 min bei 10000 g zentrifugiert und die wässrige Phase mit Phenol/Chloroform (2 Vol) und abschließend mit Chloroform extrahiert.

Das erhaltene Volumen der wässrigen Phase wurde mit 1/20 Vol 4 M Na-Acetat (pH 6) und 1 Vol Isopropanol (eiskalt) versetzet und die Nukleinsäuren bei -20°C über Nacht (= ÜN) gefällt. Anschließend wurde 30 min bei 10000 g zentrifugiert und der Überstand abgesogen. Es folgte ein Waschschritt mit 70 % EtOH und erneute Zentrifugation. Das Sediment wurde in Tris-Borat-Puffer (80 mM Tris-Borat-Puffer, 10 mM EDTA, pH 7,0) aufgenommen. Dann wurde der Überstand mit 1/3 Vol 8 M LiCl versetzt, gemischt 30 min bei 4°C inkubiert. Nach erneutem zentrifugieren wurde das Sediment mit 70 % Ethanol gewaschen, zentrifugiert und das Sediment in RNAse-freiem Wasser gelöst.

Die Isolierung von poly(A)⁺-RNA erfolgte unter Verwendung von Dyna Beads^{®} (Dynal, Oslo, Finnland) nach den Anweisungen im Protokoll des Herstellers.

Nach der Bestimmung der RNA- oder poly(A)⁺-RNA-Konzentration wurde die RNA durch Zugabe von 1/10 Volumina 3 M Natriumacetat, pH 4,6, und 2 Volumina Ethanol gefällt und bei -70°C aufbewahrt.

Für die Analyse wurden jeweils 20 µg RNA in einem Formaldehydhaltigen 1,5%igen Agarosegel aufgetrennt und auf Nylon Membranen (Hybond, Amersham) überführt. Der Nachweis spezifischer Transkripte wurde wie bei Amasino beschrieben durchgeführt ((1986) Anal. Biochem. 152, 304)).

### Beispiel 4: Konstruktion der cDNA-Bank

Zur Konstruktion der cDNA-Bank aus Phaeodactylum tricornutum wurde die Erststrangsynthese unter Verwendung von Reverser Transkriptase aus Maus-Leukämie-Virus (Roche, Mannheim, Deutschland) und Oligo-d(T)-Primern, die Zweitstrangsynthese durch Inkubation mit DNA-Polymerase I, Klenow-Enzym und RNAse H-Spaltung bei 12°C (2 Std.), 16°C (1 Std.) und 22°C (1 STd.) erzielt. Die Reaktion wurde durch Inkubation bei 65°C (10 min) gestoppt und anschließend auf Eis überführt. Doppelsträngige DNA-Moleküle wurde mit T4-DNA-Polymerase (Roche, Mannheim) bei 37°C (30 min) mit glatten Enden versehen. Die Nukleotide wurden durch Phenol/Chloroform-Extraktion und Sephadex-G50-Zentrifugiersäulen entfernt. EcoRI/XhoI-Adapter (Pharmacia, Freiburg, Deutschland) wurden mittels T4-DNA-Ligase (Roche, 12°C, über Nacht) an die cDNA-Enden ligiert, mit XhoI nachgeschnitten und durch Inkubation mit Polynukleotidkinase (Roche, 37°C, 30 min) phosphoryliert. Dieses Gemisch wurde der Trennung auf einem Low-Melting-AgaroseGel unterworfen. DNA-Moleküle über 300 Basenpaaren wurden aus dem Gel eluiert, Phenol-extrahiert, auf Elutip-D-Säulen (Schleicher und Schüll, Dassel, Deutschland) konzentriert und an Vektorarme ligiert und in lambda-ZAP-Express-Phagen unter Verwendung des Gigapack Gold-Kits (Stratagene, Amsterdam, Niederlande) verpackt, wobei Material des Herstellers verwendet und seine Anweisungen befolgt wurden.

### Beispiel 5: DNA-Sequenzierung und Computeranalyse

cDNA-Banken, wie im Beispiel 4 beschrieben, wurden zur DNA-Sequenzierung nach Standardverfahren, insbesondere durch das Kettenterminationsverfahren unter Verwendung des ABI PRISM Big Dye Terminator Cycle Sequencing Ready Reaction-Kit (Perkin-Elmer, Weiterstadt, Deutschland), verwendet. Die Sequenzierung zufälliger, vereinzelter Klone wurde anschließend an die präparative Plasmidgewinnung aus cDNA-Banken über in vivo-Massenexcision und Retransformation von DH10B auf Agarplatten durchgeführt (Einzelheiten zu Material und Protokoll von Stratagene, Amsterdam, Niederlande). Plasmid-DNA wurde aus über Nacht gezüchteten E. coli-Kulturen, die in Luria-Brühe mit Ampicillin (siehe Sambrook et al. (1989) (Cold Spring Harbor Laboratory Press: ISBN 0-87969-309-6)) gezüchtet worden waren, an einem Qiagen-DNA-Präparations-Roboter (Qiagen, Hilden) nach den Protokollen des Herstellers präpariert. Sequenzierprimer mit den folgenden Nukleotidsequenzen wurden verwendet:
5'-CAGGAAACAGCTATGACC-3'
5'-CTAAAGGGAACAAAAGCTG-3'
5'-TGTAAAACGACGGCCAGT-3'

Die Sequenzen wurden unter Verwendung des Standard-Softwarepakets EST-MAX, das kommerziell von Bio-Max (München, Deutschland) geliefert wird, prozessiert und annotiert. Durch Nutzung von Vergleichsalgorithmen und unter Verwendung der in SEQ ID NO: 8 dargestellten Suchsequenz wurde mithilfe des BLAST-Programms nach homologen Genen gesucht (Altschul et al. (1997) "Gapped BLAST and PSI-BLAST: a new generation of protein database search programs", Nucleic Acids Res. 25:3389-3402.). Zwei Sequenzen aus Phaeodactylum tricornutum mit Homologien zur Suchsequenz aus Physcomitrella patens wurden eingehender charakterisiert.

### Beispiel 5a: Isolation von Desaturasen aus Phaeodactylum tricornutum über Polymerase Kettenreaktion mithilfe degenerierter Oligonukleotide:

Mithilfe von publizierten Desaturasen können Motive identifiziert werden, die für Δ-5 und Δ-6 Desaturasen typisch sind. Im folgenden sind Oligonukleotidsequenzen mit möglichen Variationen dargestellt. Unter der Oligonukleotidsequenz ist im Ein-Buchstabencode die Aminosäure dargestellt, von der die Basenkombination abgeleitet werden kann. Z.B. bedeutet A/G, daß an dieser Position bei der Synthese des Bausteins statistisch gleichverteilt entweder ein A oder ein G in das Oligonukleotid eingebaut wird, da das von der korrespondierenden Aminosäure abgeleitete Basentriplett entweder ein AAA oder ein AAG sein kann. Die DNA Sequenz kann auch ein Inosin (i) enthalten, wenn die Bestimmung einer Base an dieser Position aufgrund des genetischen Codes drei oder vier unterschiedliche Basen erlaubt. Folgende Sequenzen und Primer können verwendet werden:
5'-Vorwärts-Primer: 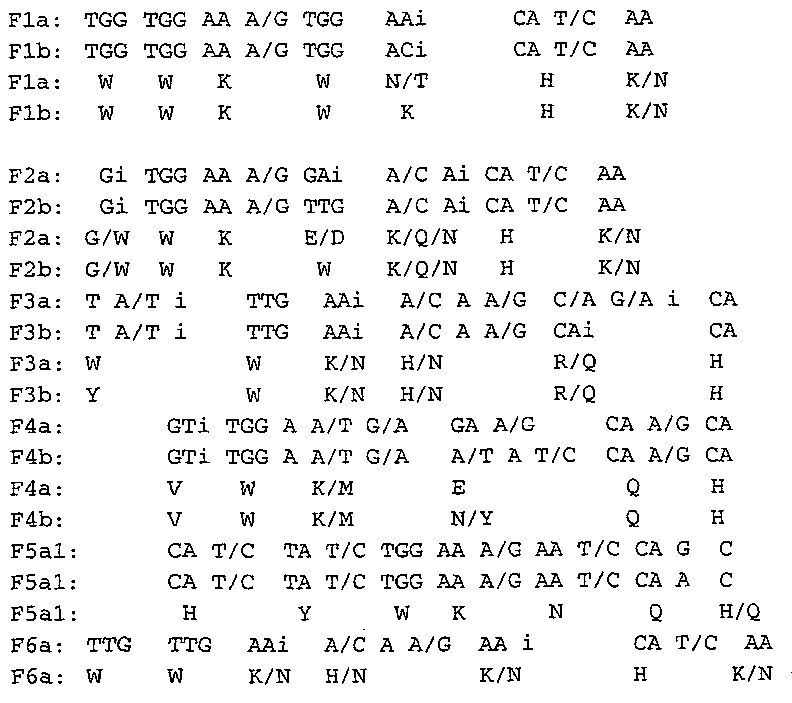
3'- Reverse Primer

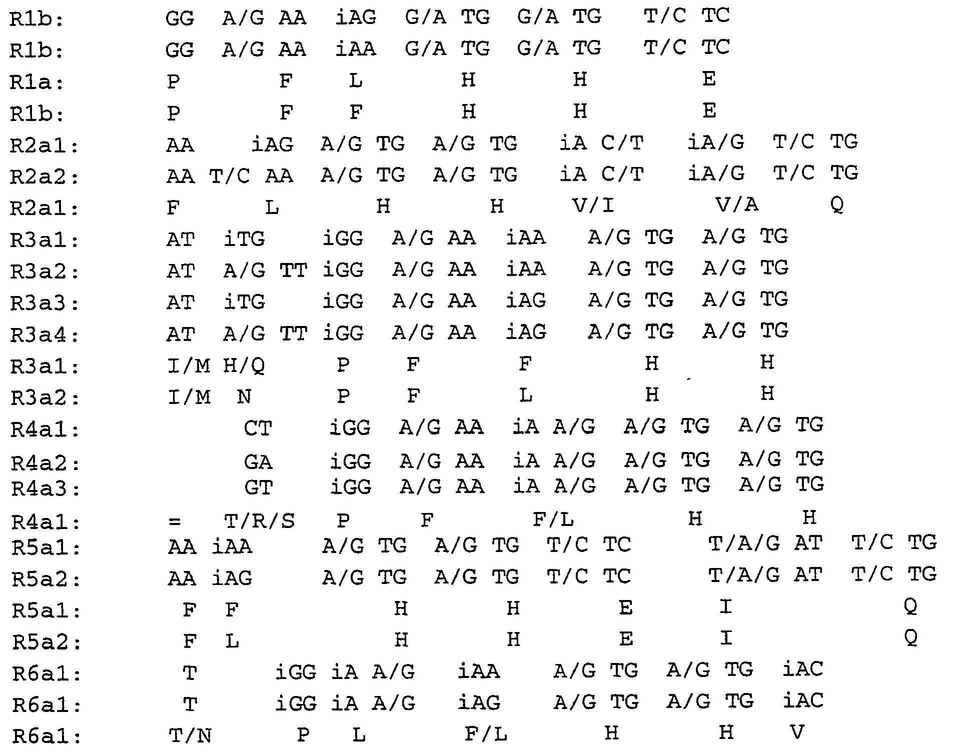

Aufgrund verschiedener Variationsmöglichkeiten sind viele abgeleitete Oligonukleotide möglich, jedoch überraschenderweise gefunden wurde, dass dargestellte Oligonukleotide besonders zur Isolation von Desaturasen geeignet sein können.

Die Primer können in allen Kombinationen für Polymerase Kettenreaktionen eingesetzt werden. Mithilfe einzelner Kombinationen konnten Desaturase-Fragmente isoliert, wenn nachfolgende Bedingungen berücksichtigt wurden: Für PCR Reaktionen wurden jeweils 10 nMol Primer und 10 ng einer durch in vivo Excision gewonnenen Plasmidbank eingesetzt. Die Plasmidbank konnte nach Protokollen des Herstellers (Stratagene) aus der Phagenbank .isoliert werden. Die PCR-Reaktion wurde in einem Thermocycler (Biometra) mit der Pfu-DNA-Polymerase (Stratagene) und dem folgenden Temperaturprogramm durchgeführt: 3 min bei 96°C, gefolgt von 35 Zyklen mit 30 s bei 96°C, 30 s bei 55°C und 1 min bei 72°C. Dabei wurde die Anlagerungstemperatur nach dem ersten Schritt von 55°C schrittweise um je 3°C erniedrigt und nach dem fünften Zyklus eine Anlagerungstemperatur von 40°C beibehalten. Letztlich wurde ein Zyklus mit 10 min bei 72°C durchgeführt und der Ansatz durch Kühlen auf 4°C beendet.

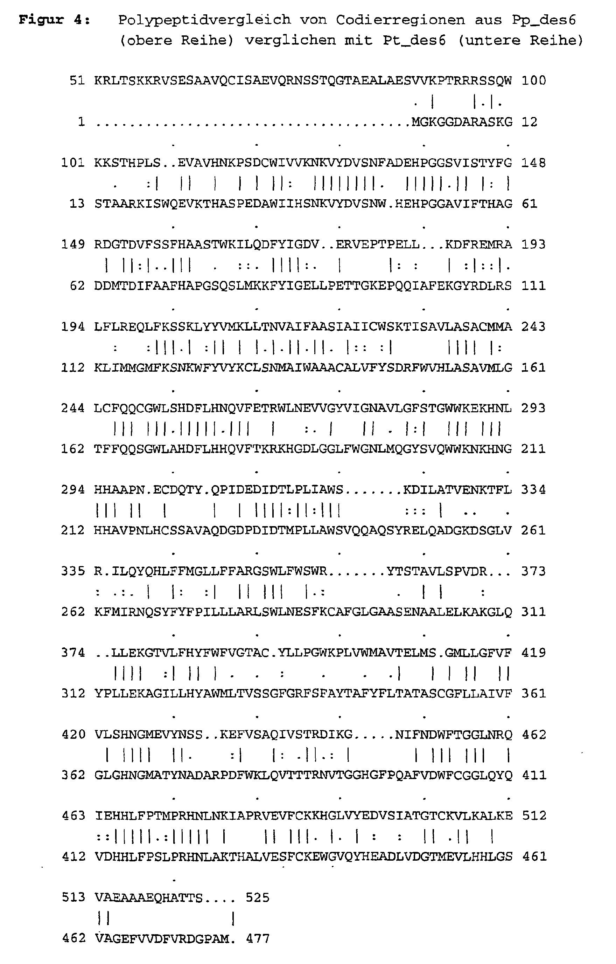

Die Primerkombination F6a und R4a2 sind im Text unterstrichen gekennzeichnet und konnten erfolgreich zur Isolierung eines Desaturasefragmentes genutzt werden. Das resultierende Fragment konnte durch Sequenzierung verifiziert werden und zeigte Homologien zu einer Desaturase mit der Genbank Accession Nr. T36617 aus Streptomyces coelicolor. Die Homologie wurde mithilfe des BLASTP Programmes erhalten. Der Vergleich ist in Figur 4 dargestellt. Es ergaben sich Identitäten von 34 % und eine Homologie von 43 % zu Sequenz T36617. Das DNA-Fragment wurde gemäß Beispiel 7 in einem Hybridisierungsexperiment zur Isolierung eines Vollängengens nach Standardbedingungen erfindungsgemäß eingesetzt.

Die Codierregion einer so isolierten DNA-Sequenz wurde durch Übersetzung des genetischen Codes in eine Polypeptidsequenz erhalten. In SEQ ID NO: 3 ist eine 1434 Basenpaare lange Sequenz dargestellt, die durch beschriebenes Verfahren isoliert werden konnte. Die Sequenz besitzt ein Startcodon in Position 1 bis 3 und ein Stopcodon in Position 1432-1434 und konnte in ein 477 Aminosäuren langes Polypeptid übersetzt werden. Durch Vergleich mit einer in WO 98 46763 beschriebenen Gensequenz wurde gefunden, dass ein nicht identisches aber homologes Fragment aus Phaeodactylum tricornutum codierend für 87 Aminosäuren vorbeschrieben wurde. Jedoch offenbart WO 98/46763 weder eine vollständige, funktionell aktive Desaturase noch Positions- oder Substratspezifiät. Dies wird auch dadurch deutlich, dass sowohl Homologien zur Δ-5, als auch zur Δ-6-Desaturase aus Mortierella alpina berichtet werden, ohne eine genaue Funktion festzulegen. Die erfindungsgemäße Sequenz hingegen codiert für eine funktionell aktive Δ-6-Acyl Lipid Desaturase.

### Beispiel 6: Identifizierung von DNA Sequenzen codierend für Desaturasen aus Phaeodactylum tricornutum

Die Volllängensequenz der Δ-6-Acyl Lipid Desaturase Pp_des6 AJ222980 (NCBI Genbank Accession Nr.) aus dem Moos Physcomitrella patens (siehe auch Tabelle 1) sowie die Δ-12-acyl Lipid Desaturase Sequenz (Tabelle 1 siehe Ma_des12) aus Mortierella alpina AF110509 (AF110509 NCBI Genbank Accession Nr.) wurden für Sequenzvergleiche mithilfe des TBLASTN Suchalgorhythmus eingesetzt.

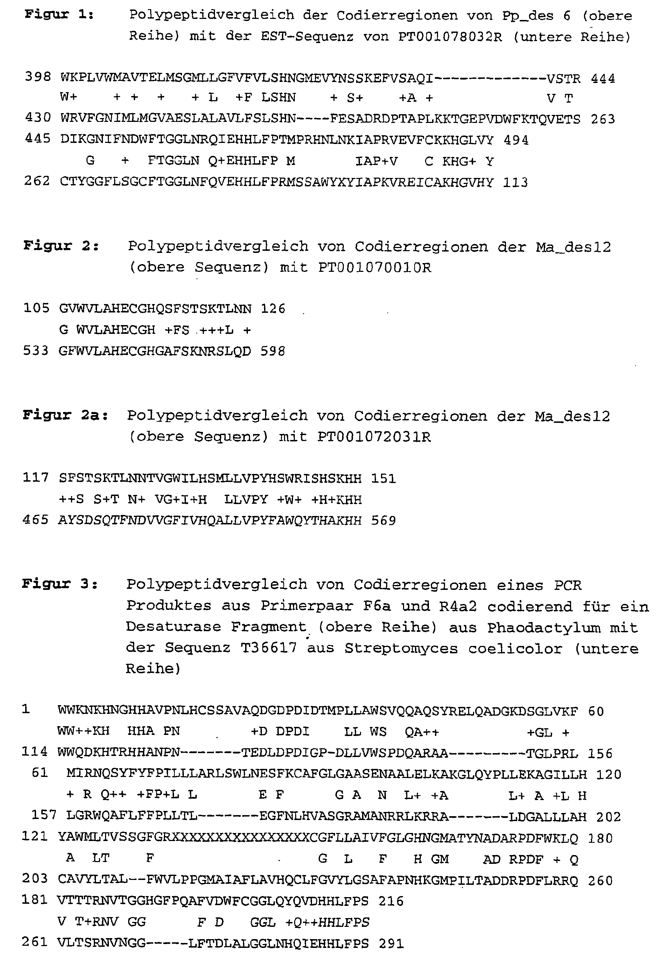

Die EST-Sequenzen PT0010070010R, PT001072031R sowie PT001078032R wurden zunächst aufgrund schwacher Homologien mit den Suchsequenzen aus Physcomitrella und Mortierella unter weiteren Kandidatengenen als Zielgen in Betracht gezogen. In Figur 1 und in Figur 2 sowie Figur 2a ist das Ergebnis der zwei gefundenen est-Sequenzen dargestellt. Die gefundenen Sequenzen sind Teil der erfindungsgemäßen Nukleinsäuren aus SEQ ID NO: 1 (Genname: Pt_des5, eigene Datenbank Nr. der Erfinder PT001078032R), SEQ ID NO: 5. (Genname: Pt_des12, eigene Datenbank NR. der Erfinder PT0010070010R) und SEQ ID NO: 11 (Genname: Pt_des12.2, eigene Datenbank des Erfinders PT001072031R). Buchstaben zeigen identische Aminosäuren an, während das Pluszeichen eine chemisch ähnliche Aminosäure bedeutet. Die Identitäten bzw. Homologien aller erfindungsgemäß gefundener Sequenzen gehen aus Tabelle 2 zusammenfassend hervor.

Desaturasen können Cytochrom b5 Domänen aufweisen, die auch in anderen nicht Desaturasen codierenden Genen vorkommen. Cytochrom b5 Domänen zeigen mithin hohe Homologien an, obwohl es sich um verschiedene Genfunktionen handelt. Desaturasen können schwach konservierter Bereiche lediglich als putative Kandidatengene identifiziert werden und müssen auf die Enzymaktivität und Positionsspezifität der enzymatischen Funktion hin geprüft werden. Beispielsweise zeigen auch verschiedene Hydroxylasen, Acetylenasen und Epoxygenasen ähnlich wie Desaturasen Histidin-Box Motive, so dass eine konkrete Funktion experimentell nachgewiesen werden muß und zusätzlich die Verifizierung der Doppelbindung erst eine sichere Enzymaktivität und Positionsspezifität einer Desaturase ermöglicht. Überraschenderweise wurde gefunden, dass erfindungsgemäße Δ-6- und Δ-5- Desaturase besonders geeignete Substratspezifitäten aufweisen und besonders geeignet sind, um in Kombination mit einer Δ-6-Elongase aus Physcomitrella zur Produktion von polyungesättigten Fettsäuren wie Arachidonsäure, Eicosapentaensäure und Docosahexaensäure genutzt werden können.

Die Sequenzierung des vollständigen cDNA Fragmentes aus Klon PT001078032R ergab eine 1652 Basenpaare lange Sequenz. Die Sequenz codiert für ein Polypeptid von 469 Aminosäuren dargestellt in SEQ ID NO: 2. Diese wurde erhalten durch Übersetzung des genetischen Codes aus SEQ ID NO: 1 mit einem Startcodon in Basenpaarposition 115-117 und mit einem Stopcodon in Basenpaarposition 1522-1524. Der Klon beinhaltet ein vollständiges Desaturase-Polypeptid, wie aus dem Sequenzvergleich in Figur 3 zu ersehen ist. Striche bedeuten identische Aminosäuren während Doppelpunkte und Einzelpunkte chemisch austauschbare, d.h. chemisch äquivalente Aminosäuren darstellen. Der Vergleich wurde mit der BLOSUM62 Austauschmatrix für Aminosäuren nach Henikoff & Henikoff durchgeführt:((1992) Amino acid substitution matrices from protein blocks. Proc. Natl. Acad.Sci. USA 89: 10915-10919). Verwendete Parameter: Gap Weight: 8; Average Match: 2.912, Length Weight: 2, Average Mismatch: -2.003.

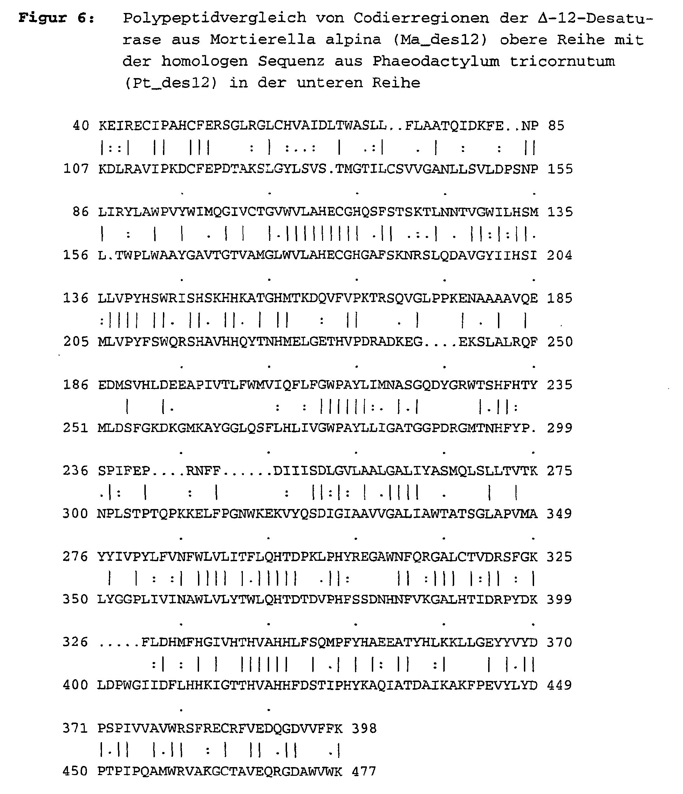

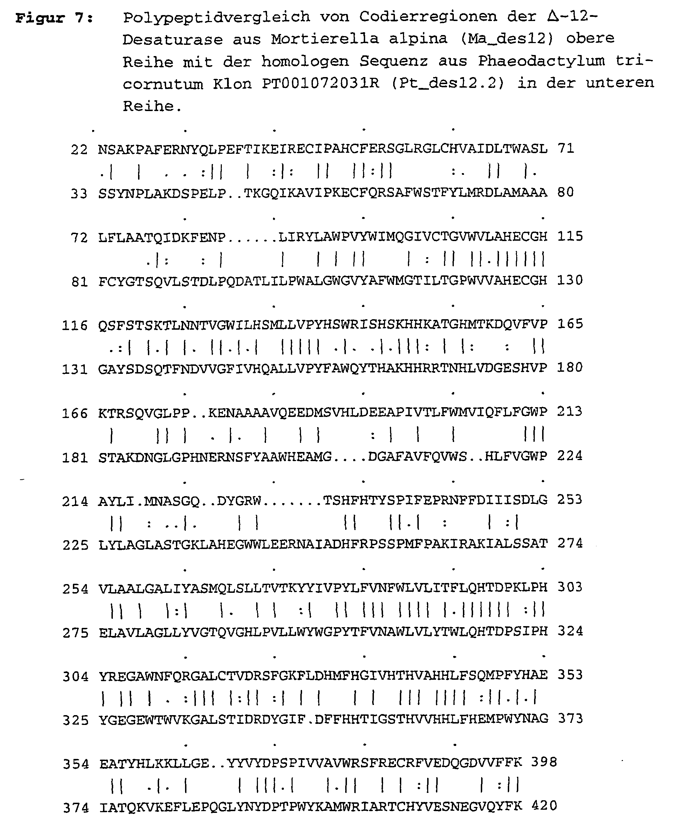

In Figur 6 und Figur 7 ist der Vergleich der MA_des12 Peptidsequenz mit den gefundenen Sequenzen dargestellt.

Die Sequenzierung des vollständigen cDNA Fragmentes aus Klon PT0010070010R ergab eine in SEQ ID NO: 5 dargestellte 1651 Basenpaare lange Sequenz mit einem Startcodon in Position 67-69 und einem Stopcodon in Position 1552-1554. Die erfindungsgemäße Polypetidsequenz ist in SEQ ID NO: 6 dargestellt.

Die Sequenzierung des vollständigen identifizierten cDNA Fragmentes aus Klon PT0010072031R ergab eine in SEQ ID NO: 11 dargestellte 1526 Basenpaare lange Sequenz mit einem Startcodon in Position 92-94 und einem Stopcodon in Position 1400-1402. Die erfindungsgemäße Polypetidsequenz ist in SEQ ID NO: 12 dargestellt.

In Tabelle 2 sind die Identitäten und Homologien erfindungsgemäßer Desaturasen untereinander und mit der Desaturase aus Physcomitrella patens und Mortierella alpina dargestellt. Die Angaben wurden mithilfe des Programms Bestfit unter gegebenen Parametern wie unten definiert als Teilprogramm folgender Software erhalten: Wisconsin Package Version 10.0 (Genetics Computer Group (GCG), Madison, Wisc., USA). Henikoff, S. and Henikoff, J.G. (1992). Amino acid substitution matrices from protein blocks. Proc. Natl. Acad. Sci. USA 89: 10915-10919.

Weiterhin ist in Figur 5 der Vergleich der Δ-6-acyl Lipid Desaturase aus Physcomitrella patens mit der Polypeptidsequenz des Klons Pt_des6 dargestellt.

**Tabelle 2:**

| Homologie / Identität in % | Suchsequenz Pp_des6 | Suchsequenz Ma_des12 |
|---|---|---|
| Pt_des5 | 34.92/26.37 | n.d. |
| Pt_des6 | 50.69/41.06 | n.d. |
| Pt_des12 | n.d. | 48.58/38.92 |
| Pt_des12.2 | n.d. | 48.37/41.60 |

| | | |
|---|---|---|
| n.d. = nicht durchgeführt | | |

Mithilfe des Algorhythmus TBLASTN 2.0.10: Altschul et al 1997, "Gapped BLAST and PSI-BLAST: a new generation of protein database search programs", Nucleic Acids Res. 25:3389-3402 wurden über einen lokalen Datenbankvergleich Sequenzen mit höchster Sequenzhomologie bzw. Identität identifiziert. Die Ergebnisse sind in folgender Tabelle 2A dargestellt.

**Tabelle 2A: Homologe mit den höchsten Sequenzhomologien bzw Identitäten zu erfindungsgemäßen Polypeptidsequenzen aus SEQ ID NO. 2, 4, 6 oder 12**

| Homologie / Identität(%) | Suchsequenz PT001070010R | Suchsequenz PT001072031R | Suchsequenz PT001078032R | Suchsequenz Pt_des6 |
|---|---|---|---|---|
| L26296: Fad2 A. thaliana | 50 % / 37 % | n.d. | n.d. | n.d. |
| U86072 Petroselinum crispum Fad2 | n.d. | 51/40 | n.d. | n.d. |
| AL358652 L. major putative desaturase | n.d. | n.d. | 45/30 | n.d. |
| AB020032 M. alpina delta 6 desaturase | n.d. | n.d. | n.d. | 53/38 |

### Beispiel 7: Identifikation von Genen mittels Hybridisierung

Gensequenzen lassen sich zur Identifikation homologer oder heterologer Gene aus cDNA- oder genomischen Banken verwenden.

Homologe Gene (d.h. Voll-Längen-cDNA-Klone, die homolog sind, oder Homologen) lassen sich über Nukleinsäurehybridisierung unter Verwendung von beispielsweise cDNA-Banken isolieren: Insbesondere zur Isolierung von funktionell aktiven Voll-Längengenen der in SEQ ID NO: 3 gezeigten kann die Methode genutzt werden. Je nach der Häufigkeit des Gens von Interesse werden 100000 bis zu 1000000 rekombinante Bakteriophagen plattiert und auf eine Nylonmembran überführt. Nach der Denaturierung mit Alkali wurde die DNA auf der Membran z.B. durch UV-Vernetzung immobilisiert. Die Hybridisierung erfolgt bei hoch-stringenten Bedingungen. In wässriger Lösung werden die Hybridisierung und die Waschschritte bei einer Ionenstärke von 1 M NaCl und einer Temperatur von 68°C durchgeführt. Hybridisierungssonden wurden z.B. durch Markierung mittels radioaktiver (³²P-) Nicktranskription (High Prime, Roche, Mannheim, Deutschland) hergestellt. Die Signale werden mittels Autoradiographie nachgewiesen.

Partiell homologe oder heterologe Gene, die verwandt, aber nicht identisch sind, lassen sich analog zum oben beschriebenen Verfahren unter Verwendung niedrig-stringenter Hybridisierungs- und Waschbedingungen identifizieren. Für die wässrige Hybridisierung wurde die Ionenstärke gewöhnlich bei 1 M NaCl gehalten, wobei die Temperatur nach und nach von 68 auf 42°C gesenkt wurde.

Die Isolierung von Gensequenzen, die nur zu einer einzelnen Domäne von beispielsweise 10 bis 20 Aminosäuren Homologien aufweisen, lässt sich unter Verwendung synthetischer, radioaktiv markierter Oligonukleotidsonden durchführen. Radioaktiv markierte Oligonukleotide werden mittels Phosphorylierung des 5'-Endes zweier komplementärer Oligonukleotide mit T4-Polynukleotidkinase hergestellt. Die komplementären Oligonukleotide werden aneinander hybridisiert und ligiert, so dass Konkatemere entstehen. Die doppelsträngigen Konkatemere werden beispielsweise durch Nicktranskription radioaktiv markiert. Die Hybridisierung erfolgt gewöhnlich bei niedrig-stringenten Bedingungen unter Verwendung hoher Oligonukleotidkonzentrationen.

### Oligonukleotid-Hybridisierungslösung:

6 x SSC
0,01 M Natriumphosphat
1 mM EDTA (pH 8)
0,5 % SDS
100 mikrog/ml denaturierte Lachssperma-DNA
0,1 % fettarme Trockenmilch

Während der Hybridisierung wird die Temperatur schrittweise auf 5 bis 10°C unter die berechnete Oligonukleotid-Tm oder bis auf Raumtemperatur (bedeutet RT = - 23°C in allen Experimenten, wenn nicht anders angegeben) gesenkt, gefolgt von Waschschritten und Autoradiographie. Das Waschen wird mit extrem niedriger Stringenz durchgeführt, zum Beispiel 3 Waschschritte unter Verwendung von 4 X SSC. Weitere Einzelheiten sind wie von Sambrook, J., et al. (1989), "Molecular Cloning: A Laboratory Manual", Cold Spring Harbor Laboratory Press, oder Ausubel, F.M., et al. (1994) "Current Protocols in Molecular Biology", John Wiley & Sons, beschrieben.

### Beispiel 8: Identifikation von Zielgenen durch Sichtung von Expressionsbanken mit Antikörpern

Es wurden cDNA-Sequenzen zur Herstellung von rekombinantem Protein zum Beispiel in E. coli verwendet (z.B. Qiagen QIAexpress pQE-System). Die rekombinanten Proteine wurden dann gewöhnlich über Ni-NTA-Affinitätschromatographie (Qiagen) affinitätsgereinigt. Die rekombinanten Proteine wurden dann zur Herstellung spezifischer Antikörper beispielsweise unter Verwendung von Standardtechniken zur Immunisierung von Kaninchen verwendet. Anschließend wurden die Antikörper dann unter Verwendung einer Ni-NTA-Säule, die mit rekombinantem Antigen vorgesättigt wird, affinitätsgereinigt, wie von Gu et al., (1994) BioTechniques 17:257-262 beschrieben. Der Antikörper kann dann zur Durchmusterung von Expressions-cDNA-Banken mittels immunologischem Sichtung verwendet werden (Sambrook, J., et al. (1989), "Molecular Cloning: A Laboratory Manual", Cold Spring Harbor Laboratory Press, oder Ausubel, F.M., et al. (1994) "Current Protocols in Molecular Biology", John Wiley & Sons).

### Beispiel 9: Transformation von Agrobacterium

Die Agrobacterium-vermittelte Pflanzentransformation kann zum Beispiel unter Verwendung des GV3101- (pMP90-) (Koncz und Schell, Mol. Gen. Genet. 204 (1986) 383-396) oder LBA4404- (Clontech) oder C58C1 pGV2260 (Deblaere et al 1984, Nucl. Acids Res. 13, 4777-4788) Agrobacterium tumefaciens-Stamms durchgeführt werden. Die Transformation kann durch Standard-Transformationstechniken durchgeführt werden (ebenfalls Deblaere et al. 1984).

### Beispiel 10: Pflanzentransformation

Die Agrobacterium-vermittelte Pflanzentransformation kann unter Verwendung von Standard-Transformations- und Regenerationstechniken durchgeführt werden (Gelvin, Stanton B., Schilperoort, Robert A., Plant Molecular Biology Manual, 2. Aufl., Dordrecht: Kluwer Academic Publ., 1995, in Sect., Ringbuc Zentrale Signatur: BT11-P ISBN 0-7923-2731-4; Glick, Bernard R., Thompson, John E., Methods in Plant Molecular Biology and Biotechnology, Boca Raton: CRC Press, 1993, 360 S., ISBN 0-8493-5164-2).

Beispielsweise kann Raps mittels Kotyledonen- oder Hypokotyltransformation transformiert werden (Moloney et al., Plant Cell 8 (1989) 238-242; De Block et al., Plant Physiol. 91 (1989) 694-701). Die Verwendung von Antibiotika für die Agrobacterium- und Pflanzenselektion hängt von dem für die Transformation verwendeten binären Vektor und Agrobacterium-Stamm ab. Die Rapsselektion wird gewöhnlich unter Verwendung von Kanamycin als selektierbarem Pflanzenmarker durchgeführt.

Der Agrobacterium-vermittelte Gentransfer in Lein (Linum usitatissimum) lässt sich unter Verwendung von beispielsweise einer von Mlynarova et al. (1994) Plant Cell Report 13:282-285 beschriebenen Technik durchführen.

Die Transformation von Soja kann unter Verwendung von beispielsweise einer in EP-A-0 0424 047 (Pioneer Hi-Bred International) oder in EP-A-0 0397 687, US 5,376,543, US 5,169,770 (University Toledo) beschriebenen Technik durchgeführt werden.

Die Pflanzentransformation unter Verwendung von Teilchenbeschuss, Polyethylenglycol-vermittelter DNA-Aufnahme oder über die Siliziumcarbonatfaser-Technik ist beispielsweise beschrieben von Freeling und Walbot "The maize handbook" (1993) ISBN 3-540-97826-7, Springer Verlag New York).

### Beispiel 11: Plasmide für die Pflanzentransformation

Zur Pflanzentransformation können binäre Vektoren, wie pBinAR (Höfgen und Willmitzer, Plant Science 66 (1990) 221-230) oder pGPTV (Becker et al 1992, Plant Mol. Biol. 20:1195-1197) oder Derivate davon verwendet werden. Die Konstruktion der binären Vektoren kann durch Ligation der cDNA in Sense- oder Antisense-Orientierung in T-DNA erfolgen. 5' der cDNA aktiviert ein Pflanzenpromotor die Transkription der cDNA. Eine Polyadenylierungssequenz befindet sich 3' von der cDNA. Die binären Vektoren können unterschiedliche Markergene tragen. Insbesondere kann das nptII-Markergen codierend für Kanamycin-Resistenz vermittelt durch Neomycinphosphotransferase gegen die herbizidresistente Form eines Acetolactat Synthasegens (Abkürzung: AHAS oder ALS) ausgetauscht werden. Das ALS-Gen ist beschrieben in Ott et al., J. Mol. Biol. 1996, 263:359-360. Der v-ATPase-c1-Promotor kann in das Plasmid pBin19 oder pGPTV kloniert werden und durch Klonierung vor das ALS Codierregion für die Markergenexpression genutzt werden. Der genannte Promotor entspricht einem 1153 Basenpaarfragment aus beta-Vulgaris (Plant Mol Biol, 1999, 39:463-475). Dabei können sowohl Sulphonylharnstoffe als auch Imidazolinone wie Imazethapyr oder Sulphonylharnstoffe als Antimetaboliten zur Selektion verwendet werden.

Die gewebespezifische Expression lässt sich unter Verwendung eines gewebespezifischen Promotors erzielen. Beispielsweise kann die samenspezifische Expression erreicht werden, indem der DC3- oder der LeB4- oder der USP-Promotor oder der Phaseolin-Promotor 5' der cDNA einkloniert wird. Auch jedes andere samenspezifische Promotorelement wie z.B. der Napin- oder Arcelin Promotor Goossens et al. 1999, Plant Phys. 120(4):1095-1103 und Gerhardt et al. 2000, Biochimica et Biophysica Acta 1490(1-2):87-98) kann verwendet werden. Zur konstitutiven Expression in der ganzen Pflanzen lässt sich der CaMV-35S-Promotor oder ein v-ATPase C1 Promotor verwenden.

Insbesondere lassen sich Gene codierend für Desaturasen und Elongasen durch Konstruktion mehrerer Expressionskassetten hintereinander in einen binären Vektor klonieren, um den Stoffwechselweg in Pflanzen nachzubilden.

Innerhalb einer Expressionskassette kann das zu exprimierende Protein unter Verwendung eines Signalpeptids, beispielsweise für Plastiden, Mitochondrien oder das Endoplasmatische Retikulum, in ein zelluläres Kompartiment dirigiert werden (Kermode, Crit. Rev. Plant Sci. 15, 4 (1996) 285-423). Das Signalpeptid wird 5' im Leseraster mit der cDNA einkloniert, um die subzelluläre Lokalisierung des Fusionsprotein zu erreichen.

Beispiele für Multiexpressionskassetten sind im folgenden gegeben.

### I.) Promotor-Terminator-Kassetten

Expressionskassetten bestehen aus wenigstens zwei funktionellen Einheiten wie einem Promotor und einem Terminator. Zwischen Promotor und Terminator können weitere gewünschte Gensequenzen wie Targetting-Sequenzen, Codierregionen von Genen oder Teilen davon etc. eingefügt werden. Zum Aufbau von Expressionskassetten werden Promotoren und Terminatoren (USP Promotor: Baeumlein et al., Mol Gen Genet, 1991, 225 (3):459-67); OCS Terminator: Gielen et al. EMBO J. 3 (1984) 835ff.) mithilfe der Polymerasekettenreaktion isoliert und mit flankierenden Sequenzen nach Wahl auf Basis von synthetischen Oligonukleotiden maßgeschneidert.

Folgende Oligonukleotide können beispielsweise verwendet werden:
USP1 vorne: CCGGAATTCGGCGCGCCGAGCTCCTCGAGCAAATTTACACATTGCCA
USP2 vorne: CCGGAATTCGGCGCGCCGAGCTCCTCGAGCAAATTTACACATTGCCA
USP3 vorne: CCGGAATTCGGCGCGCCGAGCTCCTCGAGCAAATTTACACATTGCCA
USP1 hinten:AAAACTGCAGGCGGCCGCCCACCGCGGTGGGCTGGCTATGAAGAAATT
USP2 hinten:CGCGGATCCGCTGGCTATGAAGAAATT
USP3 hinten:TCCCCCGGGATCGATGCCGGCAGATCTGCTGGCTATGAAGAAATT
OCS1 vorne: AAAACTGCAGTCTAGAAGGCCTCCTGCTTTAATGAGATAT
OCS2 vorne: CGCGGATCCGATATCGGGCCCGCTAGCGTTAACCCTGCTTTAATGAGATAT
OCS3 vorne: TCCCCCGGGCCATGGCCTGCTTTAATGAGATAT
OCS1 hinten:CCCAAGCTTGGCGCGCCGAGCTCGAATTCGTCGACGGACAATCAGTAAATTGA
OCS2 hinten:CCCAAGCTTGGCGCGCCGAGCTCGAATTCGTCGACGGACAATCAGTAAATTGA
OCS3 hinten:CCCAAGCTTGGCGCGCCGAGCTCGTCGACGGACAATCAGTAAATTGA

Die Methoden sind dem Fachmann auf dem Gebiet bekannt und sind allgemein literaturbekannt.

In einem ersten Schritt werden ein Promotor und ein Terminator über PCR amplifiziert. Dann wird der Terminator in ein Empfängerplasmid kloniert und in einem zweiten Schritt der Promotor vor den Terminator inseriert. Mithin erhält man eine Expressionskassette auf einem Trägerplasmid. Auf Basis des Plamides pUC19 werden die Plasmide pUT1, pUT2 und pUT3 erstellt.

Die Konstrukte sind erfindungsgemäß in SEQ ID NO: 13, 14 und 15 definiert. Sie enthalten auf Basis von pUC19 den USP-Promotor und den OCS Terminator. Auf Basis dieser Plasmide wird das Konstrukt pUT12 erstellt, indem pUT1 mittels SalI/ScaI geschnitten wird und pUT2 mittels XhoI/ScaI geschnitten wird. Die die Expressionskassetten enthaltenden Fragmente werden ligiert und in E. coli XLI blue MRF transformiert. Es wird nach Vereinzelung ampicillinresistenter Kolonien DNA präpariert und per Restriktionsanalyse solche Klone identifiziert, die zwei Expressionskassetten enthalten. Die XhoI/SalI Ligation kompatibler Enden hat dabei die beiden Schnittstellen XhoI und SalI zwischen den Expressionskassetten eliminiert. Es resultiert das Plasmid pUT12, das in SEQ ID NO: 16 definiert ist. Anschließend wird pUT12 wiederum mittels Sal/ScaI geschnitten und pUT3 mittels XhoI/ScaI geschnitten. Die die Expressionskassetten enthaltenden Fragmente werden ligiert und in E. coli XLI blue MRF transformiert. Es wird nach Vereinzelung ampicillinresistenter Kolonien DNA präpariert und per Restriktionsanalyse solche Klone identifiziert, die drei Expressionskassetten enthalten. Auf diese Weise wird ein Set von Multiexpressionskassetten geschaffen, dass für die Insertion gewünschter DNA genutzt werden kann und in Tabelle 3 beschrieben wird und zudem noch weitere Expressionskassetten aufnehmen kann.

Diese enthalten folgende Elemente:

**Tabelle 3**

| pUC19-Derivat | Schnittstellen vor dem USP Promotor | Multiple Klonierungs-Schnittstellen | Schnittstellen hinter dem OCS-Terminator |
|---|---|---|---|
| pUT1 | EcoRI/AscI/SacI/XhoI | BstXI/NotI/PstI/XbaI/StuI | SalI/EcoRI/SacI/AscI/HindIII |
| pUT2 | EcoRI/AscI/SacI/XhoI | BamHI/EcoRV/ApaI/NheI/HpaI | SalI/EcoRI/SacI/AseI/HindIII |
| pUT3 | EcoRI/AscI/SacI/XhoI | BglII/NaeI/ClaI/SmaI/NcoI | SalI/SacI/AscI/HindIII |
| pUT12 Doppel-expressionskassette | EcoRI/AscI/ SacI/XhoI | BstXI/NotI/PstI/XbaI/StuI Und BamHI/EcoRV/ApaI/NheI/HpaI | SalI/EcoRI/SacI/AscI/HindIII |
| pUT123 Tripel-expressionskassette | EcoRI/AscI/SacI/XhoI | 1.BstXI/NotI/ PstI/XbaI/StuI und 2.BamHI/EcoRV/ ApaI/NheI/ HpaI und 3.BglII/NaeI/ ClaI/SmaI/NcoI | SalI/SacI/AscI/HindIII |

Weiterhin lassen sich wie beschrieben und wie in Tabelle 4 näher spezifiziert weitere Multiexpressionskassetten mithilfe des
i) USP-Promotors oder mithilfe des
ii) ca. 700 Basenpaare 3'-Fragmentes des LeB4-Promotors oder mithilfe des
iii)DC3-Promotors erzeugen und für samenspezifische Genexpression einsetzen.

Der DC3-Promotor ist beschrieben bei Thomas, Plant Cell 1996, 263:359-368 und besteht lediglich aus der Region -117 bis +27 weshalb er mithin einer der kleinsten bekannten samenspezifischen Promotoren darstellt. Die Expressionskassetten können mehrfach den selben Promotor enthalten oder aber über drei verschiedene Promotoren aufgebaut werden.

**Tabelle 4: Multiple Expressionskassetten**

| Plasmidname des pUC19-Derivates | Schnittstellen vor dem jeweiligen Promotor | Multiple Klonierungs-Schnittstellen | Schnittstellen hinter dem OCS-Terminator |
|---|---|---|---|
| pUTl (pUC19 mit USP-OCS1) | EcoRI/AscI/SacI/XhoI | (1) BstXI/NotI/PstI/XbaI/StuI | SalI/EcoRI/Sacl/AscI/HindIII |
| pDCT (pUC19 mit DC3-OCS) | EcoRI/AscI/SacI/XhoI | (2) BamHI/EcoRV/Apal/NheI/HpaI | SalI/EcoRI/SacI/AscI/HindIII |
| pLeBT (pUC19-mit LeB4(700)-OCS) | EcoRI/AscI/SacI/XhoI | (3) BgIII/NaeI/ClaI/SmaI/NcoI | SalI/SacI/AscI/HindIII |
| pUD12 (pUC19 mit mit USP-OCS1 und mit DC3-OCS) | EcoRI/AscI/SacI/XhoI | (1) BstXI/NotI/PstI/XbaI/StuI und (2) BamHI/EcoRV/ApaI/NheI/HpaI | SalI/EcoRI/SacI/AscI/ HindIII |
| pUDL123 Triple expression cassette (pUC19 mit USP/DC3 und LeB4-700) | EcoRI/AscI/SacI/XhoI | (1) BstXI/NotI/PstI/XbaI/StuI und (2) BamHI/ (EcoRV*)/ApaI/ NheI/HpaI und (3) BglII/NaeI/ClaI/SmaI/NcoI | SalI/SacI/AscI/HindIII |

| | | | |
|---|---|---|---|
| * EcoRV Schnittstelle schneidet im 700 Basenpaarfragment des LeB4 Promotors (LeB4-700) | | | |

Analog lassen sich weitere Promotoren für Multigenkonstrukte erzeugen insbesondere unter Verwendung des
a) 2,7 kB Fragmentes des LeB4-Promotors oder mithilfe des
b) Phaseolin-Promotors oder mithilfe des
c) konstitutiven v-ATPase c1-Promotors.

Es kann insbesondere wünschenswert sein, weitere besonders geeignete Promotoren zum Aufbau samenspezifischer Multiexpressionskassetten wie z.B. den Napin-Promotor oder den Arcelin-5 Promotor zu verwenden.

### ii) Erstellung von Expressionskonstrukten in pUC19- oder pGPTV Derivaten, die Promotor und Terminator erhalten und in Kombination mit gewünschten Gensequenzen zur PUFA Genexpression in pflanzlichen Expressionskassetten enthalten.

Multiexpressionskassetten können mittels AscI direkt von pUC19-Derivaten aus Tabelle 3 in den Vektor pGPTV+AscI (siehe iii.)) über die AscI Schnittstelle inseriert werden und stehen zur Inserierung von Zielgenen zur Verfügung. Die entsprechenden Genkonstrukte (pBUT1 ist in SEQUENZ ID NO: 20, pBUT2 ist in SEQUENZ ID NO: 21, pBUT 3 ist in SEQUENZ ID NO: 22, pBUT12 ist in SEQUENZ ID NO: 22 und pBUT123 ist in SEQUENZ ID NO: 24 dargestellt) stehen erfindunsgemäß als Kit zur Verfügung. Alternativ können Gensequenzen in die pUC19 basierten Expressionskassetten inseriert werden und als AscI Fragment in pGPTV+AscI eingesetzt werden.

In pUT12 wird zunächst über BstXI und XbaI die D-6-Elongase Pp_PSE1 in die erste Kassette inseriert. Dann wird die D-6-Desaturase aus Moos (Pp_des6) über BamHI/NaeI in die zweite Kassette inseriert. Es entsteht das Konstrukt pUT-ED.Das AscI Fragment aus dem Plasmid pUT-ED wird in den mit AscI geschnittenen Vektor pGPTV+AscI inseriert und die Orientierung des inserierten Fragmentes mittels Restriktion oder Sequenzierung ermittelt. Es entsteht das Plasmid pB-DHGLA, dessen vollständige Sequenz in SEQUENZ ID NO. 25 dargestellt ist. Die Codierregion der Physcomitrella delta 6 Elongase ist in SEQUENZ ID NO. 26 dargestellt, die der delta 6 Desaturase aus Physcomitrella in SEQUENZ ID NO: 27.

In pUT123 wird zunächst über BstXI und XbaI die Δ-6-Elongase Pp_PSE1 in die erste Kassette inseriert. Dann wird die Δ-6-Desaturase aus Moos (Pp_des6) über BamHI/NaeI in die zweite Kassette inseriert und schließlich die Δ-5-Desaturase aus Phaeodactylum (Pt_des5) über BglII in die dritte Kassette inseriert. Das Dreifachkonstrukt erhält den Namen pARA1. Unter Berücksichtigung sequenzspezifischer Restriktionsschnittstellen können weitere Expressionskassetten gemäß Tabelle 5 mit der Bezeichnung pARA2, pARA3 und pARA4 erstellt werden.

Das AscI Fragment aus dem Plasmid pARA1 wird in den mit AscI geschnittenen Vektor pGPTV+AscI inseriert und die Orientierung des inserierten Fragmentes mittels Restriktion oder Sequenzierung ermittelt. Die vollständige Sequenz des resultierenden Plasmides pBARA1 ist in SEQUENZ ID NO. 28 dargestellt. Die Codierregion der Physcomitrella delta 6 Elongase ist in SEQUENZ ID NO. 29 dargestellt, die der delta 6 Desaturase aus Physcomitrella in SEQUENZ ID NO: 30 und die der delta-5 Desaturase aus Phaeodactylum tricornutum in SEQUENZ ID NO: 31.

**Tabelle 5: Kombinationen von Desaturasen und Elongasen**

| | Gen Plasmid | Δ-6-Desaturase | Δ-5-Desaturase | Δ-6-Elongase |
|---|---|---|---|---|
| 1 | PUT-ED | Pp_des6 | --- | Pp_PSE1 |
| 2 | pARA1 | Pt_des6 | Pt_des5 | Pp_PSE1 |
| 3 | pARA2 | Pt_des6 | Ce_des5 | Pp_PSE1 |
| 4 | pARA3 | Pt_des6 | Ce_des5 | Pp_PSE1 |
| 5 | pARA4 | Ce_des6 | Ce_des5 | Ce_PSE1 |
| 6 | PBDHGLA | Pt_des6 | --- | Pp_PSE1 |
| 7 | PBARAI | Pt_des6 | Pt_des5 | Pp_PSE1 |

Plasmide 1 bis 5 sind pUC Derivate, Plasmide 6 bis 7 sind binäre Pflanzentransformationsvektoren
Pp = Physcomitrella patens, Pt = Phaeodactylum tricornutum Pp_PSE1 entspricht der Sequenz aus SEQ ID NO: 9.
PSE = PUFA spezifische Δ-6-Elongase
Ce_des5 = Δ-5-Desaturase aus Caenorhabditis elegans (Genbank Acc. Nr. AF078796)
Ce_des6 = Δ-6-Desaturase aus Caenorhabditis elegans elegans (Genbank Acc. Nr. AF031477, Basen 11-1342)
Ce_PSE1 = Δ-6-Elongase aus Caenorhabditis elegans (Genbank Acc. Nr. AF244356, Basen 1-867)

Auch weitere Desaturasen oder Elongasegensequenzen können in Expressionskassetten beschriebener Art inseriert werden wie z.B. Genbank Acc. Nr. AF231981, NM_013402, AF206662, AF268031, AF226273, AF110510 oder AF110509.

### iii) Transfer von Expressionskassetten in Vektoren zur Transformation von Agrobakterium tumefaciens und zur Transformation von Pflanzen

Chimäre Genkonstrukte auf Basis der in pUC19 beschriebenen können mittels AscI in den binären Vektor pGPTV inseriert. Die multiple Klonierungssequenz wird zu diesem Zweck um eine AscI Schnittstelle erweitert. Zu diesem Zweck wird der Polylinker als zwei doppelsträngige Oligonukleotide neu synthetisiert, wobei eine zusätzliche AscI DNA Sequenz eingefügt wird. Das Oligonukleotid wird mittels EcoRI und HindIII in den Vektor pGPTV inseriert. Es entsteht das Plasmid pGPTV+AscI. Die notwendigen Kloniertechniken sind dem Fachmann bekannt und können einfach wie in Beispiel 1 beschrieben nachgelesen werden.

### Beispiel 12: In vivo-Mutagenese

Die in vivo-Mutagenese von Mikroorganismen kann mittels Passage der Plasmid- (oder einer anderen Vektor-) DNA durch E. coli oder andere Mikroorganismen (z.B. Bacillus spp. oder Hefen, wie Saccharomyces cerevisiae), bei denen die Fähigkeiten, die Unversehrtheit ihrer genetischen Information aufrechtzuerhalten, gestört ist, erfolgen. Übliche Mutator-Stämme haben Mutationen in den Genen für das DNA-Reparatursystem (z.B. mutHLS, mutD, mutT usw.; als Literaturstelle siehe Rupp, W.D. (1996) DNA repair mechanisms, in: Escherichia coli and Salmonella, S. 2277-2294, ASM: Washington). Diese Stämme sind dem Fachmann bekannt. Die Verwendung dieser Stämme ist beispielsweise in Greener, A., und Callahan, M. (1994) Strategies 7:32-34, erläutert. Der Transfer mutierter DNA-Moleküle in Pflanzen erfolgt vorzugsweise nach Selektion und Test der Mikrooganismen. Transgene Pflanzen werden nach verschiedenen Beispielen im Beispielteil dieses Dokumentes erzeugt.

### Beispiel 13: Untersuchung der Expression eines rekombinanten Genproduktes in einem transformierten Organismus

Die Aktivität eines rekombinanten Genproduktes im transformierten Wirtsorganismus kann auf der Transkriptions- und/oder der Translationsebene gemessen werden.

Ein geeignetes Verfahren zur Bestimmung der Menge an Transkription des Gens (ein Hinweis auf die Menge an RNA, die für die Translation des Genproduktes zur Verfügung steht) ist die Durchführung eines Northern-Blots wie unten ausgeführt (als Bezugsstelle siehe Ausubel et al. (1988) Current Protocols in Molecular Biology, Wiley: New York, oder den oben erwähnten Beispielteil), wobei ein Primer, der so gestaltet ist, dass er an das Gen von Interesse bindet, mit einer nachweisbaren Markierung (gewöhnlich radioaktiv oder chemilumineszent) markiert wird, so dass, wenn die Gesamt-RNA einer Kultur des Organismus extrahiert, auf einem Gel aufgetrennt, auf eine stabile Matrix transferiert und mit dieser Sonde inkubiert wird, die Bindung und das Ausmaß der Bindung der Sonde das Vorliegen und auch die Menge der mRNA für dieses Gen anzeigt. Diese Information zeigt den Grad der Transkription des transformierten Gens an. Zelluläre Gesamt-RNA kann aus Zellen, Geweben oder Organen mit mehreren Verfahren, die alle im Fachgebiet bekannt sind, wie zum Beispiel das von Bormann, E.R., et al. (1992) Mol. Microbiol. 6:317-326 beschriebene, präpariert werden.

### Northern-Hybridisierung

Für die RNA-Hybridisierung wurden 20 µg Gesamt-RNA oder 1 µg poly(A)⁺-RNA mittels Gelelektrophorese in Agarosegelen mit einer Stärke von 1,25 % unter Verwendung von Formaldehyd, wie beschrieben in Amasino (1986, Anal. Biochem. 152, 304) aufgetrennt, mittels Kapillaranziehung unter Verwendung von 10 x SSC auf positiv geladene Nylonmembranen (Hybond N+, Amersham, Braunschweig) übertragen, mittels UV-Licht immobilisiert und 3 Stunden bei 68°C unter Verwendung von Hybridisierungspuffer (10 % Dextransulfat Gew./Vol., 1 M NaCl, 1 % SDS, 100 mg Heringssperma-DNA) vorhybridisiert. Die Markierung der DNA-Sonde mit dem Highprime DNA labeling-Kit (Roche, Mannheim, Deutschland) erfolgte während der Vorhybridisierung unter Verwendung von alpha-³²P-dCTP (Amersham, Braunschweig, Deutschland). Die Hybridisierung wurde nach Zugabe der markierten DNA-Sonde im gleichen Puffer bei 68°C über Nacht durchgeführt. Die Waschschritte wurden zweimal für 15 min unter Verwendung von 2 X SSC und zweimal für 30 min unter Verwendung von 1 X SSC, 1 % SDS, bei 68°C durchgeführt. Die Exposition der verschlossenen Filter wurde bei -70°C für einen Zeitraum von 1 bis 14 T durchgeführt.

Zur Untersuchung des Vorliegens oder der relativen Menge an von dieser mRNA translatiertem Protein können Standardtechniken, wie ein Western-Blot, eingesetzt werden (siehe beispielsweise Ausubel et al. (1988) Current Protocols in Molecular Biology, Wiley: New York). Bei diesem Verfahren werden die zellulären Gesamt-Proteine extrahiert, mittels Gelelektrophorese aufgetrennt, auf eine Matrix, wie Nitrozellulose, übertragen und mit einer Sonde, wie einem Antikörper, der spezifisch an das gewünschte Protein bindet, inkubiert. Diese Sonde ist gewöhnlich mit einer chemilumineszenten oder kolorimetrischen Markierung versehen, die sich leicht nachweisen lässt. Das Vorliegen und die Menge der beobachteten Markierung zeigt das Vorliegen und die Menge des gewünschten, in der Zelle vorliegenden mutierten Proteins an.

### Beispiel 14: Analyse der Auswirkung der rekombinanten Proteine auf die Produktion des gewünschten Produktes

Die Auswirkung der genetischen Modifikation in Pflanzen, Pilzen, Algen, Ciliaten oder auf die Produktion einer gewünschten Verbindung (wie einer Fettsäure) kann bestimmt werden, indem die modifizierten Mikroorganismen oder die modifizierte Pflanze unter geeigneten Bedingungen (wie den vorstehend beschriebenen) gezüchtet werden und das Medium und/oder die zellulären Komponenten auf die erhöhte Produktion des gewünschten Produktes (d.h. von Lipiden oder einer Fettsäure) untersucht wird. Diese Analysetechniken sind dem Fachmann bekannt und umfassen Spektroskopie, Dünnschichtchromatographie, Färbeverfahren verschiedener Art, enzymatische und mikrobiologische Verfahren sowie analytische Chromatographie, wie Hochleistungs-Flüssigkeitschromatographie (siehe beispielsweise Ullman, Encyclopedia of Industrial Chemistry, Bd. A2, S. 89-90 und S. 443-613, VCH: Weinheim (1985); Fallon, A., et al., (1987) "Applications of HPLC in Biochemistry" in: Laboratory Techniques in Biochemistry and Molecular Biology, Bd. 17; Rehm et al. (1993) Biotechnology, Bd. 3, Kapitel III: "Product recovery and purification", S. 469-714, VCH: Weinheim; Belter, P.A., et al. (1988) Bioseparations; downstream processing for Biotechnology, John Wiley and Sons; Kennedy, J.F., und Cabral, J.M.S. (1992) Recovery processes for biological Materials, John Wiley and Sons; Shaeiwitz, J.A., und Henry, J.D. (1988) Biochemical Separations, in: Ullmann's Encyclopedia of Industrial Chemistry, Bd. B3; Kapitel 11, S. 1-27, VCH: Weinheim; und Dechow, F.J. (1989) Separation and purification techniques in biotechnology, Noyes Publications).

Neben den oben erwähnten Verfahren werden Pflanzenlipide aus Pflanzenmaterial wie von Cahoon et al. (1999) Proc. Natl. Acad. Sci. USA 96 (22):12935-12940, und Browse et al. (1986) Analytic Biochemistry 152:141-145, beschrieben extrahiert. Die qualitative und quantitative Lipid- oder Fettsäureanalyse ist beschrieben bei Christie, William W., Advances in Lipid Methodology, Ayr/ Scotland: Oily Press (Oily Press Lipid Library; 2); Christie, William W., Gas Chromatography and Lipids. A Practical Guide - Ayr, Scotland: Oily Press, 1989, Repr. 1992, IX, 307 S. (Oily Press Lipid Library; 1); "Progress in Lipid Research, Oxford: Pergamon Press, 1 (1952) - 16 (1977) u.d.T.: Progress in the Chemistry of Fats and Other Lipids CODEN.

Zusätzlich zur Messung des Endproduktes der Fermentation ist es auch möglich, andere Komponenten der Stoffwechselwege zu analysieren, die zur Produktion der gewünschten Verbindung verwendet werden, wie Zwischen- und Nebenprodukte, um die Gesamteffizienz der Produktion der Verbindung zu bestimmen. Die Analyseverfahren umfassen Messungen der Nährstoffmengen im Medium (z.B. Zucker, Kohlenwasserstoffe, Stickstoffquellen, Phosphat und andere Ionen), Messungen der Biomassezusammensetzung und des Wachstums, Analyse der Produktion üblicher Metabolite von Biosynthesewegen und Messungen von Gasen, die während der Fermentation erzeugt werden. Standardverfahren für diese Messungen sind in Applied Microbial Physiology; A Practical Approach, P.M. Rhodes und P.F. Stanbury, Hrsgb., IRL Press, S. 103-129; 131-163 und 165-192 (ISBN: 0199635773) und darin angegebenen Literaturstellen beschrieben.

Ein Beispiel ist die Analyse von Fettsäuren (Abkürzungen: FAME, Fettsäuremethylester; GC-MS, Gas-Flüssigkeitschromatographie-Massenspektrometrie; TAG, Triacylglycerin; TLC, Dünnschichtchromatographie).

Der unzweideutige Nachweis für das Vorliegen von Fettsäureprodukten kann mittels Analyse rekombinanter Organismen nach Standard-Analyseverfahren erhalten werden: GC, GC-MS oder TLC, wie verschiedentlich beschrieben von Christie und den Literaturstellen darin (1997, in: Advances on Lipid Methodology, Vierte Aufl.: Christie, Oily Press, Dundee, 119-169; 1998, Gaschromatographie-Massenspektrometrie-Verfahren, Lipide 33:343-353).

Das zu analysierende Material kann durch Ultraschallbehandlung, Mahlen in der Glasmühle, flüssigen Stickstoff und Mahlen oder über andere anwendbare Verfahren aufgebrochen werden. Das Material muss nach dem Aufbrechen zentrifugiert werden. Das Sediment wird in Aqua dest. resuspendiert, 10 min bei 100°C erhitzt, auf Eis abgekühlt und erneut zentrifugiert, gefolgt von Extraktion in 0,5 M Schwefelsäure in Methanol mit 2 % Dimethoxypropan für 1 Std. bei 90°C, was zu hydrolysierten Öl- und Lipidverbindungen führt, die transmethylierte Lipide ergeben. Diese Fettsäuremethylester werden in Petrolether extrahiert und schließlich einer GC-Analyse unter Verwendung einer Kapillarsäule (Chrompack, WCOT Fused Silica, CP-Wax-52 CB, 25 mikrom, 0,32 mm) bei einem Temperaturgradienten zwischen 170°C und 240°C für 20 min und 5 min bei 240°C unterworfen. Die Identität der erhaltenen Fettsäuremethylester muss unter Verwendung von Standards, die aus kommerziellen Quellen erhältlich sind (d.h. Sigma), definiert werden.

Bei Fettsäuren, für die keine Standards verfügbar sind, muss die Identität über Derivatisierung und anschließende GC-MS-Analyse gezeigt werden. Beispielsweise muss die Lokalisierung von Fettsäuren mit Dreifachbindung über GC-MS nach Derivatisierung mit 4,4-Dimethoxyoxazolin-Derivaten (Christie, 1998, siehe oben) gezeigt werden.

### Expressionskonstrukte in heterologen mikrobiellen Systemen

### Stämme, Wachstumsbedingungen und Plasmide

Der Escherichia coli-Stamm XL1 Blue MRF' kan (Stratagene) wurde zur Subklonierung der neuen Desaturase pPDesaturase1 aus Physcomitrella patens verwendet. Für die funktionelle Expression dieses Gens verwendeten wir den Saccharomyces cerevisiae-Stamm INVSc 1 (Invitrogen Co.). E. coli wurde in Luria-Bertini-Brühe (LB, Duchefa, Haarlem, Niederlande) bei 37°C kultiviert. Wenn nötig, wurde Ampicillin (100 mg/Liter) zugegeben, und 1,5 % Agar (Gew./ Vol.) wurde für feste LB-Medien hinzugefügt. S. cerevisiae wurde bei 30°C entweder in YPG-Medium oder in komplettem Minimalmedium ohne Uracil (CMdum; siehe in: Ausubel, F.M., Brent, R., Kingston, R.E., Moore, D.D., Seidman, J.G., Smith, J.A., Struhl, K., Albright, L.B., Coen, D.M., und Varki, A. (1995) Current Protocols in Molecular Biology, John Wiley & Sons, New York) mit entweder 2 % (Gew./Vol.) Raffinose oder Glucose kultiviert. Für feste Medien wurden 2 % (Gew./Vol.) Bacto™-Agar (Difco) hinzugefügt. Die zur Klonierung und Expression verwendeten Plasmide sindpUC18 (Pharmacia) und pYES2 (Invitrogen Co.).

### Beispiel 16: Klonierung und Expression PUFA-spezifischer Desaturasen aus Phaeodactylum tricornutum

Für die Expression in Hefe wurden die Phaeodactylum tricornutum -cDNA-Klone aus Seq ID NO: 1, 3, 5 oder 11 bzw. die Sequenzen aus SEQ ID NO: 7 oder 9 bzw andere gewünschte Sequenzen zuerst so modifiziert, dass lediglich die Codierregion mittels Polymerase Kettenreaktion unter Zuhilfenahme zweier Oligonukleotide amplifiziert werden. Dabei wurde darauf geachtet, dass eine Konsensusequenz vor dem Startcodon zur effizienten Translation eingehalten wurde. Entweder wurde hierzu die Basenfolge ATA oder AAA gewählt und vor das ATG in die Sequenz eingefügt (Kozak, M. (1986) Point mutations define a sequence flanking the AUG initiator codon that modulates translation by eukaryotic ribosomes, Cell 44, 283-292). Vor diesem Konsensustriplett wurde zusätzlich eine Restriktionsschnittstelle eingeführt, die kompatibel sein muss zur Schnittstelle des Zielvektors, in den das Fragment kloniert werden soll und mit dessen Hilfe die Genexpression in Mikroorganismen oder Pflanzen erfolgen soll.

Die PCR-Reaktion wurde mit Plasmid-DNA als Matrize in einem Thermocycler (Biometra) mit der Pfu-DNA-(Stratagene)Polymerase und dem folgenden Temperaturprogramm durchgeführt: 3 min bei 96°C, gefolgt von 30 Zyklen mit 30 s bei 96°C, 30 s bei 55°C und 2 min bei 72°C, 1 Zyklus mit 10 min bei 72°C und Stop bei 4°C. Die Anlagerungstemperatur wurde je nach gewählten Oligonukleotiden variiert. Pro Kilobasenpaare DNA ist von einer Synthesezeit von etwa einer Minute auszugehen. Weitere Parameter, die Einfluss auf die PCR haben wie z.B. Mg-Ionen, Salz, DNA Polymerase etc., sind dem Fachmann auf dem Gebiet geläufig und können nach Bedarf variiert werden.

Die korrekte Größe des amplifizierten DNA-Fragments wurde mittels Agarose-TBE-Gelelektrophorese bestätigt. Die amplifizierte DNA wurde aus dem Gel mit dem QIAquick-Gelextraktionskit (QIAGEN) extrahiert und in die SmaI-Restriktionsstelle des dephosphorylierten Vektors pUC18 unter Verwendung des Sure Clone Ligations Kit (Pharmacia) ligiert, wobei die pUC-Derivate erhalten wurden. Nach der Transformation von E. coli XL1 Blue MRF' kan wurde eine DNA-Minipräparation (Riggs, M.G., & McLachlan, A. (1986) A simplified screening procedure for large numbers of plasmid minipreparation. BioTechniques 4, 310-313) an ampicillinresistenten Transformanden durchgeführt, und positive Klone mittels BamHI-Restriktionsanalyse identifiziert. Die Sequenz des klonierten PCR-Produktes wurde mittels Resequenzierung unter Verwendung des ABI PRISM Big Dye Terminator Cycle Sequencing Ready Reaction Kit (Perkin-Elmer, Weiterstadt) bestätigt.
Δ5 Acyl Lipid desaturase, Pt_des5

| | |
|---|---|
| Primer 1 | GAG CTC ACA TAA TGG CTC CGG ATG CGG ATA AGC |
| Primer 2 | CTC GAG TTA CGC CCG TCC GGT CAA GGG |

Das PCR-Fragment (1428bp) wurde mithilfe des Sure Clone Kit (Pharmacia) in pUC 18 kloniert, das inserierte Fragment SacI/XhoI verdaut und das Fragment mithilfe entsprechender Restriktionsschnittstellen in pYES2 oder pYES6 inseriert.
Δ6 Acyl Lipid desaturase, Pt_des6

| | |
|---|---|
| Primer 3 | GGA TCC ACA TAA TGG GCA AAG GAG GGG ACG CTC GGG |
| Primer 4 | CTC GAG TTA CAT GGC GGG TCC ATC GGG |

Das PCR-Fragment (1451 bp) wurde mithilfe des Sure Clone Kit (Pharmacia) in pUC 18 kloniert, das inserierte Fragment BamtiI/XhoI verdaut und das Fragment mithilfe entsprechender Restriktionsschnittstellen in pYES2 oder pYES6 inseriert.
Δ12 Acyl Lipid desaturase, Pt_des12

| | |
|---|---|
| Primer 5 | GGA TCC ACA TAA TGG TTC GCT TTT CAA CAG CC |
| Primer 6 | CTC GAG TTA TTC GCT CGA TAA TTT GC |

Δ 12 Acyl Lipid desaturase, Pt_des12.2

| | |
|---|---|
| Primer 7 | GGA TCC ACA TAA TGG GTA AGG GAG GTC AAC G |
| Primer 8 | CTC GAG TCA TGC GGC TTT GTT TCG C |

Das PCR Fragment (1505bp) wurde mithilfe des Sure Clone Kit (Pharmacia) in pUC 18 kloniert, das inserierte Fragment BamHI/XhoI verdaut und das Fragment mithilfe entsprechender Restriktionsschnittstellen in pYES2 oder pYES6 inseriert.

Die Plasmid-DNA wurde mit Restriktionsenzym/en passend zur eingeführten Schnittstelle der Primersequenz gespalten und das erhaltene Fragment in die kompatiblen Restriktionsstellen des dephosphorylierten Hefe-E. coli-Shuttlevektors pYES2 oder pYES6 ligiert, wobei pYES-Derivate erhalten werden. Nach der Transformation von E. coli und DNA-Minipräparation aus den Transformanden wurde die Orientierung des DNA-Fragments im Vektor durch geeignete Restriktionsspaltung oder Sequenzierung überprüft. Ein Klon wurde für die DNA-Maxipräparation mit dem Nucleobon^{®} AX 500 Plasmid-DNA-Extraktionskit (Macherey-Nagel, Düringen) angezogen.

Saccharomyces cerevisiae INVSc1 wurde mit den pYES-Derivaten und pYES Leervektor mittels eines PEG/Lithiumacetat-Protokolls transformiert (Ausubel et al., 1995). Nach der Selektion auf CMdum-Agarplatten mit 2 % Glucose wurden pYES-Derivate-Transformanden und eine pYES2-Transformande zur weiteren Anzucht und funktionellen Expression ausgewählt. Bei pYES6-Derivaten wurde Blasticidin als Antimetabolit verwendet. Im Fall von Coexpressionen auf Basis von pYES2 und pYES6 wurde auf Minimalmedium mit Blasticidin selektiert.

### Funktionelle Expression einer Desaturaseaktivität in Hefe

### Vorkultur

20 ml CMdum-Flüssigmedium ohne Uracil aber mit 2 % (Gew./Vol.) Raffinose wurden mit den transgenen Hefeklonen (pYES2) angeimpft und 3 Tage bei 30°C, 200 rpm gezüchtet, bis eine optische Dichte bei 600 nm (OD₆₀₀) von 1,5 bis 2 erreicht wurde. Wurde als Vektor pYES6 verwendet, so wurde zusätzlich auf Blasticidin als Antimetabolit selektioniert.

### Hauptkultur

Für die Expression wurden 20 ml CMdum-Flüssigmedium ohne Uracil aber mit 2 % Raffinose und 1 % (Vol./Vol.) Tergitol NP-40 mit Fettsäuresubstraten auf eine Endkonzentration von 0,003 % (Gew./Vol.) angereichert. Die Medien wurden mit den Vorkulturen auf eine OD₆₀₀ von 0,05 angeimpft. Die Expression wurde bei einer OD₆₀₀ von 0,2 mit 2% (Gew./Vol.) Galaktose für 16 Std. induziert, wonach die Kulturen eine OD₆₀₀ von 0,8-1,2 geerntet wurden.

### Fettsäureanalyse

Die Gesamt-Fettsäuren wurden aus Hefekulturen extrahiert und mittels Gaschromatographie analysiert. Davon wurden Zellen von 5 ml Kultur mittels Zentrifugation (1000 x g, 10 min, 4°C) geerntet und einmal mit 100 mM NaHCO₃, pH 8,0, gewaschen, um restliches Medium und Fettsäuren zu entfernen. Zur Herstellung des Fettsäuremethylester (FAMES oder Singular FAME) wurden die Zellsedimente mit 1 M methanolischer H₂SO₄ und 2 % (Vol./Vol.) Dimethoxypropan für 1 Std. bei 80°C behandelt. Die FAMES wurden zweimal mit 2 ml Petrolether extrahiert, einmal mit 100 mM NaHCO₃, pH 8,0, und einmal mit destilliertem Wasser gewaschen und mit Na₂SO₄ getrocknet. Das organische Lösungsmittel wurde unter einem Argonstrom verdampft, und die FAMES wurden in 50 mikrol Petrolether gelöst. Die Proben wurden auf einer ZEBRON-ZB-Wax-Kapillarsäule (30 m, 0,32 mm, 0,25 mikro m; Phenomenex) in einem Hewlett Packard-6850-Gaschromatograph mit einem Flammenionisationsdetektor aufgetrennt. Die Ofentemperatur wurde von 70°C (1 min halten) bis 200°C mit einer Rate von 20°C/min, dann auf 250°C (5 min halten) mit einer Rate von 5°C/min und schließlich auf 260°C mit einer Rate von 5°C/min programmiert. Stickstoff wurde als Trägergas verwendet (4,5 ml/min bei 70°C). Die Fettsäuren wurden durch Vergleich mit Retentionszeiten von FAME-Standards (SIGMA) identifiziert.

### Expressionsanalyse

Die Verhältnisse der zugegebenen und aufgenommenen Fettsäuresubstrate wurden ermittelt und so Quantität und Qualität der Desaturasereaktion gemäß Tabelle 6, Tabelle 7 und Tabelle 8 erfasst.

Ergebnis der Expression einer Phaeodactylum tricornutum Δ-6-Acyl Lipid Desaturase in Hefe:

**Tabelle 6**

| Fettsäure | pYes2 | pYes2-Ptd6 gefüttert mit | | |
|---|---|---|---|---|
| | - | - | +18:2 | +18:3 |
| 16:0 | 13,3 | 18,9 | 28,4 | 16,7 |
| 16:1Δ9 | 45,4 | 44,7 | 12,5 | 16,9 |
| 16:2Δ6,9 | - | 4,3 | - | - |
| 18:0 | 4,9 | 6,3 | 10,4 | 9,1 |
| 18:1Δ9 | 36,4 | 24,1 | 6,8 | 11,8 |
| 18:2Δ6,9 | - | 1,8 | - | - |
| 18:2Δ9,12 | - | - | 33,4 | - |
| 18:3Δ6,12,15 | - | - | 4,9 | - |
| 18:3Δ9,12,15 | - | - | | 43,1 |
| 18:4Δ6,9,12,15 | - | - | - | 2,3 |

Die Angaben stellen Mol-% entsprechender cis-Fettsäuren dar.

Ergebnis der Expression einer Phaeodactylum tricornutum Δ-5-Acyl Lipid Desaturase in Hefe:

**Tabelle 7**

| | pYES2 | | pYES_PtD5-Konstrukt gefüttert mit | | | | | | |
|---|---|---|---|---|---|---|---|---|---|
| Fettsäure | Leer | Kontrolle | 18:2 | 18:3 | 20:1 | -20:1 | 20:2 | 20:3 | 20:3 |
| | | | | | Δ8 | Δ11 | Δ11,14 | Ω3 | Ω6 |
| 16:0Δ | 16,9 | 20,4 | 27,7 | 24,4 | 16,2 | 21 | 17,6 | 19,5 | 22,8 |
| 16:1Δ9 | 44,7 | 44,1 | 13,2 | 9,6 | 37,4 | 39,4 | 38,3 | 36,9 | 30,7 |
| 18:0 | 6,1 | 6,9 | 10,54 | 9,8 | 4,7 | 7,9 | 6,3 | 6,8 | 8,2 |
| 18:1Δ9 | 31,72 | 28,1 | 8,77 | 6 | 15 | 26 | 29,5 | 25,6 | 21,1 |
| 18 : 2 Δ5 , 9 | | 0,17 | 0 | 0 | 0 | 0,09 | 0,21 | 0,09 | 9 |
| 18:2 Δ9,12 | | - | 39,7 | - | - | - | - | - | - |
| 18:3 Δ9, 12, 15 | | - | | 49,9 | -- | - | - | - | - |
| 20:1 Δ8 | | - | | - | 25,5 | - | - | - | - |
| 20:1 Δ11 | | - | | - | - | 5,41 | - | - | - |
| 20:2 Δ5,11 | | - | | - | - | 0,21 | - | - | - |
| 20:2 Δ11, 14 | | - | - | - | - | - | 6,48 | - | - |
| 20:3 Δ5, 11, 14 | | - | | | | | 0,76 | - | - |
| 20:3 Δ11,14,17 | | - | - | - | - | - | - | 9.83 | - |
| 20:3 Δ8,11,14 | | - | - | - | - | - | - | - | 13,69 |
| 20:4 Δ5,11,14,17 | | - | - | - | - | - | - | 1,26 | - |
| 20:4 Δ5,8,11,14 | | - | - | - | - | - | - | - | 3,08 |

Die Angaben stellen Mol-% Fettsäuren von cis-Fettsäuren dar.

Aus weiteren Fütterungsversuchen wurde gefunden, dass C18:1Δ9 in der Anwesenheit von C18:2Δ9,11 oder C18:Δ9,12,15 oder C20:1Δ8 Fettsäuren nicht desaturiert wurde während in Anwesenheit von C20:1Δ11, C20:2 Δ11, 14 und C20:3 Δ8,11,14 auch C18:1 desaturiert wird. Ebenfalls keine Desaturierung erfolgte in Anwesenheit von C20:3 Δ8,11,14.

Bei Nutzung des Protease-defizienten Hefestammes C13BYS86 (Kunze I. et al., Biochemica et Biophysica Acta (1999) 1410:287-298) für die Expression der Δ-5-Desaturase aus Phaeodactylum tricornutum auf Vollmedium mit Blasticidin wurde gefunden, dass C20:4 Δ8,11,14,17 als Substrat der Δ-5-Desaturase- mit 20 % Umsatzrate ebenso gut umgesetzt wurde wie C20:3 Δ8,11,14. Alternativ können auch die Auxotrophiemarker leu2, ura3 oder his für Genexpression genutzt werden.

In einem weiteren Coexpressionsexperiment von Δ-5 Desaturase aus Phaeodactylum und Δ-6 Elongase aus Physcomitrella wurde der Stamm UTL7A (Warnecke et al., J. Biol. Chem. (1999) 274(19):13048-13059)benutzt, wobei die Δ-5 Desaturase ca 10 % C20:3 Δ8, 11, 14 zu C20:4 Δ5,8,11,14 umsetzte.

Weitere Fütterungsexperimente mit verschiedensten anderen Fettsäuren allein oder in Kombination (z.B. Linolsäure, 20:3 Δ-5,11,14-Fettsäure, alpha- oder gamma Linolensäure, Stearidonsäure, Arachidonsäure, Eicosapentaensäure etc.) können zur detaillierteren Bestätigung der Substratspezifität und -Selektivität dieser Desaturasen durchgeführt werden.

**Tabelle 8: Ergebnis der Coxpression einer Phaeodactylum tricornutum Δ-5-Acyl Lipid Desaturase und einer Δ-6 Elongase aus Moos in Hefe auf Basis der Expressionsvektoren pYes2 und pYes6**

| | pYes2-Elo | | pYes2-Elo and pYes6-Ptd5 | |
|---|---|---|---|---|
| | +18:3 | +18:4 | +18:3 | +18:4 |
| 16:0 | 15,0 | 14,8 | 15,6 | 15,1 |
| 16:1Δ9 | 27,7 | 29,2 | 27,5 | 29,0 |
| 18:0 | 5,6 | 6,3 | 5,7 | 6,4 |
| 18:1Δ9 | 17,1 | 30,8 | 27,4 | 31,6 |
| 18:3Δ6,9,12 | 7,60 | - | 7,8 | - |
| 18:4Δ6,9,12,15 | - | 6,71 | - | 6,4 |
| 20:3Δ8,11,14 | 15,92 | - | 13,55 | - |
| 20:4Δ5,8,11,14 | - | - | 1,31 | - |
| 20:4Δ8,11,14,17 | - | 11,4 | - | 10,31 |
| 20:5Δ5,8,11,14,17 | - | - | - | 0,53 |

Aus den Substratumsetzungen geht hervor, dass die verwendete Δ-5-Desaturase aus Phaeodactylum und die Δ-6-Elongase aus Physcomitrella patens bzgl. der Substrataktivität und insbesondere der Substratspezifität geeignet sind, um Arachidonsäure bzw. Eicosapentaensäure mithilfe erfindungsgemäßer Sequenzen zu produzieren.

Die Fragmentierungsmuster und Massenspektren von DMOX-Derivaten von Standards als auch den Peakfraktionen per GC identifizierter Fettsäuren der in Tabelle 6, 7 und 8 aufgeführten, zeigen vergleichsweise identische Ergebnisse, wodurch die jeweilige Position der Doppelbindung über die bloße GC-Detektion hinaus abgesichert wurde.

### Beispiel 17: Reinigung des gewünschten Produktes aus transformierten Organismen

Die Gewinnung des gewünschten Produktes aus Pflanzenmaterial oder Pilzen, Algen, Ciliaten, tierischen Zellen oder aus dem Überstand der vorstehend beschriebenen Kulturen kann durch verschiedene, im Fachgebiet bekannte Verfahren erfolgen. Wird das gewünschte Produkt nicht aus den Zellen sezerniert, können die Zellen aus der Kultur durch langsame Zentrifugation geerntet werden, die Zellen können durch Standardtechniken, wie mechanische Kraft oder Ultraschallbehandlung, lysiert werden. Organe von Pflanzen können mechanisch von anderem Gewebe oder anderen Organen getrennt werden. Nach der Homogenisation werden die Zelltrümmer durch Zentrifugation entfernt, und die Überstandsfraktion, welche die löslichen Proteine enthält, wird zur weiteren Reinigung der gewünschten Verbindung aufbewahrt. Wird das Produkt aus gewünschten Zellen sezerniert, werden die Zellen durch langsame Zentrifugation aus der Kultur entfernt, und die Überstandsfraktion wird zur weiteren Reinigung aufbewahrt.

Die Überstandsfraktion aus jedem Reinigungsverfahren wird einer Chromatographie mit einem geeigneten Harz unterworfen, wobei das gewünschte Molekül entweder auf dem Chromatographieharz zurückgehalten wird, viele Verunreinigungen in der Probe jedoch nicht, oder die Verunreinigungen auf dem Harz zurückbleiben, die Probe hingegen nicht. Diese Chromatographieschritte können wenn nötig wiederholt werden, wobei die gleichen oder andere Chromatographieharze verwendet werden. Der Fachmann ist in der Auswahl geeigneter Chromatographieharze und ihrer wirksamsten Anwendung für ein bestimmtes zu reinigendes Molekül bewandert. Das gereinigte Produkt kann durch Filtration oder Ultrafiltration konzentriert und bei einer Temperatur aufbewahrt werden, bei der die Stabilität des Produktes maximal ist.

Im Fachgebiet ist ein breites Spektrum an Reinigungsverfahren bekannt, und das vorstehende Reinigungsverfahren soll nicht beschränkend sein. Diese Reinigungsverfahren sind zum Beispiel beschrieben in Bailey, J.E., & Ollis, D.F., Biochemical Engineering Fundamentals, McGraw-Hill: New York (1986).

Die Identität und Reinheit der isolierten Verbindungen kann durch Standardtechniken des Fachgebiets bestimmt werden. Dazu gehören Hochleistungs-Flüssigkeitschromatographie (HPLC), spektroskopische Verfahren, Färbeverfahren, Dünnschichtchromatographie, insbesondere Dünnschichtchromatographie und Flammenionisationsdetektion (IATROSCAN, Iatron, Tokio, Japan), NIRS, Enzymtest oder mikrobiologisch. Eine Übersicht über diese Analyseverfahren siehe in: Patek et al. (1994) Appl. Environ. Microbiol. 60:133-140; Malakhova et al. (1996) Biotekhnologiya 11:27-32; und Schmidt et al. (1998) Bioprocess Engineer. 19:67-70. Ulmann's Encyclopedia of Industrial Chemistry (1996) Bd. A27, VCH: Weinheim, S. 89-90, S. 521-540, S. 540-547, S. 559-566, 575-581 und S. 581-587; Michal, G (1999) Biochemical Pathways: An Atlas of Biochemistry and Molecular Biology, John Wiley and Sons; Fallon, A., et al. (1987) Applications of HPLC in Biochemistry in: Laboratory Techniques in Biochemistry and Molecular Biology, Bd. 17.

### Äquivalente

Der Fachmann erkennt oder kann viele Äquivalente der hier beschriebenen erfindungsgemäßen spezifischen Ausführungsformen feststellen, indem er lediglich Routineexperimente verwendet. Diese Äquivalente sollen von den Patentansprüchen umfasst sein.

## Patentansprüche

1. Verfahren zur Herstellung von Fettsäureestern mit einem erhöhtem Gehalt an mehrfach ungesättigten Fettsäuren mit mindestens zwei Doppelbindungen **dadurch gekennzeichnet, daß** man mindestens eine Nukleinsäure ausgewählt aus der Gruppe
a) einer Nukleinsäure mit der in SEQ ID NO: 1, SEQ ID NO: 3, SEQ ID NO: 5 oder SEQ ID NO: 11 dargestellten Sequenz,
b) Nukleinsäuren, die aufgrund des degenerierten genetischen Codes durch Rückübersetzung der in SEQ ID NO: 2, SEQ ID NO: 4, SEQ ID NO: 6 oder SEQ ID NO: 12 dargestellten Aminosäuresequenzen erhalten werden,
c) Derivate der in SEQ ID NO: 1, SEQ ID NO: 3, SEQ ID NO: 5 oder SEQ ID NO: 11 dargestellten Nukleinsäuren, die für biologisch aktive Teile einer Desaturase kodieren und mindestens 50 % Identität zu der Aminosäuresequenz der SEQ ID NO: 2, 4, 6 oder 12 aufweisen
in einen Fettsäureester produzierenden nicht-humanen Organismus einbringt, anzieht und die in dem Organismus enthaltenen Fettsäureester isoliert.

2. Verfahren nach Anspruch 1, wobei die durch das Verfahren hergestellten Fettsäureester mehrfach ungesättigte C₁₉-, C₂₀- oder C₂₂-Fettsäuremoleküle mit mindestens zwei Doppelbindungen im Fettsäureester enthalten.

3. Verfahren nach Anspruch 1 oder 2, wobei die C₁₈-, C₂₀- oder C₂₂-Fettsäuremoleküle aus dem Organismus in Form eines Öls oder Lipids isoliert werden.

4. Verfahren nach den Ansprüchen 1 bis 3, wobei der Organismus ein Mikroorganismus, ein nicht-humanes Tier oder eine Pflanze ist.

5. Verfahren nach den Ansprüchen 1 bis 4, wobei der Organismus eine transgene Pflanze ist.

6. Verfahren nach den Ansprüchen 1 bis 5, wobei die Fettsäureester, C₁₈-, C₂₀- oder C₂₂-Fettsäuren mit drei, vier oder fünf Doppelbindungen im Fettsäurenester enthalten.

7. Verfahren nach den Ansprüchen 1 bis 6, **dadurch gekennzeichnet, daß** man die in den Fettsäureestern enthaltenden mehrfach ungesättigten Fettsäuren freisetzt.

8. Isolierte Nukleinsäure, die für ein Polypeptid mit Desaturaseaktivität codiert, ausgewählt aus der Gruppe:
a) einer Nukleinsäure mit der in SEQ ID NO: 1, SEQ ID NO: 3, SEQ ID NO: 5 oder SEQ ID NO: 11 dargestellten Sequenz,
b) Nukleinsäuren, die aufgrund des degenerierten genetischen Codes durch Rückübersetzung der in SEQ ID NO: 2, SEQ ID NO: 4, SEQ ID NO: 6 oder SEQ ID NO: 12 dargestellten Aminosäuresequenzen erhalten werden,
c) Derivate der in SEQ ID NO: 1, SEQ ID NO: 3, SEQ ID NO: 5 oder SEQ ID NO: 11 dargestellten Nukleinsäuren, die für biologisch aktive Teile einer Desaturase kodieren und mindestens 50 % Identität zu der Aminosäuresequenz der SEQ ID NO: 2, 4, 6 oder 12 aufweisen.

9. Polypeptid codiert durch eine Nukleinsäuresequenz gemäß Anspruch 8.

10. Polypeptid nach Anspruch 9, codiert durch die in SEQ ID NO: 1, SEQ ID NO: 3, SEQ ID NO: 5 oder SEQ ID NO: 11 dargestellte Sequenz.

11. Nukleinsäurekonstrukt enthaltend eine Nukleinsäure gemäß Anspruch 8, wobei die Nukleinsäure mit einem oder mehreren Regulationssignalen verknüpft ist.

12. Nukleinsäurekonstrukt nach Anspruch 11, wobei im Nukleinsäurekonstrukt zusätzliche Biosynthesegenen des Fettsäure- oder Lipidstoffwechsels enthalten sind.

13. Nukleinsäurekonstrukt nach Anspruch 11 oder 12, wobei als Biosynthesegen des Fettsäure- oder Lipidstoffwechsels im Nukleinsäurekonstrukt ein Gen ausgewählt aus der Gruppe Acyl-CoA-Dehydrogenase(n), Acyl-ACP[= acyl carrier protein]-Desaturase(n), Acyl-ACP-Thioesterase(n), Fettsäure-Acyl-Transferase(n), Fettsäure-Synthase(n), Fettsäure-Hydroxylase(n), Acetyl-Coenzym A-Carboxylase(n), Acyl-Coenzym A-Oxidase(n), Fettsäure-Desaturase(n), Fettsäure-Acetylenasen, Lipoxygenasen, Triacylglycerol-Lipasen, Allenoxid-Synthasen, Hydroperoxid-Lyasen oder Fettsäure-Elongase (n) enthalten ist.

14. Vektor enthaltend eine Nukleinsäure gemäß Anspruch 8 oder ein Nukleinsäurekonstrukt gemäß Anspruch 11.

15. Transgene Pflanze enthaltend eine Nukleinsäure gemäß Anspruch 8, ein Nukleinsäurekonstrukt gemäß Anspruch 11 oder einen Vektor gemäß Anspruch 14.

16. Verwendung einer Nukleinsäure gemäß Anspruch 8 oder eines Nukleinsäurekonstruktes gemäß Anspruch 11 zur Herstellung von transgenen Pflanzen.

17. Antikörper, der spezifisch ein Polypeptid, das von einer der Nukleinsäuren gemäß Anspruch 8 a) oder b) codiert wird, bindet.

18. Antisense-Nukleinsäuremolekül, das die Komplementärsequenz der Nukleinsäure gemäß Anspruch 8 umfasst.

19. Kit, umfassend die Nukleinsäure gemäß Anspruch 8, das Nukleinsäurekonstrukt nach den Ansprüchen 11 bis 13, den Antikörper gemäß Anspruch 17, oder das Antisense-Nukleinsäuremolekül gemäß Anspruch 18.

20. Zusammensetzung, enthaltend den Antikörper gemäß Anspruch 17oder das Antisense-Nukleinsäurekonstrukt gemäß Anspruch 18.

## Claims

1. A method for the production of fatty acid esters with an increased content of polyunsaturated fatty acids with at least two double bonds, which comprises introducing, into a fatty-acid-ester-producing nonhuman organism, at least one nucleic acid selected from the group consisting of
a) a nucleic acid with the sequence shown in SEQ ID NO: 1, SEQ ID NO: 3, SEQ ID NO: 5 or SEQ ID NO: 11,
b) nucleic acids which, owing to the degeneracy of the genetic code, are obtained by backtranslating the amino acid sequences shown in SEQ ID NO: 2, SEQ ID NO: 4, SEQ ID NO: 6 or SEQ ID NO: 12,
c) derivatives of the nucleic acids shown in SEQ ID NO: 1, SEQ ID NO: 3, SEQ ID NO: 5 or SEQ ID NO: 11, which encode biologically active parts of a desaturase and have at least 50% identity to the amino acid sequence of SEQ ID NO: 2, 4, 6 or 12,
growing the organism, and isolating the fatty acid esters present in the organism.

2. The method according to claim 1, wherein the fatty acid esters produced by the method comprise polyunsaturated C₁₈-, C₂₀- or C₂₂-fatty acid molecules with at least two double bonds in the fatty acid ester.

3. The method according to claim 1 or 2, wherein the C₁₈-, C₂₀- or C₂₂-fatty acid molecules are isolated from the organism in the form of an oil or lipid.

4. The method according to any of claims 1 to 3, wherein the organism is a microorganism, a nonhuman animal or a plant.

5. The method according to any of claims 1 to 4, wherein the organism is a transgenic plant.

6. The method according to any of claims 1 to 5, wherein the fatty acid esters comprise C₁₈-, C₂₀- or C₂₂-fatty acids with three, four or five double bonds in the fatty acid ester.

7. The method according to any of claims 1 to 6, wherein the polyunsaturated fatty acids comprised in the fatty acid esters are liberated.

8. An isolated nucleic acid encoding a polypeptide with desaturase activity, selected from the group consisting of:
a) a nucleic acid with the sequence shown in SEQ ID NO: 1, SEQ ID NO: 3, SEQ ID NO: 5 or SEQ ID NO: 11,
b) nucleic acids which, owing to the degeneracy of the genetic code, are obtained by backtranslating the amino acid sequences shown in SEQ ID NO: 2, SEQ ID NO: 4, SEQ ID NO: 6 or SEQ ID NO: 12,
c) derivatives of the nucleic acids shown in SEQ ID NO: 1, SEQ ID NO: 3, SEQ ID NO: 5 or SEQ ID NO: 11, which encode biologically active parts of a desaturase and have at least 50% identity to the amino acid sequence of SEQ ID NO: 2, 4, 6 or 12.

9. A polypeptide encoded by a nucleic acid sequence according to claim 8.

10. The polypeptide according to claim 9 encoded by the sequence shown in SEQ ID NO: 1, SEQ ID NO: 3, SEQ ID NO: 5 or SEQ ID NO: 11.

11. A nucleic acid construct comprising a nucleic acid according to claim 8, wherein the nucleic acid is linked to one or more regulatory signals.

12. The nucleic acid construct according to claim 11, wherein additional biosynthesis genes of the fatty acid or lipid metabolism are present in the nucleic acid construct.

13. The nucleic acid construct according to claim 11 or 12, wherein the biosynthesis gene of the fatty acid or lipid metabolism which is present in the nucleic acid construct is a gene selected from the group consisting of acyl-CoA dehydrogenase(s), acyl-ACP[= acyl carrier protein] desaturase(s), acyl-ACP thioesterase(s), fatty acid acyl transferase(s), fatty acid synthase(s), fatty acid hydroxylase(s), acetyl-coenzyme A carboxylase(s), acyl-coenzyme A oxidase(s), fatty acid desaturase(s), fatty acid acetylenases, lipoxygenases, triacylglycerol lipases, allenoxide synthases, hydroperoxide lyases or fatty acid elongase(s).

14. A vector comprising a nucleic acid according to claim 8 or a nucleic acid construct according to claim 11.

15. A transgenic plant comprising a nucleic acid according to claim 8, a nucleic acid construct according to claim 11 or a vector according to claim 14.

16. The use of a nucleic acid according to claim 8 or of a nucleic acid construct according to claim 11 for generating transgenic plants.

17. An antibody which specifically binds a polypeptide encoded by one of the nucleic acids according to claim 8 a) or b).

18. An antisense nucleic acid molecule comprising the complementary sequence of the nucleic acid according to claim 8.

19. A kit encompassing the nucleic acid according to claim 8, the nucleic acid construct according to any of claims 11 to 13, the antibody according to claim 17, or the antisense nucleic acid molecule according to claim 18.

20. A composition comprising the antibody according to claim 17 or the antisense nucleic acid construct according to claim 18.

## Revendications

1. Procédé pour la préparation d'esters d'acides gras présentant une teneur élevée en acides gras polyinsaturés présentant au moins deux doubles liaisons, **caractérisé en ce qu'**on introduit au moins un acide nucléique, choisi dans le groupe
a) d'un acide nucléique présentant la séquence représentée dans SEQ ID NO : 1, SEQ ID NO : 3, SEQ ID NO : 5 ou SEQ ID NO : 11,
b) des acides nucléiques qui sont obtenus en raison du code génétique dégénéré par traduction en retour des séquences d'acides aminés représentées dans SEQ ID NO : 2, SEQ ID NO : 4, SEQ ID NO : 6 ou SEQ ID NO : 12,
c) des dérivés des acides nucléiques représentés dans SEQ ID NO : 1, SEQ ID NO : 3, SEQ ID NO : 5 ou SEQ ID NO : 11, qui codent pour des parties biologiquement actives d'une désaturase et qui présentent au moins 50% d'identité par rapport à la séquence d'acides aminés de SEQ ID NO : 2, 4, 6 ou 12
dans un organisme non humain produisant des esters d'acides gras, on cultive et on isole les esters d'acides gras obtenus dans l'organisme.

2. Procédé selon la revendication 1, où les esters d'acides gras préparés par le procédé contiennent des molécules d'acides gras en C₁₈, C₂₀ ou C₂₂ polyinsaturés présentant au moins deux doubles liaisons dans les esters d'acides gras.

3. Procédé selon la revendication 1 ou 2, où les molécules d'acides gras en C₁₈, C₂₀ ou C₂₂ sont isolées de l'organisme sous forme d'une huile ou d'un lipide.

4. Procédé selon les revendications 1 à 3, où l'organisme est un microorganisme, un animal non humain ou une plante.

5. Procédé selon les revendications 1 à 4, où l'organisme est une plante transgénique.

6. Procédé selon les revendications 1 à 5, où les esters d'acides gras contiennent des acides gras en C₁₈, C₂₀ ou C₂₂ présentant trois, quatre ou cinq doubles liaisons dans les esters d'acide gras.

7. Procédé selon les revendications 1 à 6, **caractérisé en ce qu'**on libère les acides gras polyinsaturés contenus dans les esters d'acides gras.

8. Acide nucléique isolé qui code pour un polypeptide avec une activité de désaturase, choisi dans le groupe :
a) d'un acide nucléique présentant la séquence représentée dans SEQ ID NO : 1, SEQ ID NO : 3, SEQ ID NO : 5 ou SEQ ID NO : 11,
b) des acides nucléiques qui sont obtenus en raison du code génétique dégénérée par traduction en retour des séquences d'acides aminés représentées dans SEQ ID NO : 2, SEQ ID NO : 4, SEQ ID NO : 6 ou SEQ ID NO : 12,
c) des dérivés des acides nucléiques représentés dans SEQ ID NO : 1, SEQ ID NO : 3, SEQ ID NO : 5 ou SEQ ID NO : 11, qui codent pour des parties biologiquement actives d'une désaturase et qui présentent au moins 50% d'identité par rapport à la séquence d'acides aminés de SEQ ID NO : 2, 4, 6 ou 12.

9. Polypeptide codé par une séquence d'acides nucléiques selon la revendication 8.

10. Polypeptide selon la revendication 9, codé par la séquence représentée dans SEQ ID NO : 1, SEQ ID NO : 3, SEQ ID NO : 5 ou SEQ ID NO : 11.

11. Elément d'acide nucléique contenant un acide nucléique selon la revendication 8, l'acide nucléique étant lié à un ou plusieurs signaux de régulation.

12. Elément d'acide nucléique selon la revendication 11, où l'élément d'acide nucléique contient des gènes supplémentaires de biosynthèse du métabolisme des acides gras ou des lipides.

13. Elément d'acide nucléique selon la revendication 11 ou 12, contenant comme gène de biosynthèse du métabolisme des acides gras ou des lipides dans l'élément d'acide nucléique un gène choisi dans le groupe du/des acyl-CoA-déshydrogénase(s), acyl-ACP[= acyl carrier protein, protéine porteuse de la fonction acyle]-désaturase(s), acyl-ACP-thioestérase(s), acide gras-acyl-transférase(s), acide gras-synthase(s), acide gras-hydroxylase(s), acétylcoenzyme A-carboxylase(s), acyl-coenzyme A-oxydase(s), acide gras-désaturase(s), acide gras-acétylénases, lipoxygénases, triacylglycérol-lipases, oxyde d'allène-synthases, hydroperoxyde-lyases ou acide gras-élongase(s).

14. Vecteur contenant un acide nucléique selon la revendication 8 ou un élément d'acide nucléique selon la revendication 11.

15. Plante transgénique contenant un acide nucléique selon la revendication 8, un élément d'acide nucléique selon la revendication 11 ou un vecteur selon la revendication 14.

16. Utilisation d'un acide nucléique selon la revendication 8 ou d'un élément d'acide nucléique selon la revendication 11 pour la production de plantes transgéniques.

17. Anticorps qui lie spécifiquement un polypeptide qui est codé par un des acides nucléiques selon la revendication 8 a) ou b).

18. Molécule d'acide nucléique antisens qui comprend la séquence complémentaire de l'acide nucléique selon la revendication 8.

19. Kit, comprenant l'acide nucléique selon la revendication 8, l'élément d'acide nucléique selon les revendications 11 à 13, l'anticorps selon la revendication 17, ou la molécule d'acide nucléique antisens selon la revendication 18.

20. Composition contenant l'anticorps selon la revendication 17 ou l'élément d'acide nucléique antisens selon la revendication 18.


### SEQUENZPROTOKOLL

<110> BASF Plant Science GmbH
<120> Verfahren zur Herstellung mehrfach ungesättigter Fettsäuren, neue Biosynthesegene sowie neue pflanzliche Expressionskonstrukte
<130> 2000_873
<140> 2000_873
   <141> 2000-12-22
<160> 31
<170> PatentIn Vers. 2.0
<210> 1
   <211> 1652
   <212> DNA
   <213> Phaeodactylum tricornutum
<220>
   <221> CDS
   <222> (115) .. (1524)
<400> 1
   gacccaacaa acccaacaat cccaacaatc ccatcaacag gaattgggtt tcgttgagtc 60
   aataattgct agaatccaaa cagacagaca gagaccaacc gcatctatta caga atg   117
                                                               Met
                                                               1
   gct ccg gat gcg gat aag ctt cga caa cgc cag acg act gcg gta gcg   165
   Ala Pro Asp Ala Asp Lys Leu Arg Gln Arg Gln Thr Thr Ala Val Ala
               5                   10                  15
   aag cac aat gct gct acc ata tcg acg cag gaa cgc ctt tgc agt ctg   213
   Lys His Asn Ala Ala Thr Ile Ser Thr Gln Glu Arg Leu Cys Ser Leu
           20                  25                  30
   tct tcg ctc aaa ggc gaa gaa gtc tgc atc gac gga atc atc tat gac   261
   Ser Ser Leu Lys Gly Glu Glu Val Cys Ile Asp Gly Ile Ile Tyr Asp
       35                  40                  45
   ctc caa tca ttc gat cat ccc ggg ggt gaa acg atc aaa atg ttt ggt   309
   Leu Gln Ser Phe Asp His Pro Gly Gly Glu Thr Ile Lys Met Phe Gly
   50                  55                  60                  65
   ggc aac gat gtc act gta cag tac aag atg att cac ccg tac cat acc   357
   Gly Asn Asp Val Thr Val Gln Tyr Lys Met Ile His Pro Tyr His Thr
                   70                  75                  80
   gag aag cat ttg gaa aag atg aag cgt gtc ggc aag gtg acg gat ttc   405
   Glu Lys His Leu Glu Lys Met Lys Arg Val Gly Lys Val Thr Asp Phe
               85                  90                  95
   gtc tgc gag tac aag ttc gat acc gaa ttt gaa cgc gaa atc aaa cga   453
   Val Cys Glu Tyr Lys Phe Asp Thr Glu Phe Glu Arg Glu Ile Lys Arg
           100                 105                 110
   gaa gtc ttc aag att gtg cga cga ggc aag gat ttc ggt act ttg gga     501
   Glu Val Phe Lys Ile Val Arg Arg Gly Lys Asp Phe Gly Thr Leu Gly
       115                 120                 125
   tgg ttc ttc cgt gcg ttt tgc tac att gcc att ttc ttc tac ctg cag     549
   Trp Phe Phe Arg Ala Phe Cys Tyr Ile Ala Ile Phe Phe Tyr Leu Gln
   130                 135                 140                 145
   tac cat tgg gtc acc acg gga acc tct tgg ctg ctg gcc gtg gcc tac     597
   Tyr His Trp Val Thr Thr Gly Thr Ser Trp Leu Leu Ala Val Ala Tyr
                   150                 155                 160
   gga atc tcc caa gcg atg att ggc atg aat gtc cag cac gat gcc aac     645
   Gly Ile Ser Gln Ala Met Ile Gly Met Asn Val Gln His Asp Ala Asn
               165                 170                 175
   cac ggg gcc acc tcc aag cgt ccc tgg gtc aac gac atg cta ggc ctc     693
   His Gly Ala Thr Ser Lys Arg Pro Trp Val Asn Asp Met Leu Gly Leu
           180                 185                 190
   ggt gcg gat ttt att ggt ggt tcc aag tgg ctc tgg cag gaa caa cac     741
   Gly Ala Asp Phe Ile Gly Gly Ser Lys Trp Leu Trp Gln Glu Gln His
       195                 200                 205
   tgg acc cac cac gct tac acc aat cac gcc gag atg gat ccc gat agc     789
   Trp Thr His His Ala Tyr Thr Asn His Ala Glu Met Asp Pro Asp Ser
   210                 215                 220                 225
   ttt ggt gcc gaa cca atg ctc cta ttc aac gac tat ccc ttg gat cat     837
   Phe Gly Ala Glu Pro Met Leu Leu Phe Asn Asp Tyr Pro Leu Asp His
                   230                 235                 240
   ccc gct cgt acc tgg cta cat cgc ttt caa gca ttc ttt tac atg ccc     885
   Pro Ala Arg Thr Trp Leu His Arg Phe Gln Ala Phe Phe Tyr Met Pro
               245                 250                 255
   gtc ttg gct gga tac tgg ttg tcc gct gtc ttc aat cca caa att ctt     933
   Val Leu Ala Gly Tyr Trp Leu Ser Ala Val Phe Asn Pro Gln Ile Leu
           260                 265                 270
   gac ctc cag caa cgc ggc gca ctt tcc gtc ggt atc cgt ctc gac aac     981
   Asp Leu Gln Gln Arg Gly Ala Leu Ser Val Gly Ile Arg Leu Asp Asn
       275                 280                 285
   gct ttc att cac tcg cga cgc aag tat gcg gtt ttc tgg cgg gct gtg     1029
   Ala Phe Ile His Ser Arg Arg Lys Tyr Ala Val Phe Trp Arg Ala Val
   290                 295                 300                 305
   tac att gcg gtg aac gtg att gct ccg ttt tac aca aac tcc ggc ctc     1077
   Tyr Ile Ala Val Asn Val Ile Ala Pro Phe Tyr Thr Asn Ser Gly Leu
                   310                 315                 320
   gaa tgg tcc tgg cgt gtc ttt gga aac atc atg ctc atg ggt gtg gcg     1125
   Glu Trp Ser Trp Arg Val Phe Gly Asn Ile Met Leu Met Gly Val Ala
               325                 330                 335
   gaa tcg ctc gcg ctg gcg gtc ctg ttt tcg ttg tcg cac aat ttc gaa   1173
   Glu Ser Leu Ala Leu Ala Val Leu Phe Ser Leu Ser His Asn Phe Glu
           340                 345                 350
   tcc gcg gat cgc gat ccg acc gcc cca ctg aaa aag acg gga gaa cca   1221
   Ser Ala Asp Arg Asp Pro Thr Ala Pro Leu Lys Lys Thr Gly Glu Pro
       355                 360                 365
   gtc gac tgg ttc aag aca cag gtc gaa act tcc tgc act tac ggt gga   1269
   Val Asp Trp Phe Lys Thr Gln Val Glu Thr Ser Cys Thr Tyr Gly Gly
   370                 375                 380                 385
   ttc ctt tcc ggt tgc ttc acg gga ggt ctc aac ttt cag gtt gaa cac   1317
   Phe Leu Ser Gly Cys Phe Thr Gly Gly Leu Asn Phe Gln Val Glu His
                   390                 395                 400
   cac ttg ttc cca cgc atg agc agc gct tgg tat ccc tac att gcc ccc   1365
   His Leu Phe Pro Arg Met Ser Ser Ala Trp Tyr Pro Tyr Ile Ala Pro
               405                 410                 415
   aag gtc cgc gaa att tgc gcc aaa cac ggc gtc cac tac gcc tac tac   1413
   Lys Val Arg Glu Ile Cys Ala Lys His Gly Val His Tyr Ala Tyr Tyr
           420                 425                 430
   ccg tgg atc cac caa aac ttt ctc tcc acc gtc cgc tac atg cac gcg   1461
   Pro Trp Ile His Gln Asn Phe Leu Ser Thr Val Arg Tyr Met His Ala
       435                 440                 445
   gcc ggg acc ggt gcc aac tgg cgc cag atg gcc aga gaa aat ccc ttg   1509
   Ala Gly Thr Gly Ala Asn Trp Arg Gln Met Ala Arg Glu Asn Pro Leu
   450                 455                 460                 465
   acc gga cgg gcg taa aagtacacga cacgaccaaa ggtggcgtat ggtgatctct   1564
   Thr Gly Arg Ala
                   470
   agaaaacaga catagcctac tggaaatatc gacgtccaaa caataatttt aaagactatt 1624
   tttctgcgta aaaaaaaaaa aaaaaaaa                                      1652
<210> 2
   <211> 469
   <212> PRT
   <213> Phaeodactylum tricornutum
<400> 2
   Met Ala Pro Asp Ala Asp Lys Leu Arg Gln Arg Gln Thr Thr Ala Val
   1 5 10 15
   Ala Lys His Asn Ala Ala Thr Ile Ser Thr Gln Glu Arg Leu Cys Ser
   20 25 30
   Leu Ser Ser Leu Lys Gly Glu Glu Val Cys Ile Asp Gly Ile Ile Tyr
   35 40 45
   Asp Leu Gln Ser Phe Asp His Pro Gly Gly Glu Thr Ile Lys Met Phe
   50 55 60
   Gly Gly Asn Asp Val Thr Val Gln Tyr Lys Met Ile His Pro Tyr His
   65 70 75 80
   Thr Glu Lys His Leu Glu Lys Met Lys Arg Val Gly Lys Val Thr Asp
   85 90 95
   Phe Val Cys Glu Tyr Lys Phe Asp Thr Glu Phe Glu Arg Glu Ile Lys
   100 105 110
   Arg Glu Val Phe Lys Ile Val Arg Arg Gly Lys Asp Phe Gly Thr Leu
   115 120 125
   Gly Trp Phe Phe Arg Ala Phe Cys Tyr Ile Ala Ile Phe Phe Tyr Leu
   130 135 140
   Gln Tyr His Trp Val Thr Thr Gly Thr Ser Trp Leu Leu Ala Val Ala
   145 150 155 160
   Tyr Gly Ile Ser Gln Ala Met Ile Gly Met Asn Val Gln His Asp Ala
   165 170 175
   Asn His Gly Ala Thr Ser Lys Arg Pro Trp Val Asn Asp Met Leu Gly
   180 185 190
   Leu Gly Ala Asp Phe Ile Gly Gly Ser Lys Trp Leu Trp Gln Glu Gln
   195 200 205
   His Trp Thr His His Ala Tyr Thr Asn His Ala Glu Met Asp Pro Asp
   210 215 220
   Ser Phe Gly Ala Glu Pro Met Leu Leu Phe Asn Asp Tyr Pro Leu Asp
   225 230 235 240
   His Pro Ala Arg Thr Trp Leu His Arg Phe Gln Ala Phe Phe Tyr Met
   245 250 255
   Pro Val Leu Ala Gly Tyr Trp Leu Ser Ala Val Phe Asn Pro Gln Ile
   260 265 270
   Leu Asp Leu Gln Gln Arg Gly Ala Leu Ser Val Gly Ile Arg Leu Asp
   275 280 285
   Asn Ala Phe Ile His Ser Arg Arg Lys Tyr Ala Val Phe Trp Arg Ala
   290 295 300
   Val Tyr Ile Ala Val Asn Val Ile Ala Pro Phe Tyr Thr Asn Ser Gly
   305 310 315 320
   Leu Glu Trp Ser Trp Arg Val Phe Gly Asn Ile Met Leu Met Gly Val
   325 330 335
   Ala Glu Ser Leu Ala Leu Ala Val Leu Phe Ser Leu Ser His Asn Phe
   340 345 350
   Glu Ser Ala Asp Arg Asp Pro Thr Ala Pro Leu Lys Lys Thr Gly Glu
   355 360 365
   Pro Val Asp Trp Phe Lys Thr Gln Val Glu Thr Ser Cys Thr Tyr Gly
   370 375 380
   Gly Phe Leu Ser Gly Cys Phe Thr Gly Gly Leu Asn Phe Gln Val Glu
   385 390 395 400
   His His Leu Phe Pro Arg Met Ser Ser Ala Trp Tyr Pro Tyr Ile Ala
   405 410 415
   Pro Lys Val Arg Glu Ile Cys Ala Lys His Gly Val His Tyr Ala Tyr
   420 425 430
   Tyr Pro Trp Ile His Gln Asn Phe Leu Ser Thr Val Arg Tyr Met His
   435 440 445
   Ala Ala Gly Thr Gly Ala Asn Trp Arg Gln Met Ala Arg Glu Asn Pro
   450 455 460
   Leu Thr Gly Arg Ala
   465
<210> 3
   <211> 1434
   <212> DNA
   <213> Phaeodactylum tricornutum
<220>
   <221> CDS
   <222> (1)..(1434)
<400> 3
   atg ggc aaa gga ggg gac gct cgg gcc tcg aag ggc tca acg gcg gct     48
   Met Gly Lys Gly Gly Asp Ala Arg Ala Ser Lys Gly Ser Thr Ala Ala
    1               5                  10                  15
   cgc aag atc agt tgg cag gaa gtc aag acc cac gcg tct ccg gag gac     96
   Arg Lys Ile Ser Trp Gln Glu Val Lys Thr His Ala Ser Pro Glu Asp
               20                  25                  30
   gcc tgg atc att cac tcc aat aag gtc tac gac gtg tcc aac tgg cac    144
   Ala Trp Ile Ile His Ser Asn Lys Val Tyr Asp Val Ser Asn Trp His
           35                  40                  45
   gaa cat ccc gga ggc gcc gtc att ttc acg cac gcc ggt gac gac atg    192
   Glu His Pro Gly Gly Ala Val Ile Phe Thr His Ala Gly Asp Asp Met
       50                  55                  60
   acg gac att ttc gct gcc ttt cac gca ccc gga tcg cag tcg ctc atg    240
   Thr Asp Ile Phe Ala Ala Phe His Ala Pro Gly Ser Gln Ser Leu Met
   65                  70                  75                  80
   aag aag ttc tac att ggc gaa ttg ctc ccg gaa acc acc ggc aag gag    288
   Lys Lys Phe Tyr Ile Gly Glu Leu Leu Pro Glu Thr Thr Gly Lys Glu
                   85                  90                  95
   ccg cag caa atc gcc ttt gaa aag ggc tac cgc gat ctg cgc tcc aaa    336
   Pro Gln Gln Ile Ala Phe Glu Lys Gly Tyr Arg Asp Leu Arg Ser Lys
               100                 105                 110
   ctc atc atg atg ggc atg ttc aag tcc aac aag tgg ttc tac gtc tac    384
   Leu Ile Met Met Gly Met Phe Lys Ser Asn Lys Trp Phe Tyr Val Tyr
           115                 120                 125
   aag tgc ctc agc aac atg gcc att tgg gcc gcc gcc tgt gct ctc gtc    432
   Lys Cys Leu Ser Asn Met Ala Ile Trp Ala Ala Ala Cys Ala Leu Val
       130                 135                 140
   ttt tac tcg gac cgc ttc tgg gta cac ctg gcc agc gcc gtc atg ctg    480
   Phe Tyr Ser Asp Arg Phe Trp Val His Leu Ala Ser Ala Val Met Leu
   145                 150                 155                 160
   gga aca ttc ttt cag cag tcg gga tgg ttg gca cac gac ttt ctg cac    528
   Gly Thr Phe Phe Gln Gln Ser Gly Trp Leu Ala His Asp Phe Leu His
                   165                 170                 175
   cac cag gtc ttc acc aag cgc aag cac ggg gat ctc gga gga ctc ttt    576
   His Gln Val Phe Thr Lys Arg Lys His Gly Asp Leu Gly Gly Leu Phe
               180                 185                 190
   tgg ggg aac ctc atg cag ggt tac tcc gta cag tgg tgg aaa aac aag    624
   Trp Gly Asn Leu Met Gln Gly Tyr Ser Val Gln Trp Trp Lys Asn Lys
           195                 200                 205
   cac aac gga cac cac gcc gtc ccc aac ctc cac tgc tcc tcc gca gtc    672
   His Asn Gly His His Ala Val Pro Asn Leu His Cys Ser Ser Ala Val
       210                 215                 220
   gcg caa gat ggg gac ccg gac atc gat acc atg ccc ctt ctc gcc tgg    720
   Ala Gln Asp Gly Asp Pro Asp Ile Asp Thr Met Pro Leu Leu Ala Trp
   225                 230                 235                 240
   tcc gtc cag caa gcc cag tct tac cgg gaa ctc caa gcc gac gga aag    768
   Ser Val Gln Gln Ala Gln Ser Tyr Arg Glu Leu Gln Ala Asp Gly Lys
                   245                 250                 255
   gat tcg ggt ttg gtc aag ttc atg atc cgt aac caa tcc tac ttt tac    816
   Asp Ser Gly Leu Val Lys Phe Met Ile Arg Asn Gln Ser Tyr Phe Tyr
               260                 265                 270
   ttt ccc atc ttg ttg ctc gcc cgc ctg tcg tgg ttg aac gag tcc ttc    864
   Phe Pro Ile Leu Leu Leu Ala Arg Leu Ser Trp Leu Asn Glu Ser Phe
           275                 280                 285
   aag tgc gcc ttt ggg ctt gga gct gcg tcg gag aac gct gct ctc gaa    912
   Lys Cys Ala Phe Gly Leu Gly Ala Ala Ser Glu Asn Ala Ala Leu Glu
       290                 295                 300
   ctc aag gcc aag ggt ctt cag tac ccc ctt ttg gaa aag gct ggc atc    960
   Leu Lys Ala Lys Gly Leu Gln Tyr Pro Leu Leu Glu Lys Ala Gly Ile
   305                 310                 315                 320
   ctg ctg cac tac gct tgg atg ctt aca gtt tcg tcc ggc ttt gga cgc    1008
   Leu Leu His Tyr Ala Trp Met Leu Thr Val Ser Ser Gly Phe Gly Arg
                   325                 330                 335
   ttc tcg ttc gcg tac acc gca ttt tac ttt cta acc gcg acc gcg tcc    1056
   Phe Ser Phe Ala Tyr Thr Ala Phe Tyr Phe Leu Thr Ala Thr Ala Ser
               340                 345                 350
   tgt gga ttc ttg ctc gcc att gtc ttt ggc ctc ggc cac aac ggc atg    1104
   Cys Gly Phe Leu Leu Ala Ile Val Phe Gly Leu Gly His Asn Gly Met
           355                 360                 365
   gcc acc tac aat gcc gac gcc cgt ccg gac ttc tgg aag ctc caa gtc    1152
   Ala Thr Tyr Asn Ala Asp Ala Arg Pro Asp Phe Trp Lys Leu Gln Val
       370                 375                 380
   acc acg act cgc aac gtc acg ggc gga cac ggt ttc ccc caa gcc ttt    1200
   Thr Thr Thr Arg Asn Val Thr Gly Gly His Gly Phe Pro Gln Ala Phe
   385                 390                 395                 400
   gtc gac tgg ttc tgt ggt ggc ctc cag tac caa gtc gac cac cac tta    1248
   Val Asp Trp Phe Cys Gly Gly Leu Gln Tyr Gln Val Asp His His Leu
                   405                 410                 415
   ttc ccc agc ctg ccc cga cac aat ctg gcc aag aca cac gca ctg gtc    1296
   Phe Pro Ser Leu Pro Arg His Asn Leu Ala Lys Thr His Ala Leu Val
               420                 425                 430
   gaa tcg ttc tgc aag gag tgg ggt gtc cag tac cac gaa gcc gac ctt    1344
   Glu Ser Phe Cys Lys Glu Trp Gly Val Gln Tyr His Glu Ala Asp Leu
           435                 440                 445
   gtg gac ggg acc atg gaa gtc ttg cac cat ttg ggc agc gtg gcc ggc    1392
   Val Asp Gly Thr Met Glu Val Leu His His Leu Gly Ser Val Ala Gly
       450                 455                 460
   gaa ttc gtc gtg gat ttt gta cgc gat gga ccc gcc atg taa            1434
   Glu Phe Val Val Asp Phe Val Arg Asp Gly Pro Ala Met
   465                 470                 475
<210> 4
   <211> 477
   <212> PRT
   <213> Phaeodactylum tricornutum
<400> 4
   Met Gly Lys Gly Gly Asp Ala Arg Ala Ser Lys Gly Ser Thr Ala Ala
   1 5 10 15
   Arg Lys Ile Ser Trp Gln Glu Val Lys Thr His Ala Ser Pro Glu Asp
   20 25 30
   Ala Trp Ile Ile His Ser Asn Lys Val Tyr Asp Val Ser Asn Trp His
   35 40 45
   Glu His Pro Gly Gly Ala Val Ile Phe Thr His Ala Gly Asp Asp Met
   50 55 60
   Thr Asp Ile Phe Ala Ala Phe His Ala Pro Gly Ser Gln Ser Leu Met
   65 70 75 80
   Lys Lys Phe Tyr Ile Gly Glu Leu Leu Pro Glu Thr Thr Gly Lys Glu
   85 90 95
   Pro Gln Gln Ile Ala Phe Glu Lys Gly Tyr Arg Asp Leu Arg Ser Lys
   100 105 110
   Leu Ile Met Met Gly Met Phe Lys Ser Asn Lys Trp Phe Tyr Val Tyr
   115 120 125
   Lys Cys Leu Ser Asn Met Ala Ile Trp Ala Ala Ala Cys Ala Leu Val
   130 135 140
   Phe Tyr Ser Asp Arg Phe Trp Val His Leu Ala Ser Ala Val Met Leu
   145 150 155 160
   Gly Thr Phe Phe Gln Gln Ser Gly Trp Leu Ala His Asp Phe Leu His
   165 170 175
   His Gln Val Phe Thr Lys Arg Lys His Gly Asp Leu Gly Gly Leu Phe
   180 185 190
   Trp Gly Asn Leu Met Gln Gly Tyr Ser Val Gln Trp Trp Lys Asn Lys
   195 200 205
   His Asn Gly His His Ala Val Pro Asn Leu His Cys Ser Ser Ala Val
   210 215 220
   Ala Gln Asp Gly Asp Pro Asp Ile Asp Thr Met Pro Leu Leu Ala Trp
   225 230 235 240
   Ser Val Gln Gln Ala Gln Ser Tyr Arg Glu Leu Gln Ala Asp Gly Lys
   245 250 255
   Asp Ser Gly Leu Val Lys Phe Met Ile Arg Asn Gln Ser Tyr Phe Tyr
   260 265 270
   Phe Pro Ile Leu Leu Leu Ala Arg Leu Ser Trp Leu Asn Glu Ser Phe
   275 280 285
   Lys Cys Ala Phe Gly Leu Gly Ala Ala Ser Glu Asn Ala Ala Leu Glu
   290 295 300
   Leu Lys Ala Lys Gly Leu Gln Tyr Pro Leu Leu Glu Lys Ala Gly Ile
   305 310 315 320
   Leu Leu His Tyr Ala Trp Met Leu Thr Val Ser Ser Gly Phe Gly Arg
   325 330 335
   Phe Ser Phe Ala Tyr Thr Ala Phe Tyr Phe Leu Thr Ala Thr Ala Ser
   340 345 350
   Cys Gly Phe Leu Leu Ala Ile Val Phe Gly Leu Gly His Asn Gly Met
   355 360 365
   Ala Thr Tyr Asn Ala Asp Ala Arg Pro Asp Phe Trp Lys Leu Gln Val
   370 375 380
   Thr Thr Thr Arg Asn Val Thr Gly Gly His Gly Phe Pro Gln Ala Phe
   385                 390                 395                 400
   Val Asp Trp Phe Cys Gly Gly Leu Gln Tyr Gln Val Asp His His Leu
                   405                 410                 415
   Phe Pro Ser Leu Pro Arg His Asn Leu Ala Lys Thr His Ala Leu Val
               420                 425                 430
   Glu Ser Phe Cys Lys Glu Trp Gly Val Gln Tyr His Glu Ala Asp Leu
           435                 440                 445
   Val Asp Gly Thr Met Glu Val Leu His His Leu Gly Ser Val Ala Gly
       450                 455                 460
   Glu Phe Val Val Asp Phe Val Arg Asp Gly Pro Ala Met
   465                 470                 475
<210> 5
   <211> 1651
   <212> DNA
   <213> Phaeodactylum tricornutum
<220>
   <221> CDS
   <222> (67)..(1554)
<400> 5
   gaagaaggaa catataaaag taagccatct cctcggcacc atctaaagac ctaatatcta 60
   ctcgtc atg gtt cgc ttt tca aca gcc gct cta ctt tct ctg tcg aca    108
          Met Val Arg Phe Ser Thr Ala Ala Leu Leu Ser Leu Ser Thr
           1               5                  10
   ttg aca act tca tgt att ggt gcc ttc cag ctg tct tcg cca gca caa    156
   Leu Thr Thr Ser Cys Ile Gly Ala Phe Gln Leu Ser Ser Pro Ala Gln
    15                  20                  25                  30
   ctt ccg aca agt agg ctt cgt cgg cat acg aac acg gcg ccg ctt tcg    204
   Leu Pro Thr Ser Arg Leu Arg Arg His Thr Asn Thr Ala Pro Leu Ser
                    35                  40                  45
   gcc gtg gcc gtc gac tcc ggt tct tcc gat ccg gcc ttg gta ggc aac    252
   Ala Val Ala Val Asp Ser Gly Ser Ser Asp Pro Ala Leu Val Gly Asn
                50                  55                  60
   ctc ccc ctt ccc aac aac aat gat aat gag gac aag aac cgt aga atg    300
   Leu Pro Leu Pro Asn Asn Asn Asp Asn Glu Asp Lys Asn Arg Arg Met
            65                  70                  75
   cca atg atg gac ttg aaa ggt att gct ctg tct ggt ctc aaa ggg caa    348
   Pro Met Met Asp Leu Lys Gly Ile Ala Leu Ser Gly Leu Lys Gly Gln
        80                  85                  90
   gct ctt tcc gtc cga gcg gaa gat ttt cct cag gcg aaa gac ttg cgt    396
   Ala Leu Ser Val Arg Ala Glu Asp Phe Pro Gln Ala Lys Asp Leu Arg
    95                 100                 105                 110
   gcc gtc att ccg aaa gat tgc ttc gaa ccc gac acg gcc aaa tcg ttg   444
   Ala Val Ile Pro Lys Asp Cys Phe Glu Pro Asp Thr Ala Lys Ser Leu
                   115                 120                 125
   gga tat ctt tcc gtt tca act atg ggg aca att ctc tgc tcc gtc gtc   492
   Gly Tyr Leu Ser Val Ser Thr Met Gly Thr Ile Leu Cys Ser Val Val
               130                 135                 140
   ggc gcg aac ctc ctt agt gtg ctc gat ccc tcc aat cca tta acc tgg   540
   Gly Ala Asn Leu Leu Ser Val Leu Asp Pro Ser Asn Pro Leu Thr Trp
           145                 150                 155
   cct ctc tgg gcg gcc tac ggt gcc gtc acg ggg acg gtc gcc atg ggg   588
   Pro Leu Trp Ala Ala Tyr Gly Ala Val Thr Gly Thr Val Ala Met Gly
       160                 165                 170
   ctt tgg gtg ctg gcc cac gaa tgc gga cac ggc gcc ttt tcc aaa aac   636
   Leu Trp Val Leu Ala His Glu Cys Gly His Gly Ala Phe Ser Lys Asn
   175                 180                 185                 190
   cga tcc ctc cag gat gcc gtg ggg tac att atc cat tcc atc atg ctg   684
   Arg Ser Leu Gln Asp Ala Val Gly Tyr Ile Ile His Ser Ile Met Leu
                   195                 200                 205
   gtg cca tac ttt agt tgg cag cga tcg cat gcc gtg cat cac cag tat   732
   Val Pro Tyr Phe Ser Trp Gln Arg Ser His Ala Val His His Gln Tyr
               210                 215                 220
   acc aat cat atg gaa ctg ggg gaa aca cac gtt cct gat cga gcc gat   780
   Thr Asn His Met Glu Leu Gly Glu Thr His Val Pro Asp Arg Ala Asp
           225                 230                 235
   aag gag ggc gag aag agc ctg gcg ctc cgc cag ttc atg ttg gat tcc   828
   Lys Glu Gly Glu Lys Ser Leu Ala Leu Arg Gln Phe Met Leu Asp Ser
       240                 245                 250
   ttt ggt aaa gac aag ggc atg aaa gca tac gga ggc ctc cag tcg ttt   876
   Phe Gly Lys Asp Lys Gly Met Lys Ala Tyr Gly Gly Leu Gln Ser Phe
   255                 260                 265                 270
   ttg cat ctc atc gtg gga tgg cca gcc tac ctc ctg atc ggt gcg acc   924
   Leu His Leu Ile Val Gly Trp Pro Ala Tyr Leu Leu Ile Gly Ala Thr
                   275                 280                 285
   ggt gga ccc gac cgt ggt atg acc aac cat ttt tat ccc aac cct ttg   972
   Gly Gly Pro Asp Arg Gly Met Thr Asn His Phe Tyr Pro Asn Pro Leu
               290                 295                 300
   tcg acg cca aca cag ccc aag aaa gaa ctt ttc cct ggg aac tgg aaa   1020
   Ser Thr Pro Thr Gln Pro Lys Lys Glu Leu Phe Pro Gly Asn Trp Lys
           305                 310                 315
   gaa aag gtc tac cag tca gat att gga atc gcc gcc gtt gtc ggc gcc   1068
   Glu Lys Val Tyr Gln Ser Asp Ile Gly Ile Ala Ala Val Val Gly Ala
       320                 325                 330
   ctc att gct tgg acc gcc act tcg ggt cta gcc ccc gtc atg gcc ttg   1116
   Leu Ile Ala Trp Thr Ala Thr Ser Gly Leu Ala Pro Val Met Ala Leu
   335                 340                 345                 350
   tac ggt ggt ccc ttg atc gtc att aat gcc tgg ctg gta ctg tac acg     1164
   Tyr Gly Gly Pro Leu Ile Val Ile Asn Ala Trp Leu Val Leu Tyr Thr
                   355                 360                 365
   tgg ttg caa cat aca gat acc gat gtt ccg cac ttt tcc tcc gac aac     1212
   Trp Leu Gln His Thr Asp Thr Asp Val Pro His Phe Ser Ser Asp Asn
               370                 375                 380
   cac aac ttt gtc aag ggc gca ctg cat acg atc gat cgt ccc tac gac     1260
   His Asn Phe Val Lys Gly Ala Leu His Thr Ile Asp Arg Pro Tyr Asp
           385                 390                 395
   aaa ctt gat ccc tgg gga atc ata gac ttt ctg cac cac aag att gga     1308
   Lys Leu Asp Pro Trp Gly Ile Ile Asp Phe Leu His His Lys Ile Gly
       400                 405                 410
   aca acg cat gtg gca cac cat ttt gac agt act atc ccc cac tat aag     1356
   Thr Thr His Val Ala His His Phe Asp Ser Thr Ile Pro His Tyr Lys
   415                 420                 425                 430
   gct cag att gct acc gat gcc atc aaa gcc aag ttt cca gaa gtg tac     1404
   Ala Gln Ile Ala Thr Asp Ala Ile Lys Ala Lys Phe Pro Glu Val Tyr
                   435                 440                 445
   ctc tat gac ccg aca cca att cca caa gcc atg tgg cgc gtc gcc aag     1452
   Leu Tyr Asp Pro Thr Pro Ile Pro Gln Ala Met Trp Arg Val Ala Lys
               450                 455                 460
   gga tgt act gca gta gag caa cgc ggt gac gcc tgg gtg tgg aaa aac     1500
   Gly Cys Thr Ala Val Glu Gln Arg Gly Asp Ala Trp Val Trp Lys Asn
           465                 470                 475
   gaa gga ata gaa gat ttg gtg gaa cat cgt caa agc aaa tta tcg agc     1548
   Glu Gly Ile Glu Asp Leu Val Glu His Arg Gln Ser Lys Leu Ser Ser
       480                 485                 490
   gaa taa agcaacatat cgctttatgg aagaacaaac gtccattgtg taaaaccctg     1604
   Glu
   495
   ataatttcaa tattgtgttt tgttttaaaa aaaaaaaaaa aaaaaaa                1651
<210> 6
   <211> 495
   <212> PRT
   <213> Phaeodactylum tricornutum
<400> 6
   Met Val Arg Phe Ser Thr Ala Ala Leu Leu Ser Leu Ser Thr Leu Thr
    1               5                   10                  15
   Thr Ser Cys Ile Gly Ala Phe Gln Leu Ser Ser Pro Ala Gln Leu Pro
               20                  25                  30
   Thr Ser Arg Leu Arg Arg His Thr Asn Thr Ala Pro Leu Ser Ala Val
   35 40 45
   Ala Val Asp Ser Gly Ser Ser Asp Pro Ala Leu Val Gly Asn Leu Pro
   50 55 60
   Leu Pro Asn Asn Asn Asp Asn Glu Asp Lys Asn Arg Arg Met Pro Met
   65 70 75 80
   Met Asp Leu Lys Gly Ile Ala Leu Ser Gly Leu Lys Gly Gln Ala Leu
   85 90 95
   Ser Val Arg Ala Glu Asp Phe Pro Gln Ala Lys Asp Leu Arg Ala Val
   100 105 110
   Ile Pro Lys Asp Cys Phe Glu Pro Asp Thr Ala Lys Ser Leu Gly Tyr
   115 120 125
   Leu Ser Val Ser Thr Met Gly Thr Ile Leu Cys Ser Val Val Gly Ala
   130 135 140
   Asn Leu Leu Ser Val Leu Asp Pro Ser Asn Pro Leu Thr Trp Pro Leu
   145 150 155 160
   Trp Ala Ala Tyr Gly Ala Val Thr Gly Thr Val Ala Met Gly Leu Trp
   165 170 175
   Val Leu Ala His Glu Cys Gly His Gly Ala Phe Ser Lys Asn Arg Ser
   180 185 190
   Leu Gln Asp Ala Val Gly Tyr Ile Ile His Ser Ile Met Leu Val Pro
   195 200 205
   Tyr Phe Ser Trp Gln Arg Ser His Ala Val His His Gln Tyr Thr Asn
   210 215 220
   His Met Glu Leu Gly Glu Thr His Val Pro Asp Arg Ala Asp Lys Glu
   225 230 235 240
   Gly Glu Lys Ser Leu Ala Leu Arg Gln Phe Met Leu Asp Ser Phe Gly
   245 250 255
   Lys Asp Lys Gly Met Lys Ala Tyr Gly Gly Leu Gln Ser Phe Leu His
   260 265 270
   Leu Ile Val Gly Trp Pro Ala Tyr Leu Leu Ile Gly Ala Thr Gly Gly
   275 280 285
   Pro Asp Arg Gly Met Thr Asn His Phe Tyr Pro Asn Pro Leu Ser Thr
   290 295 300
   Pro Thr Gln Pro Lys Lys Glu Leu Phe Pro Gly Asn Trp Lys Glu Lys
   305 310 315 320
   Val Tyr Gln Ser Asp Ile Gly Ile Ala Ala Val Val Gly Ala Leu Ile
   325 330 335
   Ala Trp Thr Ala Thr Ser Gly Leu Ala Pro Val Met Ala Leu Tyr Gly
   340 345 350
   Gly Pro Leu Ile Val Ile Asn Ala Trp Leu Val Leu Tyr Thr Trp Leu
   355 360 365
   Gln His Thr Asp Thr Asp Val Pro His Phe Ser Ser Asp Asn His Asn
   370 375 380
   Phe Val Lys Gly Ala Leu His Thr Ile Asp Arg Pro Tyr Asp Lys Leu
   385 390 395 400
   Asp Pro Trp Gly Ile Ile Asp Phe Leu His His Lys Ile Gly Thr Thr
   405 410 415
   His Val Ala His His Phe Asp Ser Thr Ile Pro His Tyr Lys Ala Gln
   420 425 430
   Ile Ala Thr Asp Ala Ile Lys Ala Lys Phe Pro Glu Val Tyr Leu Tyr
   435 440 445
   Asp Pro Thr Pro Ile Pro Gln Ala Met Trp Arg Val Ala Lys Gly Cys
   450 455 460
   Thr Ala Val Glu Gln Arg Gly Asp Ala Trp Val Trp Lys Asn Glu Gly
   465 470 475 480
   Ile Glu Asp Leu Val Glu His Arg Gln Ser Lys Leu Ser Ser Glu
   485 490 495
<210> 7
   <211> 1578
   <212> DNA
   <213> Physcomitrella patens
<220>
   <221> CDS
   <222> (1) .. (1578)
<400> 7
   atg gta ttc gcg ggc ggt gga ctt cag cag ggc tct ctc gaa gaa aac     48
   Met Val Phe Ala Gly Gly Gly Leu Gln Gln Gly Ser Leu Glu Glu Asn
    1               5                  10                  15
   atc gac gtc gag cac att gcc agt atg tct ctc ttc agc gac ttc ttc     96
   Ile Asp Val Glu His Ile Ala Ser Met Ser Leu Phe Ser Asp Phe Phe
               20                  25                  30
   agt tat gtg tct tca act gtt ggt tcg tgg agc gta cac agt ata caa    144
   Ser Tyr Val Ser Ser Thr Val Gly Ser Trp Ser Val His Ser Ile Gln
           35                  40                  45
   cct ttg aag cgc ctg acg agt aag aag cgt gtt tcg gaa agc gct gcc    192
   Pro Leu Lys Arg Leu Thr Ser Lys Lys Arg Val Ser Glu Ser Ala Ala
       50                  55                  60
   gtg caa tgt ata tca gct gaa gtt cag aga aat tcg agt acc cag gga    240
   Val Gln Cys Ile Ser Ala Glu Val Gln Arg Asn Ser Ser Thr Gln Gly
   65                  70                   75                  80
   act gcg gag gca ctc gca gaa tca gtc gtg aag ccc acg aga cga agg      288
   Thr Ala Glu Ala Leu Ala Glu Ser Val Val Lys Pro Thr Arg Arg Arg
                   85                  90                  95
   tca tct cag tgg aag aag tcg aca cac ccc cta tca gaa gta gca gta      336
   Ser Ser Gln Trp Lys Lys Ser Thr His Pro Leu Ser Glu Val Ala Val
               100                 105                 110
   cac aac aag cca agc gat tgc tgg att gtt gta aaa aac aag gtg tat      384
   His Asn Lys Pro Ser Asp Cys Trp Ile Val Val Lys Asn Lys Val Tyr
           115                 120                 125
   gat gtt tcc aat ttt gcg gac gag cat ccc gga gga tca gtt att agt      432
   Asp Val Ser Asn Phe Ala Asp Glu His Pro Gly Gly Ser Val Ile Ser
       130                 135                 140
   act tat ttt gga cga gac ggc aca gat gtt ttc tct agt ttt cat gca      480
   Thr Tyr Phe Gly Arg Asp Gly Thr Asp Val Phe Ser Ser Phe His Ala
   145                 150                 155                 160
   gct tct aca tgg aaa att ctt caa gac ttt tac att ggt gac gtg gag      528
   Ala Ser Thr Trp Lys Ile Leu Gln Asp Phe Tyr Ile Gly Asp Val Glu
                   165                 170                 175
   agg gtg gag ccg act cca gag ctg ctg aaa gat ttc cga gaa atg aga      576
   Arg Val Glu Pro Thr Pro Glu Leu Leu Lys Asp Phe Arg Glu Met Arg
               180                 185                 190
   gct ctt ttc ctg agg gag caa ctt ttc aaa agt tcg aaa ttg tac tat      624
   Ala Leu Phe Leu Arg Glu Gln Leu Phe Lys Ser Ser Lys Leu Tyr Tyr
           195                 200                 205
   gtt atg aag ctg ctc acg aat gtt gct att ttt gct gcg agc att gca      672
   Val Met Lys Leu Leu Thr Asn Val Ala Ile Phe Ala Ala Ser Ile Ala
       210                 215                 220
   ata ata tgt tgg agc aag act att tca gcg gtt ttg gct tca gct tgt      720
   Ile Ile Cys Trp Ser Lys Thr Ile Ser Ala Val Leu Ala Ser Ala Cys
   225                 230                 235                 240
   atg atg gct ctg tgt ttc caa cag tgc gga tgg cta tcc cat gat ttt      768
   Met Met Ala Leu Cys Phe Gln Gln Cys Gly Trp Leu Ser His Asp Phe
                   245                 250                 255
   ctc cac aat cag gtg ttt gag aca cgc tgg ctt aat gaa gtt gtc ggg      816
   Leu His Asn Gln Val Phe Glu Thr Arg Trp Leu Asn Glu Val Val Gly
               260                 265                 270
   tat gtg atc ggc aac gcc gtt ctg ggg ttt agt aca ggg tgg tgg aag      864
   Tyr Val Ile Gly Asn Ala Val Leu Gly Phe Ser Thr Gly Trp Trp Lys
           275                 280                 285
   gag aag cat aac ctt cat cat gct gct cca aat gaa tgc gat cag act      912
   Glu Lys His Asn Leu His His Ala Ala Pro Asn Glu Cys Asp Gln Thr
       290                 295                 300
   tac caa cca att gat gaa gat att gat act ctc ccc ctc att gcc tgg     960
   Tyr Gln Pro Ile Asp Glu Asp Ile Asp Thr Leu Pro Leu Ile Ala Trp
   305                 310                 315                 320
   agc aag gac ata ctg gcc aca gtt gag aat aag aca ttc ttg cga atc    1008
   Ser Lys Asp Ile Leu Ala Thr Val Glu Asn Lys Thr Phe Leu Arg Ile
                   325                 330                 335
   ctc caa tac cag cat ctg ttc ttc atg ggt ctg tta ttt ttc gcc cgt    1056
   Leu Gln Tyr Gln His Leu Phe Phe Met Gly Leu Leu Phe Phe Ala Arg
               340                 345                 350
   ggt agt tgg ctc ttt tgg agc tgg aga tat acc tct aca gca gtg ctc    1104
   Gly Ser Trp Leu Phe Trp Ser Trp Arg Tyr Thr Ser Thr Ala Val Leu
           355                 360                 365
   tca cct gtc gac agg ttg ttg gag aag gga act gtt ctg ttt cac tac    1152
   Ser Pro Val Asp Arg Leu Leu Glu Lys Gly Thr Val Leu Phe His Tyr
       370                 375                 380
   ttt tgg ttc gtc ggg aca gcg tgc tat ctt ctc cct ggt tgg aag cca    1200
   Phe Trp Phe Val Gly Thr Ala Cys Tyr Leu Leu Pro Gly Trp Lys Pro
   385                 390                 395                 400
   tta gta tgg atg gcg gtg act gag ctc atg tcc ggc atg ctg ctg ggc    1248
   Leu Val Trp Met Ala Val Thr Glu Leu Met Ser Gly Met Leu Leu Gly
                   405                 410                 415
   ttt gta ttt gta ctt agc cac aat ggg atg gag gtt tat aat tcg tct    1296
   Phe Val Phe Val Leu Ser His Asn Gly Met Glu Val Tyr Asn Ser Ser
               420                 425                 430
   aaa gaa ttc gtg agt gca cag atc gta tcc aca cgg gat atc aaa gga    1344
   Lys Glu Phe Val Ser Ala Gln Ile Val Ser Thr Arg Asp Ile Lys Gly
           435                 440                 445
   aac ata ttc aac gac tgg ttc act ggt ggc ctt aac agg caa ata gag    1392
   Asn Ile Phe Asn Asp Trp Phe Thr Gly Gly Leu Asn Arg Gln Ile Glu
       450                 455                 460
   cat cat ctt ttc cca aca atg ccc agg cat aat tta aac aaa ata gca    1440
   His His Leu Phe Pro Thr Met Pro Arg His Asn Leu Asn Lys Ile Ala
   465                 470                 475                 480
   cct aga gtg gag gtg ttc tgt aag aaa cac ggt ctg gtg tac gaa gac    1488
   Pro Arg Val Glu Val Phe Cys Lys Lys His Gly Leu Val Tyr Glu Asp
                   485                 490                 495
   gta tct att gct acc ggc act tgc aag gtt ttg aaa gca ttg aag gaa    1536
   Val Ser Ile Ala Thr Gly Thr Cys Lys Val Leu Lys Ala Leu Lys Glu
               500                 505                 510
   gtc gcg gag gct gcg gca gag cag cat gct acc acc agt taa            1578
   Val Ala Glu Ala Ala Ala Glu Gln His Ala Thr Thr Ser
           515                 520                 525
<210> 8
   <211> 525
   <212> PRT
   <213> Physcomitrella patens
<400> 8
   Met Val Phe Ala Gly Gly Gly Leu Gln Gln Gly Ser Leu Glu Glu Asn
    1               5                  10                  15
   Ile Asp Val Glu His Ile Ala Ser Met Ser Leu Phe Ser Asp Phe Phe
               20                  25                  30
   Ser Tyr Val Ser Ser Thr Val Gly Ser Trp Ser Val His Ser Ile Gln
           35                  40                  45
   Pro Leu Lys Arg Leu Thr Ser Lys Lys Arg Val Ser Glu Ser Ala Ala
       50                  55                  60
   Val Gln Cys Ile Ser Ala Glu Val Gln Arg Asn Ser Ser Thr Gln Gly
   65                  70                  75                  80
   Thr Ala Glu Ala Leu Ala Glu Ser Val Val Lys Pro Thr Arg Arg Arg
                   85                  90                  95
   Ser Ser Gln Trp Lys Lys Ser Thr His Pro Leu Ser Glu Val Ala Val
               100                 105                 110
   His Asn Lys Pro Ser Asp Cys Trp Ile Val Val Lys Asn Lys Val Tyr
           115                 120                 125
   Asp Val Ser Asn Phe Ala Asp Glu His Pro Gly Gly Ser Val Ile Ser
       130                 135                 140
   Thr Tyr Phe Gly Arg Asp Gly Thr Asp Val Phe Ser Ser Phe His Ala
   145                 150                 155                 160
   Ala Ser Thr Trp Lys Ile Leu Gln Asp Phe Tyr Ile Gly Asp Val Glu
                   165                 170                 175
   Arg Val Glu Pro Thr Pro Glu Leu Leu Lys Asp Phe Arg Glu Met Arg
               180                 185                 190
   Ala Leu Phe Leu Arg Glu Gln Leu Phe Lys Ser Ser Lys Leu Tyr Tyr
           195                 200                 205
   Val Met Lys Leu Leu Thr Asn Val Ala Ile Phe Ala Ala Ser Ile Ala
       210                 215                 220
   Ile Ile Cys Trp Ser Lys Thr Ile Ser Ala Val Leu Ala Ser Ala Cys
   225                 230                 235                 240
   Met Met Ala Leu Cys Phe Gln Gln Cys Gly Trp Leu Ser His Asp Phe
                   245                 250                 255
   Leu His Asn Gln Val Phe Glu Thr Arg Trp Leu Asn Glu Val Val Gly
               260                 265                 270
   Tyr Val Ile Gly Asn Ala Val Leu Gly Phe Ser Thr Gly Trp Trp Lys
           275                 280                 285
   Glu Lys His Asn Leu His His Ala Ala Pro Asn Glu Cys Asp Gln Thr
   290 295 300
   Tyr Gln Pro Ile Asp Glu Asp Ile Asp Thr Leu Pro Leu Ile Ala Trp
   305 310 315 320
   Ser Lys Asp Ile Leu Ala Thr Val Glu Asn Lys Thr Phe Leu Arg Ile
   325 330 335
   Leu Gln Tyr Gln His Leu Phe Phe Met Gly Leu Leu Phe Phe Ala Arg
   340 345 350
   Gly Ser Trp Leu Phe Trp Ser Trp Arg Tyr Thr Ser Thr Ala Val Leu
   355 360 365
   Ser Pro Val Asp Arg Leu Leu Glu Lys Gly Thr Val Leu Phe His Tyr
   370 375 380
   Phe Trp Phe Val Gly Thr Ala Cys Tyr Leu Leu Pro Gly Trp Lys Pro
   385 390 395 400
   Leu Val Trp Met Ala Val Thr Glu Leu Met Ser Gly Met Leu Leu Gly
   405 410 415
   Phe Val Phe Val Leu Ser His Asn Gly Met Glu Val Tyr Asn Ser Ser
   420 425 430
   Lys Glu Phe Val Ser Ala Gln Ile Val Ser Thr Arg Asp Ile Lys Gly
   435 440 445
   Asn Ile Phe Asn Asp Trp Phe Thr Gly Gly Leu Asn Arg Gln Ile Glu
   450 455 460
   His His Leu Phe Pro Thr Met Pro Arg His Asn Leu Asn Lys Ile Ala
   465 470 475 480
   Pro Arg Val Glu Val Phe Cys Lys Lys His Gly Leu Val Tyr Glu Asp
   485 490 495
   Val Ser Ile Ala Thr Gly Thr Cys Lys Val Leu Lys Ala Leu Lys Glu
   500 505 510
   Val Ala Glu Ala Ala Ala Glu Gln His Ala Thr Thr Ser
   515 520 525
<210> 9
   <211> 873
   <212> DNA
   <213> Physcomitrella patens
<220>
   <221> CDS
   <222> (1)..(873)
<400> 9
   atg gag gtc gtg gag aga ttc tac ggt gag ttg gat ggg aag gtc tcg 48
   Met Glu Val Val Glu Arg Phe Tyr Gly Glu Leu Asp Gly Lys Val Ser
   1               5                   10                  15
   cag ggc gtg aat gca ttg ctg ggt agt ttt ggg gtg gag ttg acg gat     96
   Gln Gly Val Asn Ala Leu Leu Gly Ser Phe Gly Val Glu Leu Thr Asp
               20                  25                  30
   acg ccc act acc aaa ggc ttg ccc ctc gtt gac agt ccc aca ccc atc    144
   Thr Pro Thr Thr Lys Gly Leu Pro Leu Val Asp Ser Pro Thr Pro Ile
           35                  40                  45
   gtc ctc ggt gtt tct gta tac ttg act att gtc att gga ggg ctt ttg    192
   Val Leu Gly Val Ser Val Tyr Leu Thr Ile Val Ile Gly Gly Leu Leu
       50                  55                  60
   tgg ata aag gcc agg gat ctg aaa ccg cgc gcc tcg gag cca ttt ttg    240
   Trp Ile Lys Ala Arg Asp Leu Lys Pro Arg Ala Ser Glu Pro Phe Leu
   65                  70                  75                  80
   ctc caa gct ttg gtg ctt gtg cac aac ctg ttc tgt ttt gcg ctc agt    288
   Leu Gln Ala Leu Val Leu Val His Asn Leu Phe Cys Phe Ala Leu Ser
                   85                  90                  95
   ctg tat atg tgc gtg ggc atc gct tat cag gct att acc tgg cgg tac    336
   Leu Tyr Met Cys Val Gly Ile Ala Tyr Gln Ala Ile Thr Trp Arg Tyr
               100                 105                 110
   tct ctc tgg ggc aat gca tac aat cct aaa cat aaa gag atg gcg att    384
   Ser Leu Trp Gly Asn Ala Tyr Asn Pro Lys His Lys Glu Met Ala Ile
           115                 120                 125
   ctg gta tac ttg ttc tac atg tct aag tac gtg gaa ttc atg gat acc    432
   Leu Val Tyr Leu Phe Tyr Met Ser Lys Tyr Val Glu Phe Met Asp Thr
       130                 135                 140
   gtt atc atg ata ctg aag cgc agc acc agg caa ata agc ttc ctc cac    480
   Val Ile Met Ile Leu Lys Arg Ser Thr Arg Gln Ile Ser Phe Leu His
   145                 150                 155                 160
   gtt tat cat cat tct tca att tcc ctc att tgg tgg gct att gct cat    528
   Val Tyr His His Ser Ser Ile Ser Leu Ile Trp Trp Ala Ile Ala His
                   165                 170                 175
   cac gct cct ggc ggt gaa gca tat tgg tct gcg gct ctg aac tca gga    576
   His Ala Pro Gly Gly Glu Ala Tyr Trp Ser Ala Ala Leu Asn Ser Gly
               180                 185                 190
   gtg cat gtt ctc atg tat gcg tat tac ttc ttg gct gcc tgc ctt cga    624
   Val His Val Leu Met Tyr Ala Tyr Tyr Phe Leu Ala Ala Cys Leu Arg
           195                 200                 205
   agt agc cca aag tta aaa aat aag tac ctt ttt tgg ggc agg tac ttg    672
   Ser Ser Pro Lys Leu Lys Asn Lys Tyr Leu Phe Trp Gly Arg Tyr Leu
       210                 215                 220
   aca caa ttc caa atg ttc cag ttt atg ctg aac tta gtg cag gct tac    720
   Thr Gln Phe Gln Met Phe Gln Phe Met Leu Asn Leu Val Gln Ala Tyr
   225                 230                 235                 240
   tac gac atg aaa acg aat gcg cca tat cca caa tgg ctg atc aag att     768
   Tyr Asp Met Lys Thr Asn Ala Pro Tyr Pro Gln Trp Leu Ile Lys Ile
                   245                 250                 255
   ttg ttc tac tac atg atc tcg ttg ctg ttt ctt ttc ggc aat ttt tac     816
   Leu Phe Tyr Tyr Met Ile Ser Leu Leu Phe Leu Phe Gly Asn Phe Tyr
               260                 265                 270
   gta caa aaa tac atc aaa ccc tct gac gga aag caa aag gga gct aaa     864
   Val Gln Lys Tyr Ile Lys Pro Ser Asp Gly Lys Gln Lys Gly Ala Lys
           275                 280                 285
   act gag tga                                                         873
   Thr Glu
       290
<210> 10
   <211> 290
   <212> PRT
   <213> Physcomitrella patens
<400> 10
   Met Glu Val Val Glu Arg Phe Tyr Gly Glu Leu Asp Gly Lys Val Ser
   1 5 10 15
   Gln Gly Val Asn Ala Leu Leu Gly Ser Phe Gly Val Glu Leu Thr Asp
   20 25 30
   Thr Pro Thr Thr Lys Gly Leu Pro Leu Val Asp Ser Pro Thr Pro Ile
   35 40 45
   Val Leu Gly Val Ser Val Tyr Leu Thr Ile Val Ile Gly Gly Leu Leu
   50 55 60
   Trp Ile Lys Ala Arg Asp Leu Lys Pro Arg Ala Ser Glu Pro Phe Leu
   65 70 75 80
   Leu Gln Ala Leu Val Leu Val His Asn Leu Phe Cys Phe Ala Leu Ser
   85 90 95
   Leu Tyr Met Cys Val Gly Ile Ala Tyr Gln Ala Ile Thr Trp Arg Tyr
   100 105 110
   Ser Leu Trp Gly Asn Ala Tyr Asn Pro Lys His Lys Glu Met Ala Ile
   115 120 125
   Leu Val Tyr Leu Phe Tyr Met Ser Lys Tyr Val Glu Phe Met Asp Thr
   130 135 140
   Val Ile Met Ile Leu Lys Arg Ser Thr Arg Gln Ile Ser Phe Leu His
   145 150 155 160
   Val Tyr His His Ser Ser Ile Ser Leu Ile Trp Trp Ala Ile Ala His
   165 170 175
   His Ala Pro Gly Gly Glu Ala Tyr Trp Ser Ala Ala Leu Asn Ser Gly
   180 185 190
   Val His Val Leu Met Tyr Ala Tyr Tyr Phe Leu Ala Ala Cys Leu Arg
   195 200 205
   Ser Ser Pro Lys Leu Lys Asn Lys Tyr Leu Phe Trp Gly Arg Tyr Leu
   210 215 220
   Thr Gln Phe Gln Met Phe Gln Phe Met Leu Asn Leu Val Gln Ala Tyr
   225 230 235 240
   Tyr Asp Met Lys Thr Asn Ala Pro Tyr Pro Gln Trp Leu Ile Lys Ile
   245 250 255
   Leu Phe Tyr Tyr Met Ile Ser Leu Leu Phe Leu Phe Gly Asn Phe Tyr
   260 265 270
   Val Gln Lys Tyr Ile Lys Pro Ser Asp Gly Lys Gln Lys Gly Ala Lys
   275 280 285
   Thr Glu
   290
<210> 11
   <211> 1526
   <212> DNA
   <213> Phaeodactylum tricornutum
<220>
   <221> CDS
   <222> (92)..(1402)
<400> 11
   gcttccgtta gcgtcccata gtttgttaca cttggctgtg aaacgaatac gttcttggtc 60
   tacttactac aacgaagcaa ccaccagcag c atg ggt aag gga ggt caa cga     112
                                      Met Gly Lys Gly Gly Gln Arg
                                      1               5
   gct gta gct ccc aag agt gcc acc agc tct act ggc agt gct acc ctt    160
   Ala Val Ala Pro Lys Ser Ala Thr Ser Ser Thr Gly Ser Ala Thr Leu
           10                  15                  20
   agc caa agc aag gaa cag gta tgg act tcg tcg tac aac cct ctg gcg    208
   Ser Gln Ser Lys Glu Gln Val Trp Thr Ser Ser Tyr Asn Pro Leu Ala
       25                  30                  35
   aag gat tcc ccg gag ctg cca acc aaa ggc caa atc aag gcc gtc att    256
   Lys Asp Ser Pro Glu Leu Pro Thr Lys Gly Gln Ile Lys Ala Val Ile
   40                  45                  50                  55
   ccg aag gaa tgt ttc caa cgc tca gcc ttt tgg tct acc ttc tac ctg    304
   Pro Lys Glu Cys Phe Gln Arg Ser Ala Phe Trp Ser Thr Phe Tyr Leu
                   60                  65                  70
   atg cgc gat ctc gcc atg gct gcc gcc ttt tgc tac gga acc tca cag    352
   Met Arg Asp Leu Ala Met Ala Ala Ala Phe Cys Tyr Gly Thr Ser Gln
                   75                  80                  85
   gtc ctc tcc acc gac ctt ccc caa gac gcc acg ctc att ctg ccc tgg     400
   Val Leu Ser Thr Asp Leu Pro Gln Asp Ala Thr Leu Ile Leu Pro Trp
            90                  95                 100
   gct ctc ggc tgg ggc gtc tac gcc ttt tgg atg gga acc att ctc acc     448
   Ala Leu Gly Trp Gly Val Tyr Ala Phe Trp Met Gly Thr Ile Leu Thr
       105                 110                 115
   ggg cct tgg gta gtt gcg cac gaa tgt gga cac ggc gct tac tcc gac     496
   Gly Pro Trp Val Val Ala His Glu Cys Gly His Gly Ala Tyr Ser Asp
   120                 125                 130                 135
   tcc cag acg ttc aat gac gtg gtc ggc ttt atc gtc cac caa gct ttg     544
   Ser Gln Thr Phe Asn Asp Val Val Gly Phe Ile Val His Gln Ala Leu
                   140                 145                 150
   ctc gtc ccc tac ttt gcc tgg cag tac acc cac gcg aaa cac cac cgt     592
   Leu Val Pro Tyr Phe Ala Trp Gln Tyr Thr His Ala Lys His His Arg
               155                 160                 165
   cga acc aac cat ctg gtg gac ggc gag tcc cac gtc cct tct acc gcc     640
   Arg Thr Asn His Leu Val Asp Gly Glu Ser His Val Pro Ser Thr Ala
           170                 175                 180
   aag gat aac ggc ctc ggg ccg cac aac gag cga aac tcc ttc tac gcc     688
   Lys Asp Asn Gly Leu Gly Pro His Asn Glu Arg Asn Ser Phe Tyr Ala
       185                 190                 195
   gcg tgg cac gag gcc atg gga gac ggc gcc ttt gcc gtc ttt caa gtc     736
   Ala Trp His Glu Ala Met Gly Asp Gly Ala Phe Ala Val Phe Gln Val
   200                 205                 210                 215
   tgg tcg cac ttg ttc gtc ggc tgg cct ctc tac ttg gcc ggt ctg gcc     784
   Trp Ser His Leu Phe Val Gly Trp Pro Leu Tyr Leu Ala Gly Leu Ala
                   220                 225                 230
   agt acc gga aag ctt gcg cac gaa ggt tgg tgg ctg gaa gaa cgg aac     832
   Ser Thr Gly Lys Leu Ala His Glu Gly Trp Trp Leu Glu Glu Arg Asn
               235                 240                 245
   gcg att gcg gat cac ttt cga ccc agc tct ccc atg ttc ccc gcc aag     880
   Ala Ile Ala Asp His Phe Arg Pro Ser Ser Pro Met Phe Pro Ala Lys
           250                 255                 260
   atc cgt gcc aag att gcc ctt tcc agc gcg acg gaa ctc gcc gtg ctc     928
   Ile Arg Ala Lys Ile Ala Leu Ser Ser Ala Thr Glu Leu Ala Val Leu
       265                 270                 275
   gct gga ctc ttg tat gtc ggt aca cag gtc gga cac ctt ccc gtc ctg     976
   Ala Gly Leu Leu Tyr Val Gly Thr Gln Val Gly His Leu Pro Val Leu
   280                 285                 290                 295
   ctg tgg tac tgg gga ccg tac acc ttt gtc aac gct tgg ctt gta ctc    1024
   Leu Trp Tyr Trp Gly Pro Tyr Thr Phe Val Asn Ala Trp Leu Val Leu
                   300                 305                 310
   tac acg tgg ctg cag cat acg gac ccg tcc atc ccg cac tac ggt gaa    1072
   Tyr Thr Trp Leu Gln His Thr Asp Pro Ser Ile Pro His Tyr Gly Glu
               315                 320                 325
   ggc gag tgg acc tgg gtc aag ggc gcg ctc tct acc att gat cga gac    1120
   Gly Glu Trp Thr Trp Val Lys Gly Ala Leu Ser Thr Ile Asp Arg Asp
           330                 335                 340
   tac ggc atc ttc gat ttc ttt cac cac acc atc ggt tcc acg cac gtg    1168
   Tyr Gly Ile Phe Asp Phe Phe His His Thr Ile Gly Ser Thr His Val
       345                 350                 355
   gta cac cat ttg ttc cac gaa atg ccc tgg tac aat gcc ggc att gcc    1216
   Val His His Leu Phe His Glu Met Pro Trp Tyr Asn Ala Gly Ile Ala
   360                 365                 370                 375
   acg caa aag gtc aag gaa ttt ttg gaa ccc cag ggc ttg tac aat tac    1264
   Thr Gln Lys Val Lys Glu Phe Leu Glu Pro Gln Gly Leu Tyr Asn Tyr
                   380                 385                 390
   gat ccg acc ccc tgg tac aag gcc atg tgg cgc att gcc cgg acc tgt    1312
   Asp Pro Thr Pro Trp Tyr Lys Ala Met Trp Arg Ile Ala Arg Thr Cys
               395                 400                 405
   cac tat gtg gag tca aac gag ggt gtg cag tat ttc aag agt atg gaa    1360
   His Tyr Val Glu Ser Asn Glu Gly Val Gln Tyr Phe Lys Ser Met Glu
           410                 415                 420
   aac gtg ccg ctg act aag gat gtg cga aac aaa gcc gca tga            1402
   Asn Val Pro Leu Thr Lys Asp Val Arg Asn Lys Ala Ala
       425                 430                 435
   gaaaaagtgc caccgacgca taattttaca atcctaccaa caagaccaac attatatggt 1462
   tttcgcttaa aagatagttt tttctaccat ctgtgtagtc ggcacaaaaa aaaaaaaaaa 1522
   aaaa                                                              1526
<210> 12
   <211> 436
   <212> PRT
   <213> Phaeodactylum tricornutum
<400> 12
   Met Gly Lys Gly Gly Gln Arg Ala Val Ala Pro Lys Ser Ala Thr Ser
    1               5                  10                  15
   Ser Thr Gly Ser Ala Thr Leu Ser Gln Ser Lys Glu Gln Val Trp Thr
               20                  25                  30
   Ser Ser Tyr Asn Pro Leu Ala Lys Asp Ser Pro Glu Leu Pro Thr Lys
           35                  40                  45
   Gly Gln Ile Lys Ala Val Ile Pro Lys Glu Cys Phe Gln Arg Ser Ala
       50                  55                  60
   Phe Trp Ser Thr Phe Tyr Leu Met Arg Asp Leu Ala Met Ala Ala Ala
   65 70 75 80
   Phe Cys Tyr Gly Thr Ser Gln Val Leu Ser Thr Asp Leu Pro Gln Asp
   85 90 95
   Ala Thr Leu Ile Leu Pro Trp Ala Leu Gly Trp Gly Val Tyr Ala Phe
   100 105 110
   Trp Met Gly Thr Ile Leu Thr Gly Pro Trp Val Val Ala His Glu Cys
   115 120 125
   Gly His Gly Ala Tyr Ser Asp Ser Gln Thr Phe Asn Asp Val Val Gly
   130 135 140
   Phe Ile Val His Gln Ala Leu Leu Val Pro Tyr Phe Ala Trp Gln Tyr
   145 150 155 160
   Thr His Ala Lys His His Arg Arg Thr Asn His Leu Val Asp Gly Glu
   165 170 175
   Ser His Val Pro Ser Thr Ala Lys Asp Asn Gly Leu Gly Pro His Asn
   180 185 190
   Glu Arg Asn Ser Phe Tyr Ala Ala Trp His Glu Ala Met Gly Asp Gly
   195 200 205
   Ala Phe Ala Val Phe Gln Val Trp Ser His Leu Phe Val Gly Trp Pro
   210 215 220
   Leu Tyr Leu Ala Gly Leu Ala Ser Thr Gly Lys Leu Ala His Glu Gly
   225 230 235 240
   Trp Trp Leu Glu Glu Arg Asn Ala Ile Ala Asp His Phe Arg Pro Ser
   245 250 255
   Ser Pro Met Phe Pro Ala Lys Ile Arg Ala Lys Ile Ala Leu Ser Ser
   260 265 270
   Ala Thr Glu Leu Ala Val Leu Ala Gly Leu Leu Tyr Val Gly Thr Gln
   275 280 285
   Val Gly His Leu Pro Val Leu Leu Trp Tyr Trp Gly Pro Tyr Thr Phe
   290 295 300
   Val Asn Ala Trp Leu Val Leu Tyr Thr Trp Leu Gln His Thr Asp Pro
   305 310 315 320
   Ser Ile Pro His Tyr Gly Glu Gly Glu Trp Thr Trp Val Lys Gly Ala
   325 330 335
   Leu Ser Thr Ile Asp Arg Asp Tyr Gly Ile Phe Asp Phe Phe His His
   340 345 350
   Thr Ile Gly Ser Thr His Val Val His His Leu Phe His Glu Met Pro
   355 360 365
   Trp Tyr Asn Ala Gly Ile Ala Thr Gln Lys Val Lys Glu Phe Leu Glu
   370 375 380
   Pro Gln Gly Leu Tyr Asn Tyr Asp Pro Thr Pro Trp Tyr Lys Ala Met
   385 390 395 400
   Trp Arg Ile Ala Arg Thr Cys His Tyr Val Glu Ser Asn Glu Gly Val
   405 410 415
   Gln Tyr Phe Lys Ser Met Glu Asn Val Pro Leu Thr Lys Asp Val Arg
   420 425 430
   Asn Lys Ala Ala
   435
<210> 13
   <211> 3598
   <212> DNA
   <213> Unknown
<220>
   <223> Sequenz stellt eine pflanzliche Promotor-Terminator-Expressionskassette in Vektor pUC19 dar
<400> 13
   tcgcgcgttt cggtgatgac ggtgaaaacc tctgacacat gcagctcccg gagacggtca 60
   cagcttgtct gtaagcggat gccgggagca gacaagcccg tcagggcgcg tcagcgggtg 120
   ttggcgggtg tcggggctgg cttaactatg cggcatcaga gcagattgta ctgagagtgc 180
   accatatgcg gtgtgaaata ccgcacagat gcgtaaggag aaaataccgc atcaggcgcc 240
   attcgccatt caggctgcgc aactgttggg aagggcgatc ggtgcgggcc tcttcgctat 300
   tacgccagct ggcgaaaggg ggatgtgctg caaggcgatt aagttgggta acgccagggt 360
   tttcccagtc acgacgttgt aaaacgacgg ccagtgaatt cggcgcgccg agctcctcga 420
   gcaaatttac acattgccac taaacgtcta aacccttgta atttgttttt gttttactat 480
   gtgtgttatg tatttgattt gcgataaatt tttatatttg gtactaaatt tataacacct 540
   tttatgctaa cgtttgccaa cacttagcaa tttgcaagtt gattaattga ttctaaatta 600
   tttttgtctt ctaaatacat atactaatca actggaaatg taaatatttg ctaatatttc 660
   tactatagga gaattaaagt gagtgaatat ggtaccacaa ggtttggaga tttaattgtt 720
   gcaatgctgc atggatggca tatacaccaa acattcaata attcttgagg ataataatgg 780
   taccacacaa gatttgaggt gcatgaacgt cacgtggaca aaaggtttag taatttttca 840
   agacaacaat gttaccacac acaagttttg aggtgcatgc atggatgccc tgtggaaagt 900
   ttaaaaatat tttggaaatg atttgcatgg aagccatgtg taaaaccatg acatccactt 960
   ggaggatgca ataatgaaga aaactacaaa tttacatgca actagttatg catgtagtct 1020
   atataatgag gattttgcaa tactttcatt catacacact cactaagttt tacacgatta 1080
   taatttcttc atagccagcc caccgcggtg ggcggccgcc tgcagtctag aaggcctcct 1140
   gctttaatga gatatgcgag acgcctatga tcgcatgata tttgctttca attctgttgt 1200
   gcacgttgta aaaaacctga gcatgtgtag ctcagatcct taccgccggt ttcggttcat 1260
   tctaatgaat atatcacccg ttactatcgt atttttatga ataatattct ccgttcaatt 1320
   tactgattgt ccgtcgacga attcgagctc ggcgcgccaa gcttggcgta atcatggtca 1380
   tagctgtttc ctgtgtgaaa ttgttatccg ctcacaattc cacacaacat acgagccgga 1440
   agcataaagt gtaaagcctg gggtgcctaa tgagtgagct aactcacatt aattgcgttg 1500
   cgctcactgc ccgctttcca gtcgggaaac ctgtcgtgcc agctgcatta atgaatcggc 1560
   caacgcgcgg ggagaggcgg tttgcgtatt gggcgctctt ccgcttcctc gctcactgac 1620
   tcgctgcgct cggtcgttcg gctgcggcga gcggtatcag ctcactcaaa ggcggtaata 1680
   cggttatcca cagaatcagg ggataacgca ggaaagaaca tgtgagcaaa aggccagcaa 1740
   aaggccagga accgtaaaaa ggccgcgttg ctggcgtttt tccataggct ccgcccccct 1800
   gacgagcatc acaaaaatcg acgctcaagt cagaggtggc gaaacccgac aggactataa 1860
   agataccagg cgtttccccc tggaagctcc ctcgtgcgct ctcctgttcc gaccctgccg 1920
   cttaccggat acctgtccgc ctttctccct tcgggaagcg tggcgctttc tcatagctca 1980
   cgctgtaggt atctcagttc ggtgtaggtc gttcgctcca agctgggctg tgtgcacgaa 2040
   ccccccgttc agcccgaccg ctgcgcctta tccggtaact atcgtcttga gtccaacccg 2100
   gtaagacacg acttatcgcc actggcagca gccactggta acaggattag cagagcgagg 2160
   tatgtaggcg gtgctacaga gttcttgaag tggtggccta actacggcta cactagaagg 2220
   acagtatttg gtatctgcgc tctgctgaag ccagttacct tcggaaaaag agttggtagc 2280
   tcttgatccg gcaaacaaac caccgctggt agcggtggtt tttttgtttg caagcagcag 2340
   attacgcgca gaaaaaaagg atctcaagaa gatcctttga tcttttctac ggggtctgac 2400
   gctcagtgga acgaaaactc acgttaaggg attttggtca tgagattatc aaaaaggatc 2460
   ttcacctaga tccttttaaa ttaaaaatga agttttaaat caatctaaag tatatatgag 2520
   taaacttggt ctgacagtta ccaatgctta atcagtgagg cacctatctc agcgatctgt 2580
   ctatttcgtt catccatagt tgcctgactc cccgtcgtgt agataactac gatacgggag 2640
   ggcttaccat ctggccccag tgctgcaatg ataccgcgag acccacgctc accggctcca 2700
   gatttatcag caataaacca gccagccgga agggccgagc gcagaagtgg tcctgcaact 2760
   ttatccgcct ccatccagtc tattaattgt tgccgggaag ctagagtaag tagttcgcca 2820
   gttaatagtt tgcgcaacgt tgttgccatt gctacaggca tcgtggtgtc acgctcgtcg 2880
   tttggtatgg cttcattcag ctccggttcc caacgatcaa ggcgagttac atgatccccc 2940
   atgttgtgca aaaaagcggt tagctccttc ggtcctccga tcgttgtcag aagtaagttg 3000
   gccgcagtgt tatcactcat ggttatggca gcactgcata attctcttac tgtcatgcca 3060
   tccgtaagat gcttttctgt gactggtgag tactcaacca agtcattctg agaatagtgt 3120
   atgcggcgac cgagttgctc ttgcccggcg tcaatacggg ataataccgc gccacatagc 3180
   agaactttaa aagtgctcat cattggaaaa cgttcttcgg ggcgaaaact ctcaaggatc 3240
   ttaccgctgt tgagatccag ttcgatgtaa cccactcgtg cacccaactg atcttcagca 3300
   tcttttactt tcaccagcgt ttctgggtga gcaaaaacag gaaggcaaaa tgccgcaaaa 3360
   aagggaataa gggcgacacg gaaatgttga atactcatac tcttcctttt tcaatattat 3420
   tgaagcattt atcagggtta ttgtctcatg agcggataca tatttgaatg tatttagaaa 3480
   aataaacaaa taggggttcc gcgcacattt ccccgaaaag tgccacctga cgtctaagaa 3540
   accattatta tcatgacatt aacctataaa aataggcgta tcacgaggcc ctttcgtc 3598
<210> 14
   <211> 3590
   <212> DNA
   <213> Unknown
<220>
   <223> Sequenz stellt eine pflanzliche Promotor-Terminator-Expressionskassette in Vektor pUC19 dar
<400> 14
   tcgcgcgttt cggtgatgac ggtgaaaacc tctgacacat gcagctcccg gagacggtca 60
   cagcttgtct gtaagcggat gccgggagca gacaagcccg tcagggcgcg tcagcgggtg 120
   ttggcgggtg tcggggctgg cttaactatg cggcatcaga gcagattgta ctgagagtgc 180
   accatatgcg gtgtgaaata ccgcacagat gcgtaaggag aaaataccgc atcaggcgcc 240
   attcgccatt caggctgcgc aactgttggg aagggcgatc ggtgcgggcc tcttcgctat 300
   tacgccagct ggcgaaaggg ggatgtgctg caaggcgatt aagttgggta acgccagggt 360
   tttcccagtc acgacgttgt aaaacgacgg ccagtgaatt cggcgcgccg agctcctcga 420
   gcaaatttac acattgccac taaacgtcta aacccttgta atttgttttt gttttactat 480
   gtgtgttatg tatttgattt gcgataaatt tttatatttg gtactaaatt tataacacct 540
   tttatgctaa cgtttgccaa cacttagcaa tttgcaagtt gattaattga ttctaaatta 600
   tttttgtctt ctaaatacat atactaatca actggaaatg taaatatttg ctaatatttc 660
   tactatagga gaattaaagt gagtgaatat ggtaccacaa ggtttggaga tttaattgtt 720
   gcaatgctgc atggatggca tatacaccaa acattcaata attcttgagg ataataatgg 780
   taccacacaa gatttgaggt gcatgaacgt cacgtggaca aaaggtttag taatttttca 840
   agacaacaat gttaccacac acaagttttg aggtgcatgc atggatgccc tgtggaaagt 900
   ttaaaaatat tttggaaatg atttgcatgg aagccatgtg taaaaccatg acatccactt 960
   ggaggatgca ataatgaaga aaactacaaa tttacatgca actagttatg catgtagtct 1020
   atataatgag gattttgcaa tactttcatt catacacact cactaagttt tacacgatta 1080
   taatttcttc atagccagcg gatccgatat cgggcccgct agcgttaacc ctgctttaat 1140
   gagatatgcg agacgcctat gatcgcatga tatttgcttt caattctgtt gtgcacgttg 1200
   taaaaaacct gagcatgtgt agctcagatc cttaccgccg gtttcggttc attctaatga 1260
   atatatcacc cgttactatc gtatttttat gaataatatt ctccgttcaa tttactgatt 1320
   gtccgtcgac gaattcgagc tcggcgcgcc aagcttggcg taatcatggt catagctgtt 1380
   tcctgtgtga aattgttatc cgctcacaat tccacacaac atacgagccg gaagcataaa 1440
   gtgtaaagcc tggggtgcct aatgagtgag ctaactcaca ttaattgcgt tgcgctcact 1500
   gcccgctttc cagtcgggaa acctgtcgtg ccagctgcat taatgaatcg gccaacgcgc 1560
   ggggagaggc ggtttgcgta ttgggcgctc ttccgcttcc tcgctcactg actcgctgcg 1620
   ctcggtcgtt cggctgcggc gagcggtatc agctcactca aaggcggtaa tacggttatc 1680
   cacagaatca ggggataacg caggaaagaa catgtgagca aaaggccagc aaaaggccag 1740
   gaaccgtaaa aaggccgcgt tgctggcgtt tttccatagg ctccgccccc ctgacgagca 1800
   tcacaaaaat cgacgctcaa gtcagaggtg gcgaaacccg acaggactat aaagatacca 1860
   ggcgtttccc cctggaagct ccctcgtgcg ctctcctgtt ccgaccctgc cgcttaccgg 1920
   atacctgtcc gcctttctcc cttcgggaag cgtggcgctt tctcatagct cacgctgtag 1980
   gtatctcagt tcggtgtagg tcgttcgctc caagctgggc tgtgtgcacg aaccccccgt 2040
   tcagcccgac cgctgcgcct tatccggtaa ctatcgtctt gagtccaacc cggtaagaca 2100
   cgacttatcg ccactggcag cagccactgg taacaggatt agcagagcga ggtatgtagg 2160
   cggtgctaca gagttcttga agtggtggcc taactacggc tacactagaa ggacagtatt 2220
   tggtatctgc gctctgctga agccagttac cttcggaaaa agagttggta gctcttgatc 2280
   cggcaaacaa accaccgctg gtagcggtgg tttttttgtt tgcaagcagc agattacgcg 2340
   cagaaaaaaa ggatctcaag aagatccttt gatcttttct acggggtctg acgctcagtg 2400
   gaacgaaaac tcacgttaag ggattttggt catgagatta tcaaaaagga tcttcaccta 2460
   gatcctttta aattaaaaat gaagttttaa atcaatctaa agtatatatg agtaaacttg 2520
   gtctgacagt taccaatgct taatcagtga ggcacctatc tcagcgatct gtctatttcg 2580
   ttcatccata gttgcctgac tccccgtcgt gtagataact acgatacggg agggcttacc 2640
   atctggcccc agtgctgcaa tgataccgcg agacccacgc tcaccggctc cagatttatc 2700
   agcaataaac cagccagccg gaagggccga gcgcagaagt ggtcctgcaa ctttatccgc 2760
   ctccatccag tctattaatt gttgccggga agctagagta agtagttcgc cagttaatag 2820
   tttgcgcaac gttgttgcca ttgctacagg catcgtggtg tcacgctcgt cgtttggtat 2880
   ggcttcattc agctccggtt cccaacgatc aaggcgagtt acatgatccc ccatgttgtg 2940
   caaaaaagcg gttagctcct tcggtcctcc gatcgttgtc agaagtaagt tggccgcagt 3000
   gttatcactc atggttatgg cagcactgca taattctctt actgtcatgc catccgtaag 3060
   atgcttttct gtgactggtg agtactcaac caagtcattc tgagaatagt gtatgcggcg 3120
   accgagttgc tcttgcccgg cgtcaatacg ggataatacc gcgccacata gcagaacttt 3180
   aaaagtgctc atcattggaa aacgttcttc ggggcgaaaa ctctcaagga tcttaccgct 3240
   gttgagatcc agttcgatgt aacccactcg tgcacccaac tgatcttcag catcttttac 3300
   tttcaccagc gtttctgggt gagcaaaaac aggaaggcaa aatgccgcaa aaaagggaat 3360
   aagggcgaca cggaaatgtt gaatactcat actcttcctt tttcaatatt attgaagcat 3420
   ttatcagggt tattgtctca tgagcggata catatttgaa tgtatttaga aaaataaaca 3480
   aataggggtt ccgcgcacat ttccccgaaa agtgccacct gacgtctaag aaaccattat 3540
   tatcatgaca ttaacctata aaaataggcg tatcacgagg ccctttcgtc 3590
<210> 15
   <211> 3584
   <212> DNA
   <213> Unknown
<220>
   <223> Sequenz stellt eine pflanzliche Promotor-Terminator-Expressionskassette in Vektor pUC19 dar
<400> 15
   tcgcgcgttt cggtgatgac ggtgaaaacc tctgacacat gcagctcccg gagacggtca 60
   cagcttgtct gtaagcggat gccgggagca gacaagcccg tcagggcgcg tcagcgggtg 120
   ttggcgggtg tcggggctgg cttaactatg cggcatcaga gcagattgta ctgagagtgc 180
   accatatgcg gtgtgaaata ccgcacagat gcgtaaggag aaaataccgc atcaggcgcc 240
   attcgccatt caggctgcgc aactgttggg aagggcgatc ggtgcgggcc tcttcgctat 300
   tacgccagct ggcgaaaggg ggatgtgctg caaggcgatt aagttgggta acgccagggt 360
   tttcccagtc acgacgttgt aaaacgacgg ccagtgaatt cggcgcgccg agctcctcga 420
   gcaaatttac acattgccac taaacgtcta aacccttgta atttgttttt gttttactat 480
   gtgtgttatg tatttgattt gcgataaatt tttatatttg gtactaaatt tataacacct 540
   tttatgctaa cgtttgccaa cacttagcaa tttgcaagtt gattaattga ttctaaatta 600
   tttttgtctt ctaaatacat atactaatca actggaaatg taaatatttg ctaatatttc 660
   tactatagga gaattaaagt gagtgaatat ggtaccacaa ggtttggaga tttaattgtt 720
   gcaatgctgc atggatggca tatacaccaa acattcaata attcttgagg ataataatgg 780
   taccacacaa gatttgaggt gcatgaacgt cacgtggaca aaaggtttag taatttttca 840
   agacaacaat gttaccacac acaagttttg aggtgcatgc atggatgccc tgtggaaagt 900
   ttaaaaatat tttggaaatg atttgcatgg aagccatgtg taaaaccatg acatccactt 960
   ggaggatgca ataatgaaga aaactacaaa tttacatgca actagttatg catgtagtct 1020
   atataatgag gattttgcaa tactttcatt catacacact cactaagttt tacacgatta 1080
   taatttcttc atagccagca gatctgccgg catcgatccc gggccatggc ctgctttaat 1140
   gagatatgcg agacgcctat gatcgcatga tatttgcttt caattctgtt gtgcacgttg 1200
   taaaaaacct gagcatgtgt agctcagatc cttaccgccg gtttcggttc attctaatga 1260
   atatatcacc cgttactatc gtatttttat gaataatatt ctccgttcaa tttactgatt 1320
   gtccgtcgac gagctcggcg cgccaagctt ggcgtaatca tggtcatagc tgtttcctgt 1380
   gtgaaattgt tatccgctca caattccaca caacatacga gccggaagca taaagtgtaa 1440
   agcctggggt gcctaatgag tgagctaact cacattaatt gcgttgcgct cactgcccgc 1500
   tttccagtcg ggaaacctgt cgtgccagct gcattaatga atcggccaac gcgcggggag 1560
   aggcggtttg cgtattgggc gctcttccgc ttcctcgctc actgactcgc tgcgctcggt 1620
   cgttcggctg cggcgagcgg tatcagctca ctcaaaggcg gtaatacggt tatccacaga 1680
   atcaggggat aacgcaggaa agaacatgtg agcaaaaggc cagcaaaagg ccaggaaccg 1740
   taaaaaggcc gcgttgctgg cgtttttcca taggctccgc ccccctgacg agcatcacaa 1800
   aaatcgacgc tcaagtcaga ggtggcgaaa cccgacagga ctataaagat accaggcgtt 1860
   tccccctgga agctccctcg tgcgctctcc tgttccgacc ctgccgctta ccggatacct 1920
   gtccgccttt ctcccttcgg gaagcgtggc gctttctcat agctcacgct gtaggtatct 1980
   cagttcggtg taggtcgttc gctccaagct gggctgtgtg cacgaacccc ccgttcagcc 2040
   cgaccgctgc gccttatccg gtaactatcg tcttgagtcc aacccggtaa gacacgactt 2100
   atcgccactg gcagcagcca ctggtaacag gattagcaga gcgaggtatg taggcggtgc 2160
   tacagagttc ttgaagtggt ggcctaacta cggctacact agaaggacag tatttggtat 2220
   ctgcgctctg ctgaagccag ttaccttcgg aaaaagagtt ggtagctctt gatccggcaa 2280
   acaaaccacc gctggtagcg gtggtttttt tgtttgcaag cagcagatta cgcgcagaaa 2340
   aaaaggatct caagaagatc ctttgatctt ttctacgggg tctgacgctc agtggaacga 2400
   aaactcacgt taagggattt tggtcatgag attatcaaaa aggatcttca cctagatcct 2460
   tttaaattaa aaatgaagtt ttaaatcaat ctaaagtata tatgagtaaa cttggtctga 2520
   cagttaccaa tgcttaatca gtgaggcacc tatctcagcg atctgtctat ttcgttcatc 2580
   catagttgcc tgactccccg tcgtgtagat aactacgata cgggagggct taccatctgg 2640
   ccccagtgct gcaatgatac cgcgagaccc acgctcaccg gctccagatt tatcagcaat 2700
   aaaccagcca gccggaaggg ccgagcgcag aagtggtcct gcaactttat ccgcctccat 2760
   ccagtctatt aattgttgcc gggaagctag agtaagtagt tcgccagtta atagtttgcg 2820
   caacgttgtt gccattgcta caggcatcgt ggtgtcacgc tcgtcgtttg gtatggcttc 2880
   attcagctcc ggttcccaac gatcaaggcg agttacatga tcccccatgt tgtgcaaaaa 2940
   agcggttagc tccttcggtc ctccgatcgt tgtcagaagt aagttggccg cagtgttatc 3000
   actcatggtt atggcagcac tgcataattc tcttactgtc atgccatccg taagatgctt 3060
   ttctgtgact ggtgagtact caaccaagtc attctgagaa tagtgtatgc ggcgaccgag 3120
   ttgctcttgc ccggcgtcaa tacgggataa taccgcgcca catagcagaa ctttaaaagt 3180
   gctcatcatt ggaaaacgtt cttcggggcg aaaactctca aggatcttac cgctgttgag 3240
   atccagttcg atgtaaccca ctcgtgcacc caactgatct tcagcatctt ttactttcac 3300
   cagcgtttct gggtgagcaa aaacaggaag gcaaaatgcc gcaaaaaagg gaataagggc 3360
   gacacggaaa tgttgaatac tcatactctt cctttttcaa tattattgaa gcatttatca 3420
   gggttattgt ctcatgagcg gatacatatt tgaatgtatt tagaaaaata aacaaatagg 3480
   ggttccgcgc acatttcccc gaaaagtgcc acctgacgtc taagaaacca ttattatcat 3540
   gacattaacc tataaaaata ggcgtatcac gaggcccttt cgtc 3584
<210> 16
   <211> 4507
   <212> DNA
   <213> Unknown
<220>
   <223> Sequenz stellt eine pflanzliche Promotor-Terminator-Expressionskassette in Vektor pUC19 dar
<400> 16
   tcgcgcgttt cggtgatgac ggtgaaaacc tctgacacat gcagctcccg gagacggtca 60
   cagcttgtct gtaagcggat gccgggagca gacaagcccg tcagggcgcg tcagcgggtg 120
   ttggcgggtg tcggggctgg cttaactatg cggcatcaga gcagattgta ctgagagtgc 180
   accatatgcg gtgtgaaata ccgcacagat gcgtaaggag aaaataccgc atcaggcgcc 240
   attcgccatt caggctgcgc aactgttggg aagggcgatc ggtgcgggcc tcttcgctat 300
   tacgccagct ggcgaaaggg ggatgtgctg caaggcgatt aagttgggta acgccagggt 360
   tttcccagtc acgacgttgt aaaacgacgg ccagtgaatt cggcgcgccg agctcctcga 420
   gcaaatttac acattgccac taaacgtcta aacccttgta atttgttttt gttttactat 480
   gtgtgttatg tatttgattt gcgataaatt tttatatttg gtactaaatt tataacacct 540
   tttatgctaa cgtttgccaa cacttagcaa tttgcaagtt gattaattga ttctaaatta 600
   tttttgtctt ctaaatacat atactaatca actggaaatg taaatatttg ctaatatttc 660
   tactatagga gaattaaagt gagtgaatat ggtaccacaa ggtttggaga tttaattgtt 720
   gcaatgctgc atggatggca tatacaccaa acattcaata attcttgagg ataataatgg 780
   taccacacaa gatttgaggt gcatgaacgt cacgtggaca aaaggtttag taatttttca 840
   agacaacaat gttaccacac acaagttttg aggtgcatgc atggatgccc tgtggaaagt 900
   ttaaaaatat tttggaaatg atttgcatgg aagccatgtg taaaaccatg acatccactt 960
   ggaggatgca ataatgaaga aaactacaaa tttacatgca actagttatg catgtagtct 1020
   atataatgag gattttgcaa tactttcatt catacacact cactaagttt tacacgatta 1080
   taatttcttc atagccagcc caccgcggtg ggcggccgcc tgcagtctag aaggcctcct 1140
   gctttaatga gatatgcgag acgcctatga tcgcatgata tttgctttca attctgttgt 1200
   gcacgttgta aaaaacctga gcatgtgtag ctcagatcct taccgccggt ttcggttcat 1260
   tctaatgaat atatcacccg ttactatcgt atttttatga ataatattct ccgttcaatt 1320
   tactgattgt ccgtcgagca aatttacaca ttgccactaa acgtctaaac ccttgtaatt 1380
   tgtttttgtt ttactatgtg tgttatgtat ttgatttgcg ataaattttt atatttggta 1440
   ctaaatttat aacacctttt atgctaacgt ttgccaacac ttagcaattt gcaagttgat 1500
   taattgattc taaattattt ttgtcttcta aatacatata ctaatcaact ggaaatgtaa 1560
   atatttgcta atatttctac tataggagaa ttaaagtgag tgaatatggt accacaaggt 1620
   ttggagattt aattgttgca atgctgcatg gatggcatat acaccaaaca ttcaataatt 1680
   cttgaggata ataatggtac cacacaagat ttgaggtgca tgaacgtcac gtggacaaaa 1740
   ggtttagtaa tttttcaaga caacaatgtt accacacaca agttttgagg tgcatgcatg 1800
   gatgccctgt ggaaagttta aaaatatttt ggaaatgatt tgcatggaag ccatgtgtaa 1860
   aaccatgaca tccacttgga ggatgcaata atgaagaaaa ctacaaattt acatgcaact 1920
   agttatgcat gtagtctata taatgaggat tttgcaatac tttcattcat acacactcac 1980
   taagttttac acgattataa tttcttcata gccagcggat ccgatatcgg gcccgctagc 2040
   gttaaccctg ctttaatgag atatgcgaga cgcctatgat cgcatgatat ttgctttcaa 2100
   ttctgttgtg cacgttgtaa aaaacctgag catgtgtagc tcagatcctt accgccggtt 2160
   tcggttcatt ctaatgaata tatcacccgt tactatcgta tttttatgaa taatattctc 2220
   cgttcaattt actgattgtc cgtcgacgaa ttcgagctcg gcgcgccaag cttggcgtaa 2280
   tcatggtcat agctgtttcc tgtgtgaaat tgttatccgc tcacaattcc acacaacata 2340
   cgagccggaa gcataaagtg taaagcctgg ggtgcctaat gagtgagcta actcacatta 2400
   attgcgttgc gctcactgcc cgctttccag tcgggaaacc tgtcgtgcca gctgcattaa 2460
   tgaatcggcc aacgcgcggg gagaggcggt ttgcgtattg ggcgctcttc cgcttcctcg 2520
   ctcactgact cgctgcgctc ggtcgttcgg ctgcggcgag cggtatcagc tcactcaaag 2580
   gcggtaatac ggttatccac agaatcaggg gataacgcag gaaagaacat gtgagcaaaa 2640
   ggccagcaaa aggccaggaa ccgtaaaaag gccgcgttgc tggcgttttt ccataggctc 2700
   cgcccccctg acgagcatca caaaaatcga cgctcaagtc agaggtggcg aaacccgaca 2760
   ggactataaa gataccaggc gtttccccct ggaagctccc tcgtgcgctc tcctgttccg 2820
   accctgccgc ttaccggata cctgtccgcc tttctccctt cgggaagcgt ggcgctttct 2880
   catagctcac gctgtaggta tctcagttcg gtgtaggtcg ttcgctccaa gctgggctgt 2940
   gtgcacgaac cccccgttca gcccgaccgc tgcgccttat ccggtaacta tcgtcttgag 3000
   tccaacccgg taagacacga cttatcgcca ctggcagcag ccactggtaa caggattagc 3060
   agagcgaggt atgtaggcgg tgctacagag ttcttgaagt ggtggcctaa ctacggctac 3120
   actagaagga cagtatttgg tatctgcgct ctgctgaagc cagttacctt cggaaaaaga 3180
   gttggtagct cttgatccgg caaacaaacc accgctggta gcggtggttt ttttgtttgc 3240
   aagcagcaga ttacgcgcag aaaaaaagga tctcaagaag atcctttgat cttttctacg 3300
   gggtctgacg ctcagtggaa cgaaaactca cgttaaggga ttttggtcat gagattatca 3360
   aaaaggatct tcacctagat ccttttaaat taaaaatgaa gttttaaatc aatctaaagt 3420
   atatatgagt aaacttggtc tgacagttac caatgcttaa tcagtgaggc acctatctca 3480
   gcgatctgtc tatttcgttc atccatagtt gcctgactcc ccgtcgtgta gataactacg 3540
   atacgggagg gcttaccatc tggccccagt gctgcaatga taccgcgaga cccacgctca 3600
   ccggctccag atttatcagc aataaaccag ccagccggaa gggccgagcg cagaagtggt 3660
   cctgcaactt tatccgcctc catccagtct attaattgtt gccgggaagc tagagtaagt 3720
   agttcgccag ttaatagttt gcgcaacgtt gttgccattg ctacaggcat cgtggtgtca 3780
   cgctcgtcgt ttggtatggc ttcattcagc tccggttccc aacgatcaag gcgagttaca 3840
   tgatccccca tgttgtgcaa aaaagcggtt agctccttcg gtcctccgat cgttgtcaga 3900
   agtaagttgg ccgcagtgtt atcactcatg gttatggcag cactgcataa ttctcttact 3960
   gtcatgccat ccgtaagatg cttttctgtg actggtgagt actcaaccaa gtcattctga 4020
   gaatagtgta tgcggcgacc gagttgctct tgcccggcgt caatacggga taataccgcg 4080
   ccacatagca gaactttaaa agtgctcatc attggaaaac gttcttcggg gcgaaaactc 4140
   tcaaggatct taccgctgtt gagatccagt tcgatgtaac ccactcgtgc acccaactga 4200
   tcttcagcat cttttacttt caccagcgtt tctgggtgag caaaaacagg aaggcaaaat 4260
   gccgcaaaaa agggaataag ggcgacacgg aaatgttgaa tactcatact cttccttttt 4320
   caatattatt gaagcattta tcagggttat tgtctcatga gcggatacat atttgaatgt 4380
   atttagaaaa ataaacaaat aggggttccg cgcacatttc cccgaaaagt gccacctgac 4440
   gtctaagaaa ccattattat catgacatta acctataaaa ataggcgtat cacgaggccc 4500
   tttcgtc 4507
<210> 17
   <211> 5410
   <212> DNA
   <213> Unknown
<220>
   <223> Sequenz stellt eine pflanzliche Promotor-Terminator-Expressionskassette in Vektor pUC19 dar
<400> 17
   ttttggaaat gatttgcatg gaagccatgt gtaaaaccat gacatccact tggaggatgc 60
   aataatgaag aaaactacaa atttacatgc aactagttat gcatgtagtc tatataatga 120
   ggattttgca atactttcat tcatacacac tcactaagtt ttacacgatt ataatttctt 180
   catagccagc ggatccgata tcgggcccgc tagcgttaac cctgctttaa tgagatatgc 240
   gagacgccta tgatcgcatg atatttgctt tcaattctgt tgtgcacgtt gtaaaaaacc 300
   tgagcatgtg tagctcagat ccttaccgcc ggtttcggtt cattctaatg aatatatcac 360
   ccgttactat cgtattttta tgaataatat tctccgttca atttactgat tgtccgtcga 420
   gcaaatttac acattgccac taaacgtcta aacccttgta atttgttttt gttttactat 480
   gtgtgttatg tatttgattt gcgataaatt tttatatttg gtactaaatt tataacacct 540
   tttatgctaa cgtttgccaa cacttagcaa tttgcaagtt gattaattga ttctaaatta 600
   tttttgtctt ctaaatacat atactaatca actggaaatg taaatatttg ctaatatttc 660
   tactatagga gaattaaagt gagtgaatat ggtaccacaa ggtttggaga tttaattgtt 720
   gcaatgctgc atggatggca tatacaccaa acattcaata attcttgagg ataataatgg 780
   taccacacaa gatttgaggt gcatgaacgt cacgtggaca aaaggtttag taatttttca 840
   agacaacaat gttaccacac acaagttttg aggtgcatgc atggatgccc tgtggaaagt 900
   ttaaaaatat tttggaaatg atttgcatgg aagccatgtg taaaaccatg acatccactt 960
   ggaggatgca ataatgaaga aaactacaaa tttacatgca actagttatg catgtagtct 1020
   atataatgag gattttgcaa tactttcatt catacacact cactaagttt tacacgatta 1080
   taatttcttc atagccagca gatctgccgg catcgatccc gggccatggc ctgctttaat 1140
   gagatatgcg agacgcctat gatcgcatga tatttgcttt caattctgtt gtgcacgttg 1200
   taaaaaacct gagcatgtgt agctcagatc cttaccgccg gtttcggttc attctaatga 1260
   atatatcacc cgttactatc gtatttttat gaataatatt ctccgttcaa tttactgatt 1320
   gtccgtcgac gagctcggcg cgccaagctt ggcgtaatca tggtcatagc tgtttcctgt 1380
   gtgaaattgt tatccgctca caattccaca caacatacga gccggaagca taaagtgtaa 1440
   agcctggggt gcctaatgag tgagctaact cacattaatt gcgttgcgct cactgcccgc 1500
   tttccagtcg ggaaacctgt cgtgccagct gcattaatga atcggccaac gcgcggggag 1560
   aggcggtttg cgtattgggc gctcttccgc ttcctcgctc actgactcgc tgcgctcggt 1620
   cgttcggctg cggcgagcgg tatcagctca ctcaaaggcg gtaatacggt tatccacaga 1680
   atcaggggat aacgcaggaa agaacatgtg agcaaaaggc cagcaaaagg ccaggaaccg 1740
   taaaaaggcc gcgttgctgg cgtttttcca taggctccgc ccccctgacg agcatcacaa 1800
   aaatcgacgc tcaagtcaga ggtggcgaaa cccgacagga ctataaagat accaggcgtt 1860
   tccccctgga agctccctcg tgcgctctcc tgttccgacc ctgccgctta ccggatacct 1920
   gtccgccttt ctcccttcgg gaagcgtggc gctttctcat agctcacgct gtaggtatct 1980
   cagttcggtg taggtcgttc gctccaagct gggctgtgtg cacgaacccc ccgttcagcc 2040
   cgaccgctgc gccttatccg gtaactatcg tcttgagtcc aacccggtaa gacacgactt 2100
   atcgccactg gcagcagcca ctggtaacag gattagcaga gcgaggtatg taggcggtgc 2160
   tacagagttc ttgaagtggt ggcctaacta cggctacact agaaggacag tatttggtat 2220
   ctgcgctctg ctgaagccag ttaccttcgg aaaaagagtt ggtagctctt gatccggcaa 2280
   acaaaccacc gctggtagcg gtggtttttt tgtttgcaag cagcagatta cgcgcagaaa 2340
   aaaaggatct caagaagatc ctttgatctt ttctacgggg tctgacgctc agtggaacga 2400
   aaactcacgt taagggattt tggtcatgag attatcaaaa aggatcttca cctagatcct 2460
   tttaaattaa aaatgaagtt ttaaatcaat ctaaagtata tatgagtaaa cttggtctga 2520
   cagttaccaa tgcttaatca gtgaggcacc tatctcagcg atctgtctat ttcgttcatc 2580
   catagttgcc tgactccccg tcgtgtagat aactacgata cgggagggct taccatctgg 2640
   ccccagtgct gcaatgatac cgcgagaccc acgctcaccg gctccagatt tatcagcaat 2700
   aaaccagcca gccggaaggg ccgagcgcag aagtggtcct gcaactttat ccgcctccat 2760
   ccagtctatt aattgttgcc gggaagctag agtaagtagt tcgccagtta atagtttgcg 2820
   caacgttgtt gccattgcta caggcatcgt ggtgtcacgc tcgtcgtttg gtatggcttc 2880
   attcagctcc ggttcccaac gatcaaggcg agttacatga tcccccatgt tgtgcaaaaa 2940
   agcggttagc tccttcggtc ctccgatcgt tgtcagaagt aagttggccg cagtgttatc 3000
   actcatggtt atggcagcac tgcataattc tcttactgtc atgccatccg taagatgctt 3060
   ttctgtgact ggtgagtact caaccaagtc attctgagaa tagtgtatgc ggcgaccgag 3120
   ttgctcttgc ccggcgtcaa tacgggataa taccgcgcca catagcagaa ctttaaaagt 3180
   gctcatcatt ggaaaacgtt cttcggggcg aaaactctca aggatcttac cgctgttgag 3240
   atccagttcg atgtaaccca ctcgtgcacc caactgatct tcagcatctt ttactttcac 3300
   cagcgtttct gggtgagcaa aaacaggaag gcaaaatgcc gcaaaaaagg gaataagggc 3360
   gacacggaaa tgttgaatac tcatactctt cctttttcaa tattattgaa gcatttatca 3420
   gggttattgt ctcatgagcg gatacatatt tgaatgtatt tagaaaaata aacaaatagg 3480
   ggttccgcgc acatttcccc gaaaagtgcc acctgacgtc taagaaacca ttattatcat 3540
   gacattaacc tataaaaata ggcgtatcac gaggcccttt cgtctcgcgc gtttcggtga 3600
   tgacggtgaa aacctctgac acatgcagct cccggagacg gtcacagctt gtctgtaagc 3660
   ggatgccggg agcagacaag cccgtcaggg cgcgtcagcg ggtgttggcg ggtgtcgggg 3720
   ctggcttaac tatgcggcat cagagcagat tgtactgaga gtgcaccata tgcggtgtga 3780
   aataccgcac agatgcgtaa ggagaaaata ccgcatcagg cgccattcgc cattcaggct 3840
   gcgcaactgt tgggaagggc gatcggtgcg ggcctcttcg ctattacgcc agctggcgaa 3900
   agggggatgt gctgcaaggc gattaagttg ggtaacgcca gggttttccc agtcacgacg 3960
   ttgtaaaacg acggccagtg aattcggcgc gccgagctcc tcgagcaaat ttacacattg 4020
   ccactaaacg tctaaaccct tgtaatttgt ttttgtttta ctatgtgtgt tatgtatttg 4080
   atttgcgata aatttttata tttggtacta aatttataac accttttatg ctaacgtttg 4140
   ccaacactta gcaatttgca agttgattaa ttgattctaa attatttttg tcttctaaat 4200
   acatatacta atcaactgga aatgtaaata tttgctaata tttctactat aggagaatta 4260
   aagtgagtga atatggtacc acaaggtttg gagatttaat tgttgcaatg ctgcatggat 4320
   ggcatataca ccaaacattc aataattctt gaggataata atggtaccac acaagatttg 4380
   aggtgcatga acgtcacgtg gacaaaaggt ttagtaattt ttcaagacaa caatgttacc 4440
   acacacaagt tttgaggtgc atgcatggat gccctgtgga aagtttaaaa atattttgga 4500
   aatgatttgc atggaagcca tgtgtaaaac catgacatcc acttggagga tgcaataatg 4560
   aagaaaacta caaatttaca tgcaactagt tatgcatgta gtctatataa tgaggatttt 4620
   gcaatacttt cattcataca cactcactaa gttttacacg attataattt cttcatagcc 4680
   agcccaccgc ggtgggcggc cgcctgcagt ctagaaggcc tcctgcttta atgagatatg 4740
   cgagacgcct atgatcgcat gatatttgct ttcaattctg ttgtgcacgt tgtaaaaaac 4800
   ctgagcatgt gtagctcaga tccttaccgc cggtttcggt tcattctaat gaatatatca 4860
   cccgttacta tcgtattttt atgaataata ttctccgttc aatttactga ttgtccgtcg 4920
   agcaaattta cacattgcca ctaaacgtct aaacccttgt aatttgtttt tgttttacta 4980
   tgtgtgttat gtatttgatt tgcgataaat ttttatattt ggtactaaat ttataacacc 5040
   ttttatgcta acgtttgcca acacttagca atttgcaagt tgattaattg attctaaatt 5100
   atttttgtct tctaaataca tatactaatc aactggaaat gtaaatattt gctaatattt 5160
   ctactatagg agaattaaag tgagtgaata tggtaccaca aggtttggag atttaattgt 5220
   tgcaatgctg catggatggc atatacacca aacattcaat aattcttgag gataataatg 5280
   gtaccacaca agatttgagg tgcatgaacg tcacgtggac aaaaggttta gtaatttttc 5340
   aagacaacaa tgttaccaca cacaagtttt gaggtgcatg catggatgcc ctgtggaaag 5400
   tttaaaaata 5410
<210> 18
   <211> 648
   <212> DNA
   <213> Phaeodactylum tricornutum
<220>
   <221> CDS
   <222> (1)..(648)
<220>
   <223> .
<400> 18
   tgg tgg aaa aac aag cac aac gga cac cac gcc gtc ccc aac ctc cac    48
   Trp Trp Lys Asn Lys His Asn Gly His His Ala Val Pro Asn Leu His
    1               5                  10                  15
   tgc tcc tcc gca gtc gcg caa gat ggg gac ccg gac atc gat acc atg    96
   Cys Ser Ser Ala Val Ala Gln Asp Gly Asp Pro Asp Ile Asp Thr Met
               20                  25                  30
   ccc ctt ctc gcc tgg tcc gtc cag caa gcc cag tct tac cgg gaa ctc   144
   Pro Leu Leu Ala Trp Ser Val Gln Gln Ala Gln Ser Tyr Arg Glu Leu
           35                  40                  45
   caa gcc gac gga aag gat tcg ggt ttg gtc aag ttc atg atc cgt aac   192
   Gln Ala Asp Gly Lys Asp Ser Gly Leu Val Lys Phe Met Ile Arg Asn
       50                  55                  60
   caa tcc tac ttt tac ttt ccc atc ttg ttg ctc gcc cgc ctg tcg tgg   240
   Gln Ser Tyr Phe Tyr Phe Pro Ile Leu Leu Leu Ala Arg Leu Ser Trp
   65                  70                  75                  80
   ttg aac gag tcc ttc aag tgc gcc ttt ggg ctt gga gct gcg tcg gag   288
   Leu Asn Glu Ser Phe Lys Cys Ala Phe Gly Leu Gly Ala Ala Ser Glu
                   85                  90                  95
   aac gct gct ctc gaa ctc aag gcc aag ggt ctt cag tac ccc ctt ttg   336
   Asn Ala Ala Leu Glu Leu Lys Ala Lys Gly Leu Gln Tyr Pro Leu Leu
               100                 105                 110
   gaa aag gct ggc atc ctg ctg cac tac gct tgg atg ctt aca gtt tcg   384
   Glu Lys Ala Gly Ile Leu Leu His Tyr Ala Trp Met Leu Thr Val Ser
           115                 120                 125
   tcc ggc ttt gga cgc ttc tcg ttc gcg tac acc gca ttt tac ttt cta      432
   Ser Gly Phe Gly Arg Phe Ser Phe Ala Tyr Thr Ala Phe Tyr Phe Leu
       130                 135                     140
   acc gcg acc gcg tcc tgt gga ttc ttg ctc gcc att gtc ttt ggc ctc      480
   Thr Ala Thr Ala Ser Cys Gly Phe Leu Leu Ala Ile Val Phe Gly Leu
   145                     150                     155                 160
   ggc cac aac ggc atg gcc acc tac aat gcc gac gcc cgt ccg gac ttc      528
   Gly His Asn Gly Met Ala Thr Tyr Asn Ala Asp Ala Arg Pro Asp Phe
                   165                     170                     175
   tgg aag ctc caa gtc acc acg act cgc aac gtc acg ggc gga cac ggt      576
   Trp Lys Leu Gln Val Thr Thr Thr Arg Asn Val Thr Gly Gly His Gly
               180                     185                     190
   ttc ccc caa gcc ttt gtc gac tgg ttc tgt ggt ggc ctc cag tac caa      624
   Phe Pro Gln Ala Phe Val Asp Trp Phe Cys Gly Gly Leu Gln Tyr Gln
           195                     200                     205
   gtc gac cac cac tta ttc ccc agc                                      648
   Val Asp His His Leu Phe Pro Ser
       210                 215
<210> 19
   <211> 216
   <212> PRT
   <213> Phaeodactylum tricornutum
<400> 19
   Trp Trp Lys Asn Lys His Asn Gly His His Ala Val Pro Asn Leu His
   1               5                   10                  15
   Cys Ser Ser Ala Val Ala Gln Asp Gly Asp Pro Asp Ile Asp Thr Met
               20                  25                  30
   Pro Leu Leu Ala Trp Ser Val Gln Gln Ala Gln Ser Tyr Arg Glu Leu
           35                  40                  45
   Gln Ala Asp Gly Lys Asp Ser Gly Leu Val Lys Phe Met Ile Arg Asn
       50                  55                  60
   Gln Ser Tyr Phe Tyr Phe Pro Ile Leu Leu Leu Ala Arg Leu Ser Trp
   65                  70                  75                  80
   Leu Asn Glu Ser Phe Lys Cys Ala Phe Gly Leu Gly Ala Ala Ser Glu
                   85                  90                  95
   Asn Ala Ala Leu Glu Leu Lys Ala Lys Gly Leu Gln Tyr Pro Leu Leu
               100                 105                 110
   Glu Lys Ala Gly Ile Leu Leu His Tyr Ala Trp Met Leu Thr Val Ser
           115                 120                 125
   Ser Gly Phe Gly Arg Phe Ser Phe Ala Tyr Thr Ala Phe Tyr Phe Leu
       130                 135                 140
   Thr Ala Thr Ala Ser Cys Gly Phe Leu Leu Ala Ile Val Phe Gly Leu
   145                 150                 155                 160
   Gly His Asn Gly Met Ala Thr Tyr Asn Ala Asp Ala Arg Pro Asp Phe
                   165                 170                 175
   Trp Lys Leu Gln Val Thr Thr Thr Arg Asn Val Thr Gly Gly His Gly
               180                 185                 190
   Phe Pro Gln Ala Phe Val Asp Trp Phe Cys Gly Gly Leu Gln Tyr Gln
           195                 200                 205
   Val Asp His His Leu Phe Pro Ser
       210                 215
<210> 20
   <211> 12093
   <212> DNA
   <213> Unknown
<220>
   <223> pflanzlicher Expressionsvektor mit einer Promotor-Terminator-Expressionskassette
<400> 20
   gatctggcgc cggccagcga gacgagcaag attggccgcc gcccgaaacg atccgacagc 60
   gcgcccagca caggtgcgca ggcaaattgc accaacgcat acagcgccag cagaatgcca 120
   tagtgggcgg tgacgtcgtt cgagtgaacc agatcgcgca ggaggcccgg cagcaccggc 180
   ataatcaggc cgatgccgac agcgtcgagc gcgacagtgc tcagaattac gatcaggggt 240
   atgttgggtt tcacgtctgg cctccggacc agcctccgct ggtccgattg aacgcgcgga 300
   ttctttatca ctgataagtt ggtggacata ttatgtttat cagtgataaa gtgtcaagca 360
   tgacaaagtt gcagccgaat acagtgatcc gtgccgccct ggacctgttg aacgaggtcg 420
   gcgtagacgg tctgacgaca cgcaaactgg cggaacggtt gggggttcag cagccggcgc 480
   tttactggca cttcaggaac aagcgggcgc tgctcgacgc actggccgaa gccatgctgg 540
   cggagaatca tacgcattcg gtgccgagag ccgacgacga ctggcgctca tttctgatcg 600
   ggaatgcccg cagcttcagg caggcgctgc tcgcctaccg cgatggcgcg cgcatccatg 660
   ccggcacgcg accgggcgca ccgcagatgg aaacggccga cgcgcagctt cgcttcctct 720
   gcgaggcggg tttttcggcc ggggacgccg tcaatgcgct gatgacaatc agctacttca 780
   ctgttggggc cgtgcttgag gagcaggccg gcgacagcga tgccggcgag cgcggcggca 840
   ccgttgaaca ggctccgctc tcgccgctgt tgcgggccgc gatagacgcc ttcgacgaag 900
   ccggtccgga cgcagcgttc gagcagggac tcgcggtgat tgtcgatgga ttggcgaaaa 960
   ggaggctcgt tgtcaggaac gttgaaggac cgagaaaggg tgacgattga tcaggaccgc 1020
   tgccggagcg caacccactc actacagcag agccatgtag acaacatccc ctcccccttt 1080
   ccaccgcgtc agacgcccgt agcagcccgc tacgggcttt ttcatgccct gccctagcgt 1140
   ccaagcctca cggccgcgct cggcctctct ggcggccttc tggcgctctt ccgcttcctc 1200
   gctcactgac tcgctgcgct cggtcgttcg gctgcggcga gcggtatcag ctcactcaaa 1260
   ggcggtaata cggttatcca cagaatcagg ggataacgca ggaaagaaca tgtgagcaaa 1320
   aggccagcaa aaggccagga accgtaaaaa ggccgcgttg ctggcgtttt tccataggct 1380
   ccgcccccct gacgagcatc acaaaaatcg acgctcaagt cagaggtggc gaaacccgac 1440
   aggactataa agataccagg cgtttccccc tggaagctcc ctcgtgcgct ctcctgttcc 1500
   gaccctgccg cttaccggat acctgtccgc ctttctccct tcgggaagcg tggcgctttt 1560
   ccgctgcata accctgcttc ggggtcatta tagcgatttt ttcggtatat ccatcctttt 1620
   tcgcacgata tacaggattt tgccaaaggg ttcgtgtaga ctttccttgg tgtatccaac 1680
   ggcgtcagcc gggcaggata ggtgaagtag gcccacccgc gagcgggtgt tccttcttca 1740
   ctgtccctta ttcgcacctg gcggtgctca acgggaatcc tgctctgcga ggctggccgg 1800
   ctaccgccgg cgtaacagat gagggcaagc ggatggctga tgaaaccaag ccaaccagga 1860
   agggcagccc acctatcaag gtgtactgcc ttccagacga acgaagagcg attgaggaaa 1920
   aggcggcggc ggccggcatg agcctgtcgg cctacctgct ggccgtcggc cagggctaca 1980
   aaatcacggg cgtcgtggac tatgagcacg tccgcgagct ggcccgcatc aatggcgacc 2040
   tgggccgcct gggcggcctg ctgaaactct ggctcaccga cgacccgcgc acggcgcggt 2100
   tcggtgatgc cacgatcctc gccctgctgg cgaagatcga agagaagcag gacgagcttg 2160
   gcaaggtcat gatgggcgtg gtccgcccga gggcagagcc atgacttttt tagccgctaa 2220
   aacggccggg gggtgcgcgt gattgccaag cacgtcccca tgcgctccat caagaagagc 2280
   gacttcgcgg agctggtgaa gtacatcacc gacgagcaag gcaagaccga gcgcctttgc 2340
   gacgctcacc gggctggttg ccctcgccgc tgggctggcg gccgtctatg gccctgcaaa 2400
   cgcgccagaa acgccgtcga agccgtgtgc gagacaccgc ggccgccggc gttgtggata 2460
   cctcgcggaa aacttggccc tcactgacag atgaggggcg gacgttgaca cttgaggggc 2520
   cgactcaccc ggcgcggcgt tgacagatga ggggcaggct cgatttcggc cggcgacgtg 2580
   gagctggcca gcctcgcaaa tcggcgaaaa cgcctgattt tacgcgagtt tcccacagat 2640
   gatgtggaca agcctgggga taagtgccct gcggtattga cacttgaggg gcgcgactac 2700
   tgacagatga ggggcgcgat ccttgacact tgaggggcag agtgctgaca gatgaggggc 2760
   gcacctattg acatttgagg ggctgtccac aggcagaaaa tccagcattt gcaagggttt 2820
   ccgcccgttt ttcggccacc gctaacctgt cttttaacct gcttttaaac caatatttat 2880
   aaaccttgtt tttaaccagg gctgcgccct gtgcgcgtga ccgcgcacgc cgaagggggg 2940
   tgccccccct tctcgaaccc tcccggcccg ctaacgcggg cctcccatcc ccccaggggc 3000
   tgcgcccctc ggccgcgaac ggcctcaccc caaaaatggc agcgctggca gtccttgcca 3060
   ttgccgggat cggggcagta acgggatggg cgatcagccc gagcgcgacg cccggaagca 3120
   ttgacgtgcc gcaggtgctg gcatcgacat tcagcgacca ggtgccgggc agtgagggcg 3180
   gcggcctggg tggcggcctg cccttcactt cggccgtcgg ggcattcacg gacttcatgg 3240
   cggggccggc aatttttacc ttgggcattc ttggcatagt ggtcgcgggt gccgtgctcg 3300
   tgttcggggg tgcgataaac ccagcgaacc atttgaggtg ataggtaaga ttataccgag 3360
   gtatgaaaac gagaattgga cctttacaga attactctat gaagcgccat atttaaaaag 3420
   ctaccaagac gaagaggatg aagaggatga ggaggcagat tgccttgaat atattgacaa 3480
   tactgataag ataatatatc ttttatatag aagatatcgc cgtatgtaag gatttcaggg 3540
   ggcaaggcat aggcagcgcg cttatcaata tatctataga atgggcaaag cataaaaact 3600
   tgcatggact aatgcttgaa acccaggaca ataaccttat agcttgtaaa ttctatcata 3660
   attgggtaat gactccaact tattgatagt gttttatgtt cagataatgc ccgatgactt 3720
   tgtcatgcag ctccaccgat tttgagaacg acagcgactt ccgtcccagc cgtgccaggt 3780
   gctgcctcag attcaggtta tgccgctcaa ttcgctgcgt atatcgcttg ctgattacgt 3840
   gcagctttcc cttcaggcgg gattcataca gcggccagcc atccgtcatc catatcacca 3900
   cgtcaaaggg tgacagcagg ctcataagac gccccagcgt cgccatagtg cgttcaccga 3960
   atacgtgcgc aacaaccgtc ttccggagac tgtcatacgc gtaaaacagc cagcgctggc 4020
   gcgatttagc cccgacatag ccccactgtt cgtccatttc cgcgcagacg atgacgtcac 4080
   tgcccggctg tatgcgcgag gttaccgact gcggcctgag ttttttaagt gacgtaaaat 4140
   cgtgttgagg ccaacgccca taatgcgggc tgttgcccgg catccaacgc cattcatggc 4200
   catatcaatg attttctggt gcgtaccggg ttgagaagcg gtgtaagtga actgcagttg 4260
   ccatgtttta cggcagtgag agcagagata gcgctgatgt ccggcggtgc ttttgccgtt 4320
   acgcaccacc ccgtcagtag ctgaacagga gggacagctg atagacacag aagccactgg 4380
   agcacctcaa aaacaccatc atacactaaa tcagtaagtt ggcagcatca cccataattg 4440
   tggtttcaaa atcggctccg tcgatactat gttatacgcc aactttgaaa acaactttga 4500
   aaaagctgtt ttctggtatt taaggtttta gaatgcaagg aacagtgaat tggagttcgt 4560
   cttgttataa ttagcttctt ggggtatctt taaatactgt agaaaagagg aaggaaataa 4620
   taaatggcta aaatgagaat atcaccggaa ttgaaaaaac tgatcgaaaa ataccgctgc 4680
   gtaaaagata cggaaggaat gtctcctgct aaggtatata agctggtggg agaaaatgaa 4740
   aacctatatt taaaaatgac ggacagccgg tataaaggga ccacctatga tgtggaacgg 4800
   gaaaaggaca tgatgctatg gctggaagga aagctgcctg ttccaaaggt cctgcacttt 4860
   gaacggcatg atggctggag caatctgctc atgagtgagg ccgatggcgt cctttgctcg 4920
   gaagagtatg aagatgaaca aagccctgaa aagattatcg agctgtatgc ggagtgcatc 4980
   aggctctttc actccatcga catatcggat tgtccctata cgaatagctt agacagccgc 5040
   ttagccgaat tggattactt actgaataac gatctggccg atgtggattg cgaaaactgg 5100
   gaagaagaca ctccatttaa agatccgcgc gagctgtatg atttttttaa gacggaaaag 5160
   cccgaagagg aacttgtctt ttcccacggc gacctgggag acagcaacat ctttgtgaaa 5220
   gatggcaaag taagtggctt tattgatctt gggagaagcg gcagggcgga caagtggtat 5280
   gacattgcct tctgcgtccg gtcgatcagg gaggatatcg gggaagaaca gtatgtcgag 5340
   ctattttttg acttactggg gatcaagcct gattgggaga aaataaaata ttatatttta 5400
   ctggatgaat tgttttagta cctagatgtg gcgcaacgat gccggcgaca agcaggagcg 5460
   caccgacttc ttccgcatca agtgttttgg ctctcaggcc gaggcccacg gcaagtattt 5520
   gggcaagggg tcgctggtat tcgtgcaggg caagattcgg aataccaagt acgagaagga 5580
   cggccagacg gtctacggga ccgacttcat tgccgataag gtggattatc tggacaccaa 5640
   ggcaccaggc gggtcaaatc aggaataagg gcacattgcc ccggcgtgag tcggggcaat 5700
   cccgcaagga gggtgaatga atcggacgtt tgaccggaag gcatacaggc aagaactgat 5760
   cgacgcgggg ttttccgccg aggatgccga aaccatcgca agccgcaccg tcatgcgtgc 5820
   gccccgcgaa accttccagt ccgtcggctc gatggtccag caagctacgg ccaagatcga 5880
   gcgcgacagc gtgcaactgg ctccccctgc cctgcccgcg ccatcggccg ccgtggagcg 5940
   ttcgcgtcgt ctcgaacagg aggcggcagg tttggcgaag tcgatgacca tcgacacgcg 6000
   aggaactatg acgaccaaga agcgaaaaac cgccggcgag gacctggcaa aacaggtcag 6060
   cgaggccaag caggccgcgt tgctgaaaca cacgaagcag cagatcaagg aaatgcagct 6120
   ttccttgttc gatattgcgc cgtggccgga cacgatgcga gcgatgccaa acgacacggc 6180
   ccgctctgcc ctgttcacca cgcgcaacaa gaaaatcccg cgcgaggcgc tgcaaaacaa 6240
   ggtcattttc cacgtcaaca aggacgtgaa gatcacctac accggcgtcg agctgcgggc 6300
   cgacgatgac gaactggtgt ggcagcaggt gttggagtac gcgaagcgca cccctatcgg 6360
   cgagccgatc accttcacgt tctacgagct ttgccaggac ctgggctggt cgatcaatgg 6420
   ccggtattac acgaaggccg aggaatgcct gtcgcgccta caggcgacgg cgatgggctt 6480
   cacgtccgac cgcgttgggc acctggaatc ggtgtcgctg ctgcaccgct tccgcgtcct 6540
   ggaccgtggc aagaaaacgt cccgttgcca ggtcctgatc gacgaggaaa tcgtcgtgct 6600
   gtttgctggc gaccactaca cgaaattcat atgggagaag taccgcaagc tgtcgccgac 6660
   ggcccgacgg atgttcgact atttcagctc gcaccgggag ccgtacccgc tcaagctgga 6720
   aaccttccgc ctcatgtgcg gatcggattc cacccgcgtg aagaagtggc gcgagcaggt 6780
   cggcgaagcc tgcgaagagt tgcgaggcag cggcctggtg gaacacgcct gggtcaatga 6840
   tgacctggtg cattgcaaac gctagggcct tgtggggtca gttccggctg ggggttcagc 6900
   agccagcgct ttactggcat ttcaggaaca agcgggcact gctcgacgca cttgcttcgc 6960
   tcagtatcgc tcgggacgca cggcgcgctc tacgaactgc cgataaacag aggattaaaa 7020
   ttgacaattg tgattaaggc tcagattcga cggcttggag cggccgacgt gcaggatttc 7080
   cgcgagatcc gattgtcggc cctgaagaaa gctccagaga tgttcgggtc cgtttacgag 7140
   cacgaggaga aaaagcccat ggaggcgttc gctgaacggt tgcgagatgc cgtggcattc 7200
   ggcgcctaca tcgacggcga gatcattggg ctgtcggtct tcaaacagga ggacggcccc 7260
   aaggacgctc acaaggcgca tctgtccggc gttttcgtgg agcccgaaca gcgaggccga 7320
   ggggtcgccg gtatgctgct gcgggcgttg ccggcgggtt tattgctcgt gatgatcgtc 7380
   cgacagattc caacgggaat ctggtggatg cgcatcttca tcctcggcgc acttaatatt 7440
   tcgctattct ggagcttgtt gtttatttcg gtctaccgcc tgccgggcgg ggtcgcggcg 7500
   acggtaggcg ctgtgcagcc gctgatggtc gtgttcatct ctgccgctct gctaggtagc 7560
   ccgatacgat tgatggcggt cctgggggct atttgcggaa ctgcgggcgt ggcgctgttg 7620
   gtgttgacac caaacgcagc gctagatcct gtcggcgtcg cagcgggcct ggcgggggcg 7680
   gtttccatgg cgttcggaac cgtgctgacc cgcaagtggc aacctcccgt gcctctgctc 7740
   acctttaccg cctggcaact ggcggccgga ggacttctgc tcgttccagt agctttagtg 7800
   tttgatccgc caatcccgat gcctacagga accaatgttc tcggcctggc gtggctcggc 7860
   ctgatcggag cgggtttaac ctacttcctt tggttccggg ggatctcgcg actcgaacct 7920
   acagttgttt ccttactggg ctttctcagc cccagatctg gggtcgatca gccggggatg 7980
   catcaggccg acagtcggaa cttcgggtcc ccgacctgta ccattcggtg agcaatggat 8040
   aggggagttg atatcgtcaa cgttcacttc taaagaaata gcgccactca gcttcctcag 8100
   cggctttatc cagcgatttc ctattatgtc ggcatagttc tcaagatcga cagcctgtca 8160
   cggttaagcg agaaatgaat aagaaggctg ataattcgga tctctgcgag ggagatgata 8220
   tttgatcaca ggcagcaacg ctctgtcatc gttacaatca acatgctacc ctccgcgaga 8280
   tcatccgtgt ttcaaacccg gcagcttagt tgccgttctt ccgaatagca tcggtaacat 8340
   gagcaaagtc tgccgcctta caacggctct cccgctgacg ccgtcccgga ctgatgggct 8400
   gcctgtatcg agtggtgatt ttgtgccgag ctgccggtcg gggagctgtt ggctggctgg 8460
   tggcaggata tattgtggtg taaacaaatt gacgcttaga caacttaata acacattgcg 8520
   gacgttttta atgtactggg gtggtttttc ttttcaccag tgagacgggc aacagctgat 8580
   tgcccttcac cgcctggccc tgagagagtt gcagcaagcg gtccacgctg gtttgcccca 8640
   gcaggcgaaa atcctgtttg atggtggttc cgaaatcggc aaaatccctt ataaatcaaa 8700
   agaatagccc gagatagggt tgagtgttgt tccagtttgg aacaagagtc cactattaaa 8760
   gaacgtggac tccaacgtca aagggcgaaa aaccgtctat cagggcgatg gcccactacg 8820
   tgaaccatca cccaaatcaa gttttttggg gtcgaggtgc cgtaaagcac taaatcggaa 8880
   ccctaaaggg agcccccgat ttagagcttg acggggaaag ccggcgaacg tggcgagaaa 8940
   ggaagggaag aaagcgaaag gagcgggcgc cattcaggct gcgcaactgt tgggaagggc 9000
   gatcggtgcg ggcctcttcg ctattacgcc agctggcgaa agggggatgt gctgcaaggc 9060
   gattaagttg ggtaacgcca gggttttccc agtcacgacg ttgtaaaacg acggccagtg 9120
   aattaattcc catcttgaaa gaaatatagt ttaaatattt attgataaaa taacaagtca 9180
   ggtattatag tccaagcaaa aacataaatt tattgatgca agtttaaatt cagaaatatt 9240
   tcaataactg attatatcag ctggtacatt gccgtagatg aaagactgag tgcgatatta 9300
   tgtgtaatac ataaattgat gatatagcta gcttagctca tcgggggatc cgtcgaagct 9360
   agcttgggtc ccgctcagaa gaactcgtca agaaggcgat agaaggcgat gcgctgcgaa 9420
   tcgggagcgg cgataccgta aagcacgagg aagcggtcag cccattcgcc gccaagctct 9480
   tcagcaatat cacgggtagc caacgctatg tcctgatagc ggtccgccac acccagccgg 9540
   ccacagtcga tgaatccaga aaagcggcca ttttccacca tgatattcgg caagcaggca 9600
   tcgccatggg tcacgacgag atcctcgccg tcgggcatgc gcgccttgag cctggcgaac 9660
   agttcggctg gcgcgagccc ctgatgctct tcgtccagat catcctgatc gacaagaccg 9720
   gcttccatcc gagtacgtgc tcgctcgatg cgatgtttcg cttggtggtc gaatgggcag 9780
   gtagccggat caagcgtatg cagccgccgc attgcatcag ccatgatgga tactttctcg 9840
   gcaggagcaa ggtgagatga caggagatcc tgccccggca cttcgcccaa tagcagccag 9900
   tcccttcccg cttcagtgac aacgtcgagc acagctgcgc aaggaacgcc cgtcgtggcc 9960
   agccacgata gccgcgctgc ctcgtcctgc agttcattca gggcaccgga caggtcggtc 10020
   ttgacaaaaa gaaccgggcg cccctgcgct gacagccgga acacggcggc atcagagcag 10080
   ccgattgtct gttgtgccca gtcatagccg aatagcctct ccacccaagc ggccggagaa 10140
   cctgcgtgca atccatcttg ttcaatccaa gctcccatgg gccctcgact agagtcgaga 10200
   tctggattga gagtgaatat gagactctaa ttggataccg aggggaattt atggaacgtc 10260
   agtggagcat ttttgacaag aaatatttgc tagctgatag tgaccttagg cgacttttga 10320
   acgcgcaata atggtttctg acgtatgtgc ttagctcatt aaactccaga aacccgcggc 10380
   tgagtggctc cttcaacgtt gcggttctgt cagttccaaa cgtaaaacgg cttgtcccgc 10440
   gtcatcggcg ggggtcataa cgtgactccc ttaattctcc gctcatgatc ttgatcccct 10500
   gcgccatcag atccttggcg gcaagaaagc catccagttt actttgcagg gcttcccaac 10560
   cttaccagag ggcgccccag ctggcaattc cggttcgctt gctgtccata aaaccgccca 10620
   gtctagctat cgccatgtaa gcccactgca agctacctgc tttctctttg cgcttgcgtt 10680
   ttcccttgtc cagatagccc agtagctgac attcatccgg ggtcagcacc gtttctgcgg 10740
   actggctttc tacgtgttcc gcttccttta gcagcccttg cgccctgagt gcttgcggca 10800
   gcgtgaagct tgcatgcctg caggtcgacg gcgcgccgag ctcctcgagc aaatttacac 10860
   attgccacta aacgtctaaa cccttgtaat ttgtttttgt tttactatgt gtgttatgta 10920
   tttgatttgc gataaatttt tatatttggt actaaattta taacaccttt tatgctaacg 10980
   tttgccaaca cttagcaatt tgcaagttga ttaattgatt ctaaattatt tttgtcttct 11040
   aaatacatat actaatcaac tggaaatgta aatatttgct aatatttcta ctataggaga 11100
   attaaagtga gtgaatatgg taccacaagg tttggagatt taattgttgc aatgctgcat 11160
   ggatggcata tacaccaaac attcaataat tcttgaggat aataatggta ccacacaaga 11220
   tttgaggtgc atgaacgtca cgtggacaaa aggtttagta atttttcaag acaacaatgt 11280
   taccacacac aagttttgag gtgcatgcat ggatgccctg tggaaagttt aaaaatattt 11340
   tggaaatgat ttgcatggaa gccatgtgta aaaccatgac atccacttgg aggatgcaat 11400
   aatgaagaaa actacaaatt tacatgcaac tagttatgca tgtagtctat ataatgagga 11460
   ttttgcaata ctttcattca tacacactca ctaagtttta cacgattata atttcttcat 11520
   agccagccca ccgcggtggg cggccgcctg cagtctagaa ggcctcctgc tttaatgaga 11580
   tatgcgagac gcctatgatc gcatgatatt tgctttcaat tctgttgtgc acgttgtaaa 11640
   aaacctgagc atgtgtagct cagatcctta ccgccggttt cggttcattc taatgaatat 11700
   atcacccgtt actatcgtat ttttatgaat aatattctcc gttcaattta ctgattgtcc 11760
   gtcgacgaat tcgagctcgg cgcgcctcta gaggatcgat gaattcagat cggctgagtg 11820
   gctccttcaa cgttgcggtt ctgtcagttc caaacgtaaa acggcttgtc ccgcgtcatc 11880
   ggcgggggtc ataacgtgac tcccttaatt ctccgctcat gatcagattg tcgtttcccg 11940
   ccttcagttt aaactatcag tgtttgacag gatatattgg cgggtaaacc taagagaaaa 12000
   gagcgtttat tagaataatc ggatatttaa aagggcgtga aaaggtttat ccttcgtcca 12060
   tttgtatgtg catgccaacc acagggttcc cca 12093
<210> 21
   <211> 12085
   <212> DNA
   <213> Unknown
<220>
   <223> pflanzlicher Expressionsvektor mit einer Promotor-Terminator-Expressionskassette
<400> 21
   gatctggcgc cggccagcga gacgagcaag attggccgcc gcccgaaacg atccgacagc 60
   gcgcccagca caggtgcgca ggcaaattgc accaacgcat acagcgccag cagaatgcca 120
   tagtgggcgg tgacgtcgtt cgagtgaacc agatcgcgca ggaggcccgg cagcaccggc 180
   ataatcaggc cgatgccgac agcgtcgagc gcgacagtgc tcagaattac gatcaggggt 240
   atgttgggtt tcacgtctgg cctccggacc agcctccgct ggtccgattg aacgcgcgga 300
   ttctttatca ctgataagtt ggtggacata ttatgtttat cagtgataaa gtgtcaagca 360
   tgacaaagtt gcagccgaat acagtgatcc gtgccgccct ggacctgttg aacgaggtcg 420
   gcgtagacgg tctgacgaca cgcaaactgg cggaacggtt gggggttcag cagccggcgc 480
   tttactggca cttcaggaac aagcgggcgc tgctcgacgc actggccgaa gccatgctgg 540
   cggagaatca tacgcattcg gtgccgagag ccgacgacga ctggcgctca tttctgatcg 600
   ggaatgcccg cagcttcagg caggcgctgc tcgcctaccg cgatggcgcg cgcatccatg 660
   ccggcacgcg accgggcgca ccgcagatgg aaacggccga cgcgcagctt cgcttcctct 720
   gcgaggcggg tttttcggcc ggggacgccg tcaatgcgct gatgacaatc agctacttca 780
   ctgttggggc cgtgcttgag gagcaggccg gcgacagcga tgccggcgag cgcggcggca 840
   ccgttgaaca ggctccgctc tcgccgctgt tgcgggccgc gatagacgcc ttcgacgaag 900
   ccggtccgga cgcagcgttc gagcagggac tcgcggtgat tgtcgatgga ttggcgaaaa 960
   ggaggctcgt tgtcaggaac gttgaaggac cgagaaaggg tgacgattga tcaggaccgc 1020
   tgccggagcg caacccactc actacagcag agccatgtag acaacatccc ctcccccttt 1080
   ccaccgcgtc agacgcccgt agcagcccgc tacgggcttt ttcatgccct gccctagcgt 1140
   ccaagcctca cggccgcgct cggcctctct ggcggccttc tggcgctctt ccgcttcctc 1200
   gctcactgac tcgctgcgct cggtcgttcg gctgcggcga gcggtatcag ctcactcaaa 1260
   ggcggtaata cggttatcca cagaatcagg ggataacgca ggaaagaaca tgtgagcaaa 1320
   aggccagcaa aaggccagga accgtaaaaa ggccgcgttg ctggcgtttt tccataggct 1380
   ccgcccccct gacgagcatc acaaaaatcg acgctcaagt cagaggtggc gaaacccgac 1440
   aggactataa agataccagg cgtttccccc tggaagctcc ctcgtgcgct ctcctgttcc 1500
   gaccctgccg cttaccggat acctgtccgc ctttctccct tcgggaagcg tggcgctttt 1560
   ccgctgcata accctgcttc ggggtcatta tagcgatttt ttcggtatat ccatcctttt 1620
   tcgcacgata tacaggattt tgccaaaggg ttcgtgtaga ctttccttgg tgtatccaac 1680
   ggcgtcagcc gggcaggata ggtgaagtag gcccacccgc gagcgggtgt tccttcttca 1740
   ctgtccctta ttcgcacctg gcggtgctca acgggaatcc tgctctgcga ggctggccgg 1800
   ctaccgccgg cgtaacagat gagggcaagc ggatggctga tgaaaccaag ccaaccagga 1860
   agggcagccc acctatcaag gtgtactgcc ttccagacga acgaagagcg attgaggaaa 1920
   aggcggcggc ggccggcatg agcctgtcgg cctacctgct ggccgtcggc cagggctaca 1980
   aaatcacggg cgtcgtggac tatgagcacg tccgcgagct ggcccgcatc aatggcgacc 2040
   tgggccgcct gggcggcctg ctgaaactct ggctcaccga cgacccgcgc acggcgcggt 2100
   tcggtgatgc cacgatcctc gccctgctgg cgaagatcga agagaagcag gacgagcttg 2160
   gcaaggtcat gatgggcgtg gtccgcccga gggcagagcc atgacttttt tagccgctaa 2220
   aacggccggg gggtgcgcgt gattgccaag cacgtcccca tgcgctccat caagaagagc 2280
   gacttcgcgg agctggtgaa gtacatcacc gacgagcaag gcaagaccga gcgcctttgc 2340
   gacgctcacc gggctggttg ccctcgccgc tgggctggcg gccgtctatg gccctgcaaa 2400
   cgcgccagaa acgccgtcga agccgtgtgc gagacaccgc ggccgccggc gttgtggata 2460
   cctcgcggaa aacttggccc tcactgacag atgaggggcg gacgttgaca cttgaggggc 2520
   cgactcaccc ggcgcggcgt tgacagatga ggggcaggct cgatttcggc cggcgacgtg 2580
   gagctggcca gcctcgcaaa tcggcgaaaa cgcctgattt tacgcgagtt tcccacagat 2640
   gatgtggaca agcctgggga taagtgccct gcggtattga cacttgaggg gcgcgactac 2700
   tgacagatga ggggcgcgat ccttgacact tgaggggcag agtgctgaca gatgaggggc 2760
   gcacctattg acatttgagg ggctgtccac aggcagaaaa tccagcattt gcaagggttt 2820
   ccgcccgttt ttcggccacc gctaacctgt cttttaacct gcttttaaac caatatttat 2880
   aaaccttgtt tttaaccagg gctgcgccct gtgcgcgtga ccgcgcacgc cgaagggggg 2940
   tgccccccct tctcgaaccc tcccggcccg ctaacgcggg cctcccatcc ccccaggggc 3000
   tgcgcccctc ggccgcgaac ggcctcaccc caaaaatggc agcgctggca gtccttgcca 3060
   ttgccgggat cggggcagta acgggatggg cgatcagccc gagcgcgacg cccggaagca 3120
   ttgacgtgcc gcaggtgctg gcatcgacat tcagcgacca ggtgccgggc agtgagggcg 3180
   gcggcctggg tggcggcctg cccttcactt cggccgtcgg ggcattcacg gacttcatgg 3240
   cggggccggc aatttttacc ttgggcattc ttggcatagt ggtcgcgggt gccgtgctcg 3300
   tgttcggggg tgcgataaac ccagcgaacc atttgaggtg ataggtaaga ttataccgag 3360
   gtatgaaaac gagaattgga cctttacaga attactctat gaagcgccat atttaaaaag 3420
   ctaccaagac gaagaggatg aagaggatga ggaggcagat tgccttgaat atattgacaa 3480
   tactgataag ataatatatc ttttatatag aagatatcgc cgtatgtaag gatttcaggg 3540
   ggcaaggcat aggcagcgcg cttatcaata tatctataga atgggcaaag cataaaaact 3600
   tgcatggact aatgcttgaa acccaggaca ataaccttat agcttgtaaa ttctatcata 3660
   attgggtaat gactccaact tattgatagt gttttatgtt cagataatgc ccgatgactt 3720
   tgtcatgcag ctccaccgat tttgagaacg acagcgactt ccgtcccagc cgtgccaggt 3780
   gctgcctcag attcaggtta tgccgctcaa ttcgctgcgt atatcgcttg ctgattacgt 3840
   gcagctttcc cttcaggcgg gattcataca gcggccagcc atccgtcatc catatcacca 3900
   cgtcaaaggg tgacagcagg ctcataagac gccccagcgt cgccatagtg cgttcaccga 3960
   atacgtgcgc aacaaccgtc ttccggagac tgtcatacgc gtaaaacagc cagcgctggc 4020
   gcgatttagc cccgacatag ccccactgtt cgtccatttc cgcgcagacg atgacgtcac 4080
   tgcccggctg tatgcgcgag gttaccgact gcggcctgag ttttttaagt gacgtaaaat 4140
   cgtgttgagg ccaacgccca taatgcgggc tgttgcccgg catccaacgc cattcatggc 4200
   catatcaatg attttctggt gcgtaccggg ttgagaagcg gtgtaagtga actgcagttg 4260
   ccatgtttta cggcagtgag agcagagata gcgctgatgt ccggcggtgc ttttgccgtt 4320
   acgcaccacc ccgtcagtag ctgaacagga gggacagctg atagacacag aagccactgg 4380
   agcacctcaa aaacaccatc atacactaaa tcagtaagtt ggcagcatca cccataattg 4440
   tggtttcaaa atcggctccg tcgatactat gttatacgcc aactttgaaa acaactttga 4500
   aaaagctgtt ttctggtatt taaggtttta gaatgcaagg aacagtgaat tggagttcgt 4560
   cttgttataa ttagcttctt ggggtatctt taaatactgt agaaaagagg aaggaaataa 4620
   taaatggcta aaatgagaat atcaccggaa ttgaaaaaac tgatcgaaaa ataccgctgc 4680
   gtaaaagata cggaaggaat gtctcctgct aaggtatata agctggtggg agaaaatgaa 4740
   aacctatatt taaaaatgac ggacagccgg tataaaggga ccacctatga tgtggaacgg 4800
   gaaaaggaca tgatgctatg gctggaagga aagctgcctg ttccaaaggt cctgcacttt 4860
   gaacggcatg atggctggag caatctgctc atgagtgagg ccgatggcgt cctttgctcg 4920
   gaagagtatg aagatgaaca aagccctgaa aagattatcg agctgtatgc ggagtgcatc 4980
   aggctctttc actccatcga catatcggat tgtccctata cgaatagctt agacagccgc 5040
   ttagccgaat tggattactt actgaataac gatctggccg atgtggattg cgaaaactgg 5100
   gaagaagaca ctccatttaa agatccgcgc gagctgtatg attttttaaa gacggaaaag 5160
   cccgaagagg aacttgtctt ttcccacggc gacctgggag acagcaacat ctttgtgaaa 5220
   gatggcaaag taagtggctt tattgatctt gggagaagcg gcagggcgga caagtggtat 5280
   gacattgcct tctgcgtccg gtcgatcagg gaggatatcg gggaagaaca gtatgtcgag 5340
   ctattttttg acttactggg gatcaagcct gattgggaga aaataaaata ttatatttta 5400
   ctggatgaat tgttttagta cctagatgtg gcgcaacgat gccggcgaca agcaggagcg 5460
   caccgacttc ttccgcatca agtgttttgg ctctcaggcc gaggcccacg gcaagtattt 5520
   gggcaagggg tcgctggtat tcgtgcaggg caagattcgg aataccaagt acgagaagga 5580
   cggccagacg gtctacggga ccgacttcat tgccgataag gtggattatc tggacaccaa 5640
   ggcaccaggc gggtcaaatc aggaataagg gcacattgcc ccggcgtgag tcggggcaat 5700
   cccgcaagga gggtgaatga atcggacgtt tgaccggaag gcatacaggc aagaactgat 5760
   cgacgcgggg ttttccgccg aggatgccga aaccatcgca agccgcaccg tcatgcgtgc 5820
   gccccgcgaa accttccagt ccgtcggctc gatggtccag caagctacgg ccaagatcga 5880
   gcgcgacagc gtgcaactgg ctccccctgc cctgcccgcg ccatcggccg ccgtggagcg 5940
   ttcgcgtcgt ctcgaacagg aggcggcagg tttggcgaag tcgatgacca tcgacacgcg 6000
   aggaactatg acgaccaaga agcgaaaaac cgccggcgag gacctggcaa aacaggtcag 6060
   cgaggccaag caggccgcgt tgctgaaaca cacgaagcag cagatcaagg aaatgcagct 6120
   ttccttgttc gatattgcgc cgtggccgga cacgatgcga gcgatgccaa acgacacggc 6180
   ccgctctgcc ctgttcacca cgcgcaacaa gaaaatcccg cgcgaggcgc tgcaaaacaa 6240
   ggtcattttc cacgtcaaca aggacgtgaa gatcacctac accggcgtcg agctgcgggc 6300
   cgacgatgac gaactggtgt ggcagcaggt gttggagtac gcgaagcgca cccctatcgg 6360
   cgagccgatc accttcacgt tctacgagct ttgccaggac ctgggctggt cgatcaatgg 6420
   ccggtattac acgaaggccg aggaatgcct gtcgcgccta caggcgacgg cgatgggctt 6480
   cacgtccgac cgcgttgggc acctggaatc ggtgtcgctg ctgcaccgct tccgcgtcct 6540
   ggaccgtggc aagaaaacgt cccgttgcca ggtcctgatc gacgaggaaa tcgtcgtgct 6600
   gtttgctggc gaccactaca cgaaattcat atgggagaag taccgcaagc tgtcgccgac 6660
   ggcccgacgg atgttcgact atttcagctc gcaccgggag ccgtacccgc tcaagctgga 6720
   aaccttccgc ctcatgtgcg gatcggattc cacccgcgtg aagaagtggc gcgagcaggt 6780
   cggcgaagcc tgcgaagagt tgcgaggcag cggcctggtg gaacacgcct gggtcaatga 6840
   tgacctggtg cattgcaaac gctagggcct tgtggggtca gttccggctg ggggttcagc 6900
   agccagcgct ttactggcat ttcaggaaca agcgggcact gctcgacgca cttgcttcgc 6960
   tcagtatcgc tcgggacgca cggcgcgctc tacgaactgc cgataaacag aggattaaaa 7020
   ttgacaattg tgattaaggc tcagattcga cggcttggag cggccgacgt gcaggatttc 7080
   cgcgagatcc gattgtcggc cctgaagaaa gctccagaga tgttcgggtc cgtttacgag 7140
   cacgaggaga aaaagcccat ggaggcgttc gctgaacggt tgcgagatgc cgtggcattc 7200
   ggcgcctaca tcgacggcga gatcattggg ctgtcggtct tcaaacagga ggacggcccc 7260
   aaggacgctc acaaggcgca tctgtccggc gttttcgtgg agcccgaaca gcgaggccga 7320
   ggggtcgccg gtatgctgct gcgggcgttg ccggcgggtt tattgctcgt gatgatcgtc 7380
   cgacagattc caacgggaat ctggtggatg cgcatcttca tcctcggcgc acttaatatt 7440
   tcgctattct ggagcttgtt gtttatttcg gtctaccgcc tgccgggcgg ggtcgcggcg 7500
   acggtaggcg ctgtgcagcc gctgatggtc gtgttcatct ctgccgctct gctaggtagc 7560
   ccgatacgat tgatggcggt cctgggggct atttgcggaa ctgcgggcgt ggcgctgttg 7620
   gtgttgacac caaacgcagc gctagatcct gtcggcgtcg cagcgggcct ggcgggggcg 7680
   gtttccatgg cgttcggaac cgtgctgacc cgcaagtggc aacctcccgt gcctctgctc 7740
   acctttaccg cctggcaact ggcggccgga ggacttctgc tcgttccagt agctttagtg 7800
   tttgatccgc caatcccgat gcctacagga accaatgttc tcggcctggc gtggctcggc 7860
   ctgatcggag cgggtttaac ctacttcctt tggttccggg ggatctcgcg actcgaacct 7920
   acagttgttt ccttactggg ctttctcagc cccagatctg gggtcgatca gccggggatg 7980
   catcaggccg acagtcggaa cttcgggtcc ccgacctgta ccattcggtg agcaatggat 8040
   aggggagttg atatcgtcaa cgttcacttc taaagaaata gcgccactca gcttcctcag 8100
   cggctttatc cagcgatttc ctattatgtc ggcatagttc tcaagatcga cagcctgtca 8160
   cggttaagcg agaaatgaat aagaaggctg ataattcgga tctctgcgag ggagatgata 8220
   tttgatcaca ggcagcaacg ctctgtcatc gttacaatca acatgctacc ctccgcgaga 8280
   tcatccgtgt ttcaaacccg gcagcttagt tgccgttctt ccgaatagca tcggtaacat 8340
   gagcaaagtc tgccgcctta caacggctct cccgctgacg ccgtcccgga ctgatgggct 8400
   gcctgtatcg agtggtgatt ttgtgccgag ctgccggtcg gggagctgtt ggctggctgg 8460
   tggcaggata tattgtggtg taaacaaatt gacgcttaga caacttaata acacattgcg 8520
   gacgttttta atgtactggg gtggtttttc ttttcaccag tgagacgggc aacagctgat 8580
   tgcccttcac cgcctggccc tgagagagtt gcagcaagcg gtccacgctg gtttgcccca 8640
   gcaggcgaaa atcctgtttg atggtggttc cgaaatcggc aaaatccctt ataaatcaaa 8700
   agaatagccc gagatagggt tgagtgttgt tccagtttgg aacaagagtc cactattaaa 8760
   gaacgtggac tccaacgtca aagggcgaaa aaccgtctat cagggcgatg gcccactacg 8820
   tgaaccatca cccaaatcaa gttttttggg gtcgaggtgc cgtaaagcac taaatcggaa 8880
   ccctaaaggg agcccccgat ttagagcttg acggggaaag ccggcgaacg tggcgagaaa 8940
   ggaagggaag aaagcgaaag gagcgggcgc cattcaggct gcgcaactgt tgggaagggc 9000
   gatcggtgcg ggcctcttcg ctattacgcc agctggcgaa agggggatgt gctgcaaggc 9060
   gattaagttg ggtaacgcca gggttttccc agtcacgacg ttgtaaaacg acggccagtg 9120
   aattaattcc catcttgaaa gaaatatagt ttaaatattt attgataaaa taacaagtca 9180
   ggtattatag tccaagcaaa aacataaatt tattgatgca agtttaaatt cagaaatatt 9240
   tcaataactg attatatcag ctggtacatt gccgtagatg aaagactgag tgcgatatta 9300
   tgtgtaatac ataaattgat gatatagcta gcttagctca tcgggggatc cgtcgaagct 9360
   agcttgggtc ccgctcagaa gaactcgtca agaaggcgat agaaggcgat gcgctgcgaa 9420
   tcgggagcgg cgataccgta aagcacgagg aagcggtcag cccattcgcc gccaagctct 9480
   tcagcaatat cacgggtagc caacgctatg tcctgatagc ggtccgccac acccagccgg 9540
   ccacagtcga tgaatccaga aaagcggcca ttttccacca tgatattcgg caagcaggca 9600
   tcgccatggg tcacgacgag atcctcgccg tcgggcatgc gcgccttgag cctggcgaac 9660
   agttcggctg gcgcgagccc ctgatgctct tcgtccagat catcctgatc gacaagaccg 9720
   gcttccatcc gagtacgtgc tcgctcgatg cgatgtttcg cttggtggtc gaatgggcag 9780
   gtagccggat caagcgtatg cagccgccgc attgcatcag ccatgatgga tactttctcg 9840
   gcaggagcaa ggtgagatga caggagatcc tgccccggca cttcgcccaa tagcagccag 9900
   tcccttcccg cttcagtgac aacgtcgagc acagctgcgc aaggaacgcc cgtcgtggcc 9960
   agccacgata gccgcgctgc ctcgtcctgc agttcattca gggcaccgga caggtcggtc 10020
   ttgacaaaaa gaaccgggcg cccctgcgct gacagccgga acacggcggc atcagagcag 10080
   ccgattgtct gttgtgccca gtcatagccg aatagcctct ccacccaagc ggccggagaa 10140
   cctgcgtgca atccatcttg ttcaatccaa gctcccatgg gccctcgact agagtcgaga 10200
   tctggattga gagtgaatat gagactctaa ttggataccg aggggaattt atggaacgtc 10260
   agtggagcat ttttgacaag aaatatttgc tagctgatag tgaccttagg cgacttttga 10320
   acgcgcaata atggtttctg acgtatgtgc ttagctcatt aaactccaga aacccgcggc 10380
   tgagtggctc cttcaacgtt gcggttctgt cagttccaaa cgtaaaacgg cttgtcccgc 10440
   gtcatcggcg ggggtcataa cgtgactccc ttaattctcc gctcatgatc ttgatcccct 10500
   gcgccatcag atccttggcg gcaagaaagc catccagttt actttgcagg gcttcccaac 10560
   cttaccagag ggcgccccag ctggcaattc cggttcgctt gctgtccata aaaccgccca 10620
   gtctagctat cgccatgtaa gcccactgca agctacctgc tttctctttg cgcttgcgtt 10680
   ttcccttgtc cagatagccc agtagctgac attcatccgg ggtcagcacc gtttctgcgg 10740
   actggctttc tacgtgttcc gcttccttta gcagcccttg cgccctgagt gcttgcggca 10800
   gcgtgaagct tgcatgcctg caggtcgacg gcgcgccgag ctcctcgagc aaatttacac 10860
   attgccacta aacgtctaaa cccttgtaat ttgtttttgt tttactatgt gtgttatgta 10920
   tttgatttgc gataaatttt tatatttggt actaaattta taacaccttt tatgctaacg 10980
   tttgccaaca cttagcaatt tgcaagttga ttaattgatt ctaaattatt tttgtcttct 11040
   aaatacatat actaatcaac tggaaatgta aatatttgct aatatttcta ctataggaga 11100
   attaaagtga gtgaatatgg taccacaagg tttggagatt taattgttgc aatgctgcat 11160
   ggatggcata tacaccaaac attcaataat tcttgaggat aataatggta ccacacaaga 11220
   tttgaggtgc atgaacgtca cgtggacaaa aggtttagta atttttcaag acaacaatgt 11280
   taccacacac aagttttgag gtgcatgcat ggatgccctg tggaaagttt aaaaatattt 11340
   tggaaatgat ttgcatggaa gccatgtgta aaaccatgac atccacttgg aggatgcaat 11400
   aatgaagaaa actacaaatt tacatgcaac tagttatgca tgtagtctat ataatgagga 11460
   ttttgcaata ctttcattca tacacactca ctaagtttta cacgattata atttcttcat 11520
   agccagcgga tccgatatcg ggcccgctag cgttaaccct gctttaatga gatatgcgag 11580
   acgcctatga tcgcatgata tttgctttca attctgttgt gcacgttgta aaaaacctga 11640
   gcatgtgtag ctcagatcct taccgccggt ttcggttcat tctaatgaat atatcacccg 11700
   ttactatcgt atttttatga ataatattct ccgttcaatt tactgattgt ccgtcgacga 11760
   attcgagctc ggcgcgcctc tagaggatcg atgaattcag atcggctgag tggctccttc 11820
   aacgttgcgg ttctgtcagt tccaaacgta aaacggcttg tcccgcgtca tcggcggggg 11880
   tcataacgtg actcccttaa ttctccgctc atgatcagat tgtcgtttcc cgccttcagt 11940
   ttaaactatc agtgtttgac aggatatatt ggcgggtaaa cctaagagaa aagagcgttt 12000
   attagaataa tcggatattt aaaagggcgt gaaaaggttt atccttcgtc catttgtatg 12060
   tgcatgccaa ccacagggtt cccca 12085
<210> 22
   <211> 12079
   <212> DNA
   <213> Unknown
<220>
   <223> pflanzlicher Expressionsvektor mit einer Promotor-Terminator-Expressionskassette
<400> 22
   gatctggcgc cggccagcga gacgagcaag attggccgcc gcccgaaacg atccgacagc 60
   gcgcccagca caggtgcgca ggcaaattgc accaacgcat acagcgccag cagaatgcca 120
   tagtgggcgg tgacgtcgtt cgagtgaacc agatcgcgca ggaggcccgg cagcaccggc 180
   ataatcaggc cgatgccgac agcgtcgagc gcgacagtgc tcagaattac gatcaggggt 240
   atgttgggtt tcacgtctgg cctccggacc agcctccgct ggtccgattg aacgcgcgga 300
   ttctttatca ctgataagtt ggtggacata ttatgtttat cagtgataaa gtgtcaagca 360
   tgacaaagtt gcagccgaat acagtgatcc gtgccgccct ggacctgttg aacgaggtcg 420
   gcgtagacgg tctgacgaca cgcaaactgg cggaacggtt gggggttcag cagccggcgc 480
   tttactggca cttcaggaac aagcgggcgc tgctcgacgc actggccgaa gccatgctgg 540
   cggagaatca tacgcattcg gtgccgagag ccgacgacga ctggcgctca tttctgatcg 600
   ggaatgcccg cagcttcagg caggcgctgc tcgcctaccg cgatggcgcg cgcatccatg 660
   ccggcacgcg accgggcgca ccgcagatgg aaacggccga cgcgcagctt cgcttcctct 720
   gcgaggcggg tttttcggcc ggggacgccg tcaatgcgct gatgacaatc agctacttca 780
   ctgttggggc cgtgcttgag gagcaggccg gcgacagcga tgccggcgag cgcggcggca 840
   ccgttgaaca ggctccgctc tcgccgctgt tgcgggccgc gatagacgcc ttcgacgaag 900
   ccggtccgga cgcagcgttc gagcagggac tcgcggtgat tgtcgatgga ttggcgaaaa 960
   ggaggctcgt tgtcaggaac gttgaaggac cgagaaaggg tgacgattga tcaggaccgc 1020
   tgccggagcg caacccactc actacagcag agccatgtag acaacatccc ctcccccttt 1080
   ccaccgcgtc agacgcccgt agcagcccgc tacgggcttt ttcatgccct gccctagcgt 1140
   ccaagcctca cggccgcgct cggcctctct ggcggccttc tggcgctctt ccgcttcctc 1200
   gctcactgac tcgctgcgct cggtcgttcg gctgcggcga gcggtatcag ctcactcaaa 1260
   ggcggtaata cggttatcca cagaatcagg ggataacgca ggaaagaaca tgtgagcaaa 1320
   aggccagcaa aaggccagga accgtaaaaa ggccgcgttg ctggcgtttt tccataggct 1380
   ccgcccccct gacgagcatc acaaaaatcg acgctcaagt cagaggtggc gaaacccgac 1440
   aggactataa agataccagg cgtttccccc tggaagctcc ctcgtgcgct ctcctgttcc 1500
   gaccctgccg cttaccggat acctgtccgc ctttctccct tcgggaagcg tggcgctttt 1560
   ccgctgcata accctgcttc ggggtcatta tagcgatttt ttcggtatat ccatcctttt 1620
   tcgcacgata tacaggattt tgccaaaggg ttcgtgtaga ctttccttgg tgtatccaac 1680
   ggcgtcagcc gggcaggata ggtgaagtag gcccacccgc gagcgggtgt tccttcttca 1740
   ctgtccctta ttcgcacctg gcggtgctca acgggaatcc tgctctgcga ggctggccgg 1800
   ctaccgccgg cgtaacagat gagggcaagc ggatggctga tgaaaccaag ccaaccagga 1860
   agggcagccc acctatcaag gtgtactgcc ttccagacga acgaagagcg attgaggaaa 1920
   aggcggcggc ggccggcatg agcctgtcgg cctacctgct ggccgtcggc cagggctaca 1980
   aaatcacggg cgtcgtggac tatgagcacg tccgcgagct ggcccgcatc aatggcgacc 2040
   tgggccgcct gggcggcctg ctgaaactct ggctcaccga cgacccgcgc acggcgcggt 2100
   tcggtgatgc cacgatcctc gccctgctgg cgaagatcga agagaagcag gacgagcttg 2160
   gcaaggtcat gatgggcgtg gtccgcccga gggcagagcc atgacttttt tagccgctaa 2220
   aacggccggg gggtgcgcgt gattgccaag cacgtcccca tgcgctccat caagaagagc 2280
   gacttcgcgg agctggtgaa gtacatcacc gacgagcaag gcaagaccga gcgcctttgc 2340
   gacgctcacc gggctggttg ccctcgccgc tgggctggcg gccgtctatg gccctgcaaa 2400
   cgcgccagaa acgccgtcga agccgtgtgc gagacaccgc ggccgccggc gttgtggata 2460
   cctcgcggaa aacttggccc tcactgacag atgaggggcg gacgttgaca cttgaggggc 2520
   cgactcaccc ggcgcggcgt tgacagatga ggggcaggct cgatttcggc cggcgacgtg 2580
   gagctggcca gcctcgcaaa tcggcgaaaa cgcctgattt tacgcgagtt tcccacagat 2640
   gatgtggaca agcctgggga taagtgccct gcggtattga cacttgaggg gcgcgactac 2700
   tgacagatga ggggcgcgat ccttgacact tgaggggcag agtgctgaca gatgaggggc 2760
   gcacctattg acatttgagg ggctgtccac aggcagaaaa tccagcattt gcaagggttt 2820
   ccgcccgttt ttcggccacc gctaacctgt cttttaacct gcttttaaac caatatttat 2880
   aaaccttgtt tttaaccagg gctgcgccct gtgcgcgtga ccgcgcacgc cgaagggggg 2940
   tgccccccct tctcgaaccc tcccggcccg ctaacgcggg cctcccatcc ccccaggggc 3000
   tgcgcccctc ggccgcgaac ggcctcaccc caaaaatggc agcgctggca gtccttgcca 3060
   ttgccgggat cggggcagta acgggatggg cgatcagccc gagcgcgacg cccggaagca 3120
   ttgacgtgcc gcaggtgctg gcatcgacat tcagcgacca ggtgccgggc agtgagggcg 3180
   gcggcctggg tggcggcctg cccttcactt cggccgtcgg ggcattcacg gacttcatgg 3240
   cggggccggc aatttttacc ttgggcattc ttggcatagt ggtcgcgggt gccgtgctcg 3300
   tgttcggggg tgcgataaac ccagcgaacc atttgaggtg ataggtaaga ttataccgag 3360
   gtatgaaaac gagaattgga cctttacaga attactctat gaagcgccat atttaaaaag 3420
   ctaccaagac gaagaggatg aagaggatga ggaggcagat tgccttgaat atattgacaa 3480
   tactgataag ataatatatc ttttatatag aagatatcgc cgtatgtaag gatttcaggg 3540
   ggcaaggcat aggcagcgcg cttatcaata tatctataga atgggcaaag cataaaaact 3600
   tgcatggact aatgcttgaa acccaggaca ataaccttat agcttgtaaa ttctatcata 3660
   attgggtaat gactccaact tattgatagt gttttatgtt cagataatgc ccgatgactt 3720
   tgtcatgcag ctccaccgat tttgagaacg acagcgactt ccgtcccagc cgtgccaggt 3780
   gctgcctcag attcaggtta tgccgctcaa ttcgctgcgt atatcgcttg ctgattacgt 3840
   gcagctttcc cttcaggcgg gattcataca gcggccagcc atccgtcatc catatcacca 3900
   cgtcaaaggg tgacagcagg ctcataagac gccccagcgt cgccatagtg cgttcaccga 3960
   atacgtgcgc aacaaccgtc ttccggagac tgtcatacgc gtaaaacagc cagcgctggc 4020
   gcgatttagc cccgacatag ccccactgtt cgtccatttc cgcgcagacg atgacgtcac 4080
   tgcccggctg tatgcgcgag gttaccgact gcggcctgag ttttttaagt gacgtaaaat 4140
   cgtgttgagg ccaacgccca taatgcgggc tgttgcccgg catccaacgc cattcatggc 4200
   catatcaatg attttctggt gcgtaccggg ttgagaagcg gtgtaagtga actgcagttg 4260
   ccatgtttta cggcagtgag agcagagata gcgctgatgt ccggcggtgc ttttgccgtt 4320
   acgcaccacc ccgtcagtag ctgaacagga gggacagctg atagacacag aagccactgg 4380
   agcacctcaa aaacaccatc atacactaaa tcagtaagtt ggcagcatca cccataattg 4440
   tggtttcaaa atcggctccg tcgatactat gttatacgcc aactttgaaa acaactttga 4500
   aaaagctgtt ttctggtatt taaggtttta gaatgcaagg aacagtgaat tggagttcgt 4560
   cttgttataa ttagcttctt ggggtatctt taaatactgt agaaaagagg aaggaaataa 4620
   taaatggcta aaatgagaat atcaccggaa ttgaaaaaac tgatcgaaaa ataccgctgc 4680
   gtaaaagata cggaaggaat gtctcctgct aaggtatata agctggtggg agaaaatgaa 4740
   aacctatatt taaaaatgac ggacagccgg tataaaggga ccacctatga tgtggaacgg 4800
   gaaaaggaca tgatgctatg gctggaagga aagctgcctg ttccaaaggt cctgcacttt 4860
   gaacggcatg atggctggag caatctgctc atgagtgagg ccgatggcgt cctttgctcg 4920
   gaagagtatg aagatgaaca aagccctgaa aagattatcg agctgtatgc ggagtgcatc 4980
   aggctctttc actccatcga catatcggat tgtccctata cgaatagctt agacagccgc 5040
   ttagccgaat tggattactt actgaataac gatctggccg atgtggattg cgaaaactgg 5100
   gaagaagaca ctccatttaa agatccgcgc gagctgtatg attttttaaa gacggaaaag 5160
   cccgaagagg aacttgtctt ttcccacggc gacctgggag acagcaacat ctttgtgaaa 5220
   gatggcaaag taagtggctt tattgatctt gggagaagcg gcagggcgga caagtggtat 5280
   gacattgcct tctgcgtccg gtcgatcagg gaggatatcg gggaagaaca gtatgtcgag 5340
   ctattttttg acttactggg gatcaagcct gattgggaga aaataaaata ttatatttta 5400
   ctggatgaat tgttttagta cctagatgtg gcgcaacgat gccggcgaca agcaggagcg 5460
   caccgacttc ttccgcatca agtgttttgg ctctcaggcc gaggcccacg gcaagtattt 5520
   gggcaagggg tcgctggtat tcgtgcaggg caagattcgg aataccaagt acgagaagga 5580
   cggccagacg gtctacggga ccgacttcat tgccgataag gtggattatc tggacaccaa 5640
   ggcaccaggc gggtcaaatc aggaataagg gcacattgcc ccggcgtgag tcggggcaat 5700
   cccgcaagga gggtgaatga atcggacgtt tgaccggaag gcatacaggc aagaactgat 5760
   cgacgcgggg ttttccgccg aggatgccga aaccatcgca agccgcaccg tcatgcgtgc 5820
   gccccgcgaa accttccagt ccgtcggctc gatggtccag caagctacgg ccaagatcga 5880
   gcgcgacagc gtgcaactgg ctccccctgc cctgcccgcg ccatcggccg ccgtggagcg 5940
   ttcgcgtcgt ctcgaacagg aggcggcagg tttggcgaag tcgatgacca tcgacacgcg 6000
   aggaactatg acgaccaaga agcgaaaaac cgccggcgag gacctggcaa aacaggtcag 6060
   cgaggccaag caggccgcgt tgctgaaaca cacgaagcag cagatcaagg aaatgcagct 6120
   ttccttgttc gatattgcgc cgtggccgga cacgatgcga gcgatgccaa acgacacggc 6180
   ccgctctgcc ctgttcacca cgcgcaacaa gaaaatcccg cgcgaggcgc tgcaaaacaa 6240
   ggtcattttc cacgtcaaca aggacgtgaa gatcacctac accggcgtcg agctgcgggc 6300
   cgacgatgac gaactggtgt ggcagcaggt gttggagtac gcgaagcgca cccctatcgg 6360
   cgagccgatc accttcacgt tctacgagct ttgccaggac ctgggctggt cgatcaatgg 6420
   ccggtattac acgaaggccg aggaatgcct gtcgcgccta caggcgacgg cgatgggctt 6480
   cacgtccgac cgcgttgggc acctggaatc ggtgtcgctg ctgcaccgct tccgcgtcct 6540
   ggaccgtggc aagaaaacgt cccgttgcca ggtcctgatc gacgaggaaa tcgtcgtgct 6600
   gtttgctggc gaccactaca cgaaattcat atgggagaag taccgcaagc tgtcgccgac 6660
   ggcccgacgg atgttcgact atttcagctc gcaccgggag ccgtacccgc tcaagctgga 6720
   aaccttccgc ctcatgtgcg gatcggattc cacccgcgtg aagaagtggc gcgagcaggt 6780
   cggcgaagcc tgcgaagagt tgcgaggcag cggcctggtg gaacacgcct gggtcaatga 6840
   tgacctggtg cattgcaaac gctagggcct tgtggggtca gttccggctg ggggttcagc 6900
   agccagcgct ttactggcat ttcaggaaca agcgggcact gctcgacgca cttgcttcgc 6960
   tcagtatcgc tcgggacgca cggcgcgctc tacgaactgc cgataaacag aggattaaaa 7020
   ttgacaattg tgattaaggc tcagattcga cggcttggag cggccgacgt gcaggatttc 7080
   cgcgagatcc gattgtcggc cctgaagaaa gctccagaga tgttcgggtc cgtttacgag 7140
   cacgaggaga aaaagcccat ggaggcgttc gctgaacggt tgcgagatgc cgtggcattc 7200
   ggcgcctaca tcgacggcga gatcattggg ctgtcggtct tcaaacagga ggacggcccc 7260
   aaggacgctc acaaggcgca tctgtccggc gttttcgtgg agcccgaaca gcgaggccga 7320
   ggggtcgccg gtatgctgct gcgggcgttg ccggcgggtt tattgctcgt gatgatcgtc 7380
   cgacagattc caacgggaat ctggtggatg cgcatcttca tcctcggcgc acttaatatt 7440
   tcgctattct ggagcttgtt gtttatttcg gtctaccgcc tgccgggcgg ggtcgcggcg 7500
   acggtaggcg ctgtgcagcc gctgatggtc gtgttcatct ctgccgctct gctaggtagc 7560
   ccgatacgat tgatggcggt cctgggggct atttgcggaa ctgcgggcgt ggcgctgttg 7620
   gtgttgacac caaacgcagc gctagatcct gtcggcgtcg cagcgggcct ggcgggggcg 7680
   gtttccatgg cgttcggaac cgtgctgacc cgcaagtggc aacctcccgt gcctctgctc 7740
   acctttaccg cctggcaact ggcggccgga ggacttctgc tcgttccagt agctttagtg 7800
   tttgatccgc caatcccgat gcctacagga accaatgttc tcggcctggc gtggctcggc 7860
   ctgatcggag cgggtttaac ctacttcctt tggttccggg ggatctcgcg actcgaacct 7920
   acagttgttt ccttactggg ctttctcagc cccagatctg gggtcgatca gccggggatg 7980
   catcaggccg acagtcggaa cttcgggtcc ccgacctgta ccattcggtg agcaatggat 8040
   aggggagttg atatcgtcaa cgttcacttc taaagaaata gcgccactca gcttcctcag 8100
   cggctttatc cagcgatttc ctattatgtc ggcatagttc tcaagatcga cagcctgtca 8160
   cggttaagcg agaaatgaat aagaaggctg ataattcgga tctctgcgag ggagatgata 8220
   tttgatcaca ggcagcaacg ctctgtcatc gttacaatca acatgctacc ctccgcgaga 8280
   tcatccgtgt ttcaaacccg gcagcttagt tgccgttctt ccgaatagca tcggtaacat 8340
   gagcaaagtc tgccgcctta caacggctct cccgctgacg ccgtcccgga ctgatgggct 8400
   gcctgtatcg agtggtgatt ttgtgccgag ctgccggtcg gggagctgtt ggctggctgg 8460
   tggcaggata tattgtggtg taaacaaatt gacgcttaga caacttaata acacattgcg 8520
   gacgttttta atgtactggg gtggtttttc ttttcaccag tgagacgggc aacagctgat 8580
   tgcccttcac cgcctggccc tgagagagtt gcagcaagcg gtccacgctg gtttgcccca 8640
   gcaggcgaaa atcctgtttg atggtggttc cgaaatcggc aaaatccctt ataaatcaaa 8700
   agaatagccc gagatagggt tgagtgttgt tccagtttgg aacaagagtc cactattaaa 8760
   gaacgtggac tccaacgtca aagggcgaaa aaccgtctat cagggcgatg gcccactacg 8820
   tgaaccatca cccaaatcaa gttttttggg gtcgaggtgc cgtaaagcac taaatcggaa 8880
   ccctaaaggg agcccccgat ttagagcttg acggggaaag ccggcgaacg tggcgagaaa 8940
   ggaagggaag aaagcgaaag gagcgggcgc cattcaggct gcgcaactgt tgggaagggc 9000
   gatcggtgcg ggcctcttcg ctattacgcc agctggcgaa agggggatgt gctgcaaggc 9060
   gattaagttg ggtaacgcca gggttttccc agtcacgacg ttgtaaaacg acggccagtg 9120
   aattaattcc catcttgaaa gaaatatagt ttaaatattt attgataaaa taacaagtca 9180
   ggtattatag tccaagcaaa aacataaatt tattgatgca agtttaaatt cagaaatatt 9240
   tcaataactg attatatcag ctggtacatt gccgtagatg aaagactgag tgcgatatta 9300
   tgtgtaatac ataaattgat gatatagcta gcttagctca tcgggggatc cgtcgaagct 9360
   agcttgggtc ccgctcagaa gaactcgtca agaaggcgat agaaggcgat gcgctgcgaa 9420
   tcgggagcgg cgataccgta aagcacgagg aagcggtcag cccattcgcc gccaagctct 9480
   tcagcaatat cacgggtagc caacgctatg tcctgatagc ggtccgccac acccagccgg 9540
   ccacagtcga tgaatccaga aaagcggcca ttttccacca tgatattcgg caagcaggca 9600
   tcgccatggg tcacgacgag atcctcgccg tcgggcatgc gcgccttgag cctggcgaac 9660
   agttcggctg gcgcgagccc ctgatgctct tcgtccagat catcctgatc gacaagaccg 9720
   gcttccatcc gagtacgtgc tcgctcgatg cgatgtttcg cttggtggtc gaatgggcag 9780
   gtagccggat caagcgtatg cagccgccgc attgcatcag ccatgatgga tactttctcg 9840
   gcaggagcaa ggtgagatga caggagatcc tgccccggca cttcgcccaa tagcagccag 9900
   tcccttcccg cttcagtgac aacgtcgagc acagctgcgc aaggaacgcc cgtcgtggcc 9960
   agccacgata gccgcgctgc ctcgtcctgc agttcattca gggcaccgga caggtcggtc 10020
   ttgacaaaaa gaaccgggcg cccctgcgct gacagccgga acacggcggc atcagagcag 10080
   ccgattgtct gttgtgccca gtcatagccg aatagcctct ccacccaagc ggccggagaa 10140
   cctgcgtgca atccatcttg ttcaatccaa gctcccatgg gccctcgact agagtcgaga 10200
   tctggattga gagtgaatat gagactctaa ttggataccg aggggaattt atggaacgtc 10260
   agtggagcat ttttgacaag aaatatttgc tagctgatag tgaccttagg cgacttttga 10320
   acgcgcaata atggtttctg acgtatgtgc ttagctcatt aaactccaga aacccgcggc 10380
   tgagtggctc cttcaacgtt gcggttctgt cagttccaaa cgtaaaacgg cttgtcccgc 10440
   gtcatcggcg ggggtcataa cgtgactccc ttaattctcc gctcatgatc ttgatcccct 10500
   gcgccatcag atccttggcg gcaagaaagc catccagttt actttgcagg gcttcccaac 10560
   cttaccagag ggcgccccag ctggcaattc cggttcgctt gctgtccata aaaccgccca 10620
   gtctagctat cgccatgtaa gcccactgca agctacctgc tttctctttg cgcttgcgtt 10680
   ttcccttgtc cagatagccc agtagctgac attcatccgg ggtcagcacc gtttctgcgg 10740
   actggctttc tacgtgttcc gcttccttta gcagcccttg cgccctgagt gcttgcggca 10800
   gcgtgaagct tgcatgcctg caggtcgacg gcgcgccgag ctcctcgagc aaatttacac 10860
   attgccacta aacgtctaaa cccttgtaat ttgtttttgt tttactatgt gtgttatgta 10920
   tttgatttgc gataaatttt tatatttggt actaaattta taacaccttt tatgctaacg 10980
   tttgccaaca cttagcaatt tgcaagttga ttaattgatt ctaaattatt tttgtcttct 11040
   aaatacatat actaatcaac tggaaatgta aatatttgct aatatttcta ctataggaga 11100
   attaaagtga gtgaatatgg taccacaagg tttggagatt taattgttgc aatgctgcat 11160
   ggatggcata tacaccaaac attcaataat tcttgaggat aataatggta ccacacaaga 11220
   tttgaggtgc atgaacgtca cgtggacaaa aggtttagta atttttcaag acaacaatgt 11280
   taccacacac aagttttgag gtgcatgcat ggatgccctg tggaaagttt aaaaatattt 11340
   tggaaatgat ttgcatggaa gccatgtgta aaaccatgac atccacttgg aggatgcaat 11400
   aatgaagaaa actacaaatt tacatgcaac tagttatgca tgtagtctat ataatgagga 11460
   ttttgcaata ctttcattca tacacactca ctaagtttta cacgattata atttcttcat 11520
   agccagcaga tctgccggca tcgatcccgg gccatggcct gctttaatga gatatgcgag 11580
   acgcctatga tcgcatgata tttgctttca attctgttgt gcacgttgta aaaaacctga 11640
   gcatgtgtag ctcagatcct taccgccggt ttcggttcat tctaatgaat atatcacccg 11700
   ttactatcgt atttttatga ataatattct ccgttcaatt tactgattgt ccgtcgacga 11760
   gctcggcgcg cctctagagg atcgatgaat tcagatcggc tgagtggctc cttcaacgtt 11820
   gcggttctgt cagttccaaa cgtaaaacgg cttgtcccgc gtcatcggcg ggggtcataa 11880
   cgtgactccc ttaattctcc gctcatgatc agattgtcgt ttcccgcctt cagtttaaac 11940
   tatcagtgtt tgacaggata tattggcggg taaacctaag agaaaagagc gtttattaga 12000
   ataatcggat atttaaaagg gcgtgaaaag gtttatcctt cgtccatttg tatgtgcatg 12060
   ccaaccacag ggttcccca 12079
<210> 23
   <211> 13002
   <212> DNA
   <213> Unknown
<220>
   <223> pflanzlicher Expressionsvektor mit zwei Promotor-Terminator-Expressionskassetten
<400> 23
   gatctggcgc cggccagcga gacgagcaag attggccgcc gcccgaaacg atccgacagc 60
   gcgcccagca caggtgcgca ggcaaattgc accaacgcat acagcgccag cagaatgcca 120
   tagtgggcgg tgacgtcgtt cgagtgaacc agatcgcgca ggaggcccgg cagcaccggc 180
   ataatcaggc cgatgccgac agcgtcgagc gcgacagtgc tcagaattac gatcaggggt 240
   atgttgggtt tcacgtctgg cctccggacc agcctccgct ggtccgattg aacgcgcgga 300
   ttctttatca ctgataagtt ggtggacata ttatgtttat cagtgataaa gtgtcaagca 360
   tgacaaagtt gcagccgaat acagtgatcc gtgccgccct ggacctgttg aacgaggtcg 420
   gcgtagacgg tctgacgaca cgcaaactgg cggaacggtt gggggttcag cagccggcgc 480
   tttactggca cttcaggaac aagcgggcgc tgctcgacgc actggccgaa gccatgctgg 540
   cggagaatca tacgcattcg gtgccgagag ccgacgacga ctggcgctca tttctgatcg 600
   ggaatgcccg cagcttcagg caggcgctgc tcgcctaccg cgatggcgcg cgcatccatg 660
   ccggcacgcg accgggcgca ccgcagatgg aaacggccga cgcgcagctt cgcttcctct 720
   gcgaggcggg tttttcggcc ggggacgccg tcaatgcgct gatgacaatc agctacttca 780
   ctgttggggc cgtgcttgag gagcaggccg gcgacagcga tgccggcgag cgcggcggca 840
   ccgttgaaca ggctccgctc tcgccgctgt tgcgggccgc gatagacgcc ttcgacgaag 900
   ccggtccgga cgcagcgttc gagcagggac tcgcggtgat tgtcgatgga ttggcgaaaa 960
   ggaggctcgt tgtcaggaac gttgaaggac cgagaaaggg tgacgattga tcaggaccgc 1020
   tgccggagcg caacccactc actacagcag agccatgtag acaacatccc ctcccccttt 1080
   ccaccgcgtc agacgcccgt agcagcccgc tacgggcttt ttcatgccct gccctagcgt 1140
   ccaagcctca cggccgcgct cggcctctct ggcggccttc tggcgctctt ccgcttcctc 1200
   gctcactgac tcgctgcgct cggtcgttcg gctgcggcga gcggtatcag ctcactcaaa 1260
   ggcggtaata cggttatcca cagaatcagg ggataacgca ggaaagaaca tgtgagcaaa 1320
   aggccagcaa aaggccagga accgtaaaaa ggccgcgttg ctggcgtttt tccataggct 1380
   ccgcccccct gacgagcatc acaaaaatcg acgctcaagt cagaggtggc gaaacccgac 1440
   aggactataa agataccagg cgtttccccc tggaagctcc ctcgtgcgct ctcctgttcc 1500
   gaccctgccg cttaccggat acctgtccgc ctttctccct tcgggaagcg tggcgctttt 1560
   ccgctgcata accctgcttc ggggtcatta tagcgatttt ttcggtatat ccatcctttt 1620
   tcgcacgata tacaggattt tgccaaaggg ttcgtgtaga ctttccttgg tgtatccaac 1680
   ggcgtcagcc gggcaggata ggtgaagtag gcccacccgc gagcgggtgt tccttcttca 1740
   ctgtccctta ttcgcacctg gcggtgctca acgggaatcc tgctctgcga ggctggccgg 1800
   ctaccgccgg cgtaacagat gagggcaagc ggatggctga tgaaaccaag ccaaccagga 1860
   agggcagccc acctatcaag gtgtactgcc ttccagacga acgaagagcg attgaggaaa 1920
   aggcggcggc ggccggcatg agcctgtcgg cctacctgct ggccgtcggc cagggctaca 1980
   aaatcacggg cgtcgtggac tatgagcacg tccgcgagct ggcccgcatc aatggcgacc 2040
   tgggccgcct gggcggcctg ctgaaactct ggctcaccga cgacccgcgc acggcgcggt 2100
   tcggtgatgc cacgatcctc gccctgctgg cgaagatcga agagaagcag gacgagcttg 2160
   gcaaggtcat gatgggcgtg gtccgcccga gggcagagcc atgacttttt tagccgctaa 2220
   aacggccggg gggtgcgcgt gattgccaag cacgtcccca tgcgctccat caagaagagc 2280
   gacttcgcgg agctggtgaa gtacatcacc gacgagcaag gcaagaccga gcgcctttgc 2340
   gacgctcacc gggctggttg ccctcgccgc tgggctggcg gccgtctatg gccctgcaaa 2400
   cgcgccagaa acgccgtcga agccgtgtgc gagacaccgc ggccgccggc gttgtggata 2460
   cctcgcggaa aacttggccc tcactgacag atgaggggcg gacgttgaca cttgaggggc 2520
   cgactcaccc ggcgcggcgt tgacagatga ggggcaggct cgatttcggc cggcgacgtg 2580
   gagctggcca gcctcgcaaa tcggcgaaaa cgcctgattt tacgcgagtt tcccacagat 2640
   gatgtggaca agcctgggga taagtgccct gcggtattga cacttgaggg gcgcgactac 2700
   tgacagatga ggggcgcgat ccttgacact tgaggggcag agtgctgaca gatgaggggc 2760
   gcacctattg acatttgagg ggctgtccac aggcagaaaa tccagcattt gcaagggttt 2820
   ccgcccgttt ttcggccacc gctaacctgt cttttaacct gcttttaaac caatatttat 2880
   aaaccttgtt tttaaccagg gctgcgccct gtgcgcgtga ccgcgcacgc cgaagggggg 2940
   tgccccccct tctcgaaccc tcccggcccg ctaacgcggg cctcccatcc ccccaggggc 3000
   tgcgcccctc ggccgcgaac ggcctcaccc caaaaatggc agcgctggca gtccttgcca 3060
   ttgccgggat cggggcagta acgggatggg cgatcagccc gagcgcgacg cccggaagca 3120
   ttgacgtgcc gcaggtgctg gcatcgacat tcagcgacca ggtgccgggc agtgagggcg 3180
   gcggcctggg tggcggcctg cccttcactt cggccgtcgg ggcattcacg gacttcatgg 3240
   cggggccggc aatttttacc ttgggcattc ttggcatagt ggtcgcgggt gccgtgctcg 3300
   tgttcggggg tgcgataaac ccagcgaacc atttgaggtg ataggtaaga ttataccgag 3360
   gtatgaaaac gagaattgga cctttacaga attactctat gaagcgccat atttaaaaag 3420
   ctaccaagac gaagaggatg aagaggatga ggaggcagat tgccttgaat atattgacaa 3480
   tactgataag ataatatatc ttttatatag aagatatcgc cgtatgtaag gatttcaggg 3540
   ggcaaggcat aggcagcgcg cttatcaata tatctataga atgggcaaag cataaaaact 3600
   tgcatggact aatgcttgaa acccaggaca ataaccttat agcttgtaaa ttctatcata 3660
   attgggtaat gactccaact tattgatagt gttttatgtt cagataatgc ccgatgactt 3720
   tgtcatgcag ctccaccgat tttgagaacg acagcgactt ccgtcccagc cgtgccaggt 3780
   gctgcctcag attcaggtta tgccgctcaa ttcgctgcgt atatcgcttg ctgattacgt 3840
   gcagctttcc cttcaggcgg gattcataca gcggccagcc atccgtcatc catatcacca 3900
   cgtcaaaggg tgacagcagg ctcataagac gccccagcgt cgccatagtg cgttcaccga 3960
   atacgtgcgc aacaaccgtc ttccggagac tgtcatacgc gtaaaacagc cagcgctggc 4020
   gcgatttagc cccgacatag ccccactgtt cgtccatttc cgcgcagacg atgacgtcac 4080
   tgcccggctg tatgcgcgag gttaccgact gcggcctgag ttttttaagt gacgtaaaat 4140
   cgtgttgagg ccaacgccca taatgcgggc tgttgcccgg catccaacgc cattcatggc 4200
   catatcaatg attttctggt gcgtaccggg ttgagaagcg gtgtaagtga actgcagttg 4260
   ccatgtttta cggcagtgag agcagagata gcgctgatgt ccggcggtgc ttttgccgtt 4320
   acgcaccacc ccgtcagtag ctgaacagga gggacagctg atagacacag aagccactgg 4380
   agcacctcaa aaacaccatc atacactaaa tcagtaagtt ggcagcatca cccataattg 4440
   tggtttcaaa atcggctccg tcgatactat gttatacgcc aactttgaaa acaactttga 4500
   aaaagctgtt ttctggtatt taaggtttta gaatgcaagg aacagtgaat tggagttcgt 4560
   cttgttataa ttagcttctt ggggtatctt taaatactgt agaaaagagg aaggaaataa 4620
   taaatggcta aaatgagaat atcaccggaa ttgaaaaaac tgatcgaaaa ataccgctgc 4680
   gtaaaagata cggaaggaat gtctcctgct aaggtatata agctggtggg agaaaatgaa 4740
   aacctatatt taaaaatgac ggacagccgg tataaaggga ccacctatga tgtggaacgg 4800
   gaaaaggaca tgatgctatg gctggaagga aagctgcctg ttccaaaggt cctgcacttt 4860
   gaacggcatg atggctggag caatctgctc atgagtgagg ccgatggcgt cctttgctcg 4920
   gaagagtatg aagatgaaca aagccctgaa aagattatcg agctgtatgc ggagtgcatc 4980
   aggctctttc actccatcga catatcggat tgtccctata cgaatagctt agacagccgc 5040
   ttagccgaat tggattactt actgaataac gatctggccg atgtggattg cgaaaactgg 5100
   gaagaagaca ctccatttaa agatccgcgc gagctgtatg attttttaaa gacggaaaag 5160
   cccgaagagg aacttgtctt ttcccacggc gacctgggag acagcaacat ctttgtgaaa 5220
   gatggcaaag taagtggctt tattgatctt gggagaagcg gcagggcgga caagtggtat 5280
   gacattgcct tctgcgtccg gtcgatcagg gaggatatcg gggaagaaca gtatgtcgag 5340
   ctattttttg acttactggg gatcaagcct gattgggaga aaataaaata ttatatttta 5400
   ctggatgaat tgttttagta cctagatgtg gcgcaacgat gccggcgaca agcaggagcg 5460
   caccgacttc ttccgcatca agtgttttgg ctctcaggcc gaggcccacg gcaagtattt 5520
   gggcaagggg tcgctggtat tcgtgcaggg caagattcgg aataccaagt acgagaagga 5580
   cggccagacg gtctacggga ccgacttcat tgccgataag gtggattatc tggacaccaa 5640
   ggcaccaggc gggtcaaatc aggaataagg gcacattgcc ccggcgtgag tcggggcaat 5700
   cccgcaagga gggtgaatga atcggacgtt tgaccggaag gcatacaggc aagaactgat 5760
   cgacgcgggg ttttccgccg aggatgccga aaccatcgca agccgcaccg tcatgcgtgc 5820
   gccccgcgaa accttccagt ccgtcggctc gatggtccag caagctacgg ccaagatcga 5880
   gcgcgacagc gtgcaactgg ctccccctgc cctgcccgcg ccatcggccg ccgtggagcg 5940
   ttcgcgtcgt ctcgaacagg aggcggcagg tttggcgaag tcgatgacca tcgacacgcg 6000
   aggaactatg acgaccaaga agcgaaaaac cgccggcgag gacctggcaa aacaggtcag 6060
   cgaggccaag caggccgcgt tgctgaaaca cacgaagcag cagatcaagg aaatgcagct 6120
   ttccttgttc gatattgcgc cgtggccgga cacgatgcga gcgatgccaa acgacacggc 6180
   ccgctctgcc ctgttcacca cgcgcaacaa gaaaatcccg cgcgaggcgc tgcaaaacaa 6240
   ggtcattttc cacgtcaaca aggacgtgaa gatcacctac accggcgtcg agctgcgggc 6300
   cgacgatgac gaactggtgt ggcagcaggt gttggagtac gcgaagcgca cccctatcgg 6360
   cgagccgatc accttcacgt tctacgagct ttgccaggac ctgggctggt cgatcaatgg 6420
   ccggtattac acgaaggccg aggaatgcct gtcgcgccta caggcgacgg cgatgggctt 6480
   cacgtccgac cgcgttgggc acctggaatc ggtgtcgctg ctgcaccgct tccgcgtcct 6540
   ggaccgtggc aagaaaacgt cccgttgcca ggtcctgatc gacgaggaaa tcgtcgtgct 6600
   gtttgctggc gaccactaca cgaaattcat atgggagaag taccgcaagc tgtcgccgac 6660
   ggcccgacgg atgttcgact atttcagctc gcaccgggag ccgtacccgc tcaagctgga 6720
   aaccttccgc ctcatgtgcg gatcggattc cacccgcgtg aagaagtggc gcgagcaggt 6780
   cggcgaagcc tgcgaagagt tgcgaggcag cggcctggtg gaacacgcct gggtcaatga 6840
   tgacctggtg cattgcaaac gctagggcct tgtggggtca gttccggctg ggggttcagc 6900
   agccagcgct ttactggcat ttcaggaaca agcgggcact gctcgacgca cttgcttcgc 6960
   tcagtatcgc tcgggacgca cggcgcgctc tacgaactgc cgataaacag aggattaaaa 7020
   ttgacaattg tgattaaggc tcagattcga cggcttggag cggccgacgt gcaggatttc 7080
   cgcgagatcc gattgtcggc cctgaagaaa gctccagaga tgttcgggtc cgtttacgag 7140
   cacgaggaga aaaagcccat ggaggcgttc gctgaacggt tgcgagatgc cgtggcattc 7200
   ggcgcctaca tcgacggcga gatcattggg ctgtcggtct tcaaacagga ggacggcccc 7260
   aaggacgctc acaaggcgca tctgtccggc gttttcgtgg agcccgaaca gcgaggccga 7320
   ggggtcgccg gtatgctgct gcgggcgttg ccggcgggtt tattgctcgt gatgatcgtc 7380
   cgacagattc caacgggaat ctggtggatg cgcatcttca tcctcggcgc acttaatatt 7440
   tcgctattct ggagcttgtt gtttatttcg gtctaccgcc tgccgggcgg ggtcgcggcg 7500
   acggtaggcg ctgtgcagcc gctgatggtc gtgttcatct ctgccgctct gctaggtagc 7560
   ccgatacgat tgatggcggt cctgggggct atttgcggaa ctgcgggcgt ggcgctgttg 7620
   gtgttgacac caaacgcagc gctagatcct gtcggcgtcg cagcgggcct ggcgggggcg 7680
   gtttccatgg cgttcggaac cgtgctgacc cgcaagtggc aacctcccgt gcctctgctc 7740
   acctttaccg cctggcaact ggcggccgga ggacttctgc tcgttccagt agctttagtg 7800
   tttgatccgc caatcccgat gcctacagga accaatgttc tcggcctggc gtggctcggc 7860
   ctgatcggag cgggtttaac ctacttcctt tggttccggg ggatctcgcg actcgaacct 7920
   acagttgttt ccttactggg ctttctcagc cccagatctg gggtcgatca gccggggatg 7980
   catcaggccg acagtcggaa cttcgggtcc ccgacctgta ccattcggtg agcaatggat 8040
   aggggagttg atatcgtcaa cgttcacttc taaagaaata gcgccactca gcttcctcag 8100
   cggctttatc cagcgatttc ctattatgtc ggcatagttc tcaagatcga cagcctgtca 8160
   cggttaagcg agaaatgaat aagaaggctg ataattcgga tctctgcgag ggagatgata 8220
   tttgatcaca ggcagcaacg ctctgtcatc gttacaatca acatgctacc ctccgcgaga 8280
   tcatccgtgt ttcaaacccg gcagcttagt tgccgttctt ccgaatagca tcggtaacat 8340
   gagcaaagtc tgccgcctta caacggctct cccgctgacg ccgtcccgga ctgatgggct 8400
   gcctgtatcg agtggtgatt ttgtgccgag ctgccggtcg gggagctgtt ggctggctgg 8460
   tggcaggata tattgtggtg taaacaaatt gacgcttaga caacttaata acacattgcg 8520
   gacgttttta atgtactggg gtggtttttc ttttcaccag tgagacgggc aacagctgat 8580
   tgcccttcac cgcctggccc tgagagagtt gcagcaagcg gtccacgctg gtttgcccca 8640
   gcaggcgaaa atcctgtttg atggtggttc cgaaatcggc aaaatccctt ataaatcaaa 8700
   agaatagccc gagatagggt tgagtgttgt tccagtttgg aacaagagtc cactattaaa 8760
   gaacgtggac tccaacgtca aagggcgaaa aaccgtctat cagggcgatg gcccactacg 8820
   tgaaccatca cccaaatcaa gttttttggg gtcgaggtgc cgtaaagcac taaatcggaa 8880
   ccctaaaggg agcccccgat ttagagcttg acggggaaag ccggcgaacg tggcgagaaa 8940
   ggaagggaag aaagcgaaag gagcgggcgc cattcaggct gcgcaactgt tgggaagggc 9000
   gatcggtgcg ggcctcttcg ctattacgcc agctggcgaa agggggatgt gctgcaaggc 9060
   gattaagttg ggtaacgcca gggttttccc agtcacgacg ttgtaaaacg acggccagtg 9120
   aattaattcc catcttgaaa gaaatatagt ttaaatattt attgataaaa taacaagtca 9180
   ggtattatag tccaagcaaa aacataaatt tattgatgca agtttaaatt cagaaatatt 9240
   tcaataactg attatatcag ctggtacatt gccgtagatg aaagactgag tgcgatatta 9300
   tgtgtaatac ataaattgat gatatagcta gcttagctca tcgggggatc cgtcgaagct 9360
   agcttgggtc ccgctcagaa gaactcgtca agaaggcgat agaaggcgat gcgctgcgaa 9420
   tcgggagcgg cgataccgta aagcacgagg aagcggtcag cccattcgcc gccaagctct 9480
   tcagcaatat cacgggtagc caacgctatg tcctgatagc ggtccgccac acccagccgg 9540
   ccacagtcga tgaatccaga aaagcggcca ttttccacca tgatattcgg caagcaggca 9600
   tcgccatggg tcacgacgag atcctcgccg tcgggcatgc gcgccttgag cctggcgaac 9660
   agttcggctg gcgcgagccc ctgatgctct tcgtccagat catcctgatc gacaagaccg 9720
   gcttccatcc gagtacgtgc tcgctcgatg cgatgtttcg cttggtggtc gaatgggcag 9780
   gtagccggat caagcgtatg cagccgccgc attgcatcag ccatgatgga tactttctcg 9840
   gcaggagcaa ggtgagatga caggagatcc tgccccggca cttcgcccaa tagcagccag 9900
   tcccttcccg cttcagtgac aacgtcgagc acagctgcgc aaggaacgcc cgtcgtggcc 9960
   agccacgata gccgcgctgc ctcgtcctgc agttcattca gggcaccgga caggtcggtc 10020
   ttgacaaaaa gaaccgggcg cccctgcgct gacagccgga acacggcggc atcagagcag 10080
   ccgattgtct gttgtgccca gtcatagccg aatagcctct ccacccaagc ggccggagaa 10140
   cctgcgtgca atccatcttg ttcaatccaa gctcccatgg gccctcgact agagtcgaga 10200
   tctggattga gagtgaatat gagactctaa ttggataccg aggggaattt atggaacgtc 10260
   agtggagcat ttttgacaag aaatatttgc tagctgatag tgaccttagg cgacttttga 10320
   acgcgcaata atggtttctg acgtatgtgc ttagctcatt aaactccaga aacccgcggc 10380
   tgagtggctc cttcaacgtt gcggttctgt cagttccaaa cgtaaaacgg cttgtcccgc 10440
   gtcatcggcg ggggtcataa cgtgactccc ttaattctcc gctcatgatc ttgatcccct 10500
   gcgccatcag atccttggcg gcaagaaagc catccagttt actttgcagg gcttcccaac 10560
   cttaccagag ggcgccccag ctggcaattc cggttcgctt gctgtccata aaaccgccca 10620
   gtctagctat cgccatgtaa gcccactgca agctacctgc tttctctttg cgcttgcgtt 10680
   ttcccttgtc cagatagccc agtagctgac attcatccgg ggtcagcacc gtttctgcgg 10740
   actggctttc tacgtgttcc gcttccttta gcagcccttg cgccctgagt gcttgcggca 10800
   gcgtgaagct tgcatgcctg caggtcgacg gcgcgccgag ctcctcgagc aaatttacac 10860
   attgccacta aacgtctaaa cccttgtaat ttgtttttgt tttactatgt gtgttatgta 10920
   tttgatttgc gataaatttt tatatttggt actaaattta taacaccttt tatgctaacg 10980
   tttgccaaca cttagcaatt tgcaagttga ttaattgatt ctaaattatt tttgtcttct 11040
   aaatacatat actaatcaac tggaaatgta aatatttgct aatatttcta ctataggaga 11100
   attaaagtga gtgaatatgg taccacaagg tttggagatt taattgttgc aatgctgcat 11160
   ggatggcata tacaccaaac attcaataat tcttgaggat aataatggta ccacacaaga 11220
   tttgaggtgc atgaacgtca cgtggacaaa aggtttagta atttttcaag acaacaatgt 11280
   taccacacac aagttttgag gtgcatgcat ggatgccctg tggaaagttt aaaaatattt 11340
   tggaaatgat ttgcatggaa gccatgtgta aaaccatgac atccacttgg aggatgcaat 11400
   aatgaagaaa actacaaatt tacatgcaac tagttatgca tgtagtctat ataatgagga 11460
   ttttgcaata ctttcattca tacacactca ctaagtttta cacgattata atttcttcat 11520
   agccagccca ccgcggtggg cggccgcctg cagtctagaa ggcctcctgc tttaatgaga 11580
   tatgcgagac gcctatgatc gcatgatatt tgctttcaat tctgttgtgc acgttgtaaa 11640
   aaacctgagc atgtgtagct cagatcctta ccgccggttt cggttcattc taatgaatat 11700
   atcacccgtt actatcgtat ttttatgaat aatattctcc gttcaattta ctgattgtcc 11760
   gtcgagcaaa tttacacatt gccactaaac gtctaaaccc ttgtaatttg tttttgtttt 11820
   actatgtgtg ttatgtattt gatttgcgat aaatttttat atttggtact aaatttataa 11880
   caccttttat gctaacgttt gccaacactt agcaatttgc aagttgatta attgattcta 11940
   aattattttt gtcttctaaa tacatatact aatcaactgg aaatgtaaat atttgctaat 12000
   atttctacta taggagaatt aaagtgagtg aatatggtac cacaaggttt ggagatttaa 12060
   ttgttgcaat gctgcatgga tggcatatac accaaacatt caataattct tgaggataat 12120
   aatggtacca cacaagattt gaggtgcatg aacgtcacgt ggacaaaagg tttagtaatt 12180
   tttcaagaca acaatgttac cacacacaag ttttgaggtg catgcatgga tgccctgtgg 12240
   aaagtttaaa aatattttgg aaatgatttg catggaagcc atgtgtaaaa ccatgacatc 12300
   cacttggagg atgcaataat gaagaaaact acaaatttac atgcaactag ttatgcatgt 12360
   agtctatata atgaggattt tgcaatactt tcattcatac acactcacta agttttacac 12420
   gattataatt tcttcatagc cagcggatcc gatatcgggc ccgctagcgt taaccctgct 12480
   ttaatgagat atgcgagacg cctatgatcg catgatattt gctttcaatt ctgttgtgca 12540
   cgttgtaaaa aacctgagca tgtgtagctc agatccttac cgccggtttc ggttcattct 12600
   aatgaatata tcacccgtta ctatcgtatt tttatgaata atattctccg ttcaatttac 12660
   tgattgtccg tcgacgaatt cgagctcggc gcgcctctag aggatcgatg aattcagatc 12720
   ggctgagtgg ctccttcaac gttgcggttc tgtcagttcc aaacgtaaaa cggcttgtcc 12780
   cgcgtcatcg gcgggggtca taacgtgact cccttaattc tccgctcatg atcagattgt 12840
   cgtttcccgc cttcagttta aactatcagt gtttgacagg atatattggc gggtaaacct 12900
   aagagaaaag agcgtttatt agaataatcg gatatttaaa agggcgtgaa aaggtttatc 12960
   cttcgtccat ttgtatgtgc atgccaacca cagggttccc ca 13002
<210> 24
   <211> 13905
   <212> DNA
   <213> Unknown
<220>
   <223> pflanzlicher Expressionsvektor mit drei Promotor-Terminator-Expressionskassetten
<400> 24
   gatctggcgc cggccagcga gacgagcaag attggccgcc gcccgaaacg atccgacagc 60
   gcgcccagca caggtgcgca ggcaaattgc accaacgcat acagcgccag cagaatgcca 120
   tagtgggcgg tgacgtcgtt cgagtgaacc agatcgcgca ggaggcccgg cagcaccggc 180
   ataatcaggc cgatgccgac agcgtcgagc gcgacagtgc tcagaattac gatcaggggt 240
   atgttgggtt tcacgtctgg cctccggacc agcctccgct ggtccgattg aacgcgcgga 300
   ttctttatca ctgataagtt ggtggacata ttatgtttat cagtgataaa gtgtcaagca 360
   tgacaaagtt gcagccgaat acagtgatcc gtgccgccct ggacctgttg aacgaggtcg 420
   gcgtagacgg tctgacgaca cgcaaactgg cggaacggtt gggggttcag cagccggcgc 480
   tttactggca cttcaggaac aagcgggcgc tgctcgacgc actggccgaa gccatgctgg 540
   cggagaatca tacgcattcg gtgccgagag ccgacgacga ctggcgctca tttctgatcg 600
   ggaatgcccg cagcttcagg caggcgctgc tcgcctaccg cgatggcgcg cgcatccatg 660
   ccggcacgcg accgggcgca ccgcagatgg aaacggccga cgcgcagctt cgcttcctct 720
   gcgaggcggg tttttcggcc ggggacgccg tcaatgcgct gatgacaatc agctacttca 780
   ctgttggggc cgtgcttgag gagcaggccg gcgacagcga tgccggcgag cgcggcggca 840
   ccgttgaaca ggctccgctc tcgccgctgt tgcgggccgc gatagacgcc ttcgacgaag 900
   ccggtccgga cgcagcgttc gagcagggac tcgcggtgat tgtcgatgga ttggcgaaaa 960
   ggaggctcgt tgtcaggaac gttgaaggac cgagaaaggg tgacgattga tcaggaccgc 1020
   tgccggagcg caacccactc actacagcag agccatgtag acaacatccc ctcccccttt 1080
   ccaccgcgtc agacgcccgt agcagcccgc tacgggcttt ttcatgccct gccctagcgt 1140
   ccaagcctca cggccgcgct cggcctctct ggcggccttc tggcgctctt ccgcttcctc 1200
   gctcactgac tcgctgcgct cggtcgttcg gctgcggcga gcggtatcag ctcactcaaa 1260
   ggcggtaata cggttatcca cagaatcagg ggataacgca ggaaagaaca tgtgagcaaa 1320
   aggccagcaa aaggccagga accgtaaaaa ggccgcgttg ctggcgtttt tccataggct 1380
   ccgcccccct gacgagcatc acaaaaatcg acgctcaagt cagaggtggc gaaacccgac 1440
   aggactataa agataccagg cgtttccccc tggaagctcc ctcgtgcgct ctcctgttcc 1500
   gaccctgccg cttaccggat acctgtccgc ctttctccct tcgggaagcg tggcgctttt 1560
   ccgctgcata accctgcttc ggggtcatta tagcgatttt ttcggtatat ccatcctttt 1620
   tcgcacgata tacaggattt tgccaaaggg ttcgtgtaga ctttccttgg tgtatccaac 1680
   ggcgtcagcc gggcaggata ggtgaagtag gcccacccgc gagcgggtgt tccttcttca 1740
   ctgtccctta ttcgcacctg gcggtgctca acgggaatcc tgctctgcga ggctggccgg 1800
   ctaccgccgg cgtaacagat gagggcaagc ggatggctga tgaaaccaag ccaaccagga 1860
   agggcagccc acctatcaag gtgtactgcc ttccagacga acgaagagcg attgaggaaa 1920
   aggcggcggc ggccggcatg agcctgtcgg cctacctgct ggccgtcggc cagggctaca 1980
   aaatcacggg cgtcgtggac tatgagcacg tccgcgagct ggcccgcatc aatggcgacc 2040
   tgggccgcct gggcggcctg ctgaaactct ggctcaccga cgacccgcgc acggcgcggt 2100
   tcggtgatgc cacgatcctc gccctgctgg cgaagatcga agagaagcag gacgagcttg 2160
   gcaaggtcat gatgggcgtg gtccgcccga gggcagagcc atgacttttt tagccgctaa 2220
   aacggccggg gggtgcgcgt gattgccaag cacgtcccca tgcgctccat caagaagagc 2280
   gacttcgcgg agctggtgaa gtacatcacc gacgagcaag gcaagaccga gcgcctttgc 2340
   gacgctcacc gggctggttg ccctcgccgc tgggctggcg gccgtctatg gccctgcaaa 2400
   cgcgccagaa acgccgtcga agccgtgtgc gagacaccgc ggccgccggc gttgtggata 2460
   cctcgcggaa aacttggccc tcactgacag atgaggggcg gacgttgaca cttgaggggc 2520
   cgactcaccc ggcgcggcgt tgacagatga ggggcaggct cgatttcggc cggcgacgtg 2580
   gagctggcca gcctcgcaaa tcggcgaaaa cgcctgattt tacgcgagtt tcccacagat 2640
   gatgtggaca agcctgggga taagtgccct gcggtattga cacttgaggg gcgcgactac 2700
   tgacagatga ggggcgcgat ccttgacact tgaggggcag agtgctgaca gatgaggggc 2760
   gcacctattg acatttgagg ggctgtccac aggcagaaaa tccagcattt gcaagggttt 2820
   ccgcccgttt ttcggccacc gctaacctgt cttttaacct gcttttaaac caatatttat 2880
   aaaccttgtt tttaaccagg gctgcgccct gtgcgcgtga ccgcgcacgc cgaagggggg 2940
   tgccccccct tctcgaaccc tcccggcccg ctaacgcggg cctcccatcc ccccaggggc 3000
   tgcgcccctc ggccgcgaac ggcctcaccc caaaaatggc agcgctggca gtccttgcca 3060
   ttgccgggat cggggcagta acgggatggg cgatcagccc gagcgcgacg cccggaagca 3120
   ttgacgtgcc gcaggtgctg gcatcgacat tcagcgacca ggtgccgggc agtgagggcg 3180
   gcggcctggg tggcggcctg cccttcactt cggccgtcgg ggcattcacg gacttcatgg 3240
   cggggccggc aatttttacc ttgggcattc ttggcatagt ggtcgcgggt gccgtgctcg 3300
   tgttcggggg tgcgataaac ccagcgaacc atttgaggtg ataggtaaga ttataccgag 3360
   gtatgaaaac gagaattgga cctttacaga attactctat gaagcgccat atttaaaaag 3420
   ctaccaagac gaagaggatg aagaggatga ggaggcagat tgccttgaat atattgacaa 3480
   tactgataag ataatatatc ttttatatag aagatatcgc cgtatgtaag gatttcaggg 3540
   ggcaaggcat aggcagcgcg cttatcaata tatctataga atgggcaaag cataaaaact 3600
   tgcatggact aatgcttgaa acccaggaca ataaccttat agcttgtaaa ttctatcata 3660
   attgggtaat gactccaact tattgatagt gttttatgtt cagataatgc ccgatgactt 3720
   tgtcatgcag ctccaccgat tttgagaacg acagcgactt ccgtcccagc cgtgccaggt 3780
   gctgcctcag attcaggtta tgccgctcaa ttcgctgcgt atatcgcttg ctgattacgt 3840
   gcagctttcc cttcaggcgg gattcataca gcggccagcc atccgtcatc catatcacca 3900
   cgtcaaaggg tgacagcagg ctcataagac gccccagcgt cgccatagtg cgttcaccga 3960
   atacgtgcgc aacaaccgtc ttccggagac tgtcatacgc gtaaaacagc cagcgctggc 4020
   gcgatttagc cccgacatag ccccactgtt cgtccatttc cgcgcagacg atgacgtcac 4080
   tgcccggctg tatgcgcgag gttaccgact gcggcctgag ttttttaagt gacgtaaaat 4140
   cgtgttgagg ccaacgccca taatgcgggc tgttgcccgg catccaacgc cattcatggc 4200
   catatcaatg attttctggt gcgtaccggg ttgagaagcg gtgtaagtga actgcagttg 4260
   ccatgtttta cggcagtgag agcagagata gcgctgatgt ccggcggtgc ttttgccgtt 4320
   acgcaccacc ccgtcagtag ctgaacagga gggacagctg atagacacag aagccactgg 4380
   agcacctcaa aaacaccatc atacactaaa tcagtaagtt ggcagcatca cccataattg 4440
   tggtttcaaa atcggctccg tcgatactat gttatacgcc aactttgaaa acaactttga 4500
   aaaagctgtt ttctggtatt taaggtttta gaatgcaagg aacagtgaat tggagttcgt 4560
   cttgttataa ttagcttctt ggggtatctt taaatactgt agaaaagagg aaggaaataa 4620
   taaatggcta aaatgagaat atcaccggaa ttgaaaaaac tgatcgaaaa ataccgctgc 4680
   gtaaaagata cggaaggaat gtctcctgct aaggtatata agctggtggg agaaaatgaa 4740
   aacctatatt taaaaatgac ggacagccgg tataaaggga ccacctatga tgtggaacgg 4800
   gaaaaggaca tgatgctatg gctggaagga aagctgcctg ttccaaaggt cctgcacttt 4860
   gaacggcatg atggctggag caatctgctc atgagtgagg ccgatggcgt cctttgctcg 4920
   gaagagtatg aagatgaaca aagccctgaa aagattatcg agctgtatgc ggagtgcatc 4980
   aggctctttc actccatcga catatcggat tgtccctata cgaatagctt agacagccgc 5040
   ttagccgaat tggattactt actgaataac gatctggccg atgtggattg cgaaaactgg 5100
   gaagaagaca ctccatttaa agatccgcgc gagctgtatg attttttaaa gacggaaaag 5160
   cccgaagagg aacttgtctt ttcccacggc gacctgggag acagcaacat ctttgtgaaa 5220
   gatggcaaag taagtggctt tattgatctt gggagaagcg gcagggcgga caagtggtat 5280
   gacattgcct tctgcgtccg gtcgatcagg gaggatatcg gggaagaaca gtatgtcgag 5340
   ctattttttg acttactggg gatcaagcct gattgggaga aaataaaata ttatatttta 5400
   ctggatgaat tgttttagta cctagatgtg gcgcaacgat gccggcgaca agcaggagcg 5460
   caccgacttc ttccgcatca agtgttttgg ctctcaggcc gaggcccacg gcaagtattt 5520
   gggcaagggg tcgctggtat tcgtgcaggg caagattcgg aataccaagt acgagaagga 5580
   cggccagacg gtctacggga ccgacttcat tgccgataag gtggattatc tggacaccaa 5640
   ggcaccaggc gggtcaaatc aggaataagg gcacattgcc ccggcgtgag tcggggcaat 5700
   cccgcaagga gggtgaatga atcggacgtt tgaccggaag gcatacaggc aagaactgat 5760
   cgacgcgggg ttttccgccg aggatgccga aaccatcgca agccgcaccg tcatgcgtgc 5820
   gccccgcgaa accttccagt ccgtcggctc gatggtccag caagctacgg ccaagatcga 5880
   gcgcgacagc gtgcaactgg ctccccctgc cctgcccgcg ccatcggccg ccgtggagcg 5940
   ttcgcgtcgt ctcgaacagg aggcggcagg tttggcgaag tcgatgacca tcgacacgcg 6000
   aggaactatg acgaccaaga agcgaaaaac cgccggcgag gacctggcaa aacaggtcag 6060
   cgaggccaag caggccgcgt tgctgaaaca cacgaagcag cagatcaagg aaatgcagct 6120
   ttccttgttc gatattgcgc cgtggccgga cacgatgcga gcgatgccaa acgacacggc 6180
   ccgctctgcc ctgttcacca cgcgcaacaa gaaaatcccg cgcgaggcgc tgcaaaacaa 6240
   ggtcattttc cacgtcaaca aggacgtgaa gatcacctac accggcgtcg agctgcgggc 6300
   cgacgatgac gaactggtgt ggcagcaggt gttggagtac gcgaagcgca cccctatcgg 6360
   cgagccgatc accttcacgt tctacgagct ttgccaggac ctgggctggt cgatcaatgg 6420
   ccggtattac acgaaggccg aggaatgcct gtcgcgccta caggcgacgg cgatgggctt 6480
   cacgtccgac cgcgttgggc acctggaatc ggtgtcgctg ctgcaccgct tccgcgtcct 6540
   ggaccgtggc aagaaaacgt cccgttgcca ggtcctgatc gacgaggaaa tcgtcgtgct 6600
   gtttgctggc gaccactaca cgaaattcat atgggagaag taccgcaagc tgtcgccgac 6660
   ggcccgacgg atgttcgact atttcagctc gcaccgggag ccgtacccgc tcaagctgga 6720
   aaccttccgc ctcatgtgcg gatcggattc cacccgcgtg aagaagtggc gcgagcaggt 6780
   cggcgaagcc tgcgaagagt tgcgaggcag cggcctggtg gaacacgcct gggtcaatga 6840
   tgacctggtg cattgcaaac gctagggcct tgtggggtca gttccggctg ggggttcagc 6900
   agccagcgct ttactggcat ttcaggaaca agcgggcact gctcgacgca cttgcttcgc 6960
   tcagtatcgc tcgggacgca cggcgcgctc tacgaactgc cgataaacag aggattaaaa 7020
   ttgacaattg tgattaaggc tcagattcga cggcttggag cggccgacgt gcaggatttc 7080
   cgcgagatcc gattgtcggc cctgaagaaa gctccagaga tgttcgggtc cgtttacgag 7140
   cacgaggaga aaaagcccat ggaggcgttc gctgaacggt tgcgagatgc cgtggcattc 7200
   ggcgcctaca tcgacggcga gatcattggg ctgtcggtct tcaaacagga ggacggcccc 7260
   aaggacgctc acaaggcgca tctgtccggc gttttcgtgg agcccgaaca gcgaggccga 7320
   ggggtcgccg gtatgctgct gcgggcgttg ccggcgggtt tattgctcgt gatgatcgtc 7380
   cgacagattc caacgggaat ctggtggatg cgcatcttca tcctcggcgc acttaatatt 7440
   tcgctattct ggagcttgtt gtttatttcg gtctaccgcc tgccgggcgg ggtcgcggcg 7500
   acggtaggcg ctgtgcagcc gctgatggtc gtgttcatct ctgccgctct gctaggtagc 7560
   ccgatacgat tgatggcggt cctgggggct atttgcggaa ctgcgggcgt ggcgctgttg 7620
   gtgttgacac caaacgcagc gctagatcct gtcggcgtcg cagcgggcct ggcgggggcg 7680
   gtttccatgg cgttcggaac cgtgctgacc cgcaagtggc aacctcccgt gcctctgctc 7740
   acctttaccg cctggcaact ggcggccgga ggacttctgc tcgttccagt agctttagtg 7800
   tttgatccgc caatcccgat gcctacagga accaatgttc tcggcctggc gtggctcggc 7860
   ctgatcggag cgggtttaac ctacttcctt tggttccggg ggatctcgcg actcgaacct 7920
   acagttgttt ccttactggg ctttctcagc cccagatctg gggtcgatca gccggggatg 7980
   catcaggccg acagtcggaa cttcgggtcc ccgacctgta ccattcggtg agcaatggat 8040
   aggggagttg atatcgtcaa cgttcacttc taaagaaata gcgccactca gcttcctcag 8100
   cggctttatc cagcgatttc ctattatgtc ggcatagttc tcaagatcga cagcctgtca 8160
   cggttaagcg agaaatgaat aagaaggctg ataattcgga tctctgcgag ggagatgata 8220
   tttgatcaca ggcagcaacg ctctgtcatc gttacaatca acatgctacc ctccgcgaga 8280
   tcatccgtgt ttcaaacccg gcagcttagt tgccgttctt ccgaatagca tcggtaacat 8340
   gagcaaagtc tgccgcctta caacggctct cccgctgacg ccgtcccgga ctgatgggct 8400
   gcctgtatcg agtggtgatt ttgtgccgag ctgccggtcg gggagctgtt ggctggctgg 8460
   tggcaggata tattgtggtg taaacaaatt gacgcttaga caacttaata acacattgcg 8520
   gacgttttta atgtactggg gtggtttttc ttttcaccag tgagacgggc aacagctgat 8580
   tgcccttcac cgcctggccc tgagagagtt gcagcaagcg gtccacgctg gtttgcccca 8640
   gcaggcgaaa atcctgtttg atggtggttc cgaaatcggc aaaatccctt ataaatcaaa 8700
   agaatagccc gagatagggt tgagtgttgt tccagtttgg aacaagagtc cactattaaa 8760
   gaacgtggac tccaacgtca aagggcgaaa aaccgtctat cagggcgatg gcccactacg 8820
   tgaaccatca cccaaatcaa gtttttgggg gtcgaggtgc cgtaaagcac taaatcggaa 8880
   ccctaaaggg agcccccgat ttagagcttg acggggaaag ccggcgaacg tggcgagaaa 8940
   ggaagggaag aaagcgaaag gagcgggcgc cattcaggct gcgcaactgt tgggaagggc 9000
   gatcggtgcg ggcctcttcg ctattacgcc agctggcgaa agggggatgt gctgcaaggc 9060
   gattaagttg ggtaacgcca gggttttccc agtcacgacg ttgtaaaacg acggccagtg 9120
   aattaattcc catcttgaaa gaaatatagt ttaaatattt attgataaaa taacaagtca 9180
   ggtattatag tccaagcaaa aacataaatt tattgatgca agtttaaatt cagaaatatt 9240
   tcaataactg attatatcag ctggtacatt gccgtagatg aaagactgag tgcgatatta 9300
   tgtgtaatac ataaattgat gatatagcta gcttagctca tcgggggatc cgtcgaagct 9360
   agcttgggtc ccgctcagaa gaactcgtca agaaggcgat agaaggcgat gcgctgcgaa 9420
   tcgggagcgg cgataccgta aagcacgagg aagcggtcag cccattcgcc gccaagctct 9480
   tcagcaatat cacgggtagc caacgctatg tcctgatagc ggtccgccac acccagccgg 9540
   ccacagtcga tgaatccaga aaagcggcca ttttccacca tgatattcgg caagcaggca 9600
   tcgccatggg tcacgacgag atcctcgccg tcgggcatgc gcgccttgag cctggcgaac 9660
   agttcggctg gcgcgagccc ctgatgctct tcgtccagat catcctgatc gacaagaccg 9720
   gcttccatcc gagtacgtgc tcgctcgatg cgatgtttcg cttggtggtc gaatgggcag 9780
   gtagccggat caagcgtatg cagccgccgc attgcatcag ccatgatgga tactttctcg 9840
   gcaggagcaa ggtgagatga caggagatcc tgccccggca cttcgcccaa tagcagccag 9900
   tcccttcccg cttcagtgac aacgtcgagc acagctgcgc aaggaacgcc cgtcgtggcc 9960
   agccacgata gccgcgctgc ctcgtcctgc agttcattca gggcaccgga caggtcggtc 10020
   ttgacaaaaa gaaccgggcg cccctgcgct gacagccgga acacggcggc atcagagcag 10080
   ccgattgtct gttgtgccca gtcatagccg aatagcctct ccacccaagc ggccggagaa 10140
   cctgcgtgca atccatcttg ttcaatccaa gctcccatgg gccctcgact agagtcgaga 10200
   tctggattga gagtgaatat gagactctaa ttggataccg aggggaattt atggaacgtc 10260
   agtggagcat ttttgacaag aaatatttgc tagctgatag tgaccttagg cgacttttga 10320
   acgcgcaata atggtttctg acgtatgtgc ttagctcatt aaactccaga aacccgcggc 10380
   tgagtggctc cttcaacgtt gcggttctgt cagttccaaa cgtaaaacgg cttgtcccgc 10440
   gtcatcggcg ggggtcataa cgtgactccc ttaattctcc gctcatgatc ttgatcccct 10500
   gcgccatcag atccttggcg gcaagaaagc catccagttt actttgcagg gcttcccaac 10560
   cttaccagag ggcgccccag ctggcaattc cggttcgctt gctgtccata aaaccgccca 10620
   gtctagctat cgccatgtaa gcccactgca agctacctgc tttctctttg cgcttgcgtt 10680
   ttcccttgtc cagatagccc agtagctgac attcatccgg ggtcagcacc gtttctgcgg 10740
   actggctttc tacgtgttcc gcttccttta gcagcccttg cgccctgagt gcttgcggca 10800
   gcgtgaagct tgcatgcctg caggtcgacg gcgcgccgag ctcctcgagc aaatttacac 10860
   attgccacta aacgtctaaa cccttgtaat ttgtttttgt tttactatgt gtgttatgta 10920
   tttgatttgc gataaatttt tatatttggt actaaattta taacaccttt tatgctaacg 10980
   tttgccaaca cttagcaatt tgcaagttga ttaattgatt ctaaattatt tttgtcttct 11040
   aaatacatat actaatcaac tggaaatgta aatatttgct aatatttcta ctataggaga 11100
   attaaagtga gtgaatatgg taccacaagg tttggagatt taattgttgc aatgctgcat 11160
   ggatggcata tacaccaaac attcaataat tcttgaggat aataatggta ccacacaaga 11220
   tttgaggtgc atgaacgtca cgtggacaaa aggtttagta atttttcaag acaacaatgt 11280
   taccacacac aagttttgag gtgcatgcat ggatgccctg tggaaagttt aaaaatattt 11340
   tggaaatgat ttgcatggaa gccatgtgta aaaccatgac atccacttgg aggatgcaat 11400
   aatgaagaaa actacaaatt tacatgcaac tagttatgca tgtagtctat ataatgagga 11460
   ttttgcaata ctttcattca tacacactca ctaagtttta cacgattata atttcttcat 11520
   agccagccca ccgcggtggg cggccgcctg cagtctagaa ggcctcctgc tttaatgaga 11580
   tatgcgagac gcctatgatc gcatgatatt tgctttcaat tctgttgtgc acgttgtaaa 11640
   aaacctgagc atgtgtagct cagatcctta ccgccggttt cggttcattc taatgaatat 11700
   atcacccgtt actatcgtat ttttatgaat aatattctcc gttcaattta ctgattgtcc 11760
   gtcgagcaaa tttacacatt gccactaaac gtctaaaccc ttgtaatttg tttttgtttt 11820
   actatgtgtg ttatgtattt gatttgcgat aaatttttat atttggtact aaatttataa 11880
   caccttttat gctaacgttt gccaacactt agcaatttgc aagttgatta attgattcta 11940
   aattattttt gtcttctaaa tacatatact aatcaactgg aaatgtaaat atttgctaat 12000
   atttctacta taggagaatt aaagtgagtg aatatggtac cacaaggttt ggagatttaa 12060
   ttgttgcaat gctgcatgga tggcatatac accaaacatt caataattct tgaggataat 12120
   aatggtacca cacaagattt gaggtgcatg aacgtcacgt ggacaaaagg tttagtaatt 12180
   tttcaagaca acaatgttac cacacacaag ttttgaggtg catgcatgga tgccctgtgg 12240
   aaagtttaaa aatattttgg aaatgatttg catggaagcc atgtgtaaaa ccatgacatc 12300
   cacttggagg atgcaataat gaagaaaact acaaatttac atgcaactag ttatgcatgt 12360
   agtctatata atgaggattt tgcaatactt tcattcatac acactcacta agttttacac 12420
   gattataatt tcttcatagc cagcggatcc gatatcgggc ccgctagcgt taaccctgct 12480
   ttaatgagat atgcgagacg cctatgatcg catgatattt gctttcaatt ctgttgtgca 12540
   cgttgtaaaa aacctgagca tgtgtagctc agatccttac cgccggtttc ggttcattct 12600
   aatgaatata tcacccgtta ctatcgtatt tttatgaata atattctccg ttcaatttac 12660
   tgattgtccg tcgagcaaat ttacacattg ccactaaacg tctaaaccct tgtaatttgt 12720
   ttttgtttta ctatgtgtgt tatgtatttg atttgcgata aatttttata tttggtacta 12780
   aatttataac accttttatg ctaacgtttg ccaacactta gcaatttgca agttgattaa 12840
   ttgattctaa attatttttg tcttctaaat acatatacta atcaactgga aatgtaaata 12900
   tttgctaata tttctactat aggagaatta aagtgagtga atatggtacc acaaggtttg 12960
   gagatttaat tgttgcaatg ctgcatggat ggcatataca ccaaacattc aataattctt 13020
   gaggataata atggtaccac acaagatttg aggtgcatga acgtcacgtg gacaaaaggt 13080
   ttagtaattt ttcaagacaa caatgttacc acacacaagt tttgaggtgc atgcatggat 13140
   gccctgtgga aagtttaaaa atattttgga aatgatttgc atggaagcca tgtgtaaaac 13200
   catgacatcc acttggagga tgcaataatg aagaaaacta caaatttaca tgcaactagt 13260
   tatgcatgta gtctatataa tgaggatttt gcaatacttt cattcataca cactcactaa 13320
   gttttacacg attataattt cttcatagcc agcagatctg ccggcatcga tcccgggcca 13380
   tggcctgctt taatgagata tgcgagacgc ctatgatcgc atgatatttg ctttcaattc 13440
   tgttgtgcac gttgtaaaaa acctgagcat gtgtagctca gatccttacc gccggtttcg 13500
   gttcattcta atgaatatat cacccgttac tatcgtattt ttatgaataa tattctccgt 13560
   tcaatttact gattgtccgt cgacgagctc ggcgcgcctc tagaggatcg atgaattcag 13620
   atcggctgag tggctccttc aacgttgcgg ttctgtcagt tccaaacgta aaacggcttg 13680
   tcccgcgtca tcggcggggg tcataacgtg actcccttaa ttctccgctc atgatcagat 13740
   tgtcgtttcc cgccttcagt ttaaactatc agtgtttgac aggatatatt ggcgggtaaa 13800
   cctaagagaa aagagcgttt attagaataa tcggatattt aaaagggcgt gaaaaggttt 13860
   atccttcgtc catttgtatg tgcatgccaa ccacagggtt cccca                 13905
<210> 25
   <211> 15430
   <212> DNA
   <213> Unknown
<220>
   <223> pflanz. Expressionsvektor mit zwei Promotor-Terminator-Expressionskassetten inseriert ist Physcomitrella patens Elongase und Desaturase
<220>
   <221> CDS
   <222> (11543)..(12415)
<220>
   <221> CDS
   <222> (13313)..(14890)
<400> 25
   gatctggcgc cggccagcga gacgagcaag attggccgcc gcccgaaacg atccgacagc 60
   gcgcccagca caggtgcgca ggcaaattgc accaacgcat acagcgccag cagaatgcca 120
   tagtgggcgg tgacgtcgtt cgagtgaacc agatcgcgca ggaggcccgg cagcaccggc 180
   ataatcaggc cgatgccgac agcgtcgagc gcgacagtgc tcagaattac gatcaggggt 240
   atgttgggtt tcacgtctgg cctccggacc agcctccgct ggtccgattg aacgcgcgga 300
   ttctttatca ctgataagtt ggtggacata ttatgtttat cagtgataaa gtgtcaagca 360
   tgacaaagtt gcagccgaat acagtgatcc gtgccgccct ggacctgttg aacgaggtcg 420
   gcgtagacgg tctgacgaca cgcaaactgg cggaacggtt gggggttcag cagccggcgc 480
   tttactggca cttcaggaac aagcgggcgc tgctcgacgc actggccgaa gccatgctgg 540
   cggagaatca tacgcattcg gtgccgagag ccgacgacga ctggcgctca tttctgatcg 600
   ggaatgcccg cagcttcagg caggcgctgc tcgcctaccg cgatggcgcg cgcatccatg 660
   ccggcacgcg accgggcgca ccgcagatgg aaacggccga cgcgcagctt cgcttcctct 720
   gcgaggcggg tttttcggcc ggggacgccg tcaatgcgct gatgacaatc agctacttca 780
   ctgttggggc cgtgcttgag gagcaggccg gcgacagcga tgccggcgag cgcggcggca 840
   ccgttgaaca ggctccgctc tcgccgctgt tgcgggccgc gatagacgcc ttcgacgaag 900
   ccggtccgga cgcagcgttc gagcagggac tcgcggtgat tgtcgatgga ttggcgaaaa 960
   ggaggctcgt tgtcaggaac gttgaaggac cgagaaaggg tgacgattga tcaggaccgc 1020
   tgccggagcg caacccactc actacagcag agccatgtag acaacatccc ctcccccttt 1080
   ccaccgcgtc agacgcccgt agcagcccgc tacgggcttt ttcatgccct gccctagcgt 1140
   ccaagcctca cggccgcgct cggcctctct ggcggccttc tggcgctctt ccgcttcctc 1200
   gctcactgac tcgctgcgct cggtcgttcg gctgcggcga gcggtatcag ctcactcaaa 1260
   ggcggtaata cggttatcca cagaatcagg ggataacgca ggaaagaaca tgtgagcaaa 1320
   aggccagcaa aaggccagga accgtaaaaa ggccgcgttg ctggcgtttt tccataggct 1380
   ccgcccccct gacgagcatc acaaaaatcg acgctcaagt cagaggtggc gaaacccgac 1440
   aggactataa agataccagg cgtttccccc tggaagctcc ctcgtgcgct ctcctgttcc 1500
   gaccctgccg cttaccggat acctgtccgc ctttctccct tcgggaagcg tggcgctttt 1560
   ccgctgcata accctgcttc ggggtcatta tagcgatttt ttcggtatat ccatcctttt 1620
   tcgcacgata tacaggattt tgccaaaggg ttcgtgtaga ctttccttgg tgtatccaac 1680
   ggcgtcagcc gggcaggata ggtgaagtag gcccacccgc gagcgggtgt tccttcttca 1740
   ctgtccctta ttcgcacctg gcggtgctca acgggaatcc tgctctgcga ggctggccgg 1800
   ctaccgccgg cgtaacagat gagggcaagc ggatggctga tgaaaccaag ccaaccagga 1860
   agggcagccc acctatcaag gtgtactgcc ttccagacga acgaagagcg attgaggaaa 1920
   aggcggcggc ggccggcatg agcctgtcgg cctacctgct ggccgtcggc cagggctaca 1980
   aaatcacggg cgtcgtggac tatgagcacg tccgcgagct ggcccgcatc aatggcgacc 2040
   tgggccgcct gggcggcctg ctgaaactct ggctcaccga cgacccgcgc acggcgcggt 2100
   tcggtgatgc cacgatcctc gccctgctgg cgaagatcga agagaagcag gacgagcttg 2160
   gcaaggtcat gatgggcgtg gtccgcccga gggcagagcc atgacttttt tagccgctaa 2220
   aacggccggg gggtgcgcgt gattgccaag cacgtcccca tgcgctccat caagaagagc 2280
   gacttcgcgg agctggtgaa gtacatcacc gacgagcaag gcaagaccga gcgcctttgc 2340
   gacgctcacc gggctggttg ccctcgccgc tgggctggcg gccgtctatg gccctgcaaa 2400
   cgcgccagaa acgccgtcga agccgtgtgc gagacaccgc ggccgccggc gttgtggata 2460
   cctcgcggaa aacttggccc tcactgacag atgaggggcg gacgttgaca cttgaggggc 2520
   cgactcaccc ggcgcggcgt tgacagatga ggggcaggct cgatttcggc cggcgacgtg 2580
   gagctggcca gcctcgcaaa tcggcgaaaa cgcctgattt tacgcgagtt tcccacagat 2640
   gatgtggaca agcctgggga taagtgccct gcggtattga cacttgaggg gcgcgactac 2700
   tgacagatga ggggcgcgat ccttgacact tgaggggcag agtgctgaca gatgaggggc 2760
   gcacctattg acatttgagg ggctgtccac aggcagaaaa tccagcattt gcaagggttt 2820
   ccgcccgttt ttcggccacc gctaacctgt cttttaacct gcttttaaac caatatttat 2880
   aaaccttgtt tttaaccagg gctgcgccct gtgcgcgtga ccgcgcacgc cgaagggggg 2940
   tgccccccct tctcgaaccc tcccggcccg ctaacgcggg cctcccatcc ccccaggggc 3000
   tgcgcccctc ggccgcgaac ggcctcaccc caaaaatggc agcgctggca gtccttgcca 3060
   ttgccgggat cggggcagta acgggatggg cgatcagccc gagcgcgacg cccggaagca 3120
   ttgacgtgcc gcaggtgctg gcatcgacat tcagcgacca ggtgccgggc agtgagggcg 3180
   gcggcctggg tggcggcctg cccttcactt cggccgtcgg ggcattcacg gacttcatgg 3240
   cggggccggc aatttttacc ttgggcattc ttggcatagt ggtcgcgggt gccgtgctcg 3300
   tgttcggggg tgcgataaac ccagcgaacc atttgaggtg ataggtaaga ttataccgag 3360
   gtatgaaaac gagaattgga cctttacaga attactctat gaagcgccat atttaaaaag 3420
   ctaccaagac gaagaggatg aagaggatga ggaggcagat tgccttgaat atattgacaa 3480
   tactgataag ataatatatc ttttatatag aagatatcgc cgtatgtaag gatttcaggg 3540
   ggcaaggcat aggcagcgcg cttatcaata tatctataga atgggcaaag cataaaaact 3600
   tgcatggact aatgcttgaa acccaggaca ataaccttat agcttgtaaa ttctatcata 3660
   attgggtaat gactccaact tattgatagt gttttatgtt cagataatgc ccgatgactt 3720
   tgtcatgcag ctccaccgat tttgagaacg acagcgactt ccgtcccagc cgtgccaggt 3780
   gctgcctcag attcaggtta tgccgctcaa ttcgctgcgt atatcgcttg ctgattacgt 3840
   gcagctttcc cttcaggcgg gattcataca gcggccagcc atccgtcatc catatcacca 3900
   cgtcaaaggg tgacagcagg ctcataagac gccccagcgt cgccatagtg cgttcaccga 3960
   atacgtgcgc aacaaccgtc ttccggagac tgtcatacgc gtaaaacagc cagcgctggc 4020
   gcgatttagc cccgacatag ccccactgtt cgtccatttc cgcgcagacg atgacgtcac 4080
   tgcccggctg tatgcgcgag gttaccgact gcggcctgag ttttttaagt gacgtaaaat 4140
   cgtgttgagg ccaacgccca taatgcgggc tgttgcccgg catccaacgc cattcatggc 4200
   catatcaatg attttctggt gcgtaccggg ttgagaagcg gtgtaagtga actgcagttg 4260
   ccatgtttta cggcagtgag agcagagata gcgctgatgt ccggcggtgc ttttgccgtt 4320
   acgcaccacc ccgtcagtag ctgaacagga gggacagctg atagacacag aagccactgg 4380
   agcacctcaa aaacaccatc atacactaaa tcagtaagtt ggcagcatca cccataattg 4440
   tggtttcaaa atcggctccg tcgatactat gttatacgcc aactttgaaa acaactttga 4500
   aaaagctgtt ttctggtatt taaggtttta gaatgcaagg aacagtgaat tggagttcgt 4560
   cttgttataa ttagcttctt ggggtatctt taaatactgt agaaaagagg aaggaaataa 4620
   taaatggcta aaatgagaat atcaccggaa ttgaaaaaac tgatcgaaaa ataccgctgc 4680
   gtaaaagata cggaaggaat gtctcctgct aaggtatata agctggtggg agaaaatgaa 4740
   aacctatatt taaaaatgac ggacagccgg tataaaggga ccacctatga tgtggaacgg 4800
   gaaaaggaca tgatgctatg gctggaagga aagctgcctg ttccaaaggt cctgcacttt 4860
   gaacggcatg atggctggag caatctgctc atgagtgagg ccgatggcgt cctttgctcg 4920
   gaagagtatg aagatgaaca aagccctgaa aagattatcg agctgtatgc ggagtgcatc 4980
   aggctctttc actccatcga catatcggat tgtccctata cgaatagctt agacagccgc 5040
   ttagccgaat tggattactt actgaataac gatctggccg atgtggattg cgaaaactgg 5100
   gaagaagaca ctccatttaa agatccgcgc gagctgtatg attttttaaa gacggaaaag 5160
   cccgaagagg aacttgtctt ttcccacggc gacctgggag acagcaacat ctttgtgaaa 5220
   gatggcaaag taagtggctt tattgatctt gggagaagcg gcagggcgga caagtggtat 5280
   gacattgcct tctgcgtccg gtcgatcagg gaggatatcg gggaagaaca gtatgtcgag 5340
   ctattttttg acttactggg gatcaagcct gattgggaga aaataaaata ttatatttta 5400
   ctggatgaat tgttttagta cctagatgtg gcgcaacgat gccggcgaca agcaggagcg 5460
   caccgacttc ttccgcatca agtgttttgg ctctcaggcc gaggcccacg gcaagtattt 5520
   gggcaagggg tcgctggtat tcgtgcaggg caagattcgg aataccaagt acgagaagga 5580
   cggccagacg gtctacggga ccgacttcat tgccgataag gtggattatc tggacaccaa 5640
   ggcaccaggc gggtcaaatc aggaataagg gcacattgcc ccggcgtgag tcggggcaat 5700
   cccgcaagga gggtgaatga atcggacgtt tgaccggaag gcatacaggc aagaactgat 5760
   cgacgcgggg ttttccgccg aggatgccga aaccatcgca agccgcaccg tcatgcgtgc 5820
   gccccgcgaa accttccagt ccgtcggctc gatggtccag caagctacgg ccaagatcga 5880
   gcgcgacagc gtgcaactgg ctccccctgc cctgcccgcg ccatcggccg ccgtggagcg 5940
   ttcgcgtcgt ctcgaacagg aggcggcagg tttggcgaag tcgatgacca tcgacacgcg 6000
   aggaactatg acgaccaaga agcgaaaaac cgccggcgag gacctggcaa aacaggtcag 6060
   cgaggccaag caggccgcgt tgctgaaaca cacgaagcag cagatcaagg aaatgcagct 6120
   ttccttgttc gatattgcgc cgtggccgga cacgatgcga gcgatgccaa acgacacggc 6180
   ccgctctgcc ctgttcacca cgcgcaacaa gaaaatcccg cgcgaggcgc tgcaaaacaa 6240
   ggtcattttc cacgtcaaca aggacgtgaa gatcacctac accggcgtcg agctgcgggc 6300
   cgacgatgac gaactggtgt ggcagcaggt gttggagtac gcgaagcgca cccctatcgg 6360
   cgagccgatc accttcacgt tctacgagct ttgccaggac ctgggctggt cgatcaatgg 6420
   ccggtattac acgaaggccg aggaatgcct gtcgcgccta caggcgacgg cgatgggctt 6480
   cacgtccgac cgcgttgggc acctggaatc ggtgtcgctg ctgcaccgct tccgcgtcct 6540
   ggaccgtggc aagaaaacgt cccgttgcca ggtcctgatc gacgaggaaa tcgtcgtgct 6600
   gtttgctggc gaccactaca cgaaattcat atgggagaag taccgcaagc tgtcgccgac 6660
   ggcccgacgg atgttcgact atttcagctc gcaccgggag ccgtacccgc tcaagctgga 6720
   aaccttccgc ctcatgtgcg gatcggattc cacccgcgtg aagaagtggc gcgagcaggt 6780
   cggcgaagcc tgcgaagagt tgcgaggcag cggcctggtg gaacacgcct gggtcaatga 6840
   tgacctggtg cattgcaaac gctagggcct tgtggggtca gttccggctg ggggttcagc 6900
   agccagcgct ttactggcat ttcaggaaca agcgggcact gctcgacgca cttgcttcgc 6960
   tcagtatcgc tcgggacgca cggcgcgctc tacgaactgc cgataaacag aggattaaaa 7020
   ttgacaattg tgattaaggc tcagattcga cggcttggag cggccgacgt gcaggatttc 7080
   cgcgagatcc gattgtcggc cctgaagaaa gctccagaga tgttcgggtc cgtttacgag 7140
   cacgaggaga aaaagcccat ggaggcgttc gctgaacggt tgcgagatgc cgtggcattc 7200
   ggcgcctaca tcgacggcga gatcattggg ctgtcggtct tcaaacagga ggacggcccc 7260
   aaggacgctc acaaggcgca tctgtccggc gttttcgtgg agcccgaaca gcgaggccga 7320
   ggggtcgccg gtatgctgct gcgggcgttg ccggcgggtt tattgctcgt gatgatcgtc 7380
   cgacagattc caacgggaat ctggtggatg cgcatcttca tcctcggcgc acttaatatt 7440
   tcgctattct ggagcttgtt gtttatttcg gtctaccgcc tgccgggcgg ggtcgcggcg 7500
   acggtaggcg ctgtgcagcc gctgatggtc gtgttcatct ctgccgctct gctaggtagc 7560
   ccgatacgat tgatggcggt cctgggggct atttgcggaa ctgcgggcgt ggcgctgttg 7620
   gtgttgacac caaacgcagc gctagatcct gtcggcgtcg cagcgggcct ggcgggggcg 7680
   gtttccatgg cgttcggaac cgtgctgacc cgcaagtggc aacctcccgt gcctctgctc 7740
   acctttaccg cctggcaact ggcggccgga ggacttctgc tcgttccagt agctttagtg 7800
   tttgatccgc caatcccgat gcctacagga accaatgttc tcggcctggc gtggctcggc 7860
   ctgatcggag cgggtttaac ctacttcctt tggttccggg ggatctcgcg actcgaacct 7920
   acagttgttt ccttactggg ctttctcagc cccagatctg gggtcgatca gccggggatg 7980
   catcaggccg acagtcggaa cttcgggtcc ccgacctgta ccattcggtg agcaatggat 8040
   aggggagttg atatcgtcaa cgttcacttc taaagaaata gcgccactca gcttcctcag 8100
   cggctttatc cagcgatttc ctattatgtc ggcatagttc tcaagatcga cagcctgtca 8160
   cggttaagcg agaaatgaat aagaaggctg ataattcgga tctctgcgag ggagatgata 8220
   tttgatcaca ggcagcaacg ctctgtcatc gttacaatca acatgctacc ctccgcgaga 8280
   tcatccgtgt ttcaaacccg gcagcttagt tgccgttctt ccgaatagca tcggtaacat 8340
   gagcaaagtc tgccgcctta caacggctct cccgctgacg ccgtcccgga ctgatgggct 8400
   gcctgtatcg agtggtgatt ttgtgccgag ctgccggtcg gggagctgtt ggctggctgg 8460
   tggcaggata tattgtggtg taaacaaatt gacgcttaga caacttaata acacattgcg 8520
   gacgttttta atgtactggg gtggtttttc ttttcaccag tgagacgggc aacagctgat 8580
   tgcccttcac cgcctggccc tgagagagtt gcagcaagcg gtccacgctg gtttgcccca 8640
   gcaggcgaaa atcctgtttg atggtggttc cgaaatcggc aaaatccctt ataaatcaaa 8700
   agaatagccc gagatagggt tgagtgttgt tccagtttgg aacaagagtc cactattaaa 8760
   gaacgtggac tccaacgtca aagggcgaaa aaccgtctat cagggcgatg gcccactacg 8820
   tgaaccatca cccaaatcaa gttttttggg gtcgaggtgc cgtaaagcac taaatcggaa 8880
   ccctaaaggg agcccccgat ttagagcttg acggggaaag ccggcgaacg tggcgagaaa 8940
   ggaagggaag aaagcgaaag gagcgggcgc cattcaggct gcgcaactgt tgggaagggc 9000
   gatcggtgcg ggcctcttcg ctattacgcc agctggcgaa agggggatgt gctgcaaggc 9060
   gattaagttg ggtaacgcca gggttttccc agtcacgacg ttgtaaaacg acggccagtg 9120
   aattaattcc catcttgaaa gaaatatagt ttaaatattt attgataaaa taacaagtca 9180
   ggtattatag tccaagcaaa aacataaatt tattgatgca agtttaaatt cagaaatatt 9240
   tcaataactg attatatcag ctggtacatt gccgtagatg aaagactgag tgcgatatta 9300
   tgtgtaatac ataaattgat gatatagcta gcttagctca tcgggggatc cgtcgaagct 9360
   agcttgggtc ccgctcagaa gaactcgtca agaaggcgat agaaggcgat gcgctgcgaa 9420
   tcgggagcgg cgataccgta aagcacgagg aagcggtcag cccattcgcc gccaagctct 9480
   tcagcaatat cacgggtagc caacgctatg tcctgatagc ggtccgccac acccagccgg 9540
   ccacagtcga tgaatccaga aaagcggcca ttttccacca tgatattcgg caagcaggca 9600
   tcgccatggg tcacgacgag atcctcgccg tcgggcatgc gcgccttgag cctggcgaac 9660
   agttcggctg gcgcgagccc ctgatgctct tcgtccagat catcctgatc gacaagaccg 9720
   gcttccatcc gagtacgtgc tcgctcgatg cgatgtttcg cttggtggtc gaatgggcag 9780
   gtagccggat caagcgtatg cagccgccgc attgcatcag ccatgatgga tactttctcg 9840
   gcaggagcaa ggtgagatga caggagatcc tgccccggca cttcgcccaa tagcagccag 9900
   tcccttcccg cttcagtgac aacgtcgagc acagctgcgc aaggaacgcc cgtcgtggcc 9960
   agccacgata gccgcgctgc ctcgtcctgc agttcattca gggcaccgga caggtcggtc 10020
   ttgacaaaaa gaaccgggcg cccctgcgct gacagccgga acacggcggc atcagagcag 10080
   ccgattgtct gttgtgccca gtcatagccg aatagcctct ccacccaagc ggccggagaa 10140
   cctgcgtgca atccatcttg ttcaatccaa gctcccatgg gccctcgact agagtcgaga 10200
   tctggattga gagtgaatat gagactctaa ttggataccg aggggaattt atggaacgtc 10260
   agtggagcat ttttgacaag aaatatttgc tagctgatag tgaccttagg cgacttttga 10320
   acgcgcaata atggtttctg acgtatgtgc ttagctcatt aaactccaga aacccgcggc 10380
   tgagtggctc cttcaacgtt gcggttctgt cagttccaaa cgtaaaacgg cttgtcccgc 10440
   gtcatcggcg ggggtcataa cgtgactccc ttaattctcc gctcatgatc ttgatcccct 10500
   gcgccatcag atccttggcg gcaagaaagc catccagttt actttgcagg gcttcccaac 10560
   cttaccagag ggcgccccag ctggcaattc cggttcgctt gctgtccata aaaccgccca 10620
   gtctagctat cgccatgtaa gcccactgca agctacctgc tttctctttg cgcttgcgtt 10680
   ttcccttgtc cagatagccc agtagctgac attcatccgg ggtcagcacc gtttctgcgg 10740
   actggctttc tacgtgttcc gcttccttta gcagcccttg cgccctgagt gcttgcggca 10800
   gcgtgaagct tgcatgcctg caggtcgacg gcgcgccgag ctcctcgagc aaatttacac 10860
   attgccacta aacgtctaaa cccttgtaat ttgtttttgt tttactatgt gtgttatgta 10920
   tttgatttgc gataaatttt tatatttggt actaaattta taacaccttt tatgctaacg 10980
   tttgccaaca cttagcaatt tgcaagttga ttaattgatt ctaaattatt tttgtcttct 11040
   aaatacatat actaatcaac tggaaatgta aatatttgct aatatttcta ctataggaga 11100
   attaaagtga gtgaatatgg taccacaagg tttggagatt taattgttgc aatgctgcat 11160
   ggatggcata tacaccaaac attcaataat tcttgaggat aataatggta ccacacaaga 11220
   tttgaggtgc atgaacgtca cgtggacaaa aggtttagta atttttcaag acaacaatgt 11280
   taccacacac aagttttgag gtgcatgcat ggatgccctg tggaaagttt aaaaatattt 11340
   tggaaatgat ttgcatggaa gccatgtgta aaaccatgac atccacttgg aggatgcaat 11400
   aatgaagaaa actacaaatt tacatgcaac tagttatgca tgtagtctat ataatgagga 11460
   ttttgcaata ctttcattca tacacactca ctaagtttta cacgattata atttcttcat 11520
   agccagccca ccgcggtgga aa atg gag gtc gtg gag aga ttc tac ggt gag   11572
                            Met Glu Val Val Glu Arg Phe Tyr Gly Glu
                            1               5                   10
   ttg gat ggg aag gtc tcg cag ggc gtg aat gca ttg ctg ggt agt ttt   11620
   Leu Asp Gly Lys Val Ser Gln Gly Val Asn Ala Leu Leu Gly Ser Phe
                   15                  20                  25
   ggg gtg gag ttg acg gat acg ccc act acc aaa ggc ttg ccc ctc gtt   11668
   Gly Val Glu Leu Thr Asp Thr Pro Thr Thr Lys Gly Leu Pro Leu Val
               30                  35                  40
   gac agt ccc aca ccc atc gtc ctc ggt gtt tct gta tac ttg act att   11716
   Asp Ser Pro Thr Pro Ile Val Leu Gly Val Ser Val Tyr Leu Thr Ile
           45                  50                  55
   gtc att gga ggg ctt ttg tgg ata aag gcc agg gat ctg aaa ccg cgc   11764
   Val Ile Gly Gly Leu Leu Trp Ile Lys Ala Arg Asp Leu Lys Pro Arg
       60                  65                  70
   gcc tcg gag cca ttt ttg ctc caa gct ttg gtg ctt gtg cac aac ctg   11812
   Ala Ser Glu Pro Phe Leu Leu Gln Ala Leu Val Leu Val His Asn Leu
   75                  80                  85                  90
   ttc tgt ttt gcg ctc agt ctg tat atg tgc gtg ggc atc gct tat cag   11860
   Phe Cys Phe Ala Leu Ser Leu Tyr Met Cys Val Gly Ile Ala Tyr Gln
                   95                  100                 105
   gct att acc tgg cgg tac tct ctc tgg ggc aat gca tac aat cct aaa    11908
   Ala Ile Thr Trp Arg Tyr Ser Leu Trp Gly Asn Ala Tyr Asn Pro Lys
               110                 115                 120
   cat aaa gag atg gcg att ctg gta tac ttg ttc tac atg tct aag tac    11956
   His Lys Glu Met Ala Ile Leu Val Tyr Leu Phe Tyr Met Ser Lys Tyr
           125                 130                 135
   gtg gaa ttc atg gat acc gtt atc atg ata ctg aag cgc agc acc agg    12004
   Val Glu Phe Met Asp Thr Val Ile Met Ile Leu Lys Arg Ser Thr Arg
       140                 145                 150
   caa ata agc ttc ctc cac gtt tat cat cat tct tca att tcc ctc att    12052
   Gln Ile Ser Phe Leu His Val Tyr His His Ser Ser Ile Ser Leu Ile
   155                 160                 165                 170
   tgg tgg gct att gct cat cac gct cct ggc ggt gaa gca tat tgg tct    12100
   Trp Trp Ala Ile Ala His His Ala Pro Gly Gly Glu Ala Tyr Trp Ser
                   175                 180                 185
   gcg gct ctg aac tca gga gtg cat gtt ctc atg tat gcg tat tac ttc    12148
   Ala Ala Leu Asn Ser Gly Val His Val Leu Met Tyr Ala Tyr Tyr Phe
               190                 195                 200
   ttg gct gcc tgc ctt cga agt agc cca aag tta aaa aat aag tac ctt    12196
   Leu Ala Ala Cys Leu Arg Ser Ser Pro Lys Leu Lys Asn Lys Tyr Leu
           205                 210                 215
   ttt tgg ggc agg tac ttg aca caa ttc caa atg ttc cag ttt atg ctg    12244
   Phe Trp Gly Arg Tyr Leu Thr Gln Phe Gln Met Phe Gln Phe Met Leu
       220                 225                 230
   aac tta gtg cag gct tac tac gac atg aaa acg aat gcg cca tat cca    12292
   Asn Leu Val Gln Ala Tyr Tyr Asp Met Lys Thr Asn Ala Pro Tyr Pro
   235                 240                 245                 250
   caa tgg ctg atc aag att ttg ttc tac tac atg atc tcg ttg ctg ttt    12340
   Gln Trp Leu Ile Lys Ile Leu Phe Tyr Tyr Met Ile Ser Leu Leu Phe
                   255                 260                 265
   ctt ttc ggc aat ttt tac gta caa aaa tac atc aaa ccc tct gac gga    12388
   Leu Phe Gly Asn Phe Tyr Val Gln Lys Tyr Ile Lys Pro Ser Asp Gly
               270                 275                 280
   aag caa aag gga gct aaa act gag tga tctagaaggc ctcctgcttt          12435
   Lys Gln Lys Gly Ala Lys Thr Glu
           285                 290
   aatgagatat gcgagacgcc tatgatcgca tgatatttgc tttcaattct gttgtgcacg 12495
   ttgtaaaaaa cctgagcatg tgtagctcag atccttaccg ccggtttcgg ttcattctaa 12555
   tgaatatatc acccgttact atcgtatttt tatgaataat attctccgtt caatttactg 12615
   attgtccgtc gagcaaattt acacattgcc actaaacgtc taaacccttg taatttgttt 12675
   ttgttttact atgtgtgtta tgtatttgat ttgcgataaa tttttatatt tggtactaaa 12735
   tttataacac cttttatgct aacgtttgcc aacacttagc aatttgcaag ttgattaatt 12795
   gattctaaat tatttttgtc ttctaaatac atatactaat caactggaaa tgtaaatatt 12855
   tgctaatatt tctactatag gagaattaaa gtgagtgaat atggtaccac aaggtttgga 12915
   gatttaattg ttgcaatgct gcatggatgg catatacacc aaacattcaa taattcttga 12975
   ggataataat ggtaccacac aagatttgag gtgcatgaac gtcacgtgga caaaaggttt 13035
   agtaattttt caagacaaca atgttaccac acacaagttt tgaggtgcat gcatggatgc 13095
   cctgtggaaa gtttaaaaat attttggaaa tgatttgcat ggaagccatg tgtaaaacca 13155
   tgacatccac ttggaggatg caataatgaa gaaaactaca aatttacatg caactagtta 13215
   tgcatgtagt ctatataatg aggattttgc aatactttca ttcatacaca ctcactaagt 13275
   tttacacgat tataatttct tcatagccag cggatcc atg gta ttc gcg ggc ggt  13330
                                            Met Val Phe Ala Gly Gly
                                                            295
   gga ctt cag cag ggc tct ctc gaa gaa aac atc gac gtc gag cac att   13378
   Gly Leu Gln Gln Gly Ser Leu Glu Glu Asn Ile Asp Val Glu His Ile
           300                 305                 310
   gcc agt atg tct ctc ttc agc gac ttc ttc agt tat gtg tct tca act   13426
   Ala Ser Met Ser Leu Phe Ser Asp Phe Phe Ser Tyr Val Ser Ser Thr
       315                 320                 325
   gtt ggt tcg tgg agc gta cac agt ata caa cct ttg aag cgc ctg acg   13474
   Val Gly Ser Trp Ser Val His Ser Ile Gln Pro Leu Lys Arg Leu Thr
   330                 335                 340                 345
   agt aag aag cgt gtt tcg gaa agc gct gcc gtg caa tgt ata tca gct   13522
   Ser Lys Lys Arg Val Ser Glu Ser Ala Ala Val Gln Cys Ile Ser Ala
                   350                 355                 360
   gaa gtt cag aga aat tcg agt acc cag gga act gcg gag gca ctc gca   13570
   Glu Val Gln Arg Asn Ser Ser Thr Gln Gly Thr Ala Glu Ala Leu Ala
               365                 370                 375
   gaa tca gtc gtg aag ccc acg aga cga agg tca tct cag tgg aag aag   13618
   Glu Ser Val Val Lys Pro Thr Arg Arg Arg Ser Ser Gln Trp Lys Lys
           380                 385                 390
   tcg aca cac ccc cta tca gaa gta gca gta cac aac aag cca agc gat   13666
   Ser Thr His Pro Leu Ser Glu Val Ala Val His Asn Lys Pro Ser Asp
       395                 400                 405
   tgc tgg att gtt gta aaa aac aag gtg tat gat gtt tcc aat ttt gcg   13714
   Cys Trp Ile Val Val Lys Asn Lys Val Tyr Asp Val Ser Asn Phe Ala
   410                 415                 420                 425
   gac gag cat ccc gga gga tca gtt att agt act tat ttt gga cga gac   13762
   Asp Glu His Pro Gly Gly Ser Val Ile Ser Thr Tyr Phe Gly Arg Asp
                   430                 435                 440
   ggc aca gat gtt ttc tct agt ttt cat gca gct tct aca tgg aaa att     13810
   Gly Thr Asp Val Phe Ser Ser Phe His Ala Ala Ser Thr Trp Lys Ile
               445                 450                 455
   ctt caa gac ttt tac att ggt gac gtg gag agg gtg gag ccg act cca     13858
   Leu Gln Asp Phe Tyr Ile Gly Asp Val Glu Arg Val Glu Pro Thr Pro
           460                 465                 470
   gag ctg ctg aaa gat ttc cga gaa atg aga gct ctt ttc ctg agg gag     13906
   Glu Leu Leu Lys Asp Phe Arg Glu Met Arg Ala Leu Phe Leu Arg Glu
       475                 480                 485
   caa ctt ttc aaa agt tcg aaa ttg tac tat gtt atg aag ctg ctc acg     13954
   Gln Leu Phe Lys Ser Ser Lys Leu Tyr Tyr Val Met Lys Leu Leu Thr
   490                 495                 500                 505
   aat gtt gct att ttt gct gcg agc att gca ata ata tgt tgg agc aag     14002
   Asn Val Ala Ile Phe Ala Ala Ser Ile Ala Ile Ile Cys Trp Ser Lys
                   510                 515                 520
   act att tca gcg gtt ttg gct tca gct tgt atg atg gct ctg tgt ttc     14050
   Thr Ile Ser Ala Val Leu Ala Ser Ala Cys Met Met Ala Leu Cys Phe
               525                 530                 535
   caa cag tgc gga tgg cta tcc cat gat ttt ctc cac aat cag gtg ttt     14098
   Gln Gln Cys Gly Trp Leu Ser His Asp Phe Leu His Asn Gln Val Phe
           540                 545                 550
   gag aca cgc tgg ctt aat gaa gtt gtc ggg tat gtg atc ggc aac gcc     14146
   Glu Thr Arg Trp Leu Asn Glu Val Val Gly Tyr Val Ile Gly Asn Ala
       555                 560                 565
   gtt ctg ggg ttt agt aca ggg tgg tgg aag gag aag cat aac ctt cat     14194
   Val Leu Gly Phe Ser Thr Gly Trp Trp Lys Glu Lys His Asn Leu His
   570                 575                 580                 585
   cat gct gct cca aat gaa tgc gat cag act tac caa cca att gat gaa     14242
   His Ala Ala Pro Asn Glu Cys Asp Gln Thr Tyr Gln Pro Ile Asp Glu
                   590                 595                 600
   gat att gat act ctc ccc ctc att gcc tgg agc aag gac ata ctg gcc     14290
   Asp Ile Asp Thr Leu Pro Leu Ile Ala Trp Ser Lys Asp Ile Leu Ala
               605                 610                 615
   aca gtt gag aat aag aca ttc ttg cga atc ctc caa tac cag cat ctg     14338
   Thr Val Glu Asn Lys Thr Phe Leu Arg Ile Leu Gln Tyr Gln His Leu
           620                 625                 630
   ttc ttc atg ggt ctg tta ttt ttc gcc cgt ggt agt tgg ctc ttt tgg     14386
   Phe Phe Met Gly Leu Leu Phe Phe Ala Arg Gly Ser Trp Leu Phe Trp
       635                 640                 645
   agc tgg aga tat acc tct aca gca gtg ctc tca cct gtc gac agg ttg     14434
   Ser Trp Arg Tyr Thr Ser Thr Ala Val Leu Ser Pro Val Asp Arg Leu
   650                 655                 660                 665
   ttg gag aag gga act gtt ctg ttt cac tac ttt tgg ttc gtc ggg aca   14482
   Leu Glu Lys Gly Thr Val Leu Phe His Tyr Phe Trp Phe Val Gly Thr
                   670                 675                 680
   gcg tgc tat ctt ctc cct ggt tgg aag cca tta gta tgg atg gcg gtg   14530
   Ala Cys Tyr Leu Leu Pro Gly Trp Lys Pro Leu Val Trp Met Ala Val
               685                 690                 695
   act gag ctc atg tcc ggc atg ctg ctg ggc ttt gta ttt gta ctt agc   14578
   Thr Glu Leu Met Ser Gly Met Leu Leu Gly Phe Val Phe Val Leu Ser
           700                 705                 710
   cac aat ggg atg gag gtt tat aat tcg tct aaa gaa ttc gtg agt gca   14626
   His Asn Gly Met Glu Val Tyr Asn Ser Ser Lys Glu Phe Val Ser Ala
       715                 720                 725
   cag atc gta tcc aca cgg gat atc aaa gga aac ata ttc aac gac tgg   14674
   Gln Ile Val Ser Thr Arg Asp Ile Lys Gly Asn Ile Phe Asn Asp Trp
   730                 735                 740                 745
   ttc act ggt ggc ctt aac agg caa ata gag cat cat ctt ttc cca aca   14722
   Phe Thr Gly Gly Leu Asn Arg Gln Ile Glu His His Leu Phe Pro Thr
                   750                 755                 760
   atg ccc agg cat aat tta aac aaa ata gca cct aga gtg gag gtg ttc   14770
   Met Pro Arg His Asn Leu Asn Lys Ile Ala Pro Arg Val Glu Val Phe
               765                 770                 775
   tgt aag aaa cac ggt ctg gtg tac gaa gac gta tct att gct acc ggc   14818
   Cys Lys Lys His Gly Leu Val Tyr Glu Asp Val Ser Ile Ala Thr Gly
           780                 785                 790
   act tgc aag gtt ttg aaa gca ttg aag gaa gtc gcg gag gct gcg gca   14866
   Thr Cys Lys Val Leu Lys Ala Leu Lys Glu Val Ala Glu Ala Ala Ala
       795                 800                 805
   gag cag cat gct acc acc agt taa gctagcgtta accctgcttt aatgagatat  14920
   Glu Gln His Ala Thr Thr Ser
   810                 815
   gcgagacgcc tatgatcgca tgatatttgc tttcaattct gttgtgcacg ttgtaaaaaa 14980
   cctgagcatg tgtagctcag atccttaccg ccggtttcgg ttcattctaa tgaatatatc 15040
   acccgttact atcgtatttt tatgaataat attctccgtt caatttactg attgtccgtc 15100
   gacgaattcg agctcggcgc gcctctagag gatcgatgaa ttcagatcgg ctgagtggct 15160
   ccttcaacgt tgcggttctg tcagttccaa acgtaaaacg gcttgtcccg cgtcatcggc 15220
   gggggtcata acgtgactcc cttaattctc cgctcatgat cagattgtcg tttcccgcct 15280
   tcagtttaaa ctatcagtgt ttgacaggat atattggcgg gtaaacctaa gagaaaagag 15340
   cgtttattag aataatcgga tatttaaaag ggcgtgaaaa ggtttatcct tcgtccattt 15400
   gtatgtgcat gccaaccaca gggttcccca                                  15430
<210> 26
   <211> 290
   <212> PRT
   <213> Unknown
<400> 26
   Met Glu Val Val Glu Arg Phe Tyr Gly Glu Leu Asp Gly Lys Val Ser
   1 5 10 15
   Gln Gly Val Asn Ala Leu Leu Gly Ser Phe Gly Val Glu Leu Thr Asp
   20 25 30
   Thr Pro Thr Thr Lys Gly Leu Pro Leu Val Asp Ser Pro Thr Pro Ile
   35 40 45
   Val Leu Gly Val Ser Val Tyr Leu Thr Ile Val Ile Gly Gly Leu Leu
   50 55 60
   Trp Ile Lys Ala Arg Asp Leu Lys Pro Arg Ala Ser Glu Pro Phe Leu
   65 70 75 80
   Leu Gln Ala Leu Val Leu Val His Asn Leu Phe Cys Phe Ala Leu Ser
   85 90 95
   Leu Tyr Met Cys Val Gly Ile Ala Tyr Gln Ala Ile Thr Trp Arg Tyr
   100 105 110
   Ser Leu Trp Gly Asn Ala Tyr Asn Pro Lys His Lys Glu Met Ala Ile
   115 120 125
   Leu Val Tyr Leu Phe Tyr Met Ser Lys Tyr Val Glu Phe Met Asp Thr
   130 135 140
   Val Ile Met Ile Leu Lys Arg Ser Thr Arg Gln Ile Ser Phe Leu His
   145 150 155 160
   Val Tyr His His Ser Ser Ile Ser Leu Ile Trp Trp Ala Ile Ala His
   165 170 175
   His Ala Pro Gly Gly Glu Ala Tyr Trp Ser Ala Ala Leu Asn Ser Gly
   180 185 190
   Val His Val Leu Met Tyr Ala Tyr Tyr Phe Leu Ala Ala Cys Leu Arg
   195 200 205
   Ser Ser Pro Lys Leu Lys Asn Lys Tyr Leu Phe Trp Gly Arg Tyr Leu
   210 215 220
   Thr Gln Phe Gln Met Phe Gln Phe Met Leu Asn Leu Val Gln Ala Tyr
   225 230 235 240
   Tyr Asp Met Lys Thr Asn Ala Pro Tyr Pro Gln Trp Leu Ile Lys Ile
   245 250 255
   Leu Phe Tyr Tyr Met Ile Ser Leu Leu Phe Leu Phe Gly Asn Phe Tyr
   260 265 270
   Val Gln Lys Tyr Ile Lys Pro Ser Asp Gly Lys Gln Lys Gly Ala Lys
   275 280 285
   Thr Glu
   290
<210> 27
   <211> 525
   <212> PRT
   <213> Unknown
<400> 27
   Met Val Phe Ala Gly Gly Gly Leu Gln Gln Gly Ser Leu Glu Glu Asn
   1               5                   10                  15
   Ile Asp Val Glu His Ile Ala Ser Met Ser Leu Phe Ser Asp Phe Phe
               20                  25                  30
   Ser Tyr Val Ser Ser Thr Val Gly Ser Trp Ser Val His Ser Ile Gln
           35                  40                  45
   Pro Leu Lys Arg Leu Thr Ser Lys Lys Arg Val Ser Glu Ser Ala Ala
       50                  55                  60
   Val Gln Cys Ile Ser Ala Glu Val Gln Arg Asn Ser Ser Thr Gln Gly
   65                  70                  75                  80
   Thr Ala Glu Ala Leu Ala Glu Ser Val Val Lys Pro Thr Arg Arg Arg
                   85                  90                  95
   Ser Ser Gln Trp Lys Lys Ser Thr His Pro Leu Ser Glu Val Ala Val
               100                 105                 110
   His Asn Lys Pro Ser Asp Cys Trp Ile Val Val Lys Asn Lys Val Tyr
           115                 120                 125
   Asp Val Ser Asn Phe Ala Asp Glu His Pro Gly Gly Ser Val Ile Ser
       130                 135                 140
   Thr Tyr Phe Gly Arg Asp Gly Thr Asp Val Phe Ser Ser Phe His Ala
   145                 150                 155                 160
   Ala Ser Thr Trp Lys Ile Leu Gln Asp Phe Tyr Ile Gly Asp Val Glu
                   165                 170                 175
   Arg Val Glu Pro Thr Pro Glu Leu Leu Lys Asp Phe Arg Glu Met Arg
               180                 185                 190
   Ala Leu Phe Leu Arg Glu Gln Leu Phe Lys Ser Ser Lys Leu Tyr Tyr
           195                 200                 205
   Val Met Lys Leu Leu Thr Asn Val Ala Ile Phe Ala Ala Ser Ile Ala
       210                 215                 220
   Ile Ile Cys Trp Ser Lys Thr Ile Ser Ala Val Leu Ala Ser Ala Cys
   225                 230                 235                 240
   Met Met Ala Leu Cys Phe Gln Gln Cys Gly Trp Leu Ser His Asp Phe
   245 250 255
   Leu His Asn Gln Val Phe Glu Thr Arg Trp Leu Asn Glu Val Val Gly
   260 265 270
   Tyr Val Ile Gly Asn Ala Val Leu Gly Phe Ser Thr Gly Trp Trp Lys
   275 280 285
   Glu Lys His Asn Leu His His Ala Ala Pro Asn Glu Cys Asp Gln Thr
   290 295 300
   Tyr Gln Pro Ile Asp Glu Asp Ile Asp Thr Leu Pro Leu Ile Ala Trp
   305 310 315 320
   Ser Lys Asp Ile Leu Ala Thr Val Glu Asn Lys Thr Phe Leu Arg Ile
   325 330 335
   Leu Gln Tyr Gln His Leu Phe Phe Met Gly Leu Leu Phe Phe Ala Arg
   340 345 350
   Gly Ser Trp Leu Phe Trp Ser Trp Arg Tyr Thr Ser Thr Ala Val Leu
   355 360 365
   Ser Pro Val Asp Arg Leu Leu Glu Lys Gly Thr Val Leu Phe His Tyr
   370 375 380
   Phe Trp Phe Val Gly Thr Ala Cys Tyr Leu Leu Pro Gly Trp Lys Pro
   385 390 395 400
   Leu Val Trp Met Ala Val Thr Glu Leu Met Ser Gly Met Leu Leu Gly
   405 410 415
   Phe Val Phe Val Leu Ser His Asn Gly Met Glu Val Tyr Asn Ser Ser
   420 425 430
   Lys Glu Phe Val Ser Ala Gln Ile Val Ser Thr Arg Asp Ile Lys Gly
   435 440 445
   Asn Ile Phe Asn Asp Trp Phe Thr Gly Gly Leu Asn Arg Gln Ile Glu
   450 455 460
   His His Leu Phe Pro Thr Met Pro Arg His Asn Leu Asn Lys Ile Ala
   465 470 475 480
   Pro Arg Val Glu Val Phe Cys Lys Lys His Gly Leu Val Tyr Glu Asp
   485 490 495
   Val Ser Ile Ala Thr Gly Thr Cys Lys Val Leu Lys Ala Leu Lys Glu
   500 505 510
   Val Ala Glu Ala Ala Ala Glu Gln His Ala Thr Thr Ser
   515 520 525
<210> 28
   <211> 17752
   <212> DNA
   <213> Unknown
<220>
   <223> pflanz. Expressionsvektor mit 3 Promotor-Terminator- Expressionskassetten inseriert mit Physcomitrella Elongase + Desaturase + Phaeodactylum Desaturase
<220>
   <221> CDS
   <222> (11543)..(12415)
<220>
   <221> CDS
   <222> (13313)..(14890)
<220>
   <221> CDS
   <222> (15791) .. (17200)
<400> 28
<210> 29
   <211> 290
   <212> PRT
   <213> Unknown
<400> 29
   Met Glu Val Val Glu Arg Phe Tyr Gly Glu Leu Asp Gly Lys Val Ser
   1 5 10 15
   Gln Gly Val Asn Ala Leu Leu Gly Ser Phe Gly Val Glu Leu Thr Asp
   20 25 30
   Thr Pro Thr Thr Lys Gly Leu Pro Leu Val Asp Ser Pro Thr Pro Ile
   35 40 45
   Val Leu Gly Val Ser Val Tyr Leu Thr Ile Val Ile Gly Gly Leu Leu
   50 55 60
   Trp Ile Lys Ala Arg Asp Leu Lys Pro Arg Ala Ser Glu Pro Phe Leu
   65 70 75 80
   Leu Gln Ala Leu Val Leu Val His Asn Leu Phe Cys Phe Ala Leu Ser
   85 90 95
   Leu Tyr Met Cys Val Gly Ile Ala Tyr Gln Ala Ile Thr Trp Arg Tyr
   100 105 110
   Ser Leu Trp Gly Asn Ala Tyr Asn Pro Lys His Lys Glu Met Ala Ile
   115 120 125
   Leu Val Tyr Leu Phe Tyr Met Ser Lys Tyr Val Glu Phe Met Asp Thr
   130 135 140
   Val Ile Met Ile Leu Lys Arg Ser Thr Arg Gln Ile Ser Phe Leu His
   145 150 155 160
   Val Tyr His His Ser Ser Ile Ser Leu Ile Trp Trp Ala Ile Ala His
   165 170 175
   His Ala Pro Gly Gly Glu Ala Tyr Trp Ser Ala Ala Leu Asn Ser Gly
   180 185 190
   Val His Val Leu Met Tyr Ala Tyr Tyr Phe Leu Ala Ala Cys Leu Arg
   195 200 205
   Ser Ser Pro Lys Leu Lys Asn Lys Tyr Leu Phe Trp Gly Arg Tyr Leu
   210 215 220
   Thr Gln Phe Gln Met Phe Gln Phe Met Leu Asn Leu Val Gln Ala Tyr
   225 230 235 240
   Tyr Asp Met Lys Thr Asn Ala Pro Tyr Pro Gln Trp Leu Ile Lys Ile
   245 250 255
   Leu Phe Tyr Tyr Met Ile Ser Leu Leu Phe Leu Phe Gly Asn Phe Tyr
                   260                     265                     270
   Val Gln Lys Tyr Ile Lys Pro Ser Asp Gly Lys Gln Lys Gly Ala Lys
           275                     280                     285
   Thr Glu
       290
<210> 30
   <211> 525
   <212> PRT
   <213> Unknown
<400> 30
   Met Val Phe Ala Gly Gly Gly Leu Gln Gln Gly Ser Leu Glu Glu Asn
   1 5 10 15
   Ile Asp Val Glu His Ile Ala Ser Met Ser Leu Phe Ser Asp Phe Phe
   20 25 30
   Ser Tyr Val Ser Ser Thr Val Gly Ser Trp Ser Val His Ser Ile Gln
   35 40 45
   Pro Leu Lys Arg Leu Thr Ser Lys Lys Arg Val Ser Glu Ser Ala Ala
   50 55 60
   Val Gln Cys Ile Ser Ala Glu Val Gln Arg Asn Ser Ser Thr Gln Gly
   65 70 75 80
   Thr Ala Glu Ala Leu Ala Glu Ser Val Val Lys Pro Thr Arg Arg Arg
   85 90 95
   Ser Ser Gln Trp Lys Lys Ser Thr His Pro Leu Ser Glu Val Ala Val
   100 105 110
   His Asn Lys Pro Ser Asp Cys Trp Ile Val Val Lys Asn Lys Val Tyr
   115 120 125
   Asp Val Ser Asn Phe Ala Asp Glu His Pro Gly Gly Ser Val Ile Ser
   130 135 140
   Thr Tyr Phe Gly Arg Asp Gly Thr Asp Val Phe Ser Ser Phe His Ala
   145 150 155 160
   Ala Ser Thr Trp Lys Ile Leu Gln Asp Phe Tyr Ile Gly Asp Val Glu
   165 170 175
   Arg Val Glu Pro Thr Pro Glu Leu Leu Lys Asp Phe Arg Glu Met Arg
   180 185 190
   Ala Leu Phe Leu Arg Glu Gln Leu Phe Lys Ser Ser Lys Leu Tyr Tyr
   195 200 205
   Val Met Lys Leu Leu Thr Asn Val Ala Ile Phe Ala Ala Ser Ile Ala
   210 215 220
   Ile Ile Cys Trp Ser Lys Thr Ile Ser Ala Val Leu Ala Ser Ala Cys
   225 230 235 240
   Met Met Ala Leu Cys Phe Gln Gln Cys Gly Trp Leu Ser His Asp Phe
   245 250 255
   Leu His Asn Gln Val Phe Glu Thr Arg Trp Leu Asn Glu Val Val Gly
   260 265 270
   Tyr Val Ile Gly Asn Ala Val Leu Gly Phe Ser Thr Gly Trp Trp Lys
   275 280 285
   Glu Lys His Asn Leu His His Ala Ala Pro Asn Glu Cys Asp Gln Thr
   290 295 300
   Tyr Gln Pro Ile Asp Glu Asp Ile Asp Thr Leu Pro Leu Ile Ala Trp
   305 310 315 320
   Ser Lys Asp Ile Leu Ala Thr Val Glu Asn Lys Thr Phe Leu Arg Ile
   325 330 335
   Leu Gln Tyr Gln His Leu Phe Phe Met Gly Leu Leu Phe Phe Ala Arg
   340 345 350
   Gly Ser Trp Leu Phe Trp Ser Trp Arg Tyr Thr Ser Thr Ala Val Leu
   355 360 365
   Ser Pro Val Asp Arg Leu Leu Glu Lys Gly Thr Val Leu Phe His Tyr
   370 375 380
   Phe Trp Phe Val Gly Thr Ala Cys Tyr Leu Leu Pro Gly Trp Lys Pro
   385 390 395 400
   Leu Val Trp Met Ala Val Thr Glu Leu Met Ser Gly Met Leu Leu Gly
   405 410 415
   Phe Val Phe Val Leu Ser His Asn Gly Met Glu Val Tyr Asn Ser Ser
   420 425 430
   Lys Glu Phe Val Ser Ala Gln Ile Val Ser Thr Arg Asp Ile Lys Gly
   435 440 445
   Asn Ile Phe Asn Asp Trp Phe Thr Gly Gly Leu Asn Arg Gln Ile Glu
   450 455 460
   His His Leu Phe Pro Thr Met Pro Arg His Asn Leu Asn Lys Ile Ala
   465 470 475 480
   Pro Arg Val Glu Val Phe Cys Lys Lys His Gly Leu Val Tyr Glu Asp
   485 490 495
   Val Ser Ile Ala Thr Gly Thr Cys Lys Val Leu Lys Ala Leu Lys Glu
   500 505 510
   Val Ala Glu Ala Ala Ala Glu Gln His Ala Thr Thr Ser
   515 520 525
<210> 31
   <211> 469
   <212> PRT
   <213> Unknown
<400> 31
   Met Ala Pro Asp Ala Asp Lys Leu Arg Gln Arg Gln Thr Thr Ala Val
   1 5 10 15
   Ala Lys His Asn Ala Ala Thr Ile Ser Thr Gln Glu Arg Leu Cys Ser
   20 25 30
   Leu Ser Ser Leu Lys Gly Glu Glu Val Cys Ile Asp Gly Ile Ile Tyr
   35 40 45
   Asp Leu Gln Ser Phe Asp His Pro Gly Gly Glu Thr Ile Lys Met Phe
   50 55 60
   Gly Gly Asn Asp Val Thr Val Gln Tyr Lys Met Ile His Pro Tyr His
   65 70 75 80
   Thr Glu Lys His Leu Glu Lys Met Lys Arg Val Gly Lys Val Thr Asp
   85 90 95
   Phe Val Cys Glu Tyr Lys Phe Asp Thr Glu Phe Glu Arg Glu Ile Lys
   100 105 110
   Arg Glu Val Phe Lys Ile Val Arg Arg Gly Lys Asp Phe Gly Thr Leu
   115 120 125
   Gly Trp Phe Phe Arg Ala Phe Cys Tyr Ile Ala Ile Phe Phe Tyr Leu
   130 135 140
   Gln Tyr His Trp Val Thr Thr Gly Thr Ser Trp Leu Leu Ala Val Ala
   145 150 155 160
   Tyr Gly Ile Ser Gln Ala Met Ile Gly Met Asn Val Gln His Asp Ala
   165 170 175
   Asn His Gly Ala Thr Ser Lys Arg Pro Trp Val Asn Asp Met Leu Gly
   180 185 190
   Leu Gly Ala Asp Phe Ile Gly Gly Ser Lys Trp Leu Trp Gln Glu Gln
   195 200 205
   His Trp Thr His His Ala Tyr Thr Asn His Ala Glu Met Asp Pro Asp
   210 215 220
   Ser Phe Gly Ala Glu Pro Met Leu Leu Phe Asn Asp Tyr Pro Leu Asp
   225 230 235 240
   His Pro Ala Arg Thr Trp Leu His Arg Phe Gln Ala Phe Phe Tyr Met
   245 250 255
   Pro Val Leu Ala Gly Tyr Trp Leu Ser Ala Val Phe Asn Pro Gln Ile
   260 265 270
   Leu Asp Leu Gln Gln Arg Gly Ala Leu Ser Val Gly Ile Arg Leu Asp
   275 280 285
   Asn Ala Phe Ile His Ser Arg Arg Lys Tyr Ala Val Phe Trp Arg Ala
       290                 295                 300
   Val Tyr Ile Ala Val Asn Val Ile Ala Pro Phe Tyr Thr Asn Ser Gly
   305                 310                 315                 320
   Leu Glu Trp Ser Trp Arg Val Phe Gly Asn Ile Met Leu Met Gly Val
                   325                 330                 335
   Ala Glu Ser Leu Ala Leu Ala Val Leu Phe Ser Leu Ser His Asn Phe
               340                 345                 350
   Glu Ser Ala Asp Arg Asp Pro Thr Ala Pro Leu Lys Lys Thr Gly Glu
           355                 360                 365
   Pro Val Asp Trp Phe Lys Thr Gln Val Glu Thr Ser Cys Thr Tyr Gly
       370                 375                 380
   Gly Phe Leu Ser Gly Cys Phe Thr Gly Gly Leu Asn Phe Gln Val Glu
   385                 390                 395                 400
   His His Leu Phe Pro Arg Met Ser Ser Ala Trp Tyr Pro Tyr Ile Ala
                   405                 410                 415
   Pro Lys Val Arg Glu Ile Cys Ala Lys His Gly Val His Tyr Ala Tyr
               420                 425                 430
   Tyr Pro Trp Ile His Gln Asn Phe Leu Ser Thr Val Arg Tyr Met His
           435                 440                 445
   Ala Ala Gly Thr Gly Ala Asn Trp Arg Gln Met Ala Arg Glu Asn Pro
       450                 455                 460
   Leu Thr Gly Arg Ala
   465